# EUROPEAN PATENT APPLICATION

(11) **EP 3 753 952 A1**
(43) Date of publication of application: **23.12.2020**
(21) Application number: 18889359.8
(22) Date of filing: 13.12.2018
(51) Int. Cl.: C07K 16/28, C07K 16/46, C12N 15/13, A61K 39/395, A61P 35/00, G01N 33/68

(54) **EGFRVIII ANTIBODY AND CONJUGATE, AND PREPARATION METHOD AND USE THEREOF**

(30) Priority: 13.12.2017 CN 201711329680
(71) Applicant: XDCExplorer (Shanghai) Co., Ltd., Shanghai 201210 (CN)
(72) Inventor: ZHANG, Ying, Shanghai 201210 (CN); YANG, Cuiqing, Shanghai 201210 (CN); LIU, Siqi, Shanghai 201210 (CN); ZHANG, Yu, Shanghai 201210 (CN); WANG, Lina, Shanghai 201210 (CN); LIU, Lile, Shanghai 201210 (CN)
(74) Representative: Mewburn Ellis LLP
(86) International application number: PCT/CN2018/120959
(87) International publication number: WO 2019/114804

(57) **Abstract**

Disclosed are an EGFRvIII antibody and a conjugate, and a preparation method and the use thereof. The antibody comprises complementarity-determining regions (CDRs) of the EGFRvIII antibody: one or more of heavy chain CDR1, heavy chain CDR2 and heavy chain CDR3, and/or one or more of light chain CDR1, light chain CDR2 and light chain CDR3, with the amino acid sequence thereof being as described in the present invention. The EGFRvIII antibody has a high affinity with the EGFRvIII protein, and can enter cells after coupling with small molecule toxins such as MMAE and has a cytotoxic killing effect on EGFRvIII positive cells. The antibody therefore can be used for preparing drugs for treating tumors and other diseases.

## Description

The present application claims the priority for Chinese patent application CN 201711329680.1 filed on December 13, 2017. The aforementioned Chinese patent application is incorporated into the present application by reference in its entirety.

### Technical Field

The present invention relates to the field of biomedicine, in particular to an EGFRvIII antibody and a conjugate thereof, a preparation method therefor and the use thereof.

### Background arts

Human epidermal growth factor receptor (EGFR, also known as her-1 or Erb-B1) is a 170-kDa transmembrane protein receptor encoded by proto-oncogene c-erbB, and has a tyrosine kinase activity in the intramembrane region (Modjtahedi et al., Br. J. Cancer 73: 228-235, 1996; Herbst and Shin, Cancer 94: 1593-1611, 2002). The full-length sequence of EGFR has the entry number P00533 in SwissProt database. Through tyrosine kinase-mediated signal transduction pathways, EGFR regulates a variety of cell physiological processes, mainly including cell proliferation and differentiation, cell survival and apoptosis, angiogenesis, and mitosis and cell transfer (Atalay et al., Ann. Oncology 14: 1346-1363, 2003; Tsao and Herbst, Signal 4: 4-9, 2003; Herbst and Shin, Cancer 94: 1593-1611, 2002; Modjtahedi et al., Br. J. Cancer 73: 228-235, 1996).

Ligands of EGFR include EGF, TGFA/TGF-alpha, amphiregulin, epigen/EPGN, BTC/betacellulin, epiregulin/EREG and HBEGF/heparin-binding EGF. Receptor-ligand binding would trigger EGFR to form a homodimer or heterodimer, thereby resulting in autophosphorylation in the intracellular region and further activating the complex downstream signaling cascade reaction, which mainly includes the following signaling pathways: RAS-RAF-MEK-ERK signaling pathway, phosphatidylinositol 3-kinase (PI3K) signaling pathway, PLC gamma-PKC signaling pathway and STATs modules signaling pathway.

Overexpression of EGFR has been found in a variety of tumors, including bladder cancer, brain cancer, head and neck cancer, pancreatic cancer, lung cancer, breast cancer, ovarian cancer, colon cancer, prostate cancer, renal cancer, etc. (Atalay et al., Ann. Oncology 14: 1346-1363, 2003; Herbst and Shin, Cancer 94: 1593-1611, 2002; and Modjtahedi et al., Br. J. Cancer 73: 228-235, 1996). In many cases, overexpression of EGFR is associated with poor prognosis of patients (Herbst and Shin, Cancer 94: 1593-1611, 2002; Modjtahedi et al., Br. J. Cancer 73: 228-235, 1996). EGFR is also expressed in normal tissues and has relatively high expression levels in epithelial tissues of skin, liver and gastrointestinal tract; however, its expression level in normal tissues is much lower than that in tumor tissues (Herbst and Shin, Cancer 94: 1593-1611, 2002).

EGFR gene amplification and mutation can be detected simultaneously in a large portion of tumor samples. EGFRvIII is one of the mutant types, and is also known as de2-7 EGFR, ΔFGFR, or Δ2-7 (Olapade-Olaopa et al., Br. J. Cancer. 82, 186-94, 2000).

In a mature EGFRvIII mRNA, 801 nucleotides of exons 2-7 are deleted; correspondingly, in an EGFRvIII protein, 267 amino acids (6-273) are deleted and a glycine residue is inserted, forming an unique linker peptide (Wong et al., Proc. Natl. Acad. Sci. U.S.A. 89, 2965-9, 1992; Yamazaki et al., Jpn. J. Cancer Res. 81, 773-9, 1990; Yamazaki et al., Mol. Cell. Biol. 8, 1816-20, 1988; and Sugawa et al., Proc. Natl. Acad. Sci. U.S.A. 87, 8602-6, 1990).

Expression of EGFRvIII has been reported in various tumor types, including glioma, breast cancer, lung cancer, ovarian cancer and prostate cancer (Wikstrand et al., Cancer Res. 57, 4130-40, 1997; Olapade-Olaopa et al., Br. J. Cancer. 82, 186-94, 2000; Wikstrand, et al., Cancer Res. 55, 3140-8, 1995; Garcia de Palazzo et al., Cancer Res. 53, 3217-20, 1993). Although EGFRvIII cannot bind to a ligand, it is in a continuous low activation state. In a nude mouse tumor-xenograft model, the expression of EGFRvIII can significantly promote the growth of glioma cells (Nishikawa et al., Proc. Natl. Acad. Sci. U.S.A. 91, 7727-31, 1994). In addition, the expression of EGFRvIII can transform NIH3T3 cells and MCF-7 cells to produce carcinogenicity (Batra et al., Cell Growth Differ. 6, 1251-9, 1995). The mechanism of action of EGFRvIII in gliomas is not completely clear; however, according to existing reports, EGFRvIII can reduce the apoptosis of glioma cells and slightly increase the proliferation of glioma cells (Nagane et al., Cancer Res. 56, 5079-86, 1996). EGFRvIII is specifically expressed in tumor tissues but not in normal tissues, so it is a highly specific target in antibody therapies.

Monoclonal antibodies have been developed into new therapeutic drugs due to the targeting property, specificity, selectivity, high affinity, and other advantages. However, early clinical trials have revealed that the use of non-human monoclonal antibodies in human bodies often results in severe immune responses due to human anti-mouse antibody (HAMA) and human anti-rat antibody (HARA) responses, and therefore, the non-human monoclonal antibodies are quickly cleared. Subsequently, the antibodies having a low immunogenicity are developed, including chimeric antibodies, humanized antibodies, and complete human antibodies. According to the degree of humanization, therapeutic monoclonal antibody drugs can be divided into 4 types: murine antibody (having no humanized amino acid sequence), chimeric antibody (having 60% to 70% of humanized amino acid sequences), CDR-grafted antibody (having 90% to 95% of humanized amino acid sequences), and complete human antibody (having 100% of humanized amino acid sequences). As the degree of humanization increases, non-murine monoclonal antibodies may alleviate human anti-murine antibody responses (HAMA and HARA responses) during the treatment in human body, gradually eliminate the issue of causing immunogenicity by heterologous antibodies, and improve the pharmacokinetics of the antibodies while maintaining a high affinity for antigens; clinically, these antibody drugs have been widely used for targeted therapies.

An antibody-drug conjugate (ADC) is a new biotherapy method, which produces a specific killing effect on tumor cells by linking a biologically active small-molecule drug to a monoclonal antibody via a chemical linker, wherein the monoclonal antibody is acted as a carrier for targeting transport of the small-molecule drug to the surface of target tumor cells, and the small-molecule drug is released after the ADC is endocytosed by the tumor cells.

In the past 100 years, antibody-based immunotherapies and chemical drug-based chemotherapies have been the two major clinical strategies for cancer treatment. Antibodies target antigens that are overexpressed on tumor cells, and various therapeutic monoclonal antibodies have achieved great success clinically. In clinical practice, although therapeutic antibodies have a good targeting, but the killing effect thereof is limited. Although small-molecule chemical drugs have an efficient killing effect on cancer cells, they also cause the same damage to non-cancer cells. Therefore, the antibody drugs and small-molecule drugs have their own clinical limitations, which puts forward new requirements for drug research and development. A new generation of antibody-drug conjugates uses the specific binding ability of antibodies to target cells to deliver highly cytotoxic chemical drugs, thereby achieving targeted and efficient killing effect on cancer cells. With the emergence of new chemical linking techniques, antibody-drug conjugates began to enter clinical research in the late 1980s, and currently, there are 4 ADC drugs approved by the FDA for marketing.

The development of the ADC drugs involves several aspects such as screening of drug targets, preparation of recombinant antibodies, development of linker techniques, and screening and optimization of highly cytotoxic compounds. EGFRvIII is merely expressed in tumor tissues and not in normal tissues, so it is a highly specific target in antibody therapies.

### Content of the present invention

The technical problem to be solved in the present invention is for overcoming the existing deficiency of lacking an EGFRvIII antibody, thus providing an EGFRvIII antibody with a high affinity and a strong specificity, and a preparation method therefor and the use thereof. The EGFRvIII antibody has a high affinity with an EGFRvIII protein, and can enter cells after coupling with small molecule toxins such as MMAF and has a cytotoxic killing effect on EGFRvIII positive cells. The antibody therefore can be used for preparing drugs for treating tumors and other diseases.

The inventors take the EGFRvIII protein or a recombinant cell line overexpressing the EGFRvIII protein as an immunogen, and use a traditional hybridoma preparation technique. This technique was established by Kohler and Milstein 40 years ago (Kohler and Milstein1975, Nature 256: 495). Through a series of adjustments and improvements, a leading antibody of the EGFRvIII antibody is obtained. Then through preliminary production, purification and detection and identification of the leading antibody, the EGFRvIII antibody having excellent biological properties, such as a high antibody affinity and a cytotoxic killing effect on EGFRvIII positive cells produced by entering the cells after coupling with small-molecule toxin MMAF, is obtained. Subsequently, the amino acid sequence of the heavy chain variable region of the EGFRvIII antibody and the light chain variable region of the EGFRvIII antibody are obtained by means of sequencing using molecular biological methods.

In the art, the binding regions of antibodies or antigens contain a light chain variable region and a heavy chain variable region, each variable region containing three domains: CDR1, CDR2, and CDR3.

The present invention provides an EGFRvIII antibody, which comprises complementarity determining regions (CDRs): one or more of heavy chain CDR1, heavy chain CDR2 and heavy chain CDR3, and/or one or more of light chain CDR1, light chain CDR2 and light chain CDR3, wherein the amino acid sequence of the heavy chain CDR1 is as shown in SEQ ID No. 2, SEQ ID No. 10, SEQ ID No. 18, SEQ ID No. 26, SEQ ID No. 34, SEQ ID No. 42, SEQ ID No. 50, SEQ ID No. 58, SEQ ID No. 66, SEQ ID No. 74, SEQ ID No. 82, SEQ ID No. 90 or SEQ ID No. 98 in the sequence listing; the amino acid sequence of the heavy chain CDR2 is as shown in SEQ ID No. 3, SEQ ID No. 11, SEQ ID No. 19, SEQ ID No. 27, SEQ ID No. 35, SEQ ID No. 43, SEQ ID No. 51, SEQ ID No. 59, SEQ ID No. 67, SEQ ID No. 75, SEQ ID No. 83, SEQ ID No. 91, SEQ ID No. 99, SEQ ID NO. 184 or SEQ ID NO. 186 in the sequence listing; the amino acid sequence of the heavy chain CDR3 is as shown in SEQ ID No. 4, SEQ ID No. 12, SEQ ID No. 20, SEQ ID No. 28, SEQ ID No. 36, SEQ ID No. 44, SEQ ID No. 52, SEQ ID No. 60, SEQ ID No. 68, SEQ ID No. 76, SEQ ID No. 84, SEQ ID No. 92 or SEQ ID No. 100 in the sequence listing; the amino acid sequence of the light chain CDR1 is as shown in SEQ ID No. 6, SEQ ID No. 14, SEQ ID No. 22, SEQ ID No. 30, SEQ ID No. 38, SEQ ID No. 46, SEQ ID No. 54, SEQ ID No. 62, SEQ ID No. 70, SEQ ID No. 78, SEQ ID No. 86, SEQ ID No. 94 or SEQ ID No. 102 in the sequence listing; the amino acid sequence of the light chain CDR2 is as shown in SEQ ID No. 7, SEQ ID No. 15, SEQ ID No. 23, SEQ ID No. 31, SEQ ID No. 39, SEQ ID No. 47, SEQ ID No. 55, SEQ ID No. 63, SEQ ID No. 71, SEQ ID No. 79, SEQ ID No. 87, SEQ ID No. 95 or SEQ ID No. 103 in the sequence listing; the amino acid sequence of the light chain CDR3 is as shown in SEQ ID No. 8, SEQ ID No. 16, SEQ ID No. 24, SEQ ID No. 32, SEQ ID No. 40, SEQ ID No. 48, SEQ ID No. 56, SEQ ID No. 64, SEQ ID No. 72 or SEQ ID No. 80, SEQ ID No. 88, SEQ ID No. 96 or SEQ ID No. 104 in the sequence listing;
or the amino acid sequence of the heavy chain CDR1 is as shown in an amino acid sequence which has at least 80% sequence homology with the amino acid sequence as shown in SEQ ID No. 2, SEQ ID No. 10, SEQ ID No. 18, SEQ ID No. 26, SEQ ID No. 34, SEQ ID No. 42, SEQ ID No. 50, SEQ ID No. 58, SEQ ID No. 66, SEQ ID No. 74, SEQ ID No. 82, SEQ ID No. 90 and SEQ ID No. 98 in the sequence listing; the amino acid sequence of the heavy chain CDR2 is as shown in an amino acid sequence which has at least 80% sequence homology with the amino acid sequence as shown in SEQ ID No. 3, SEQ ID No. 11, SEQ ID No. 19, SEQ ID No. 27, SEQ ID No. 35, SEQ ID No. 43, SEQ ID No. 51, SEQ ID No. 59, SEQ ID No. 67, SEQ ID No. 75, SEQ ID No. 83, SEQ ID No. 91, SEQ ID No. 99, SEQ ID NO. 184 or SEQ ID NO. 186 in the sequence listing; the amino acid sequence of the heavy chain CDR3 is as shown in an amino acid sequence which has at least 80% sequence homology with the amino acid sequence as shown in SEQ ID No. 4, SEQ ID No. 12, SEQ ID No. 20, SEQ ID No. 28, SEQ ID No. 36, SEQ ID No. 44, SEQ ID No. 52, SEQ ID No. 60, SEQ ID No. 68, SEQ ID No. 76, SEQ ID No. 84, SEQ ID No. 92 and SEQ ID No. 100 in the sequence listing; the amino acid sequence of the light chain CDR1 is as shown in an amino acid sequence which has at least 80% sequence homology with the amino acid sequence as shown in SEQ ID No. 6, SEQ ID No. 14, SEQ ID No. 22, SEQ ID No. 30, SEQ ID No. 38, SEQ ID No. 46, SEQ ID No. 54, SEQ ID No. 62, SEQ ID No. 70, SEQ ID No. 78, SEQ ID No. 86, SEQ ID No. 94 and SEQ ID No. 102 in the sequence listing; the amino acid sequence of the light chain CDR2 is as shown in an amino acid sequence which has at least 80% sequence homology with the amino acid sequence as shown in SEQ ID No. 7, SEQ ID No. 15, SEQ ID No. 23, SEQ ID No. 31, SEQ ID No. 39, SEQ ID No. 47, SEQ ID No. 55, SEQ ID No. 63, SEQ ID No. 71, SEQ ID No. 79, SEQ ID No. 87, SEQ ID No. 95 and SEQ ID No. 103 in the sequence listing; the amino acid sequence of the light chain CDR3 is as shown in an amino acid sequence which has at least 80% sequence homology with the amino acid sequence as shown in SEQ ID No. 8, SEQ ID No. 16, SEQ ID No. 24, SEQ ID No. 32, SEQ ID No. 40, SEQ ID No. 48, SEQ ID No. 56, SEQ ID No. 64, SEQ ID No. 72, SEQ ID No. 80, SEQ ID No. 88, SEQ ID No. 96 and SEQ ID No. 104 in the sequence listing; preferably, the amino acid sequence of the heavy chain CDR1 is as shown in an amino acid sequence which has at least 90% sequence homology with the amino acid sequence as shown in SEQ ID No. 2, SEQ ID No. 10, SEQ ID No. 18, SEQ ID No. 26, SEQ ID No. 34, SEQ ID No. 42, SEQ ID No. 50, SEQ ID No. 58, SEQ ID No. 66, SEQ ID No. 74, SEQ ID No. 82, SEQ ID No. 90 and SEQ ID No. 98 in the sequence listing; the amino acid sequence of the heavy chain CDR2 is as shown in an amino acid sequence which has at least 90% sequence homology with the amino acid sequence as shown in SEQ ID No. 3, SEQ ID No. 11, SEQ ID No. 19, SEQ ID No. 27, SEQ ID No. 35, SEQ ID No. 43, SEQ ID No. 51, SEQ ID No. 59, SEQ ID No. 67, SEQ ID No. 75, SEQ ID No. 83, SEQ ID No. 91, SEQ ID No. 99, SEQ ID NO. 184 or SEQ ID NO. 186 in the sequence listing; the amino acid sequence of the heavy chain CDR3 is as shown in an amino acid sequence which has at least 90% sequence homology with the amino acid sequence as shown in SEQ ID No. 4, SEQ ID No. 12, SEQ ID No. 20, SEQ ID No. 28, SEQ ID No. 36, SEQ ID No. 44, SEQ ID No. 52, SEQ ID No. 60, SEQ ID No. 68, SEQ ID No. 76, SEQ ID No. 84, SEQ ID No. 92 and SEQ ID No. 100 in the sequence listing; the amino acid sequence of the light chain CDR1 is as shown in an amino acid sequence which has at least 90% sequence homology with the amino acid sequence as shown in SEQ ID No. 6, SEQ ID No. 14, SEQ ID No. 22, SEQ ID No. 30, SEQ ID No. 38, SEQ ID No. 46, SEQ ID No. 54, SEQ ID No. 62, SEQ ID No. 70, SEQ ID No. 78, SEQ ID No. 86, SEQ ID No. 94 and SEQ ID No. 102 in the sequence listing; the amino acid sequence of the light chain CDR2 is as shown in an amino acid sequence which has at least 90% sequence homology with the amino acid sequence as shown in SEQ ID No. 7, SEQ ID No. 15, SEQ ID No. 23, SEQ ID No. 31, SEQ ID No. 39, SEQ ID No. 47, SEQ ID No. 55, SEQ ID No. 63, SEQ ID No. 71, SEQ ID No. 79, SEQ ID No. 87, SEQ ID No. 95 and SEQ ID No. 103 in the sequence listing; the amino acid sequence of the light chain CDR3 is as shown in an amino acid sequence which has at least 90% sequence homology with the amino acid sequence as shown in SEQ ID No. 8, SEQ ID No. 16, SEQ ID No. 24, SEQ ID No. 32, SEQ ID No. 40, SEQ ID No. 48, SEQ ID No. 56, SEQ ID No. 64, SEQ ID No. 72, SEQ ID No. 80, SEQ ID No. 88, SEQ ID No. 96 and SEQ ID No. 104 in the sequence listing; more preferably, the amino acid sequence of the heavy chain CDR1 is as shown in an amino acid sequence which has at least 95% sequence homology with the amino acid sequence as shown in SEQ ID No. 2, SEQ ID No. 10, SEQ ID No. 18, SEQ ID No. 26, SEQ ID No. 34, SEQ ID No. 42, SEQ ID No. 50, SEQ ID No. 58, SEQ ID No. 66, SEQ ID No. 74, SEQ ID No. 82, SEQ ID No. 90 and SEQ ID No. 98 in the sequence listing; the amino acid sequence of the heavy chain CDR2 is as shown in an amino acid sequence which has at least 95% sequence homology with the amino acid sequence as shown in SEQ ID No. 3, SEQ ID No. 11, SEQ ID No. 19, SEQ ID No. 27, SEQ ID No. 35, SEQ ID No. 43, SEQ ID No. 51, SEQ ID No. 59, SEQ ID No. 67, SEQ ID No. 75, SEQ ID No. 83, SEQ ID No. 91, SEQ ID No. 99, SEQ ID NO. 184 or SEQ ID NO. 186 in the sequence listing; the amino acid sequence of the heavy chain CDR3 is as shown in an amino acid sequence which has at least 95% sequence homology with the amino acid sequence as shown in SEQ ID No. 4, SEQ ID No. 12, SEQ ID No. 20, SEQ ID No. 28, SEQ ID No. 36, SEQ ID No. 44, SEQ ID No. 52, SEQ ID No. 60, SEQ ID No. 68, SEQ ID No. 76, SEQ ID No. 84, SEQ ID No. 92 and SEQ ID No. 100 in the sequence listing; the amino acid sequence of the light chain CDR1 is as shown in an amino acid sequence which has at least 95% sequence homology with the amino acid sequence as shown in SEQ ID No. 6, SEQ ID No. 14, SEQ ID No. 22, SEQ ID No. 30, SEQ ID No. 38, SEQ ID No. 46, SEQ ID No. 54, SEQ ID No. 62, SEQ ID No. 70, SEQ ID No. 78, SEQ ID No. 86, SEQ ID No. 94 and SEQ ID No. 102 in the sequence listing; the amino acid sequence of the light chain CDR2 is as shown in an amino acid sequence which has at least 95% sequence homology with the amino acid sequence as shown in SEQ ID No. 7, SEQ ID No. 15, SEQ ID No. 23, SEQ ID No. 31, SEQ ID No. 39, SEQ ID No. 47, SEQ ID No. 55, SEQ ID No. 63, SEQ ID No. 71, SEQ ID No. 79, SEQ ID No. 87, SEQ ID No. 95 and SEQ ID No. 103 in the sequence listing; the amino acid sequence of the light chain CDR3 is as shown in an amino acid sequence which has at least 95% sequence homology with the amino acid sequence as shown in SEQ ID No. 8, SEQ ID No. 16, SEQ ID No. 24, SEQ ID No. 32, SEQ ID No. 40, SEQ ID No. 48, SEQ ID No. 56, SEQ ID No. 64, SEQ ID No. 72, SEQ ID No. 80, SEQ ID No. 88, SEQ ID No. 96 and SEQ ID No. 104 in the sequence listing; further more preferably, the amino acid sequence of the heavy chain CDR1 is as shown in an amino acid sequence which has at least 99% sequence homology with the amino acid sequence as shown in SEQ ID No. 2, SEQ ID No. 10, SEQ ID No. 18, SEQ ID No. 26, SEQ ID No. 34, SEQ ID No. 42, SEQ ID No. 50, SEQ ID No. 58, SEQ ID No. 66, SEQ ID No. 74, SEQ ID No. 82, SEQ ID No. 90 and SEQ ID No. 98 in the sequence listing; the amino acid sequence of the heavy chain CDR2 is as shown in an amino acid sequence which has at least 99% sequence homology with the amino acid sequence as shown in SEQ ID No. 3, SEQ ID No. 11, SEQ ID No. 19, SEQ ID No. 27, SEQ ID No. 35, SEQ ID No. 43, SEQ ID No. 51, SEQ ID No. 59, SEQ ID No. 67, SEQ ID No. 75, SEQ ID No. 83, SEQ ID No. 91, SEQ ID No. 99, SEQ ID NO. 184 or SEQ ID NO. 186 in the sequence listing; the amino acid sequence of the heavy chain CDR3 is as shown in an amino acid sequence which has at least 99% sequence homology with the amino acid sequence as shown in SEQ ID No. 4, SEQ ID No. 12, SEQ ID No. 20, SEQ ID No. 28, SEQ ID No. 36, SEQ ID No. 44, SEQ ID No. 52, SEQ ID No. 60, SEQ ID No. 68, SEQ ID No. 76, SEQ ID No. 84, SEQ ID No. 92 and SEQ ID No. 100 in the sequence listing; the amino acid sequence of the light chain CDR1 is as shown in an amino acid sequence which has at least 99% sequence homology with the amino acid sequence as shown in SEQ ID No. 6, SEQ ID No. 14, SEQ ID No. 22, SEQ ID No. 30, SEQ ID No. 38, SEQ ID No. 46, SEQ ID No. 54, SEQ ID No. 62, SEQ ID No. 70, SEQ ID No. 78, SEQ ID No. 86, SEQ ID No. 94 and SEQ ID No. 102 in the sequence listing; the amino acid sequence of the light chain CDR2 is as shown in an amino acid sequence which has at least 99% sequence homology with the amino acid sequence as shown in SEQ ID No. 7, SEQ ID No. 15, SEQ ID No. 23, SEQ ID No. 31, SEQ ID No. 39, SEQ ID No. 47, SEQ ID No. 55, SEQ ID No. 63, SEQ ID No. 71, SEQ ID No. 79, SEQ ID No. 87, SEQ ID No. 95 and SEQ ID No. 103 in the sequence listing; the amino acid sequence of the light chain CDR3 is as shown in an amino acid sequence which has at least 99% sequence homology with the amino acid sequence as shown in SEQ ID No. 8, SEQ ID No. 16, SEQ ID No. 24, SEQ ID No. 32, SEQ ID No. 40, SEQ ID No. 48, SEQ ID No. 56, SEQ ID No. 64, SEQ ID No. 72, SEQ ID No. 80, SEQ ID No. 88, SEQ ID No. 96 and SEQ ID No. 104 in the sequence listing.

It should be noted that although the antibodies used in antibody drugs are monoclonal antibodies obtained from the clones of single antibody-producing cells, the antibodies are biological macromolecules with a very complicated structure. Therefore, various post-translational modifications and degradation reactions occur during production, transportation, storage, and in vivo use, such as N-terminal cyclization, glycosylation, deamidation, isomerization, oxidation, fragmentation, and disulfide bond mismatch. These quality attributes may affect the antibody stability, biological activity and bioavailability of final products. Therefore, it is very important to control the stability and consistency of the product quality. The amino acid sequences having "at least 80% (or 90%, 95%, 99%) sequence homology" described in the present invention are obtained by performing insertion, deletion or substitution on the amino acid sequences shown in the preceding sequences, thereby overcoming the above-mentioned problem of possible instability. For example, the computer structure simulation analysis is performed on the sequences, and the site analysis is performed on potential post-translational modifications (PTMs), especially in CDR regions, including analysis and substitution on antibody aggregation, asparagine deamidation sensitive sites (NG, NS, NH, etc.), aspartic acid isomerization sensitive sites (DG and DP), N glycosylation sensitive site (N-{P}S/T) and oxidation sensitive sites.

Among them, deamidation refers to the reaction of converting the amide on the side chain of asparagine and aspartic acid into carboxylic acid; although the deamidation speed of glutamine is usually one-tenth that of asparagine, the mechanism is the same. Isomerization means that the carboxyl group located on the side chain of asparagine and aspartic acid is attacked by a nitrogen atom electron pair located on the residues of the C-terminal side, resulting in deamidation of asparagine or dehydration of aspartic acid to form an unstable cyclic imide intermediate, wherein through cleavage, most of the intermediate becomes isoaspartic acid, and the remaining part becomes aspartic acid.

In addition, it has been reported that the deamidation reaction is particularly likely to occur at the adjacent site (Asn-Gly) of asparagine and glycine (Geiger et al., (J. Biol. Chem. 1987, 262: 785-794)). It has also been reported that aspartic acid undergoes a peptide chain breakage through a hydrolysis reaction, and especially the sequence (Asp-Pro) containing proline at the C-terminal side is susceptible to decomposition under acidic conditions (Segalas et al., FEBS Letters, 1995, 371: 171-175). Deamidation and isomerization reactions that occur during storage of these antibodies and proteins become the cause of product heterogeneity, so it is desired to be controlled as much as possible. In order to suppress deamidation, isomerization and hydrolysis, amino acid modifications of removing glutamyl residues and aspartoyl residues that are used as sites undergoing deamidation can be appropriately performed. As an non-limiting scheme of removing particularly effective deamidation sites, it is preferred to list sites promoting the deamidation reaction; i.e., glycine residues, asparagine residues, or glutamine residues in motifs represented by NG and QG sequences, wherein the substitution of any of the amino acid residues (N, Q or G) can significantly inhibit the deamidation reaction (WO 2003/057881, WO 2005/067620, etc.). In addition, by adjusting the method for culturing antibody-producing cells or by appropriately using a deamidation reaction, a method for inhibiting antibodies can be obtained.

Preferably, the amino acid sequence of the heavy chain CDR1 is as shown in SEQ ID No. 2 in the sequence listing, the amino acid sequence of the heavy chain CDR2 is as shown in SEQ ID No. 3 in the sequence listing, and the amino acid sequence of the heavy chain CDR3 is as shown in SEQ ID No. 4 in the sequence listing; the amino acid sequence of the heavy chain CDR1 is as shown in SEQ ID No. 10 in the sequence listing, the amino acid sequence of the heavy chain CDR2 is as shown in SEQ ID No. 11 in the sequence listing, and the amino acid sequence of the heavy chain CDR3 is as shown in SEQ ID No. 12 in the sequence listing; the amino acid sequence of the heavy chain CDR1 is as shown in SEQ ID No. 18 in the sequence listing, the amino acid sequence of the heavy chain CDR2 is as shown in SEQ ID No. 19 in the sequence listing, and the amino acid sequence of the heavy chain CDR3 is as shown in SEQ ID No. 20 in the sequence listing; the amino acid sequence of the heavy chain CDR1 is as shown in SEQ ID No. 26 in the sequence listing, the amino acid sequence of the heavy chain CDR2 is as shown in SEQ ID No. 27 in the sequence listing, and the amino acid sequence of the heavy chain CDR3 is as shown in SEQ ID No. 28 in the sequence listing; the amino acid sequence of the heavy chain CDR1 is as shown in SEQ ID No. 34 in the sequence listing, the amino acid sequence of the heavy chain CDR2 is as shown in SEQ ID No. 35 in the sequence listing, and the amino acid sequence of the heavy chain CDR3 is as shown in SEQ ID No. 36 in the sequence listing; the amino acid sequence of the heavy chain CDR1 is as shown in SEQ ID No. 42 in the sequence listing, the amino acid sequence of the heavy chain CDR2 is as shown in SEQ ID No. 43 in the sequence listing, and the amino acid sequence of the heavy chain CDR3 is as shown in SEQ ID No. 44 in the sequence listing; the amino acid sequence of the heavy chain CDR1 is as shown in SEQ ID No. 50 in the sequence listing, the amino acid sequence of the heavy chain CDR2 is as shown in SEQ ID No. 51 in the sequence listing, and the amino acid sequence of the heavy chain CDR3 is as shown in SEQ ID No. 52 in the sequence listing; the amino acid sequence of the heavy chain CDR1 is as shown in SEQ ID No. 58 in the sequence listing, the amino acid sequence of the heavy chain CDR2 is as shown in SEQ ID No. 59 in the sequence listing, and the amino acid sequence of the heavy chain CDR3 is as shown in SEQ ID No. 60 in the sequence listing; the amino acid sequence of the heavy chain CDR1 is as shown in SEQ ID No. 66 in the sequence listing, the amino acid sequence of the heavy chain CDR2 is as shown in SEQ ID No. 67 in the sequence listing, and the amino acid sequence of the heavy chain CDR3 is as shown in SEQ ID No. 68 in the sequence listing; the amino acid sequence of the heavy chain CDR1 is as shown in SEQ ID No. 74 in the sequence listing, the amino acid sequence of the heavy chain CDR2 is as shown in SEQ ID No. 75 in the sequence listing, and the amino acid sequence of the heavy chain CDR3 is as shown in SEQ ID No. 76 in the sequence listing; the amino acid sequence of the heavy chain CDR1 is as shown in SEQ ID No. 82 in the sequence listing, the amino acid sequence of the heavy chain CDR2 is as shown in SEQ ID No. 83 in the sequence listing, and the amino acid sequence of the heavy chain CDR3 is as shown in SEQ ID No. 84 in the sequence listing; the amino acid sequence of the heavy chain CDR1 is as shown in SEQ ID No. 90 in the sequence listing, the amino acid sequence of the heavy chain CDR2 is as shown in SEQ ID No. 91 in the sequence listing, and the amino acid sequence of the heavy chain CDR3 is as shown in SEQ ID No. 92 in the sequence listing; the amino acid sequence of the heavy chain CDR1 is as shown in SEQ ID No. 98 in the sequence listing, the amino acid sequence of the heavy chain CDR2 is as shown in SEQ ID No. 99 in the sequence listing, and the amino acid sequence of the heavy chain CDR3 is as shown in SEQ ID No. 100 in the sequence listing; the amino acid sequence of the heavy chain CDR1 is as shown in SEQ ID No. 2 in the sequence listing, the amino acid sequence of the heavy chain CDR2 is as shown in SEQ ID No. 184 in the sequence listing, and the amino acid sequence of the heavy chain CDR3 is as shown in SEQ ID No. 4 in the sequence listing; or the amino acid sequence of the heavy chain CDR1 is as shown in SEQ ID No. 2 in the sequence listing, the amino acid sequence of the heavy chain CDR2 is as shown in SEQ ID No. 186 in the sequence listing, and the amino acid sequence of the heavy chain CDR3 is as shown in SEQ ID No. 4 in the sequence listing;
the amino acid sequence of the light chain CDR1 is as shown in SEQ ID No. 6 in the sequence listing, the amino acid sequence of the light chain CDR2 is as shown in SEQ ID No. 7 in the sequence listing, and the amino acid sequence of the light chain CDR3 is as shown in SEQ ID No. 8 in the sequence listing; the amino acid sequence of the light chain CDR1 is as shown in SEQ ID No. 14 in the sequence listing, the amino acid sequence of the light chain CDR2 is as shown in SEQ ID No. 15 in the sequence listing, and the amino acid sequence of the light chain CDR3 is as shown in SEQ ID No. 16 in the sequence listing; or the amino acid sequence of the light chain CDR1 is as shown in SEQ ID No. 22 in the sequence listing, the amino acid sequence of the light chain CDR2 is as shown in SEQ ID No. 23 in the sequence listing, and the amino acid sequence of the light chain CDR3 is as shown in SEQ ID No. 24 in the sequence listing; the amino acid sequence of the light chain CDR1 is as shown in SEQ ID No. 30 in the sequence listing, the amino acid sequence of the light chain CDR2 is as shown in SEQ ID No. 31 in the sequence listing, and the amino acid sequence of the light chain CDR3 is as shown in SEQ ID No. 32 in the sequence listing; the amino acid sequence of the light chain CDR1 is as shown in SEQ ID No. 38 in the sequence listing, the amino acid sequence of the light chain CDR2 is as shown in SEQ ID No. 39 in the sequence listing, and the amino acid sequence of the light chain CDR3 is as shown in SEQ ID No. 40 in the sequence listing; the amino acid sequence of the light chain CDR1 is as shown in SEQ ID No. 46 in the sequence listing, the amino acid sequence of the light chain CDR2 is as shown in SEQ ID No. 47 in the sequence listing, and the amino acid sequence of the light chain CDR3 is as shown in SEQ ID No. 48 in the sequence listing; the amino acid sequence of the light chain CDR1 is as shown in SEQ ID No. 54 in the sequence listing, the amino acid sequence of the light chain CDR2 is as shown in SEQ ID No. 55 in the sequence listing, and the amino acid sequence of the light chain CDR3 is as shown in SEQ ID No. 56 in the sequence listing; the amino acid sequence of the light chain CDR1 is as shown in SEQ ID No. 62 in the sequence listing, the amino acid sequence of the light chain CDR2 is as shown in SEQ ID No. 63 in the sequence listing, and the amino acid sequence of the light chain CDR3 is as shown in SEQ ID No. 64 in the sequence listing; the amino acid sequence of the light chain CDR1 is as shown in SEQ ID No. 70 in the sequence listing, the amino acid sequence of the light chain CDR2 is as shown in SEQ ID No. 71 in the sequence listing, and the amino acid sequence of the light chain CDR3 is as shown in SEQ ID No. 72 in the sequence listing; the amino acid sequence of the light chain CDR1 is as shown in SEQ ID No. 78 in the sequence listing, the amino acid sequence of the light chain CDR2 is as shown in SEQ ID No. 79 in the sequence listing, and the amino acid sequence of the light chain CDR3 is as shown in SEQ ID No. 80 in the sequence listing; the amino acid sequence of the light chain CDR1 is as shown in SEQ ID No. 86 in the sequence listing, the amino acid sequence of the light chain CDR2 is as shown in SEQ ID No. 87 in the sequence listing, and the amino acid sequence of the light chain CDR3 is as shown in SEQ ID No. 88 in the sequence listing; the amino acid sequence of the light chain CDR1 is as shown in SEQ ID No. 94 in the sequence listing, the amino acid sequence of the light chain CDR2 is as shown in SEQ ID No. 95 in the sequence listing, and the amino acid sequence of the light chain CDR3 is as shown in SEQ ID No. 96 in the sequence listing; or the amino acid sequence of the light chain CDR1 is as shown in SEQ ID No. 102 in the sequence listing, the amino acid sequence of the light chain CDR2 is as shown in SEQ ID No. 103 in the sequence listing, and the amino acid sequence of the light chain CDR3 is as shown in SEQ ID No. 104 in the sequence listing.

More preferably, the EGFRvIII antibody comprises a heavy chain variable region and/or a light chain variable region containing the CDRs, wherein the amino acid sequence of the heavy chain variable region is as shown in SEQ ID No. 1, SEQ ID No. 9, SEQ ID No. 17, SEQ ID No. 25, SEQ ID No. 33, SEQ ID No. 41, SEQ ID No. 49, SEQ ID No. 57, SEQ ID No. 65, SEQ ID No. 73, SEQ ID No. 81, SEQ ID No. 89, SEQ ID No. 97, SEQ ID No. 179 or SEQ ID No. 180 in the sequence listing; and the amino acid sequence of the light chain variable region is as shown in SEQ ID No. 5, SEQ ID No. 13, SEQ ID No. 21, SEQ ID No. 29, SEQ ID No. 37, SEQ ID No. 45, SEQ ID No. 53, SEQ ID No. 61, SEQ ID No. 69, SEQ ID No. 77, SEQ ID No. 85, SEQ ID No. 93 or SEQ ID No. 101 in the sequence listing.

Preferably, the amino acid sequence of the heavy chain variable region is as shown in SEQ ID No. 1 in the sequence listing, and the amino acid sequence of the light chain variable region is as shown in SEQ ID No. 5 in the sequence listing; the amino acid sequence of the heavy chain variable region is as shown in SEQ ID No. 9 in the sequence listing, and the amino acid sequence of the light chain variable region is as shown in SEQ ID No. 13 in the sequence listing; the amino acid sequence of the heavy chain variable region is as shown in SEQ ID No. 17 in the sequence listing, and the amino acid sequence of the light chain variable region is as shown in SEQ ID No. 21 in the sequence listing; the amino acid sequence of the heavy chain variable region is as shown in SEQ ID No. 25 in the sequence listing, and the amino acid sequence of the light chain variable region is as shown in SEQ ID No. 29 in the sequence listing; the amino acid sequence of the heavy chain variable region is as shown in SEQ ID No. 33 in the sequence listing, and the amino acid sequence of the light chain variable region is as shown in SEQ ID No. 37 in the sequence listing; the amino acid sequence of the heavy chain variable region is as shown in SEQ ID No. 41 in the sequence listing, and the amino acid sequence of the light chain variable region is as shown in SEQ ID No. 45 in the sequence listing; the amino acid sequence of the heavy chain variable region is as shown in SEQ ID No. 49 in the sequence listing, and the amino acid sequence of the light chain variable region is as shown in SEQ ID No. 53 in the sequence listing; the amino acid sequence of the heavy chain variable region is as shown in SEQ ID No. 57 in the sequence listing, and the amino acid sequence of the light chain variable region is as shown in SEQ ID No. 61 in the sequence listing; the amino acid sequence of the heavy chain variable region is as shown in SEQ ID No. 65 in the sequence listing, and the amino acid sequence of the light chain variable region is as shown in SEQ ID No. 69 in the sequence listing; the amino acid sequence of the heavy chain variable region is as shown in SEQ ID No. 73 in the sequence listing, and the amino acid sequence of the light chain variable region is as shown in SEQ ID No. 77 in the sequence listing; the amino acid sequence of the heavy chain variable region is as shown in SEQ ID No. 81 in the sequence listing, and the amino acid sequence of the light chain variable region is as shown in SEQ ID No. 85 in the sequence listing; the amino acid sequence of the heavy chain variable region is as shown in SEQ ID No. 89 in the sequence listing, and the amino acid sequence of the light chain variable region is as shown in SEQ ID No. 93 in the sequence listing; the amino acid sequence of the heavy chain variable region is as shown in SEQ ID No. 97 in the sequence listing, and the amino acid sequence of the light chain variable region is as shown in SEQ ID No. 101 in the sequence listing; the amino acid sequence of the heavy chain variable region is as shown in SEQ ID No. 179 in the sequence listing, and the amino acid sequence of the light chain variable region is as shown in SEQ ID No. 5 in the sequence listing; or the amino acid sequence of the heavy chain variable region is as shown in SEQ ID No. 180 in the sequence listing, and the amino acid sequence of the light chain variable region is as shown in SEQ ID No. 5 in the sequence listing.

In summary, the numbers of the above-mentioned amino acid sequences are as shown in table 1:

**Table 1 Numbers of amino acid sequences of EGFRvIII antibody**

| Clone number | Heavy chain protein | | | | Light chain protein | | | |
|---|---|---|---|---|---|---|---|---|
| | Variable region | CDR1 | CDR2 | CDR3 | Variable region | CDR1 | CDR2 | CDR3 |
| 75G7C6 | 1 | 2 | 3 | 4 | 5 | 6 | 7 | 8 |
| 63A10A7 | 9 | 10 | 11 | 12 | 13 | 14 | 15 | 16 |
| 64F1F8 | 17 | 18 | 19 | 20 | 21 | 22 | 23 | 24 |
| 7E5-2A9 | 25 | 26 | 27 | 28 | 29 | 30 | 31 | 32 |
| 43H6A1 | 33 | 34 | 35 | 36 | 37 | 38 | 39 | 40 |
| 46C4A6 | 41 | 42 | 43 | 44 | 45 | 46 | 47 | 48 |
| 49G12G5 | 49 | 50 | 51 | 52 | 53 | 54 | 55 | 56 |
| 51G7C2 | 57 | 58 | 59 | 60 | 61 | 62 | 63 | 64 |
| 53D8H4 | 65 | 66 | 67 | 68 | 69 | 70 | 71 | 72 |
| 54D2G10 | 73 | 74 | 75 | 76 | 77 | 78 | 79 | 80 |
| 56F10G2 | 81 | 82 | 83 | 84 | 85 | 86 | 87 | 88 |
| 59G3F12 | 89 | 90 | 91 | 92 | 93 | 94 | 95 | 96 |
| 64B11F2 | 97 | 98 | 99 | 100 | 101 | 102 | 103 | 104 |

The numbers in table 1 are the sequence numbers in the sequence listing. For example, the amino acid sequence of the heavy chain protein variable region of 75G7C6 is SEQ ID No. 1, and the amino acid sequence of the CDR1 in the heavy chain protein variable region of 75G7C6 is SEQ ID No. 2.

Preferably, the EGFRvIII antibody further comprises a framework region (or also known as a frame region or a backbone region), wherein the framework region further comprises a heavy chain framework region and/or a light chain framework region; preferably, the heavy chain framework region is a human or murine antibody heavy chain framework region, and/or the light chain framework region is a human or murine antibody light chain framework region; more preferably, the heavy chain framework region is a human antibody heavy chain framework region, and the light chain framework region is a human antibody light chain framework region. The framework residues are a part of the light chain variable region or the heavy chain variable region, and are residues of the antibody variable regions other than hypervariable residues (mostly referring to complementarity determining regions or CDRs) or CDR residues, wherein the residues of the antibody variable regions serve as a scaffold for the antigen-binding loop (CDR) of the variable domain. The framework residues may be derived from naturally occurring human antibodies, for example, the framework regions of human antibodies substantially similar to those of the murine anti-EGFRvIII antibody 75G7C6 or 63A10A7. Artificial framework sequences that represent consensus sequences among individual sequences can also be used. Once humanized framework regions are selected and used, sequences widely presented in humans may be superior to less common sequences. Additional mutations in the human framework receptor sequences may be prepared to restore murine residues thought to be involved in antigen contact and/or residues involved in the structural integrity of the antigen-binding sites, or to improve antibody expression. Peptide structure prediction can be used to analyze humanized heavy chain variable region sequences and light chain variable region sequences so as to identify and avoid post-translational protein modification sites introduced by humanized design.

More preferably, when the EGFRvIII antibody of the present invention is a humanized antibody or complete human antibody, the heavy chain framework region is a human antibody heavy chain framework region, and the residues of the human antibody light chain framework region may comprise sequences encoded by germlines IGKV1-12, IGKV1-13, IGKV1-16, IGKV1-17, IGKV1-22, IGKV1-27, IGKV1-32, IGKV1-37, IGKV1-39, IGKV1-5, IGKV1-8, IGKV1-9, IGKV3-34, IGKV2-36, IGKV3-11, IGKV2-40, IGKV3-25, IGKV6-21, IGKV7-3, IGKV5-2, IGKV4-1, IGKV2-4, IGKV2-19, IGKV2-18, IGKV3-20, IGKV2-26 and IGKV2-24, particularly FR1, FR2 and FR3 of these germlines; and Jk fragments Jk1, Jk2, Jk3, Jk4 and Jk5, particularly FR4 of these germlines. The residues of the human antibody heavy chain framework region may comprise sequences encoded by germlines IGHV7-81, IGHV4-80, IGHV3-79, IGHV(II)-78-1, IGHV5-78, IGHV3-76, IGHV3-73, IGHV3-72, IGHV2-70, IGHV1-69-2, IGHV2-70D, IGHV3-49, IGHV3-43, IGHV1-2, IGHV1-3, IGHV1-8, IGHV1-18, IGHV1-24, IGHV1-45, IGHV1-46, IGHV2-5, IGHV2-21, IGHV2-26, IGHV2-70, IGHD3-7, IGHV3-9, IGHV3-11, IGHV3-7, IGHV3-7 and IGHD3-9, particularly FR1, FR2 and FR3 of these germlines, and JH fragments JH1, JH2, JH3, JH4, JH4b, JH5 and JH6, particularly FR4 of these germlines; or consensus sequences of the heavy chain framework region. Such framework region sequences can be obtained from public DNA databases or published references that include germline antibody gene sequences. For example, the germline DNA sequences of human heavy and light chain variable region genes can be obtained from the "VBase" human germline sequence database (www.mrcco8.com.ac.uk/vbase), and found in Kabat, E.A et al., 1991 Sequences of Proteins of Immunological Interest, 5th edition. In a more preferred embodiment of the humanized antibody of the present invention, the human acceptable sequences of the humanized EGFRvIII antibody are selected from human germline exons V_{H}, J_{H}, Vₖ and Jₖ sequences, wherein the template of the antibody heavy chain variable region is preferably IGHV1-46^{∗}01 of the human germline antibody heavy chain exon V_{H}, and J_{H}-4 of the exon J_{H}; or IGHV1-46^{∗}01 of the human germline antibody heavy chain exon V_{H}, and J_{H}-6b of the exon J_{H}. The template of the antibody light chain variable region is preferably IGKV1-39^{∗}01 of the human germline antibody light chain exon V_{K}, and J_{K}-2 of the exon J_{K}; or IGKV3-11^{∗}01 of the human germline antibody light chain exon V_{K}, and J_{K}-4 of the exon J_{K}.

Preferably, the protein further comprises an antibody heavy chain constant region and/or an antibody light chain constant region, wherein the antibody heavy chain constant region is conventional in the art, preferably a mouse antibody heavy chain constant region or a human antibody heavy chain constant region, and more preferably a human antibody heavy chain constant region. The antibody light chain constant region is conventional in the art, preferably a mouse light chain antibody constant region or a human antibody light chain constant region, and more preferably a human antibody light chain constant region.

The heavy chain variable region having an amino acid sequence as shown in SEQ ID NO. 1, 9, 17, 25 or 179 and the light chain variable region having an amino acid sequence as shown in SEQ ID NO. 5, 13, 21 or 29 can form a murine EGFRvIII antibody with a murine heavy chain constant region, and also can form an EGFRvIII chimeric antibody with a human heavy chain constant region and a human light chain constant region (wherein the amino acid sequence shown in SEQ ID NO. 179 is obtained by mutating the amino acid sequence shown in SEQ ID NO. 1, with NG in the CDR2 region of the latter mutated to NA).

Further, in the above-mentioned chimeric antibody, the heavy chain CDR having a sequence as shown in SEQ ID NOs 2, 184 and 4 (corresponding to CDR1, 2 and 3, respectively) or SEQ ID NOs 10 to 12, determined according to the definition of Kabat, in the heavy chain variable region having an amino acid sequence as shown in SEQ ID NO. 179 or 9, and the light chain CDR having a sequence as shown in SEQ ID NOs 6 to 8 or SEQ ID NOs 14 to 16, determined according to the definition of Kabat, in the light chain variable region having an amino acid sequence as shown in SEQ ID NO. 5 or 13 are respectively grafted into the selected human germline template, thereby replacing the CDR regions of the human germline template to obtain a humanized antibody; the light chain framework region and the heavy chain framework region in the germline template are as described above; upon selection of the human antibody variable region frameworks, the light chain FR sequences on the antibody light chain variable region are derived from human germline light chain sequences comprising a combination of human antibody light chain framework segments of 1) FR1, FR2 and FR3 of IGKV1-39^{∗}01 or IGKV3-11^{∗}01, and 2) FR4 of J_{K}-2 or J_{K}-4; the heavy chain FR sequences on the antibody heavy chain variable region are derived from human germline heavy chain sequences comprising a combination of human antibody heavy chain framework segments of 1) FR1, FR2 and FR3 of IGHV1-46^{∗}01, and 2) FR4 of J_{H}-4 or J_{H}-6b. In general, the framework regions of a human receptor should be selected similar to those of the donor antibody or most similar to the consensus sequences of the variable region subfamilies. After graft, sequence mutations can be made in the donor and/or receptor sequences to optimize antigen binding, functionality, codon usage, expression levels, etc., including the introduction of non-human residues into the framework regions.

Preferably, after the above-mentioned CDR regions are grafted into the selected human germline template and the framework region residues are subjected to back mutation, the amino acid sequence of the heavy chain variable region is preferably as shown in SEQ ID NO. 133, 134, 135, 136, 143, 144, 145, 146, 147, 141, 142 or 147; and the amino acid sequence of the light chain variable region is preferably as shown in SEQ ID NO. 137, 138, 139, 140, 148, 149, 150, 151, 152 or 153.

The humanized EGFRvIII antibody of the present invention preferably comprises at least one heavy chain variable region and/or at least one light chain variable region, wherein the amino acid sequence of the heavy chain variable region is as shown in SEQ ID NO. 133, SEQ ID NO. 134, SEQ ID NO. 135, SEQ ID NO. 136, SEQ ID NO. 141, SEQ ID NO. 142, SEQ ID NO. 143, SEQ ID NO. 144, SEQ ID NO. 145, SEQ ID NO. 146 or SEQ ID NO. 147 in the sequence listing; the sequence of the light chain variable region is as shown in SEQ ID NO. 137, SEQ ID NO. 138, SEQ ID NO. 139, SEQ ID NO. 140, SEQ ID NO. 148, SEQ ID NO. 149, SEQ ID NO. 150, SEQ ID NO. 151, SEQ ID NO. 152 or SEQ ID NO. 153 in the sequence listing;
or the amino acid sequence of the heavy chain variable region is as shown in an amino acid sequence which has at least 80% sequence homology with the amino acid sequence as shown in SEQ ID NO. 133, SEQ ID NO. 134, SEQ ID NO. 135, SEQ ID NO. 136, SEQ ID NO. 141, SEQ ID NO. 142, SEQ ID NO. 143, SEQ ID NO. 144, SEQ ID NO. 145, SEQ ID NO. 146 or SEQ ID NO. 147 in the sequence listing; the sequence of the light chain variable region is as shown in an amino acid sequence which has at least 80% sequence homology with the amino acid sequence as shown in SEQ ID NO. 137, SEQ ID NO. 138, SEQ ID NO. 139, SEQ ID NO. 140, SEQ ID NO. 148, SEQ ID NO. 149, SEQ ID NO. 150, SEQ ID NO. 151, SEQ ID NO. 152 or SEQ ID NO. 153 in the sequence listing; more preferably, the amino acid sequence of the heavy chain variable region is an amino acid sequence which has 90% sequence homology with the amino acid sequence encoded by the nucleotide sequence as shown in SEQ ID No. 154, SEQ ID No. 155, SEQ ID No. 156, SEQ ID No. 157, SEQ ID No. 162, SEQ ID No. 163, SEQ ID No. 164, SEQ ID No. 165, SEQ ID No. 166, SEQ ID No. 167 or SEQ ID No. 168 in the sequence listing; the amino acid sequence of the light chain variable region is an amino acid sequence which has 90% sequence homology with the amino acid sequence encoded by the nucleotide sequence as shown in SEQ ID No. 158, SEQ ID No. 159, SEQ ID No. 160, SEQ ID No. 161, SEQ ID No. 169, SEQ ID No. 170, SEQ ID No. 171, SEQ ID No. 172, SEQ ID No. 173 or SEQ ID No. 174 in the sequence listing; further more preferably, the amino acid sequence of the heavy chain variable region is an amino acid sequence which has 95% sequence homology with the amino acid sequence encoded by the nucleotide sequence as shown in SEQ ID No. 154, SEQ ID No. 155, SEQ ID No. 156, SEQ ID No. 157, SEQ ID No. 162, SEQ ID No. 163, SEQ ID No. 164, SEQ ID No. 165, SEQ ID No. 166, SEQ ID No. 167 or SEQ ID No. 168 in the sequence listing; the amino acid sequence of the light chain variable region is an amino acid sequence which has 95% sequence homology with the amino acid sequence encoded by the nucleotide sequence as shown in SEQ ID No. 158, SEQ ID No. 159, SEQ ID No. 160, SEQ ID No. 161, SEQ ID No. 169, SEQ ID No. 170, SEQ ID No. 171, SEQ ID No. 172, SEQ ID No. 173 or SEQ ID No. 174 in the sequence listing; most preferably, the amino acid sequence of the heavy chain variable region is an amino acid sequence which has 99% sequence homology with the amino acid sequence encoded by the nucleotide sequence as shown in SEQ ID No. 154, SEQ ID No. 155, SEQ ID No. 156, SEQ ID No. 157, SEQ ID No. 162, SEQ ID No. 163, SEQ ID No. 164, SEQ ID No. 165, SEQ ID No. 166, SEQ ID No. 167 or SEQ ID No. 168 in the sequence listing; the amino acid sequence of the light chain variable region is an amino acid sequence which has 99% sequence homology with the amino acid sequence encoded by the nucleotide sequence as shown in SEQ ID No. 158, SEQ ID No. 159, SEQ ID No. 160, SEQ ID No. 161, SEQ ID No. 169, SEQ ID No. 170, SEQ ID No. 171, SEQ ID No. 172, SEQ ID No. 173 or SEQ ID No. 174 in the sequence listing.

Preferably, the amino acid sequence of the heavy chain variable region is an amino acid sequence which has 80% sequence homology with the amino acid sequence encoded by the nucleotide sequence as shown in SEQ ID No. 154, SEQ ID No. 155, SEQ ID No. 156, SEQ ID No. 157, SEQ ID No. 162, SEQ ID No. 163, SEQ ID No. 164, SEQ ID No. 165, SEQ ID No. 166, SEQ ID No. 167 or SEQ ID No. 168 in the sequence listing; the amino acid sequence of the light chain variable region is an amino acid sequence which has 80% sequence homology with the amino acid sequence encoded by the nucleotide sequence as shown in SEQ ID No. 158, SEQ ID No. 159, SEQ ID No. 160, SEQ ID No. 161, SEQ ID No. 169, SEQ ID No. 170, SEQ ID No. 171, SEQ ID No. 172, SEQ ID No. 173 or SEQ ID No. 174 in the sequence listing; more preferably, the amino acid sequence of the heavy chain variable region is an amino acid sequence which has 90% sequence homology with the amino acid sequence encoded by the nucleotide sequence as shown in SEQ ID No. 154, SEQ ID No. 155, SEQ ID No. 156, SEQ ID No. 157, SEQ ID No. 162, SEQ ID No. 163, SEQ ID No. 164, SEQ ID No. 165, SEQ ID No. 166, SEQ ID No. 167 or SEQ ID No. 168 in the sequence listing; the amino acid sequence of the light chain variable region is an amino acid sequence which has 90% sequence homology with the amino acid sequence encoded by the nucleotide sequence as shown in SEQ ID No. 158, SEQ ID No. 159, SEQ ID No. 160, SEQ ID No. 161, SEQ ID No. 169, SEQ ID No. 170, SEQ ID No. 171, SEQ ID No. 172, SEQ ID No. 173 or SEQ ID No. 174 in the sequence listing; further more preferably, the amino acid sequence of the heavy chain variable region is an amino acid sequence which has 95% sequence homology with the amino acid sequence encoded by the nucleotide sequence as shown in SEQ ID No. 154, SEQ ID No. 155, SEQ ID No. 156, SEQ ID No. 157, SEQ ID No. 162, SEQ ID No. 163, SEQ ID No. 164, SEQ ID No. 165, SEQ ID No. 166, SEQ ID No. 167 or SEQ ID No. 168 in the sequence listing; the amino acid sequence of the light chain variable region is an amino acid sequence which has 95% sequence homology with the amino acid sequence encoded by the nucleotide sequence as shown in SEQ ID No. 158, SEQ ID No. 159, SEQ ID No. 160, SEQ ID No. 161, SEQ ID No. 169, SEQ ID No. 170, SEQ ID No. 171, SEQ ID No. 172, SEQ ID No. 173 or SEQ ID No. 174 in the sequence listing; most preferably, the amino acid sequence of the heavy chain variable region is an amino acid sequence which has 99% sequence homology with the amino acid sequence encoded by the nucleotide sequence as shown in SEQ ID No. 154, SEQ ID No. 155, SEQ ID No. 156, SEQ ID No. 157, SEQ ID No. 162, SEQ ID No. 163, SEQ ID No. 164, SEQ ID No. 165, SEQ ID No. 166, SEQ ID No. 167 or SEQ ID No. 168 in the sequence listing; the amino acid sequence of the light chain variable region is an amino acid sequence which has 99% sequence homology with the amino acid sequence encoded by the nucleotide sequence as shown in SEQ ID No. 158, SEQ ID No. 159, SEQ ID No. 160, SEQ ID No. 161, SEQ ID No. 169, SEQ ID No. 170, SEQ ID No. 171, SEQ ID No. 172, SEQ ID No. 173 or SEQ ID No. 174 in the sequence listing.

Preferably, the amino acid sequence of the heavy chain variable region is a sequence as shown in SEQ ID No. 133 in the sequence listing, and the amino acid sequence of the light chain variable region is a sequence as shown in SEQ ID No. 137 in the sequence listing; the amino acid sequence of the heavy chain variable region is a sequence as shown in SEQ ID No. 133 in the sequence listing, and the amino acid sequence of the light chain variable region is a sequence as shown in SEQ ID No. 138 in the sequence listing; the amino acid sequence of the heavy chain variable region is a sequence as shown in SEQ ID No. 133 in the sequence listing, and the amino acid sequence of the light chain variable region is a sequence as shown in SEQ ID No. 139 in the sequence listing; the amino acid sequence of the heavy chain variable region is a sequence as shown in SEQ ID No. 133 in the sequence listing, and the amino acid sequence of the light chain variable region is a sequence as shown in SEQ ID No. 140 in the sequence listing; the amino acid sequence of the heavy chain variable region is a sequence as shown in SEQ ID No. 134 in the sequence listing, and the amino acid sequence of the light chain variable region is a sequence as shown in SEQ ID No. 137 in the sequence listing; the amino acid sequence of the heavy chain variable region is a sequence as shown in SEQ ID No. 134 in the sequence listing, and the amino acid sequence of the light chain variable region is a sequence as shown in SEQ ID No. 138 in the sequence listing; the amino acid sequence of the heavy chain variable region is a sequence as shown in SEQ ID No. 134 in the sequence listing, and the amino acid sequence of the light chain variable region is a sequence as shown in SEQ ID No. 139 in the sequence listing; the amino acid sequence of the heavy chain variable region is a sequence as shown in SEQ ID No. 134 in the sequence listing, and the amino acid sequence of the light chain variable region is a sequence as shown in SEQ ID No. 140 in the sequence listing; the amino acid sequence of the heavy chain variable region is a sequence as shown in SEQ ID No. 135 in the sequence listing, and the amino acid sequence of the light chain variable region is a sequence as shown in SEQ ID No. 137 in the sequence listing; the amino acid sequence of the heavy chain variable region is a sequence as shown in SEQ ID No. 135 in the sequence listing, and the amino acid sequence of the light chain variable region is a sequence as shown in SEQ ID No. 138 in the sequence listing; the amino acid sequence of the heavy chain variable region is a sequence as shown in SEQ ID No. 135 in the sequence listing, and the amino acid sequence of the light chain variable region is a sequence as shown in SEQ ID No. 139 in the sequence listing; the amino acid sequence of the heavy chain variable region is a sequence as shown in SEQ ID No. 135 in the sequence listing, and the amino acid sequence of the light chain variable region is a sequence as shown in SEQ ID No. 140 in the sequence listing; the amino acid sequence of the heavy chain variable region is a sequence as shown in SEQ ID No. 136 in the sequence listing, and the amino acid sequence of the light chain variable region is a sequence as shown in SEQ ID No. 137 in the sequence listing; the amino acid sequence of the heavy chain variable region is a sequence as shown in SEQ ID No. 136 in the sequence listing, and the amino acid sequence of the light chain variable region is a sequence as shown in SEQ ID No. 138 in the sequence listing; the amino acid sequence of the heavy chain variable region is a sequence as shown in SEQ ID No. 136 in the sequence listing, and the amino acid sequence of the light chain variable region is a sequence as shown in SEQ ID No. 139 in the sequence listing; the amino acid sequence of the heavy chain variable region is a sequence as shown in SEQ ID No. 136 in the sequence listing, and the amino acid sequence of the light chain variable region is a sequence as shown in SEQ ID No. 140 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 144 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 145 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 146 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 147 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 148 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 150 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 151 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 152 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 153 in the sequence listing; the amino acid sequence of the heavy chain variable region is a sequence as shown in SEQ ID No. 141 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 141 in the sequence listing, and the amino acid sequence of the light chain variable region is a sequence as shown in SEQ ID No. 150 in the sequence listing; the amino acid sequence of the heavy chain variable region is a sequence as shown in SEQ ID No. 141 in the sequence listing, and the amino acid sequence of the light chain variable region is a sequence as shown in SEQ ID No. 151 in the sequence listing; the amino acid sequence of the heavy chain variable region is a sequence as shown in SEQ ID No. 141 in the sequence listing, and the amino acid sequence of the light chain variable region is a sequence as shown in SEQ ID No. 152 in the sequence listing; the amino acid sequence of the heavy chain variable region is a sequence as shown in SEQ ID No. 141 in the sequence listing, and the amino acid sequence of the light chain variable region is a sequence as shown in SEQ ID No. 153 in the sequence listing; the amino acid sequence of the heavy chain variable region is a sequence as shown in SEQ ID No. 142 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 142 in the sequence listing, and the amino acid sequence of the light chain variable region is a sequence as shown in SEQ ID No. 150 in the sequence listing; the amino acid sequence of the heavy chain variable region is a sequence as shown in SEQ ID No. 142 in the sequence listing, and the amino acid sequence of the light chain variable region is a sequence as shown in SEQ ID No. 151 in the sequence listing; the amino acid sequence of the heavy chain variable region is a sequence as shown in SEQ ID No. 142 in the sequence listing, and the amino acid sequence of the light chain variable region is a sequence as shown in SEQ ID No. 152 in the sequence listing; the amino acid sequence of the heavy chain variable region is a sequence as shown in SEQ ID No. 142 in the sequence listing, and the amino acid sequence of the light chain variable region is a sequence as shown in SEQ ID No. 153 in the sequence listing; the amino acid sequence of the heavy chain variable region is a sequence as shown in SEQ ID No. 145 in the sequence listing, and the amino acid sequence of the light chain variable region is a sequence as shown in SEQ ID No. 148 in the sequence listing; the amino acid sequence of the heavy chain variable region is a sequence as shown in SEQ ID No. 146 in the sequence listing, and the amino acid sequence of the light chain variable region is a sequence as shown in SEQ ID No. 148 in the sequence listing; or the amino acid sequence of the heavy chain variable region is a sequence as shown in SEQ ID No. 147 in the sequence listing, and the amino acid sequence of the light chain variable region is a sequence as shown in SEQ ID No. 148 in the sequence listing.

In summary, the numbers of the above-mentioned amino acid sequences are as shown in table 1-1.

**Table 1-1 Numbers of amino acid sequences of humanized EGFRvIII antibody**

| Antibody number | Heavy chain variable region SEQ ID NO. | Light chain variable region SEQ ID NO. |
|---|---|---|
| c75G7C6-2 | 180 | 5 |
| c75G7C6-1 | 179 | 5 |
| h75G7C6-1 | 133 | 137 |
| h75G7C6-2 | 133 | 138 |
| h75G7C6-3 | 133 | 139 |
| h75G7C6-4 | 133 | 140 |
| h75G7C6-5 | 134 | 137 |
| h75G7C6-6 | 134 | 138 |
| h75G7C6-7 | 134 | 139 |
| h75G7C6-8 | 134 | 140 |
| h75G7C6-9 | 135 | 137 |
| h75G7C6-10 | 135 | 138 |
| h75G7C6-11 | 135 | 139 |
| h75G7C6-12 | 135 | 140 |
| h75G7C6-13 | 136 | 137 |
| h75G7C6-14 | 136 | 138 |
| h75G7C6-15 | 136 | 139 |
| h75G7C6-16 | 136 | 140 |
| c63A10A7 | 9 | 13 |
| h63A10A7-17 | 143 | 149 |
| h63A10A7-18 | 144 | 149 |
| h63A10A7-19 | 145 | 149 |
| h63A10A7-20 | 146 | 149 |
| h63A10A7-21 | 147 | 149 |
| h63A10A7-22 | 143 | 148 |
| h63A10A7-23 | 143 | 150 |
| h63A10A7-24 | 143 | 151 |
| h63A10A7-25 | 143 | 152 |
| h63A10A7-26 | 143 | 153 |
| h63A10A7-27 | 141 | 149 |
| h63A10A7-28 | 141 | 150 |
| h63A10A7-29 | 141 | 151 |
| h63A10A7-30 | 141 | 152 |
| h63A10A7-31 | 141 | 153 |
| h63A10A7-32 | 142 | 149 |
| h63A10A7-33 | 142 | 150 |
| h63A10A7-34 | 142 | 151 |
| h63A10A7-35 | 142 | 152 |
| h63A10A7-36 | 142 | 153 |
| h63A10A7-37 | 145 | 148 |
| h63A10A7-38 | 146 | 148 |
| h63A10A7-39 | 147 | 148 |

The numbers in table 1-1 are the sequence numbers in the sequence listing. For example, the amino acid sequence of the heavy chain protein variable region of h75G7C6-1 is SEQ ID No. 133, and the amino acid sequence of the light chain protein variable region of h75G7C6-1 is SEQ ID No. 137.

The CDR1, CDR2 and CDR3 of the heavy chain variable region of c75G7C6-1 are as shown in SEQ ID NO. 2, SEQ ID NO. 184 and SEQ ID NO. 4 in the sequence listing, respectively.

The CDR1, CDR2 and CDR3 of the heavy chain variable region of c75G7C6-2 are as shown in SEQ ID NO. 2, SEQ ID NO. 186 and SEQ ID NO. 4 in the sequence listing, respectively.

Preferably, the humanized anti-EGFRvIII antibody further comprises a human antibody heavy chain constant region and/or a human antibody light chain constant region. The heavy chain variable region and the light chain variable region form a full-length humanized antibody protein with the human heavy chain constant region and the human light chain constant region. The human antibody heavy chain constant region described therein is conventional in the art, and may comprise a constant region derived from a human constant region, which further comprises a heavy chain constant region of human IgG1, IgG2, IgG3, IgG4 or a variant thereof; the human antibody light chain constant region described therein is conventional in the art, and may comprise a constant region derived from a human constant region, which further comprises a light chain constant region of human κ and λ chains or a variant thereof.

The humanized anti-EGFRvIII antibody of the present invention is prepared by using any of various methods, including veneering and graft of complementarity determining regions (CDRs), graft of shortened CDRs, graft of specificity determining regions (SDRs), and Frankenstein assembly. Specificity determining regions (SDRs) are the residues within CDRs that interact directly with antigens. SDRs correspond to hypervariable residues. See Padlan et al. (1995) FASEB J. 9: 133-139).

The preparation of the representative humanized anti-EGFRvIII antibody of the present invention is described in example 1.

The human antibodies of the present invention are also a class of chimeric antibodies in a broad sense, in which the variable region residues responsible for antigen binding include complementarity determining regions derived from non-human species, shortened complementarity determining regions, or any other residues involved in antigen binding; the remaining variable region residues, such as framework region residues and constant region residues, are derived at least in part from human antibody sequences. A subset of the framework region residues and constant region residues of the humanized antibody may be derived from non-human sources. The variable regions of the humanized antibody are also described as a humanized light chain variable region and/or heavy chain variable region. Non-human species are generally species used for immunization with antigens, such as mice, rats, rabbits, non-human primates, or other non-human mammal species. Humanized antibodies are generally less immunogenic than traditional chimeric antibodies and show improved stability after administration to humans.

The humanized antibodies also include super-humanized antibodies, which are used in a method for preparing humanized antibodies. This method does not rely on human framework sequences as analysis points, but on the comparison of the canonical CDR structure types of non-human antibodies and CDR structure types of human antibodies, especially human antibodies encoded by human germline sequences, from which candidate human antibody sequences that can be used to obtain suitable human framework sequences are identified. For example, human residues can replace non-human residues in CDRs, where one or more changes have been introduced into the CDRs. One premise of veneering is that the immunogenicity of the murine antibody variable regions originates from the surface residues thereof, and the mobility of the residues and the accessibility of solvents are the basic conditions thereof to become an antigenic determinant. According to the analysis results of the existing antibody crystal structure data, the fidelity of the relative solvent accessibility distribution of human and murine antibody variable region residues at the sequence pairing position reaches 98%, indicating that the residues that induce the immune response among the heterologous species are caused by the remaining species-specific solvent accessible surface residues. Therefore, by replacing the murine-specific surface residues with human-specific surface residues, the surface profile of human antibodies can be simulated to escape the recognition of the human immune system and achieve the purpose of humanization. In short, veneering is based on the concept of reducing the potential immunogenic amino acid sequences in rodent or other non-human antibodies by reconstructing a solvent-accessible surface of the antibody with human amino acid sequences. See Padlan (1991) Mol. Immunol. 28: 489-980. Veneering is performed by identifying the external framework residues of the solvent accessible residues exposed on the surface in non-human antibodies (the residues are different from those at the same position in the framework regions of human antibodies), and replacing the identified residues with those amino acids occupying the same positions in human antibodies; that is to say, the surface residues of the veneered antibodies are mainly human sequences, while the residues wrapped inside are mainly original murine sequences. The graft of CDRs is performed by replacing one or more CDRs of a receptor antibody (e.g., a human antibody or other antibodies containing the desired framework residues) with the CDRs of a donor antibody (e.g., a non-human antibody). The receptor antibody may be selected based on the similarity of framework residues between a candidate receptor antibody and donor antibody. For instance, according to the Frankenstein method, human framework regions having substantial sequence homology with each framework region of the relevant non-human antibody are identified, and the CDRs of the non-human antibody are grafted onto the complex of these different human framework regions. The above-mentioned methods may be combined to produce the anti-EGFRvIII antibody having any desired sequence.

The EGFRvIII antibody of the present invention is preferably one or more of an antibody full-length protein, antigen-antibody binding domain protein fragment, bispecific antibody, multispecific antibody, single chain antibody (scFv), single domain antibody (sdAb) and signle-domain antibody, and a monoclonal antibody or polyclonal antibody prepared from the above-mentioned antibodies. The monoclonal antibody may be developed by various ways and techniques, including a hybridoma technique, phage display technique, single lymphocyte gene cloning technique, etc, wherein the monoclonal antibody is mainly prepared from wild-type or transgenic mice by hybridoma technique.

The antibody full-length protein is a conventional full-length antibody protein in the art, comprising a heavy chain variable region, light chain variable region, heavy chain constant region, and light chain constant region. The heavy chain variable region and light chain variable region of the protein form a complete human antibody full-length protein with a human heavy chain constant region and a human light chain constant region. Preferably, the full-length antibody protein is IgG1, IgG2, IgG3 or IgG4.

The single chain antibody is a conventional single chain antibody in the art, comprising a heavy chain variable region, light chain variable region and short peptide of 15 to 20 amino acids.

The antigen-antibody binding domain protein fragment is a conventional antigen-antibody binding domain protein fragment in the art, comprising a light chain variable region, light chain constant region, and Fd segment of the heavy chain constant region. Preferably, the antigen-antibody binding domain protein fragment is Fab and F(ab').

The single domain antibody is a conventional single domain antibody in the art, comprising a heavy chain variable region and heavy chain constant region.

The signle-domain antibody is a conventional signle-domain antibody in the art, only comprising a heavy chain variable region.

The EGFRvIII antibody of the present invention also includes a super-humanized antibody, diabody, etc.

The method for preparing the EGFRvIII antibody is a conventional preparation method in the art. The preparation method preferably comprises: obtaining the antibody by isolation from an expression transformant recombinantly expressing the protein or by artificial synthesis of sequences of the protein. The method for obtaining the antibody by isolation from an expression transformant recombinantly expressing the protein preferably comprises: cloning a nucleic acid molecule encoding the EGFRvIII antibody and having a point mutation into a recombinant vector, transforming the obtained recombinant vector into a transformant to obtain a recombinant expression transformant, and culturing the obtained recombinant expression transformant to obtain the EGFRvIII antibody by isolation and purification.

The present invention further provides a nucleic acid encoding the above-mentioned EGFRvIII antibody.

Preferably, the nucleotide sequence of the nucleic acid encoding the heavy chain variable region is as shown in SEQ ID No. 105, SEQ ID No. 107, SEQ ID No. No. 109, SEQ ID No. 111, SEQ ID No. 113, SEQ ID No. 115, SEQ ID 117, SEQ ID No. 119, SEQ ID No. 121, SEQ ID No. 123, SEQ ID No. 125, SEQ ID No. 127, SEQ ID No. 129, SEQ ID No. 185, SEQ ID No. 154, SEQ ID No. 155, SEQ ID No. 156, SEQ ID No. 157, SEQ ID No. 162, SEQ ID No. 163, SEQ ID No. 164, SEQ ID No. 165, SEQ ID No. 166, SEQ ID No. 167 or SEQ ID No. 168 in the sequence listing; and/or the nucleotide sequence of the nucleic acid encoding the light chain variable region is as shown in SEQ ID No. 106, SEQ ID No. 108, SEQ ID No. 110, SEQ ID No. 112, SEQ ID No. 114, SEQ ID No. 116, SEQ ID No. 118, SEQ ID No. 120, SEQ ID No. 122, SEQ ID No. 124, SEQ ID No. 126, SEQ ID No. 128, SEQ ID No. 130, SEQ ID No. 158, SEQ ID No. 159, SEQ ID No. 160, SEQ ID No. 161, SEQ ID No. 169, SEQ ID No. 170, SEQ ID No. 171, SEQ ID No. 172, SEQ ID No. 173 or SEQ ID No. 174 in the sequence listing.

More preferably, the nucleotide sequence of the nucleic acid encoding the heavy chain variable region is as shown in SEQ ID No. 105 in the sequence listing, and the nucleotide sequence of the nucleic acid encoding the light chain variable region is as shown in SEQ ID No. 106 in the sequence listing; the nucleotide sequence of the nucleic acid encoding the heavy chain variable region is as shown in SEQ ID No. 107 in the sequence listing, and the nucleotide sequence of the nucleic acid encoding the light chain variable region is as shown in SEQ ID No. 108 in the sequence listing; the nucleotide sequence of the nucleic acid encoding the heavy chain variable region is as shown in SEQ ID No. 109 in the sequence listing, and the nucleotide sequence of the nucleic acid encoding the light chain variable region is as shown in SEQ ID No. 110 in the sequence listing; the nucleotide sequence of the nucleic acid encoding the heavy chain variable region is as shown in SEQ ID No. 111 in the sequence listing, and the nucleotide sequence of the nucleic acid encoding the light chain variable region is as shown in SEQ ID No. 112 in the sequence listing; the nucleotide sequence of the nucleic acid encoding the heavy chain variable region is as shown in SEQ ID No. 113 in the sequence listing, and the nucleotide sequence of the nucleic acid encoding the light chain variable region is as shown in SEQ ID No. 114 in the sequence listing; the nucleotide sequence of the nucleic acid encoding the heavy chain variable region is as shown in SEQ ID No. 115 in the sequence listing, and the nucleotide sequence of the nucleic acid encoding the light chain variable region is as shown in SEQ ID No. 116 in the sequence listing; the nucleotide sequence of the nucleic acid encoding the heavy chain variable region is as shown in SEQ ID No. 117 in the sequence listing, and the nucleotide sequence of the nucleic acid encoding the light chain variable region is as shown in SEQ ID No. 118 in the sequence listing; the nucleotide sequence of the nucleic acid encoding the heavy chain variable region is as shown in SEQ ID No. 119 in the sequence listing, and the nucleotide sequence of the nucleic acid encoding the light chain variable region is as shown in SEQ ID No. 120 in the sequence listing; the nucleotide sequence of the nucleic acid encoding the heavy chain variable region is as shown in SEQ ID No. 121 in the sequence listing, and the nucleotide sequence of the nucleic acid encoding the light chain variable region is as shown in SEQ ID No. 122 in the sequence listing; the nucleotide sequence of the nucleic acid encoding the heavy chain variable region is as shown in SEQ ID No. 123 in the sequence listing, and the nucleotide sequence of the nucleic acid encoding the light chain variable region is as shown in SEQ ID No. 124 in the sequence listing; the nucleotide sequence of the nucleic acid encoding the heavy chain variable region is as shown in SEQ ID No. 125 in the sequence listing, and the nucleotide sequence of the nucleic acid encoding the light chain variable region is as shown in SEQ ID No126 in the sequence listing; the nucleotide sequence of the nucleic acid encoding the heavy chain variable region is as shown in SEQ ID No. 127 in the sequence listing, and the nucleotide sequence of the nucleic acid encoding the light chain variable region is as shown in SEQ ID No. 128 in the sequence listing; the nucleotide sequence of the nucleic acid encoding the heavy chain variable region is as shown in SEQ ID No. 129 in the sequence listing, and the nucleotide sequence of the nucleic acid encoding the light chain variable region is as shown in SEQ ID No. 130 in the sequence listing; the nucleotide sequence of the nucleic acid encoding the heavy chain variable region is as shown in SEQ ID No. 185 in the sequence listing, and the nucleotide sequence of the nucleic acid encoding the light chain variable region is as shown in SEQ ID No. 106 in the sequence listing; the nucleotide sequence of the nucleic acid encoding the heavy chain variable region is as shown in SEQ ID No. 154 in the sequence listing, and the nucleotide sequence of the nucleic acid encoding the light chain variable region is as shown in SEQ ID No. 158 in the sequence listing; the nucleotide sequence of the nucleic acid encoding the heavy chain variable region is as shown in SEQ ID No. 154 in the sequence listing, and the nucleotide sequence of the nucleic acid encoding the light chain variable region is as shown in SEQ ID No. 159 in the sequence listing; the nucleotide sequence of the nucleic acid encoding the heavy chain variable region is as shown in SEQ ID No. 154 in the sequence listing, and the nucleotide sequence of the nucleic acid encoding the light chain variable region is as shown in SEQ ID No. 160 in the sequence listing; the nucleotide sequence of the nucleic acid encoding the heavy chain variable region is as shown in SEQ ID No. 154 in the sequence listing, and the nucleotide sequence of the nucleic acid encoding the light chain variable region is as shown in SEQ ID No. 161 in the sequence listing; the nucleotide sequence of the nucleic acid encoding the heavy chain variable region is as shown in SEQ ID No. 155 in the sequence listing, and the nucleotide sequence of the nucleic acid encoding the light chain variable region is as shown in SEQ ID No. 158 in the sequence listing; the nucleotide sequence of the nucleic acid encoding the heavy chain variable region is as shown in SEQ ID No. 155 in the sequence listing, and the nucleotide sequence of the nucleic acid encoding the light chain variable region is as shown in SEQ ID No. 159 in the sequence listing; the nucleotide sequence of the nucleic acid encoding the heavy chain variable region is as shown in SEQ ID No. 155 in the sequence listing, and the nucleotide sequence of the nucleic acid encoding the light chain variable region is as shown in SEQ ID No. 160 in the sequence listing; the nucleotide sequence of the nucleic acid encoding the heavy chain variable region is as shown in SEQ ID No. 155 in the sequence listing, and the nucleotide sequence of the nucleic acid encoding the light chain variable region is as shown in SEQ ID No. 161 in the sequence listing; the nucleotide sequence of the nucleic acid encoding the heavy chain variable region is as shown in SEQ ID No. 156 in the sequence listing, and the nucleotide sequence of the nucleic acid encoding the light chain variable region is as shown in SEQ ID No. 158 in the sequence listing; the nucleotide sequence of the nucleic acid encoding the heavy chain variable region is as shown in SEQ ID No. 156 in the sequence listing, and the nucleotide sequence of the nucleic acid encoding the light chain variable region is as shown in SEQ ID No. 159 in the sequence listing; the nucleotide sequence of the nucleic acid encoding the heavy chain variable region is as shown in SEQ ID No. 156 in the sequence listing, and the nucleotide sequence of the nucleic acid encoding the light chain variable region is as shown in SEQ ID No. 160 in the sequence listing; the nucleotide sequence of the nucleic acid encoding the heavy chain variable region is as shown in SEQ ID No. 156 in the sequence listing, and the nucleotide sequence of the nucleic acid encoding the light chain variable region is as shown in SEQ ID No. 161 in the sequence listing; the nucleotide sequence of the nucleic acid encoding the heavy chain variable region is as shown in SEQ ID No. 157 in the sequence listing, and the nucleotide sequence of the nucleic acid encoding the light chain variable region is as shown in SEQ ID No. 158 in the sequence listing; the nucleotide sequence of the nucleic acid encoding the heavy chain variable region is as shown in SEQ ID No. 157 in the sequence listing, and the nucleotide sequence of the nucleic acid encoding the light chain variable region is as shown in SEQ ID No. 159 in the sequence listing; the nucleotide sequence of the nucleic acid encoding the heavy chain variable region is as shown in SEQ ID No. 157 in the sequence listing, and the nucleotide sequence of the nucleic acid encoding the light chain variable region is as shown in SEQ ID No. 160 in the sequence listing; the nucleotide sequence of the nucleic acid encoding the heavy chain variable region is as shown in SEQ ID No. 157 in the sequence listing, and the nucleotide sequence of the nucleic acid encoding the light chain variable region is as shown in SEQ ID No. 161 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 165 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 166 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 167 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 168 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 169 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 171 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 172 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 173 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 174 in the sequence listing; the nucleotide sequence of the nucleic acid encoding the heavy chain variable region is as shown in SEQ ID No. 162 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 162 in the sequence listing, and the nucleotide sequence of the nucleic acid encoding the light chain variable region is as shown in SEQ ID No. 171 in the sequence listing; the nucleotide sequence of the nucleic acid encoding the heavy chain variable region is as shown in SEQ ID No. 162 in the sequence listing, and the nucleotide sequence of the nucleic acid encoding the light chain variable region is as shown in SEQ ID No. 172 in the sequence listing; the nucleotide sequence of the nucleic acid encoding the heavy chain variable region is as shown in SEQ ID No. 162 in the sequence listing, and the nucleotide sequence of the nucleic acid encoding the light chain variable region is as shown in SEQ ID No. 173 in the sequence listing; the nucleotide sequence of the nucleic acid encoding the heavy chain variable region is as shown in SEQ ID No. 162 in the sequence listing, and the nucleotide sequence of the nucleic acid encoding the light chain variable region is as shown in SEQ ID No. 174 in the sequence listing; the nucleotide sequence of the nucleic acid encoding the heavy chain variable region is as shown in SEQ ID No. 163 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 163 in the sequence listing, and the nucleotide sequence of the nucleic acid encoding the light chain variable region is as shown in SEQ ID No. 171 in the sequence listing; the nucleotide sequence of the nucleic acid encoding the heavy chain variable region is as shown in SEQ ID No. 163 in the sequence listing, and the nucleotide sequence of the nucleic acid encoding the light chain variable region is as shown in SEQ ID No. 172 in the sequence listing; the nucleotide sequence of the nucleic acid encoding the heavy chain variable region is as shown in SEQ ID No. 163 in the sequence listing, and the nucleotide sequence of the nucleic acid encoding the light chain variable region is as shown in SEQ ID No. 173 in the sequence listing; the nucleotide sequence of the nucleic acid encoding the heavy chain variable region is as shown in SEQ ID No. 163 in the sequence listing, and the nucleotide sequence of the nucleic acid encoding the light chain variable region is as shown in SEQ ID No. 174 in the sequence listing; the nucleotide sequence of the nucleic acid encoding the heavy chain variable region is as shown in SEQ ID No. 166 in the sequence listing, and the nucleotide sequence of the nucleic acid encoding the light chain variable region is as shown in SEQ ID No. 169 in the sequence listing; the nucleotide sequence of the nucleic acid encoding the heavy chain variable region is as shown in SEQ ID No. 167 in the sequence listing, and the nucleotide sequence of the nucleic acid encoding the light chain variable region is as shown in SEQ ID No. 169 in the sequence listing; or the nucleotide sequence of the nucleic acid encoding the heavy chain variable region is as shown in SEQ ID No. 168 in the sequence listing, and the nucleotide sequence of the nucleic acid encoding the light chain variable region is as shown in SEQ ID No. 169 in the sequence listing.

In summary, the numbers of the above-mentioned nucleotide sequences are as shown in tables 1-2 and 2.

**Table 1-2. Numbers of nucleotide sequences of EGFRvIII antibody**

| Antibody number | Heavy chain variable region SEQ ID NO. | Light chain variable region SEQ ID NO. |
|---|---|---|
| c75G7C6 | 105 | 106 |
| c75G7C6-1 | 185 | 106 |
| c75G7C6-2 | 187 | 106 |
| h75G7C6-1 | 154 | 158 |
| h75G7C6-2 | 154 | 159 |
| h75G7C6-3 | 154 | 160 |
| h75G7C6-4 | 154 | 161 |
| h75G7C6-5 | 155 | 158 |
| h75G7C6-6 | 155 | 159 |
| h75G7C6-7 | 155 | 160 |
| h75G7C6-8 | 155 | 161 |
| h75G7C6-9 | 156 | 158 |
| h75G7C6-10 | 156 | 159 |
| h75G7C6-11 | 156 | 160 |
| h75G7C6-12 | 156 | 161 |
| h75G7C6-13 | 157 | 158 |
| h75G7C6-14 | 157 | 159 |
| h75G7C6-15 | 157 | 160 |
| h75G7C6-16 | 157 | 161 |
| c63A10A7 | 107 | 108 |
| h63A10A7-17 | 164 | 170 |
| h63A10A7-18 | 165 | 170 |
| h63A10A7-19 | 166 | 170 |
| h63A10A7-20 | 167 | 170 |
| h63A10A7-21 | 168 | 170 |
| h63A10A7-22 | 164 | 169 |
| h63A10A7-23 | 164 | 171 |
| h63A10A7-24 | 164 | 172 |
| h63A10A7-25 | 164 | 173 |
| h63A10A7-26 | 164 | 174 |
| h63A10A7-27 | 162 | 170 |
| h63A10A7-28 | 162 | 171 |
| h63A10A7-29 | 162 | 172 |
| h63A10A7-30 | 162 | 173 |
| h63A10A7-31 | 162 | 174 |
| h63A10A7-32 | 163 | 170 |
| h63A10A7-33 | 163 | 171 |
| h63A10A7-34 | 163 | 172 |
| h63A10A7-35 | 163 | 173 |
| h63A10A7-36 | 163 | 174 |
| h63A10A7-37 | 166 | 169 |
| h63A10A7-38 | 167 | 169 |
| h63A10A7-39 | 168 | 169 |

The numbers in table 1-2 are the sequence numbers in the sequence listing. For example, the nucleotide sequence of the heavy chain protein variable region of h75G7C6-1 is SEQ ID No. 154, and the nucleotide sequence of the light chain protein variable region of h75G7C6-1 is SEQ ID No. 158.

The method for preparing the nucleic acid is a conventional preparation method in the art, and preferably, the method comprises the following steps: using a gene cloning technique to obtain a nucleic acid molecule encoding the above-mentioned humanized anti-EGFRvIII antibody, or using an artificial complete sequence synthesis method to obtain a nucleic acid molecule encoding the above-mentioned humanized anti-EGFRvIII antibody.

Those of skill in the art is aware that the nucleic acid encoding the above-mentioned humanized anti-EGFRvIII antibody can be appropriately introduced a substitution, deletion, alteration, insertion or addition to provide a homolog of a polynucleotide. The homologue of a polynucleotide in the present invention can be prepared by performing substitution, deletion or addition on one or more nucleotides of the nucleic acid encoding the humanized anti-EGFRvIII antibody within the range of maintaining antibody activity.

**Table 2 Numbers of nucleotide sequences of EGFRvIII antibody**

| Clone number | Heavy chain protein variable region | Light chain protein variable region |
|---|---|---|
| 75G7C6 | 105 | 106 |
| 63A10A7 | 107 | 108 |
| 64F1F8 | 109 | 110 |
| 7E5-2A9 | 111 | 112 |
| 43H6A1 | 113 | 114 |
| 46C4A6 | 115 | 116 |
| 49G12G5 | 117 | 118 |
| 51G7C2 | 119 | 120 |
| 53D8H4 | 121 | 122 |
| 54D2G10 | 123 | 124 |
| 56F10G2 | 125 | 126 |
| 59G3F12 | 127 | 128 |
| 64B11F2 | 129 | 130 |

The numbers in table 2 are the sequence numbers in the sequence listing. For example, the nucleotide sequence encoding the heavy chain protein variable region of 75G7C6 is SEQ ID No. 105.

The complementarity determining regions (CDRs) of the present invention are the residues of antibody variable regions involved in antigen binding. Several numbering systems for identifying CDRs are commonly used, including for example, Kabat definition, Chothia definition, and AbM definition. To sum up, the Kabat definition is based on sequence variability; the Chothia definition is based on the location of structural loop regions; and the AbM definition is a compromise between the Kabat method and the Chothia method. According to the Kabat, Chothia, or AbM algorithm, the light chain variable region has three CDR regions, where CDR1 is located at amino acids at positions 24-34 (CDR1-L), CDR2 located at amino acids at positions 50-56 (CDR2-L), and CDR3 located at amino acids at positions 89-97 (CDR3-L). Due to the change in length of the variable regions, in different centers or different subgroups, there may be 1-6 amino acids at position 27 and there may also be 1-6 amino acids at position 95; and the amino acids are numbered by adding English letters based on the original numbering, such as 27A, 27B, 95A and 95B. According to the Kabat definition, the CDRs of the heavy chain variable region are defined by residues at positions 31 and 35B (CDR1-H), positions 50 and 65 (CDR2-H), and positions 95 and 102 (CDR3-H) (according to Kabat numbering). According to the Chothia definition, the CDRs of the heavy chain variable region are defined by residues at positions 26 and 32 (CDR1-H), positions 52 and 56 (CDR2-H), and positions 95 and 102 (CDR3-H) (according to Chothia numbering). According to the AbM definition, the CDRs of the heavy chain variable region are defined by residues at positions 26 and 35B (CDR1-H), positions 50 and 58 (CDR2-H), and positions 95 and 102 (CDR3-H) (according to Kabat numbering). Similar to the light chain variable region, there may also be multiple amino acids at positions 35, 52, 82, and 100, numbered A, B, C, etc. See Martin et al., (1989) Proc. Nat 1. Acad. Sci. USA 86: 9268-9272; Martin et al., (1991) Methods Enzymol. 203: 121-153; Pedersen et al., (1992) Immunomethods 1: 126; Protein Structure Prediction, Oxford University Press, Oxford, pp. 141-172. In the present invention, the CDRs of the following murine antibodies are numbered by Chothia numbering system, and those of the subsequent humanized antibodies are numbered by Kabat numbering system.

The nucleotide sequence encoding CDR1 in the heavy chain protein variable region of 75G7C6 is position 76 to position 96 of SEQ ID No. 105 in the sequence listing;
the nucleotide sequence encoding CDR2 in the heavy chain protein variable region of 75G7C6 is position 154 to position 171 of SEQ ID No. 105 in the sequence listing;
the nucleotide sequence encoding CDR3 in the heavy chain protein variable region of 75G7C6 is position 295 to position 339 of SEQ ID No. 105 in the sequence listing;
the nucleotide sequence encoding CDR1 in the light chain protein variable region of 75G7C6 is position 70 to position 105 of SEQ ID No. 106 in the sequence listing;
the nucleotide sequence encoding CDR2 in the light chain protein variable region of 75G7C6 is position 151 to position 171 of SEQ ID No. 106 in the sequence listing;
the nucleotide sequence encoding CDR3 in the light chain protein variable region of 75G7C6 is position 268 to position 294 of SEQ ID No. 106 in the sequence listing;
the nucleotide sequence encoding CDR1 in the heavy chain protein variable region of 63A10A7 is position 76 to position 96 of SEQ ID No. 107 in the sequence listing;
the nucleotide sequence encoding CDR2 in the heavy chain protein variable region of 63A10A7 is position 154 to position 171 of SEQ ID No. 107 in the sequence listing;
the nucleotide sequence encoding CDR2 in the heavy chain protein variable region of c75G7C6-1 are as shown in position 154 to position 171 of SEQ ID No. 185 in the sequence listing;
the nucleotide sequence encoding CDR2 in the heavy chain protein variable region of c75G7C6-2 are as shown in position 154 to position 171 of SEQ ID No. 187 in the sequence listing.
the nucleotide sequence encoding CDR3 in the heavy chain protein variable region of 63A10A7 is position 295 to position 318 of SEQ ID No. 107 in the sequence listing;
the nucleotide sequence encoding CDR1 in the light chain protein variable region of 63A10A7 is position 70 to position 102 of SEQ ID No. 108 in the sequence listing;
the nucleotide sequence encoding CDR2 in the light chain protein variable region of 63A10A7 is position 148 to position 168 of SEQ ID No. 108 in the sequence listing;
the nucleotide sequence encoding CDR3 in the light chain protein variable region of 63A10A7 is position 265 to position 291 of SEQ ID No. 108 in the sequence listing.

The nucleotide sequence encoding CDR1 in the heavy chain protein variable region of 64F1F8 is position 76 to position 96 of SEQ ID No. 109 in the sequence listing;
the nucleotide sequence encoding CDR2 in the heavy chain protein variable region of 64F1F8 is position 154 to position 171 of SEQ ID No. 109 in the sequence listing;
the nucleotide sequence encoding CDR3 in the heavy chain protein variable region of 64F1F8 is position 295 to position 318 of SEQ ID No. 109 in the sequence listing;
the nucleotide sequence encoding CDR1 in the light chain protein variable region of 64F1F8 is position 70 to position 99 of SEQ ID No. 110 in the sequence listing;
the nucleotide sequence encoding CDR2 in the light chain protein variable region of 64F1F8 is position 145 to position 165 of SEQ ID No. 110 in the sequence listing;
the nucleotide sequence encoding CDR3 in the light chain protein variable region of 64F1F8 is position 262 to position 288 of SEQ ID No. 110 in the sequence listing.

The nucleotide sequence encoding CDR1 in the heavy chain protein variable region of 7E5-2A9 is position 76 to position 96 of SEQ ID No. 111 in the sequence listing;
the nucleotide sequence encoding CDR2 in the heavy chain protein variable region of 7E5-2A9 is position 154 to position 171 of SEQ ID No. 111 in the sequence listing;
the nucleotide sequence encoding CDR3 in the heavy chain protein variable region of 7E5-2A9 is position 295 to position 309 of SEQ ID No. 111 in the sequence listing;
the nucleotide sequence encoding CDR1 in the light chain protein variable region of 7E5-2A9 is position 70 to position 120 of SEQ ID No. 112 in the sequence listing;
the nucleotide sequence encoding CDR2 in the light chain protein variable region of 7E5-2A9 is position 166 to position 186 of SEQ ID No. 112 in the sequence listing;
the nucleotide sequence encoding CDR3 in the light chain protein variable region of 7E5-2A9 is position 283 to position 309 of SEQ ID No. 112 in the sequence listing.

The nucleotide sequence encoding CDR1 in the heavy chain protein variable region of 43H6A1 is position 127 to position 150 of SEQ ID No. 113 in the sequence listing;
the nucleotide sequence encoding CDR2 in the heavy chain protein variable region of 43H6A1 is position 208 to position 222 of SEQ ID No. 113 in the sequence listing;
the nucleotide sequence encoding CDR3 in the heavy chain protein variable region of 43H6A1 is position 346 to position 366 of SEQ ID No. 113 in the sequence listing;
the nucleotide sequence encoding CDR1 in the light chain protein variable region of 43H6A1 is position 70 to position 102 of SEQ ID No. 114 in the sequence listing;
the nucleotide sequence encoding CDR2 in the light chain protein variable region of 43H6A1 is position 148 to position 168 of SEQ ID No. 114 in the sequence listing;
the nucleotide sequence encoding CDR3 in the light chain protein variable region of 43H6A1 is position 265 to position 291 of SEQ ID No. 114 in the sequence listing;
The nucleotide sequence encoding CDR1 in the heavy chain protein variable region of 46C4A6 is position 76 to position 96 of SEQ ID No. 115 in the sequence listing;
The nucleotide sequence encoding CDR2 in the heavy chain protein variable region of 46C4A6 is position 154 to position 171 of SEQ ID No. 115 in the sequence listing;
The nucleotide sequence encoding CDR3 in the heavy chain protein variable region of 46C4A6 is position 295 to position 306 of SEQ ID No. 115 in the sequence listing;
the nucleotide sequence encoding CDR1 in the light chain protein variable region of 46C4A6 is position 70 to position 117 of SEQ ID No. 116 in the sequence listing;
the nucleotide sequence encoding CDR2 in the light chain protein variable region of 46C4A6 is position 163 to position 183 of SEQ ID No. 116 in the sequence listing;
the nucleotide sequence encoding CDR3 in the light chain protein variable region of 46C4A6 is position 280 to position 306 of SEQ ID No. 116 in the sequence listing;
the nucleotide sequence encoding CDR1 in the heavy chain protein variable region of 49G12G5 is position 76 to position 96 of SEQ ID No. 117 in the sequence listing;
the nucleotide sequence encoding CDR2 in the heavy chain protein variable region of 49G12G5 is position 154 to position 171 of SEQ ID No. 117 in the sequence listing;
the nucleotide sequence encoding CDR3 in the heavy chain protein variable region of 49G12G5 is position 295 to position 318 of SEQ ID No. 117 in the sequence listing;
the nucleotide sequence encoding CDR1 in the light chain protein variable region of 49G12G5 is position 70 to position 105 of SEQ ID No. 118 in the sequence listing;
the nucleotide sequence encoding CDR2 in the light chain protein variable region of 49G12G5 is position 151 to position 171 of SEQ ID No. 118 in the sequence listing;
the nucleotide sequence encoding CDR3 in the light chain protein variable region of 49G12G5 is position 268 to position 294 of SEQ ID No. 118 in the sequence listing.

The nucleotide sequence encoding CDR1 in the heavy chain protein variable region of 51G7C2 is position 76 to position 96 of SEQ ID No. 119 in the sequence listing;
the nucleotide sequence encoding CDR2 in the heavy chain protein variable region of 51G7C2 is position 154 to position 171 of SEQ ID No. 119 in the sequence listing;
the nucleotide sequence encoding CDR3 in the heavy chain protein variable region of 51G7C2 is position 295 to position 318 of SEQ ID No. 119 in the sequence listing;
the nucleotide sequence encoding CDR1 in the light chain protein variable region of 51G7C2 is position 70 to position 105 of SEQ ID No. 120 in the sequence listing;
the nucleotide sequence encoding CDR2 in the light chain protein variable region of 51G7C2 is position 151 to position 171 of SEQ ID No. 120 in the sequence listing;
the nucleotide sequence encoding CDR3 in the light chain protein variable region of 51G7C2 is position 268 to position 294 of SEQ ID No. 120 in the sequence listing.
The nucleotide sequence encoding CDR1 in the heavy chain protein variable region of 53D8H4 is position 76 to position 96 of SEQ ID No. 121 in the sequence listing;
the nucleotide sequence encoding CDR2 in the heavy chain protein variable region of 53D8H4 is position 154 to position 171 of SEQ ID No. 121 in the sequence listing;
the nucleotide sequence encoding CDR3 in the heavy chain protein variable region of 53D8H4 is position 295 to position 318 of SEQ ID No. 121 in the sequence listing;
the nucleotide sequence encoding CDR1 in the light chain protein variable region of 53D8H4 is position 70 to position 102 of SEQ ID No. 122 in the sequence listing;
the nucleotide sequence encoding CDR2 in the light chain protein variable region of 53D8H4 is position 148 to position 168 of SEQ ID No. 122 in the sequence listing;
the nucleotide sequence encoding CDR3 in the light chain protein variable region of 53D8H4 is position 265 to position 291 of SEQ ID No. 122 in the sequence listing.

The nucleotide sequence encoding CDR1 in the heavy chain protein variable region of 54D2G10 is position 76 to position 96 of SEQ ID No. 123 in the sequence listing;
the nucleotide sequence encoding CDR2 in the heavy chain protein variable region of 54D2G10 is position 154 to position 171 of SEQ ID No. 123 in the sequence listing;
the nucleotide sequence encoding CDR3 in the heavy chain protein variable region of 54D2G10 is position 295 to position 315 of SEQ ID No. 123 in the sequence listing;
the nucleotide sequence encoding CDR1 in the light chain protein variable region of 54D2G10 is position 70 to position 99 of SEQ ID No. 124 in the sequence listing;
the nucleotide sequence encoding CDR2 in the light chain protein variable region of 54D2G10 is position 145 to position 165 of SEQ ID No. 124 in the sequence listing;
the nucleotide sequence encoding CDR3 in the light chain protein variable region of 54D2G10 is position 262 to position 288 of SEQ ID No. 124 in the sequence listing.

The nucleotide sequence encoding CDR1 in the heavy chain protein variable region of 56F10G2 is position 76 to position 96 of SEQ ID No. 125 in the sequence listing;
the nucleotide sequence encoding CDR2 in the heavy chain protein variable region of 56F10G2 is position 154 to position 171 of SEQ ID No. 125 in the sequence listing;
The nucleotide sequence encoding CDR3 in the heavy chain protein variable region of 56F10G2 is position 295 to position 315 of SEQ ID No. 125 in the sequence listing;
the nucleotide sequence encoding CDR1 in the light chain protein variable region of 56F10G2 is position 70 to position 99 of SEQ ID No. 126 in the sequence listing;
the nucleotide sequence encoding CDR2 in the light chain protein variable region of 56F10G2 is position 145 to position 165 of SEQ ID No. 126 in the sequence listing;
the nucleotide sequence encoding CDR3 in the light chain protein variable region of 56F10G2 is position 262 to position 288 of SEQ ID No. 126 in the sequence listing.
The nucleotide sequence encoding CDR1 in the heavy chain protein variable region of 59G3F12 is position 76 to position 99 of SEQ ID No. 127 in the sequence listing;
the nucleotide sequence encoding CDR2 in the heavy chain protein variable region of 59G3F12 is position 157 to position 171 of SEQ ID No. 127 in the sequence listing;
the nucleotide sequence encoding CDR3 in the heavy chain protein variable region of 59G3F12 is position 295 to position 318 of SEQ ID No. 127 in the sequence listing;
the nucleotide sequence encoding CDR1 in the light chain protein variable region of 59G3F12 is position 70 to position 99 of SEQ ID No. 128 in the sequence listing;
the nucleotide sequence encoding CDR2 in the light chain protein variable region of 59G3F12 is position 145 to position 165 of SEQ ID No. 128 in the sequence listing;
the nucleotide sequence encoding CDR3 in the light chain protein variable region of 59G3F12 is position 262 to position 288 of SEQ ID No. 128 in the sequence listing.

The nucleotide sequence encoding CDR1 in the heavy chain protein variable region of 64B11F2 is position 76 to position 96 of SEQ ID No. 129 in the sequence listing;
the nucleotide sequence encoding CDR2 in the heavy chain protein variable region of 64B11F2 is position 154 to position 171 of SEQ ID No. 129 in the sequence listing;
the nucleotide sequence encoding CDR3 in the heavy chain protein variable region of 64B11F2 is position 295 to position 315 of SEQ ID No. 129 in the sequence listing;
the nucleotide sequence encoding CDR1 in the light chain protein variable region of 64B11F2 is position 70 to position 102 of SEQ ID No. 130 in the sequence listing;
the nucleotide sequence encoding CDR2 in the light chain protein variable region of 64B11F2 is position 148 to position 168 of SEQ ID No. 130 in the sequence listing;
the nucleotide sequence encoding CDR3 in the light chain protein variable region of 64B11F2 is position 265 to position 291 of SEQ ID No. 130 in the sequence listing.

The method for preparing the nucleic acid is a conventional preparation method in the art, and preferably, the method comprises the following steps: using a gene cloning technique to obtain a nucleic acid molecule encoding the above-mentioned protein, or using an artificial complete sequence synthesis method to obtain a nucleic acid molecule encoding the above-mentioned protein.

Those of skill in the art is aware that the base sequence encoding the amino acid sequence of the above-mentioned protein can be appropriately introduced a substitution, deletion, alteration, insertion or addition to provide a homolog of a polynucleotide. The homolog of a polynucleotide in the present invention can be prepared by performing substitution, deletion or addition on one or more bases of the gene encoding the sequence of protein within the range of maintaining antibody activity.

The present invention further provides a recombinant expression vector containing the nucleic acid,
wherein the recombinant expression vector can be obtained by conventional methods in the art, that is, it is constructed by linking the nucleic acid molecule of the present invention to various expression vectors. The expression vectors are various conventional vectors in the art, as long as it can carry the aforementioned nucleic acid molecule. The vector preferably comprises: various plasmids, cosmids, phage or viral vectors, etc.

The present invention further provides a recombinant expression transformant comprising the above-mentioned recombinant expression vector,
wherein the method for preparing the recombinant expression transformant is a conventional preparation method in the art, and preferably: it is obtained by transforming the above-mentioned recombinant expression vector into host cells. The host cells are various conventional host cells in the art, as long as it meets the following requirements: the above-mentioned recombinant expression vector is stably replicated by itself, and the carried nucleic acid can be efficiently expressed. Preferably, the host cells are *E. coli* TG1 or BL21 cells (expressing a single chain antibody or Fab antibody), or CHO-K1 cells (expressing a full-length IgG antibody). By transforming the aforementioned recombinant expression plasmid into the host cells, a preferred recombinant expression transformant of the present invention can be obtained, wherein the transformation method is a conventional transformation method in the art, preferably a chemical transformation method, a heat shock method or an electric transformation method.

The present invention further provides a method for preparing an EGFRvIII antibody, comprising the following steps: culturing the above-mentioned recombinant expression transformant, and obtaining the EGFRvIII antibody from the culture.

The present invention further provides an immunoconjugate, comprising the above-mentioned EGFRvIII antibody covalently attached to a cytotoxic agent.

Preferably, in the immunoconjugate, the aforementioned 1 equivalent of the protein is linked to y equivalent of the cytotoxic agent via x equivalent of the linker, which has the structure of formula 1,

Ab-(L)ₓ-(D)_{y} formula 1

where Ab is the above-mentioned EGFRvIII antibody; L is a linker; D is a cytotoxic agent; the x represents a conventional degree of cross linking in the art, with x being a natural number, preferably 1 to 20; y is a natural number greater than, preferably an integer of 1 to 20; x and y are each independently and preferably 2w, with w being an integer of 1 to 5, further preferably 3 and 4; and the ratio of x and y is preferably 1 : 1.

The L is a conventional linker (or cross-linking agent or coupling agent) in the art. The L contains 2 functional groups, i.e., a group that reacts with an antibody and a group that reacts with a drug (such as aldehyde or ketone).

The drug is coupled to the aforementioned EGFRvIII antibody via a linker molecule. The L is released after entering cells, including but not limited to the following functional groups: active esters, carbonates, carbamates, imidophosphates, oximes, hydrazones, acetals, orthoesters, aminos, small peptide segments or nucleotide fragments, and for another example, maleimidocaproyl (MC), maleimidocaproyl-L-valine-L-citrulline-p-aminobenzyl alcohol (MC-VC-PAB) or 4-(N-maleimidomethyl)cyclohexane-1-carboxylic acid succinimide ester (SMCC).

Preferably, the L mainly comprises the structure of formula 2, which is the remaining part in L after the leaving group leaves:

(CO-Alk¹-Sp¹-Ar-Sp²-Alk²-C(Z¹)=Q-Sp) formula 2

where Alk¹ and Alk² are independently bonds or branched or unbranched (C₁-C₁₀) alkylene chains; Sp¹ is -S-, -O-, -CONH-, -NHCO-, -NR'-, -N(CH₂CH₂)₂N-, or -X-Ar'-Y-(CH₂)ₙ-Z, where X, Y and Z are independently bonds, -NR'-, -S- or -O-, provided that when n = 0, at least one of Y and Z must be a bond, and Ar' is 1,2-, 1,3- or 1,4-phenylene optionally substituted with 1, 2 or 3 groups selected from (C₁-C₅)alkyl, (Ci-C₄)alkoxy, (C₁-C₄)thioalkoxy, halogen, nitro, -COOR', -CONHR', -(CH₂)ₙCOOR', S(CH₂)ₙCOOR', -O(CH₂)ₙCONHR' or -S(CH₂)ₙCONHR', with n being an integer of 0 to 5, provided that when Alk¹ is a bond, Sp¹ is a bond; R' is a branched or unbranched (C₁-C₅) chain optionally substituted with 1 or 2 groups selected from -OH, (Ci-C₄)alkoxy, (C₁-C₄)thioalkoxy, halogen, nitro, (C₁-C₃)dialkylamino, or (Ci-C₃)trialkylammonium-A, where A is a pharmaceutically acceptable anion to accomplish the formation of the salt; Ar is 1,2-, 1,3- or 1,4-phenylene optionally substituted with 1, 2 or 3 groups selected from (C₁-C₆)alkyl, (C₁-C₅)alkoxy, (Ci-C₄)thioalkoxy, halogen, nitro, -COOR', -CONHR', -O(CH₂)ₙCOOR', -S(CH₂)ₙCOOR', -O(CH₂)ₙCONHR" or -S(CH₂)ₙCONHR', where n and R' are as defined above, or Ar is 1,2-, 1,3-, 1,4-, 1,5-, 1,6-, 1,7-, 1,8-, 2,3-, 2,6- or 2,7-naphthylene, wherein naphthylene or phenothiazine each is optionally substituted with 1, 2, 3 or 4 groups selected from (C₁-C₆)alkyl, (C₁-C₅)alkoxy, (C₁-C₄)thioalkoxy, halogen, nitro, -COOR', -CONHR', -O(CH₂)ₙCOOR', -S(CH2)nCOOR', or -S(CH2)nCONHR', where n and R' are as defined above, provided that when Ar is phenothiazine, Sp¹ is a bond linked to nitrogen only;
Sp² is a bond, -S- or -O-, provided that when Alk² is a bond, Sp² is a bond;
Z¹ is H, (C₁-C₅)alkyl, or phenyl optionally substituted with 1, 2, or 3 groups selected from (C₁-C₅)alkyl, (C₁-C₅)alkoxy, (C₁-C₄)thioalkoxy, halogen, nitro, -COOR', - CONHR', -O(CH₂)ₙCOOR', -S(CH2)nCOOR', -O(CH₂)ₙCONHR' or - S(CH₂)ₙCONHR', where n and R' are as defined above;
Sp is a linear or branched divalent or trivalent (C₁-C₁₈) group, divalent or trivalent aryl or heteroaryl group, divalent or trivalent (C₃-C₁₈) cycloalkyl or heterocycloalkyl group, divalent or trivalent aryl or heteroaryl-aryl(C₁-C₁₈) group, divalent or trivalent cycloalkyl or heterocycloalkyl-alkyl(C₁-C₁₈) group, or divalent or trivalent (C₂-C₁₈) unsaturated alkyl group, wherein the heteroaryl is preferably furyl, thienyl, N-methylpyrrolyl, pyridyl, N-methylimidazolyl, oxazolyl, pyrimidinyl, quinolinyl, isoquinolinyl, N-methylcarbazolyl, aminocoumarin, or phenazinyl, and wherein if Sp is a trivalent group, the Sp may also be optionally substituted with a lower (Ci-C₅)dialkylamino group, lower (C₁-C₅)alkoxy group, hydroxyl, or lower (Ci-C₅)alkylthio group; and Q is =NHNCO-, =NHNCS-, =NHNCONH-, =NHNCSNH- or =NHO-.

Preferably, Alk¹ is a branched or unbranched (C₁-C₅)alkylene chain, and Sp¹ is a bond, -S-, -O-, -CONH-, -NHCO- or -NR', where R' is as defined above, provided that when Alk¹ is a bond, Sp¹ is a bond;
Ar is 1,2-, 1,3- or 1,4-phenylene optionally substituted with 1, 2 or 3 groups selected from (C₁-C₆)alkyl, (C₁-C₅)alkoxy, (C₁-C₄)thioalkoxy, halogen, nitro, -COOR', - CONHR', -O(CH₂)ₙCOOR', -S(CH2)nCOOR', -O(CH₂)ₙCONHR' or - S(CH₂)ₙCONHR', where n and R' are as defined above, or Ar is 1,2-, 1,3-, 1,4-, 1,5-, 1,6-, 1,7-, 1,8-, 2,3-, 2,6- or 2,7-naphthylene each optionally substituted with 1, 2, 3 or 4 groups selected from (C₁-C₆)alkyl, (C₁-C₅)alkoxy, (C₁-C₄)thioalkoxy, halogen, nitro, -COOR', -CONHR', -O(CH₂)ₙCOOR', -S(CH2)nCOOR', -O(CH₂)ₙCONHR' or - S(CH₂)ₙCONHR'.
Z¹ is (C₁-C₅)alkyl, or phenyl optionally substituted with 1, 2, or 3 groups selected from (C₁-C₅)alkyl, (C₁-C₄)alkoxy, (C₁-C₄)thioalkoxy, halogen, nitro, -COOR', -CONHR', - O(CH₂)ₙCOOR', -S(CH2)nCOOR', -O(CH₂)ₙCONHR' or -S(CH2)nCONHR'; Alk² and Sp² are both bonds; and Sp and Q are only as defined above. The aforementioned bonds have the meaning of covalent bonds.

The L is maleimidocaproyl (MC).

The D may be a conventional cytotoxic agent in the art, preferably selected from a cytotoxin, chemotherapeutic agent, radioisotope, therapeutic nucleic acid, immunomodulator, anti-angiogenic agent, anti-proliferative and pro-apoptotic agent or cytolytic enzyme.

The cytotoxin is a conventional cytotoxin in the art, and generally refers to an active agent that inhibits or prevents cell functions and/or causes cell destruction. Preferably, the cytotoxin is selected from an antibiotic, inhibitor of tubulin polymerization, alkylating agent, protein synthesis inhibitor, protein kinase inhibitor, phosphatase inhibitor, topoisomerase inhibitor, protein kinase, phosphatase, topoisomerase or cyclin. More preferably, the cytotoxin is selected from doxorubicin, daunorubicin, idarubicin, aclarubicin, zorubicin, mitoxantrone, epirubicin, carubicin, nogalamycin, menogaril, pirarubicin, valrubicin, cytarabine, gemcitabine, trifluridine, ancitabine, enocitabine, azacitidine, doxifluridine, pentostatin, broxuridine, capecitabine, cladribine, decitabine, floxuridine, fludarabine, gougerotin, puromycin, tegafur, tiazofurin, adriamycin, cisplatin, carboplatin, cyclophosphamide, dacarbazine, vinblastine, vincristine, bleomycin, nitrogen mustard, prednisone, methyhydrazine, methotrexate, fluorouracil, etoposide, taxol, taxol analog, platinum (such as cisplatin and carboplatin), mitomycin, thiotepa, taxane, daunorubicin, actinomycin, anthramycin, azaserine, tamoxifen, dolastatin, auristatin and a derivative thereof, hemiasterlin, esperamicin or maytansine compounds, and further more preferably selected from monomethylauristatin E (MMAE), monomethylauristatin F (MMAF) or N2'-deacetyl-N2'-3-mercapto-1 oxopropyl-maytansine (DM1). Most preferably, the cytotoxin is monomethylauristatin F (MMAF) or monomethylauristatin E (MMAE).

The chemotherapeutic agent is a conventional chemotherapeutic agent in the art, preferably selected from an alkylating agent, alkyl sulfonate chemotherapeutic agent, aziridine chemotherapeutic agent, vinylamide and methylmelamine chemotherapeutic agents, nitrogen mustard, nitrourea chemotherapeutic agent, antibiotic, antimetabolite, folic acid chemotherapeutic agent, purine analog, pyrimidine analog, androgen, anti-adrenaline, folic acid supplement, maytansinol, polysaccharide complex, taxane, platinum analog or retinoid, or a pharmaceutically acceptable salt, acid and derivative thereof.

The alkylating agent is a conventional alkylating agent in the art, preferably selected from thiotepa or cyclophosphamide. The alkyl sulfonate chemotherapeutic agent is a conventional alkyl sulfonate chemotherapeutic agent in the art, preferably selected from busulfan, improsulfan or piposulfan. The aziridine chemotherapeutic agent is a conventional aziridine chemotherapeutic agent in the art, preferably selected from aziridines such as carboquone, meturedepa or uredepa. The vinylamide and methylmelamine chemotherapeutic agents are conventional vinylamide and methylmelamine chemotherapeutic agents in the art, preferably selected from hexamethylmelamine, triethylenemelamine, triethylenephosphoramide, triethylenethiophosphoramide or trimethylolmelamine. The nitrogen mustard is a conventional nitrogen mustard in the art, preferably selected from chlorambucil, chlornaphazine, estramustine, ifosfamide, nitrogen mustard, nitromin hydrochloride, phenylalanine mustard, novembichin, phenesterine, prednimustine, trofosfamide or uramustine. The nitrourea chemotherapeutic agent is a conventional nitrourea chemotherapeutic agent in the art, preferably selected from carmustine, chlorozotocin, fotemustine, lomustine, nimustine or ranimustine. The antibiotic is a conventional antibiotic in the art, preferably selected from clarithromycin, actinomycin, anthramycin, azaserine, bleomycin, actinomycin c, calicheamicin, carubicin, carminomycin, carcinophylin, chromomycin, dactinomycin, daunorubicin, detorubicin, 6-diazo-5-oxo-L-norleucine, doxorubicin, esorubicin, idarubicin, narbomycin, mitomycin, mycophenolic acid, nogalamycin, olivomycin, peplomycin, porfiromycin, puromycin, triferricdoxorubicin, rodorubicin, streptonigrin, streptozocin, tuberculocidin, ubenimex, zinostatin or zorubicin. The antimetabolite is a conventional antimetabolite in the art, preferably selected from methotrexate or 5-fluorouracil (5-FU). The folic acid chemotherapeutic agent is a conventional folic acid chemotherapeutic agent in the art, preferably selected from denopterin, pteropterin or trimetrexate. The purine analog is a conventional purine analog in the art, preferably selected from fludarabine, 6-mercaptopurine, thiamiprine or thioguanine. The pyrimidine analog is a conventional pyrimidine analog in the art, preferably selected from ancitabine, azacitidine, 6-azauridine, carmofur, cytarabine, dideoxyuridine, doxifluridine, enocitabine, floxuridine or 5-EU. The androgen is a conventional androgen in the art, preferably selected from calusterone, methyl androstanone propionate, epithioandrostanol, mepitiostane or testolactone. The anti-adrenaline is a conventional anti-adrenaline in the art, preferably selected from aminoglutethimide, mitotane or trilostane. The folic acid supplement is a conventional folic acid supplement in the art, preferably selected from folinic acid, aceglatone, aldophosphamide glycoside, aminolevulinic acid, amsacrine, atrimustine, bisantrene, edatrexate, defosfamide, demecolcine, diaziquone, eflornithine, elliptinium acetate, epothilone, etoglucid, gallium nitrate, hydroxyurea, lentinan or lonidamine. The maytansinol is a conventional maytansinol in the art, preferably selected from maytansine, ansamitocin, mitoguazone, mitoxantrone, mopidamol, nitracrine, pentostatin, phenamet, pirarubicin, losoxantrone, podophyllic acid, 2-ethyl hydrazide or procarbazine. The polysaccharide complex is a conventional polysaccharide complex in the art, preferably selected from razoxane, rhizomycin, sizofiran, spirogermanium, tenuazonic acid, triaziquone, 2,2',2"-trichlorotriethylamine, trichothecene mycotoxins, urethane, vindesine, dacarbazine, mannomustine, dibromannitol, dibromodulcitol, pipobroman, gacytosine, cytarabine, cyclophosphamide or thiotepa. More preferably, the polysaccharide complex is selected from T-2 toxin, verrucarine A, bacillosporin A or anguidine. The taxane is a conventional taxane in the art, preferably selected from taxol, unhydrogenated castor oil, an albumin engineered nanoparticle preparation (American Pharmaceutical Partners, Schaumberg, Illinois) of taxol, docetaxel, chlorambucil, gemcitabine, 6-thioguanine, mercaptopurine or methotrexate. The platinum analog is a conventional platinum analog in the art, preferably selected from cisplatin, carboplatin, vinblastine, etoposide, ifosfamide, mitoxantrone, vincristine, novantrone, teniposide, edatrexate, daunorubicin, aminopterin, capecitabine ibandronate, CPT-11, topoisomerase inhibitor RFS 2000 or difluoromethylornithine. The retinoid is a retinoid in the art, preferably retinoic acid.

The radioisotope is a conventional radioisotope in the art, and preferably it directly binds to the above-mentioned EGFRvIII antibody, or binds to the above-mentioned EGFRvIII antibody through a chelating agent. More preferably, it directly binds to the cysteine residue of the EGFRvIII antibody. Preferably, the radioisotope is selected from an α-emitter, β-emitter and Auger electron suitable for radiotherapy, and a positron emitter or γ-emitter suitable for diagnosis. More preferably, the radioisotope is selected from ¹⁸fluorine , ⁶⁴copper, ⁶⁵copper, ⁶⁷gallium, ⁶⁸gallium, ⁷⁷bromine, ^{80m}bromine, ⁹⁵ruthenium, ⁹⁷ruthenium, ¹⁰³ruthenium, ¹⁰⁵ruthenium, ⁹⁹technetium, ¹⁰⁷mercury, ²⁰³mercury, ¹²³iodine, ¹²⁴iodine, ¹²⁵iodine, ¹²⁶iodine, ¹³¹iodine, ¹³³iodine, ¹¹¹indium, ¹¹³indium, ^{99m}rhenium, ¹⁰⁵rhenium, ¹⁰¹rhenium , ¹⁸⁶rhenium, ¹⁸⁸rhenium, ^{121m}tellurium, ⁹⁹technetium, ^{122m}tellurium, ¹²⁵tellurium, ¹⁶⁵thulium, ¹⁶⁷thulium, ¹⁶⁸thulium, ⁹⁰yttrium, ²¹³bismuth, ²¹³lead or ²²⁵actinium, or nitrides or oxides derived therefrom.

The therapeutic nucleic acid is a conventional nucleic acid in the art, and preferably is a gene encoding an immunomodulator, anti-angiogenic agent, anti-proliferative agent or pro-apoptotic agent. The therapeutic agent includes the therapeutic agent described herein, a derivative thereof, and a pharmaceutically acceptable salt, acid and derivative of the therapeutic agent.

The immunomodulator is a conventional immunomodulator in the art, which is an agent that triggers an immune response, including a humoral immune response (e.g., the production of antigen-specific antibodies) and a cell-mediated immune response (e.g., lymphocyte proliferation). Preferably, the immunomodulator is selected from a cytokine, growth factor, hormone, anti-hormonal drug, immunosuppressive agent or corticosteroid. The cytokine is a conventional cytokine in the art, preferably selected from xanthine, interleukin or interferon. The growth factor is a conventional growth factor in the art, preferably selected from TNF, CSF, GM-CSF or G-CSF. The hormone is a conventional hormone in the art, preferably selected from estrogen, androgen or progestogen. More preferably, the estrogen is diethylstilbestrol or estradiol. More preferably, the androgen is testosterone or fluoxymesterone. More preferably, the progestogen is megestrol acetate or medroxyprogesterone acetate. The corticosteroid is a conventional corticosteroid in the art, preferably selected from prednisone, dexamethasone or hydrocortisone. The anti-hormonal drug is a conventional anti-hormonal drug in the art, which can block the effects of hormones on tumors, inhibit the production of cytokines, down-regulate the expression of autoantigens, or mask the immunosuppressive agent of MHC antigens. Preferably, the anti-hormonal drug is selected from an anti-estrogen drug, anti-androgen drug or anti-adrenaline drug. More preferably, the anti-estrogen drug is selected from tamoxifen, raloxifene, aromatase inhibitory 4 (5)-imidazoles, 4-hydroxy tamoxifen, trioxifene or toremifene. The anti-androgen drug is selected from flutamide, nilutamide, bicalutamide, leuprolide or goserelin. The immunosuppressive agent is a conventional immunosuppressive agent in the art, preferably selected from 2-amino-6 aryl-5 substituted pyrimidines, azathioprine, cyclophosphamide, bromocriptine, danazol, dapsone, glutaraldehyde, anti-idiotypic antibodies against MHC antigens and MHC fragments, cyclosporin A, steroids for example glucocorticosteroids, streptokinase, TGFb, rapamycin, T cell receptors, T cell receptor fragments, cytokine receptor antagonists or T cell receptor antibodies. More preferably, the cytokine receptor antagonists are selected from an anti-interferon antibody, anti-IL10 antibody, anti-TNFa antibody or anti-IL2 antibody.

The anti-angiogenic agent is a conventional anti-angiogenic agent in the art, preferably selected from a farnesyl transferase inhibitor, COX-2 inhibitor, VEGF inhibitor, bFGF inhibitor, steroid sulfatase inhibitor, interleukin-24, thrombospondin, metallospondin protein, type I interferon, interleukin 12, protamine, angiostatin, laminin, endostatin or prolactin fragment. More preferably, the anti-angiogenic agent is 2-methoxyestradiol disulfamate (2-MeOE2bisMATE).

The anti-proliferative and pro-apoptotic agent is a conventional anti-proliferative and pro-apoptotic agent in the art, preferably selected from a PPAR-γ activator, retinoid, triterpenoid compound, EGF receptor inhibitor, telomere terminal transferase inhibitor, iron chelator, apoptin, Bcl-2 and Bcl-X (L) inhibitors, TNF-α/FAS ligand/TNF-related apoptosis-inducing ligand and a signal transduction activator thereof, or PI3K-Akt survival pathway signaling inhibitor. The PPAR-γ activator is a conventional PPAR-γ activator in the art, and preferably is cyclopentenone prostaglandins (cyPGs). The triterpenoid compound is a conventional triterpenoid compound in the art, preferably selected from cycloartane, lupinane, ursane, oleanane, friedelane, dammarane, cucurbitacine, limonin analog or other triterpenoid compounds. The EGF receptor inhibitor is a conventional EGF receptor inhibitor in the art, preferably selected from HER4, rapamycin or 1,25-dihydroxycholecalciferol (vitamin D). The iron chelator is a conventional iron chelator in the art, and preferably is 3-aminopyridine-2-carbaldehyde thiosemicarbazone. The apoptin is a conventional apoptin in the art, and preferably is a viral protein 3-VP3 of the chicken anemia virus. The PI3K-Akt survival pathway signaling inhibitor is a conventional PI3K-Akt survival pathway signaling inhibitor in the art, and preferably is UCN-01 or geldanamycin.

The cytolytic enzyme is a conventional cytolytic enzyme in the art, and preferably is RNase.

Preferably, in the present invention, x = y = n in formula 1; the D is a tubulin synthetase inhibitor - monomethylauristatin F (MMAF), and the linker L is maleimidocaproyl (MC), and the structure of the immunoconjugate is as shown in formula 3-1 or 3-2, 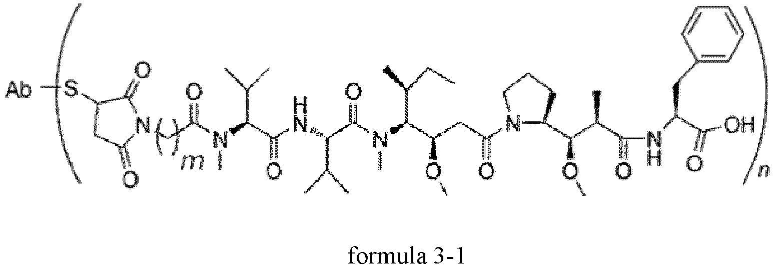 where mAb is the above-mentioned EGFRvIII antibody; where n is a natural number, preferably an integer of 1 to 20, and more preferably 2w, with w being an integer of 1 to 5, further preferably 3 and 4; 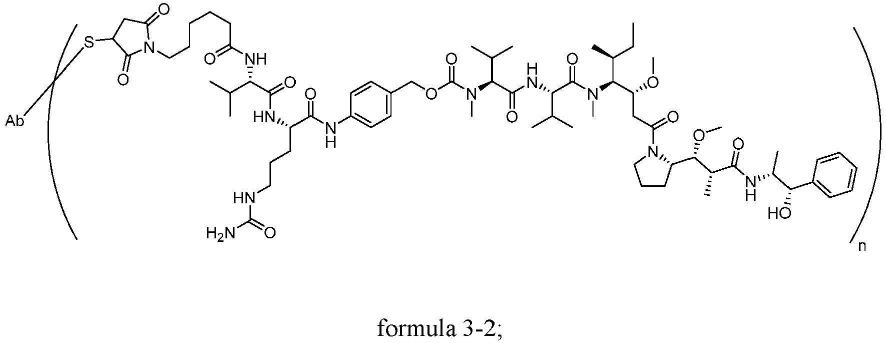 in the formula 3-2, m is 1 to 10, preferably m is 5, and L is maleimidocaproyl-L-valine-L-citrulline-p-aminobenzyl alcohol; D is monomethylauristatin E (MMAE); where n is a natural number, preferably an integer of 1 to 20, and more preferably 2w, with w being an integer of 1 to 5, further preferably 3 and 4.

The method for preparing the immunoconjugate is conventional in the art, and preferably the preparation method described in Doronina, 2006, Bioconjugate Chem. 17, 114-124 is used. Preferably, by the preparation method, an immunoconjugate with a minimum low coupling fraction (LCF) of less than 10% is produced.

In a preferred embodiment of the present invention, the preparation method comprises the following steps: the above-mentioned EGFRvIII antibody is dialyzed with a sodium borate buffer at pH 6.5 to 8.5, and then tris(2-carboxyethyl)phosphine (TCEP) is added thereto, with the molar ratio of the TCEP to the above-mentioned EGFRvIII antibody being 2 to 10; reduction is performed for 2 to 4 hours at room temperature, excess TCEPs are removed through a G25 desalting filler; and a certain proportion of MC-MMAF (with drug/antibody ratio being 5-20) is added and reacted for 4 hours. Then cysteine is added to neutralize excess drugs, and excess small molecules are removed through G25. A purified antibody-drug conjugate is afforded (for the coupling method, see Doronina, 2006, Bioconjugate Chem. 17,114-124).

The immunoconjugate may exist in any physical form known in the art, and preferably is a clear solution.

The present invention further provides a pharmaceutical composition, comprising the above-mentioned EGFRvIII antibody or immunoconjugate, and a pharmaceutically acceptable carrier.

The pharmaceutically acceptable carrier is a conventional carrier in the art, which may be any suitable physiologically or pharmaceutically acceptable pharmaceutical auxiliary material. The pharmaceutical auxiliary material is a conventional pharmaceutical auxiliary material in the art, preferably comprising a pharmaceutically acceptable excipient, filler, diluent, etc. More preferably, the pharmaceutical composition comprises 0.01% to 99.99% of the above-mentioned EGFRvIII antibody or the above-mentioned immunoconjugate, and 0.01% to 99.99% of the pharmaceutically acceptable carrier, wherein the percentage is the mass percentage accounting for the pharmaceutical composition.

The administration route of the pharmaceutical composition of the present invention is preferably parenteral administration, injection administration or oral administration. The injection administration preferably includes intravenous injection, intramuscular injection, intraperitoneal injection, intradermal injection, or subcutaneous injection. The pharmaceutical composition is in various conventional dosage forms in the art, preferably in the form of solid, semisolid or liquid, namely, the pharmaceutical composition may be an aqueous solution, non-aqueous solution or suspension, and more preferably in the form of a tablet, capsule, granule, injection, infusion, etc. More preferably, the pharmaceutical composition is administered intravascularly, subcutaneously, intraperitoneally, or intramuscularly. Preferably, the pharmaceutical composition may also be administered as an aerosol or crude spray, i.e., administered nasally; or administered intrathecally, intramedullarily, or intraventricularly. More preferably, the pharmaceutical composition may further be administered transdermally, percutaneously, topically, enterally, intravaginally, sublingually or rectally.

The administration dose level of the pharmaceutical composition of the present invention can be adjusted according to the amount of the composition achieving the desired diagnosis or treatment result. The administration schedule may also be a single injection or multiple injections, or be adjusted. The selected dosage level and schedule are reasonably adjusted depending on various factors including the activity and stability (i.e., half-life) of the pharmaceutical composition, formulation, administration route, combination with other drugs or therapies, diseases or conditions to be detected and/or treated, and health status and previous medical history of a subject to be treated.

The therapeutically effective dose for the pharmaceutical composition of the present invention can be estimated initially in cell culture experiments or animal models such as rodents, rabbits, dogs, pigs, and/or primates. Animal models may also be used to determine the appropriate concentration range and route of administration. The models may then be used to determine useful doses and routes for administration in humans. In general, the determination and adjustments of the effective amount or dosage administered and the evaluation of when and how such adjustments are made are known to those of skill in the art.

For combination therapies, the above-mentioned EGFRvIII antibody, the above-mentioned immunoconjugate, and/or additional therapeutic or diagnostic agents can each be used as a single agent within any time range suitable for performing the intended treatment or diagnosis. Therefore, these single agents can be administered substantially simultaneously (i.e., administered as a single formulation or administered within a few minutes or hours) or administered continuously in sequence. For example, these single agents may be administered within one year, or within 10, 8, 6, 4, or 2 months, or within 4, 3, 2, or 1 week, or within 5, 4, 3, 2, or 1 day.

For additional guidance on formulation, dosage, administration schedule, and measurable treatment results, see Berkow et al., (2000) The Merck Manual of Medical Information and Merck & Co. Inc., Whitehouse Station, New Jersey; Ebadi (1998) CRC Desk Reference of Clinical Pharmacology and other works.

The present invention further provides the use of the above-mentioned antibody, the above-mentioned conjugate or the above-mentioned pharmaceutical composition in the preparation of an anti-tumor drug.

The present invention further provides a method for detecting a cell overexpressing an EGFRvIII protein, comprising the following steps: contacting the above-mentioned EGFRvIII antibody with a sample to be detected *in vitro,* then detecting the binding of the EGFRvIII antibody to the sample to be detected.

The present invention further provides a composition for detecting a cell overexpressing an EGFRvIII protein, comprising the above-mentioned EGFRvIII antibody as an active ingredient.

The present invention further provides the use of the above-mentioned antibody, immunoconjugate or pharmaceutical composition in the preparation of a drug for preventing or treating a disease related to abnormal expression or function of the EGFRvIII; preferably, the disease related to abnormal expression or function of the EGFRvIII is a tumor, wherein the tumor is preferably bladder cancer, brain cancer, head and neck cancer, pancreatic cancer, lung cancer, breast cancer, ovarian cancer, colon cancer, prostate cancer, or renal cancer.

On the basis of meeting common knowledge in the art, the above-mentioned various preferred conditions can be combined in any form, such that various preferred examples of the present invention are obtained.

Reagents and raw materials used in the present invention are all commercially available.

The positive effect of the present invention lies in that the protein of the present invention is an EGFRvIII antibody, which has a high affinity with an EGFRvIII protein and can bind to the extracellular region of the EGFRvIII protein receptor at the protein level and the cellular level. The antibody cross-linking drug obtained by coupling the EGFRvIII antibody with MC-MMAF and other small-molecule compounds can effectively perform a cytotoxic killing effect on EGFRvIIIpositive cells. The EGFRvIII antibody brings the small-molecule toxin (e.g., MMAF) into cells via endocytosis, and degrades and releases small-molecule compounds in the cells, thereby playing the role of cytotoxicity. Therefore, the EGFRvIII antibody can be used for preparing an antibody cross-linking drug and effectively killing tumor cells, or can be used in the preparation of a drug for treating tumors.

### Brief description of the drawings

Figure 1 shows the detection results of CHO-K1 cells transfected with a human EGFRvIII protein by FACS.
Figure 2 shows the detection results of CHO-K1 cells transfected with a human EGFR protein by FACS.
Figure 3 shows the detection results of U87MG cells transfected with a human EGFRvIII protein by FACS.
Figure 4 shows the detection results of 293F cells transfected with a human EGFRvIII protein by FACS.
Figures 5A and 5B show the serum antibody titer in mice immunized with the EGFRvIII protein detected by ELISA.
Figure 6 shows the binding reaction of the EGFRvIII antibody with a human EGFRvIII-hFc protein detected by ELISA.
Figure 7A shows the binding reaction of the EGFRvIII antibody with U87MG-hEGFRvIII detected by FACS; Figure 7B shows the binding reaction of the EGFRvIII antibody with A431 detected by FACS; Figure 7C shows the binding reaction of the EGFRvIII antibody with U87MG detected by FACS; Figure 7D shows the binding reaction of the EGFRvIII antibody with HEB detected by FACS.
Figure 8A shows a cell killing effect of an EGFRvIII antibody-MMAF antibody conjugate on U87MG-EGFRvIII; Figure 8B shows a cell killing effect of an EGFRvIII antibody-MMAF antibody conjugate on A431; Figure 8C shows a cell killing effect of an EGFRvIII antibody-MMAF antibody conjugate on U87MG; Figure 8D shows a cell killing effect of an EGFRvIII antibody-MMAF antibody conjugate on HEB.

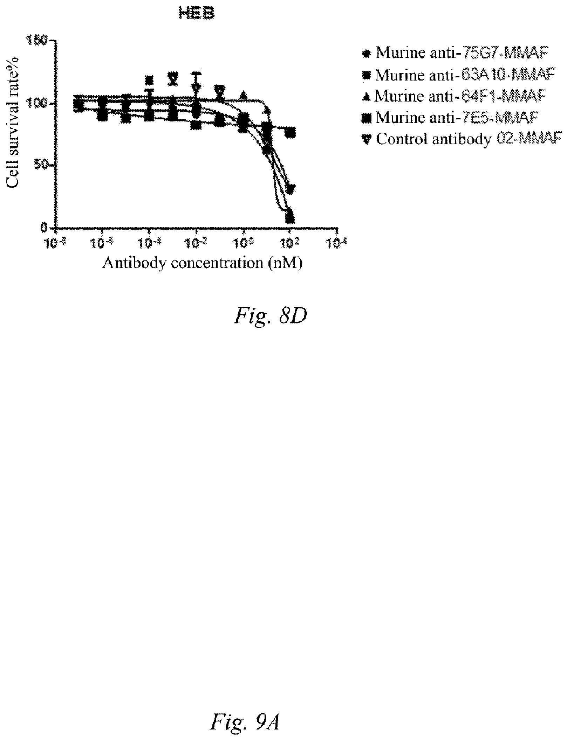

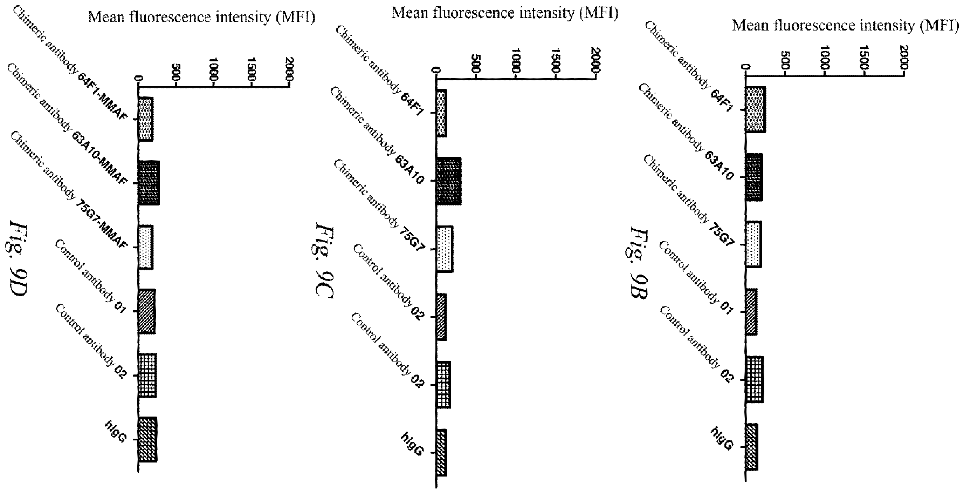

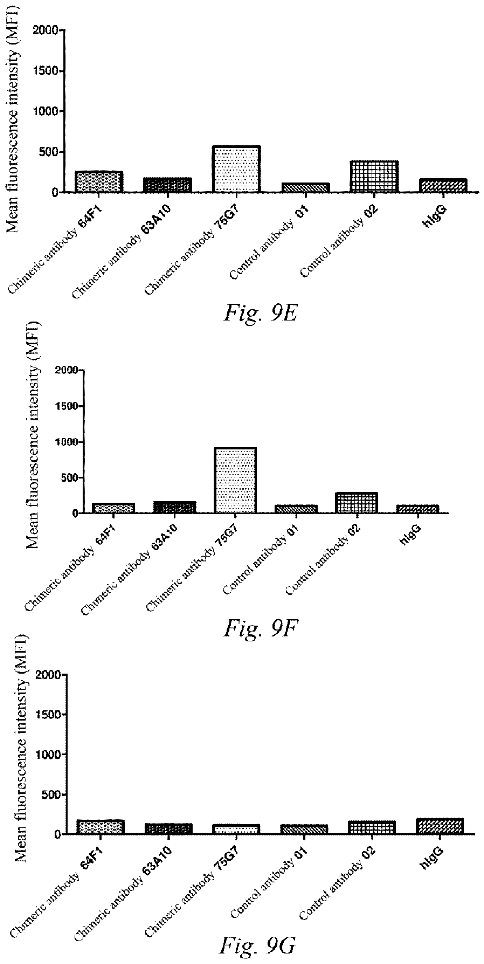

Figure 9A shows the binding reaction of the EGFRvIII antibody with a human normal tissue HFF-1 cell line detected by FACS; Figure 9B shows the binding reaction of the EGFRvIII antibody with a human normal tissue HFL-I cell line detected by FACS; Figure 9C shows the binding reaction of the EGFRvIII antibody with a human normal tissue QSG-7701 cell line detected by FACS; Figure 9D shows the binding reaction of the EGFRvIII antibody with a human normal tissue HEEC cell line detected by FACS; Figure 9E shows the binding reaction of the EGFRvIII antibody with a human normal tissue HEB cell line detected by FACS; Figure 9F shows the binding reaction of the EGFRvIII antibody with a human normal tissue WPMY-1 cell line detected by FACS; Figure 9G shows the binding reaction of the EGFRvIII antibody with a human normal tissue MCF-10A cell line detected by FACS.

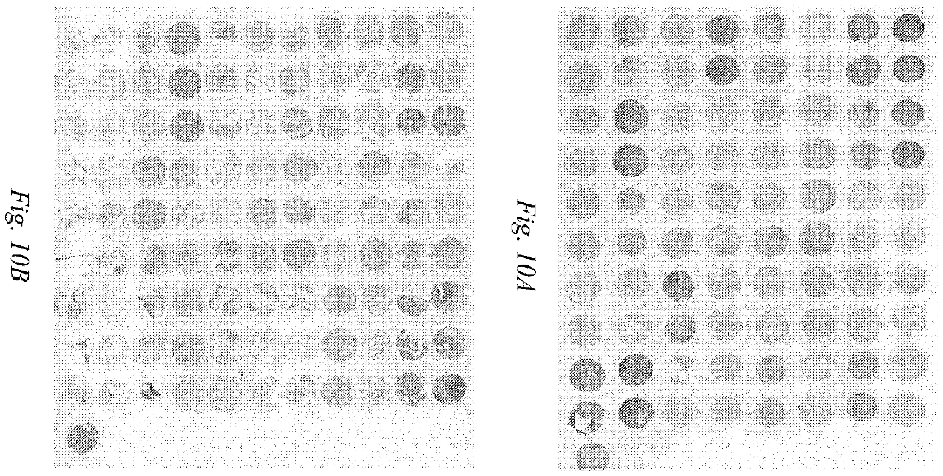

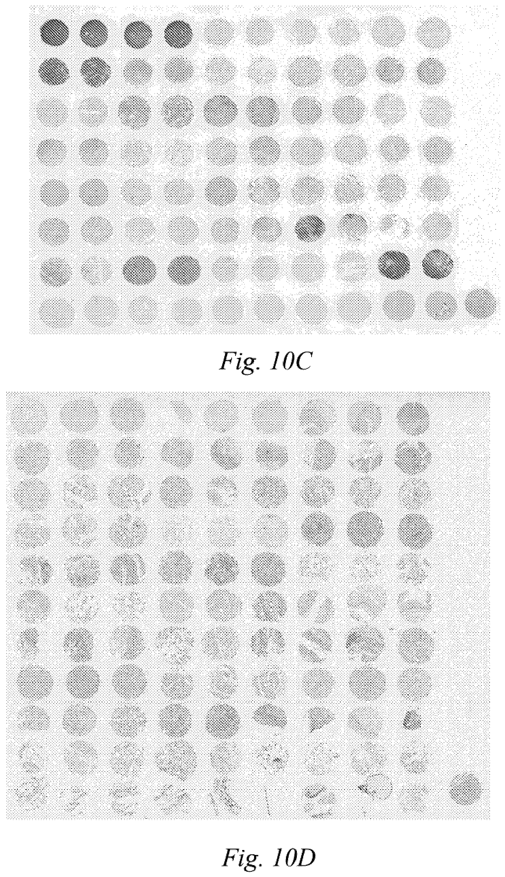

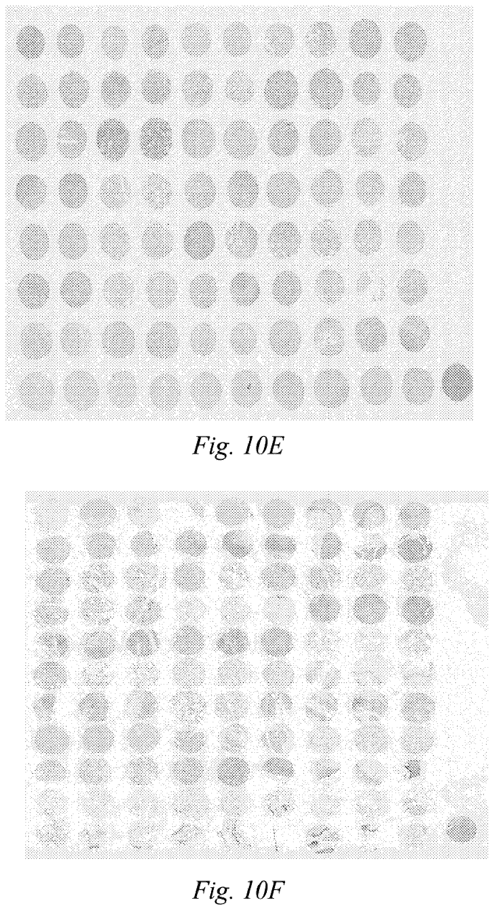

Figure 10A shows the results of the immunohistochemical staining of a human glioma tissue chip with the EGFR antibody (murine anti-80E11); Figure 10B shows the results of the immunohistochemical staining of a human normal tissue chip with the EGFR antibody (murine anti-80E11); Figure 10C shows the results of the immunohistochemical staining of a human glioma tissue chip with the EGFRvIII antibody (murine anti-63A10); Figure 10D shows the results of the immunohistochemical staining of a human normal tissue chip with the EGFRvIII antibody (murine anti-63A10); Figure 10E shows the results of the immunohistochemical staining of a human glioma tissue chip with the EGFRvIII antibody (murine anti-75G7); Figure 10F shows the results of the immunohistochemical staining of a human normal tissue chip with the EGFRvIII antibody (murine anti-75G7).
Figure 11 shows the binding of a chimeric antibody to an EGFRvIII protein detected by an enzyme-linked immunosorbent assay (ELISA).
Figure 12A shows the binding reaction of the EGFRvIII chimeric antibody with U87MG-hEGFRvIII detected by FACS; Figure 12B shows the binding reaction of the EGFRvIII chimeric antibody with A431 detected by FACS; Figure 12C shows the binding reaction of the EGFRvIII chimeric antibody with U87MG detected by FACS; Figure 12D shows the binding reaction of the EGFRvIII chimeric antibody with HEB detected by FACS.
Figure 13A shows a cell killing effect of an EGFRvIII antibody-MMAF antibody conjugate on U87MG-EGFRvIII; Figure 13B shows a cell killing effect of an EGFRvIII antibody-MMAF antibody conjugate on A431; Figure 13C shows a cell killing effect of an EGFRvIII antibody-MMAF antibody conjugate on U87MG; Figure 13D shows a cell killing effect of an EGFRvIII antibody-MMAF antibody conjugate on HEB.
Figure 14A shows the change in tumor volume of U87MG-EGFRvIII xenograft tumor-bearing mice; Figure 14B shows the change in body weight of U87MG-EGFRvIII xenograft tumor-bearing mice.
Figure 15 shows the ELISA binding activity identification of the 75G7C6 antibody, in which NG of the CDR2 in the heavy chain variable region of the antibody is mutated.
Figure 16 shows the ELISA binding activity identification of the 75G7C6 antibody, in which NG of the CDR3 in the heavy chain variable region of the antibody is mutated.
Figure 17A shows the binding activity in CHOK-EGFRvIII cells of the 75G7C6 antibody, in which NG of the CDR2 in the heavy chain variable region of the antibody is mutated, detected by FACS; Figure 17B shows the binding activity in CHOK-EGFR cells of the 75G7C6 antibody, in which NG of the CDR2 in the heavy chain variable region is mutated, detected by FACS.
Figure 18A shows the binding activity in CHOK-EGFRvIII cells of the 75G7C6 antibody, in which DG of the CDR3 in the heavy chain variable region is mutated to SG, EG or DA, detected by FACS; Figure 18B shows the binding activity in CHOK-EGFR cells of the 75G7C6 antibody, in which DG of the CDR3 in the heavy chain variable region is mutated to SG, EG or DA, detected by FACS;
Figure 19 shows the sequence comparison of the heavy chain variable region h75G7C6.VH of the humanized anti-EGFRvIII antibody h75G7C6 and a variant thereof and the chimeric antibody c75G7C6.VH and human germline VH exon IGHV1-46^{∗}01/JH6b, with CDRs in the box.
Figure 20 shows the sequence comparison of the light chain variable region h75G7C6.VL of the humanized anti-EGFRvIII antibody h75G7C6 and a variant thereof and the chimeric antibody c75G7C6.VL and human germline VL exon IGKV3-11^{∗}01/JK4, with CDRs in the box.
Figure 21 shows the sequence comparison of the heavy chain variable region h63A10A7.VH of the humanized anti-EGFRvIII antibody h63A10A7 and a variant thereof and the chimeric antibody c63A10A7.VH and human germline VH exon IGHV1-46^{∗}01/JH4, with CDRs in the box.
Figure 22 shows the sequence comparison of the light chain variable region h63A10A7.VL of the humanized anti-EGFRvIII antibody h63A10A7 and a variant thereof and the chimeric antibody c63A10A7.VL and human germline VL exon IGKVI-39^{∗}01/JK2, with CDRs in the box.
Figures 23A-C show the binding reaction of the humanized antibody h75G7C6 variant with the human EGFRvIII-hFc protein detected by ELISA.
Figures 24A-E show the binding reaction of the humanized antibody h63A10A7 variant with the human EGFRvIII-hFc protein detected by ELISA.
Figures 25A-C show the binding reaction of the humanized antibody h75G7C6 variant with U87MG-EGFRvIII cells detected by FACS.
Figures 26A-E show the binding reaction of the humanized antibody h63A10A7 variant with U87MG-EGFRvIII cells detected by FACS.
Figure 27 shows the binding of the humanized h75G7C6 variant to A431 tumor cells expressing EGFR on the surface and normal human primary hepatocytes detected by FACS.
Figures 28A-B show the binding of the humanized h63A10A7 variant to A431 tumor cells expressing EGFR on the surface and normal human primary hepatocytes detected by FACS.

### Detailed description of the preferred embodiment

The following examples further illustrate the present invention, but the present invention is not limited thereto. In experimental methods in the following examples where no specific conditions are indicated, choices can be made according to conventional methods and conditions or commodity instructions.

The following examples further illustrate the present invention, but the present invention is not limited thereto. In experimental methods in the following examples where no specific conditions are indicated, choices can be made according to conventional methods and conditions or commodity instructions.

The room temperature described in the examples is a conventional room temperature in the art, generally is 10°C to 30°C.

### Example 1 Preparation of EGFRvIII antibody

### (I) Preparation of immunogen A

The nucleotide sequences containing the amino acid sequences (NCBI: NP_005219.2, with deletion of amino acids at positions 30-297) encoding the extracellular region of the human EGFRvIIIprotein are cloned into a pCpC vector (purchased from Invitrogen, V044-50) with a human IgG Fc fragment (hFc); and plasmids are prepared according to established standard molecular biology methods, and for specific methods, please see Sambrook, J., Fritsch, E. F., and Maniatis, T. (1989). Molecular Cloning: A Laboratory Manual, Second Edition (Plainview, New York: Cold Spring Harbor Laboratory Press). HEK293 cells (purchased from Invitrogen) are subjected to transient transfection (PEI, Polysciences) and an expanded culture is performed using FreeStyle ™293 (Invitrogen) at 37°C. After 4 days, the cell culture broth is collected, and the cell components are removed by centrifugation to obtain a culture supernatant containing the extracellular region of the EGFRvIII protein. The culture supernatant is loaded onto a protein A affinity chromatography column (Mabselect Sure, purchased from GE Healthcare), and the change in ultraviolet absorption values (A280 nm) is monitored with an ultraviolet (UV) detector. After loading, the protein A affinity chromatography column is washed with PBS phosphate buffer (pH 7.2) until the UV absorption value returns back to the baseline, and then eluted with 0.1 M glycine hydrochloric acid (pH 2.5) to collect the hFc-tagged EGFRvIII protein (EGFRvIII-hFc) eluted from the protein A affinity chromatography column, which is dialyzed with the PBS phosphate buffer (pH 7.2) in the refrigerator at 4°C overnight. The dialyzed protein is sterile filtered through 0.22 microns, and then packaged and stored at -80°C to obtain a purified immunogen A.

### (II) Preparation of immunogen B

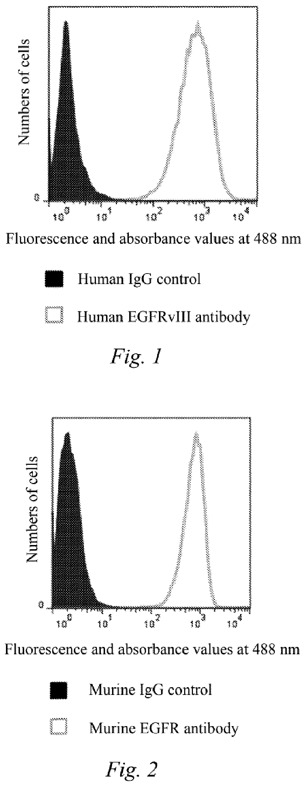

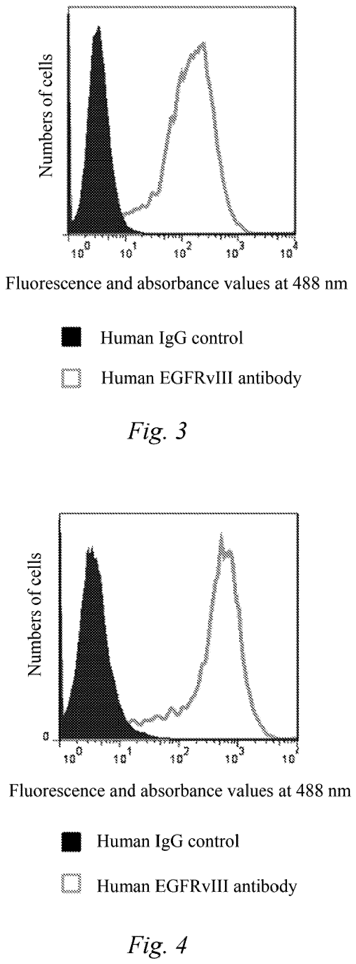

The nucleotide sequence encoding the full-length amino acid sequence of the human EGFRvIII (NCBI: NP_005219.2, with deletion of amino acids at positions 30-297) and the nucleotide sequence encoding the full-length amino acid sequence of the human EGFR (NCBI: NP_005219.2) are cloned into a pIRES vector (purchased from Clontech), and plasmids are prepared. The HEK293 cell line, U87MG cell line and CHO-K1 cell line (all purchased from Invitrogen) are subjected to plasmid transfection (PEI, purchased from Polysciences), and then selectively cultured for 2 weeks in a DMEM medium containing 0.5 µg/ml of 10% (w/w) fetal bovine serum. The culture is subcloned in a 96-well culture plate by using a limiting dilution method, and cultured at 37°C, 5% (v/v) CO₂; after about 2 weeks, a part of monoclonal wells are selected and amplified into a 6-well plate. The amplified clones are screened by a flow cytometry analysis method using a known EGFRvIII antibody (purchased from Absoluteantibody, #Ab00184-1.1). The monoclonal cell line with better growth vigour and higher fluorescence intensity is selected for further expanded culture and same was cryopreserved in liquid nitrogen to obtain an immunogen B. The specific selection results are as shown in table 3 and figure 1. The IgG subtype control is a mouse IgG control. Table 3 shows that a series of CHO-K1, U87MG and 293F cell lines with positive EGFRvIII expression and the CHO-K1 cell line with positive EGFR expression have been prepared. In figures 1-4, the horizontal axis is the cell fluorescence intensity, and the vertical axis is the number of cells. The results in figures 1, 2, 3 and 4 indicate that CHO-K1-hEGFRvIII 1G10, CHO-K1-hEGFR 3G2, U87MG-hEGFRvIII 2G2, and 293F-hEGFRvIII 1C10 are cell lines with a high expression of the EGFRvIII; CHO-K1-hEGFR 3G2 is a cell line with a high expression of the EGFR. The EGFR antibody is also obtained via the same immunization route, which is mainly used as a control to prove that the EGFRvIII antibody has a better tissue specificity than the EGFR antibody.

**Table 3 Results of FACS detection of CHO-K1 stably transfecting cell lines of human EGFRvIII protein**

| Serial number | Clone numbers of stably transfected cell lines | Cell mean fluorescence intensity | |
|---|---|---|---|
| | | IgG subtype control | EGFRvIII antibody |
| 1 | CHO-K1-hEGFRvIII 1G10 | 2.4 | 563.0 |
| 2 | CHO-K1-hEGFRvIII 1D9 | 1.9 | 457.0 |
| 3 | CHO-K1-hEGFRvIII 2B3 | 1.9 | 109.0 |
| 4 | CHO-K1-hEGFRvIII 1E10 | 2.0 | 92.0 |
| 5 | CHO-K1-hEGFRvIII 2D 11 | 1.8 | 77.0 |
| 6 | CHO-K1-hEGFRvIII 1E3 | 2.4 | 42.0 |

**Table 4 Results of FACS detection of CHO-K1 stably transfecting cell lines of human EGFR protein**

| Serial number | Clone numbers of stably transfected cell lines | Cell mean fluorescence intensity | |
|---|---|---|---|
| | | IgG subtype control | EGFR antibody |
| 1 | CHO-K1-hEGFR 3G2 | 2.3 | 396.9 |
| 2 | CHO-K1-hEGFR 3B4 | 2.1 | 285.6 |
| 3 | CHO-K1-hEGFR 1G10 | 2.2 | 191.5 |
| 4 | CHO-K1-hEGFR 2F5 | 2.0 | 321.5 |

**Table 5 Results of FACS detection of HEK293F stably transfecting cell lines of human EGFRvIII protein**

| Serial number | Clone numbers of stably transfected cell lines | Cell mean fluorescence intensity | |
|---|---|---|---|
| | | IgG subtype control | EGFRvIII antibody |
| 1 | 293F-hEGFRvIII 1C10 | 4.3 | 458.1 |
| 2 | 293F-hEGFRvIII 1F3 | 4.1 | 395.0 |
| 3 | 293F-hEGFRvIII 1D11 | 3.6 | 388.0 |

**Table 6 Results of FACS detection of U87MG stably transfecting cell lines of human EGFRvIII protein**

| Serial number | Clone numbers of stably transfected cell lines | Cell mean fluorescence intensity | |
|---|---|---|---|
| | | IgG subtype control | EGFRvIII antibody |
| 1 | U87MG-hEGFRvIII 2G2 | 1.8 | 181.9 |
| 2 | U87MG-hEGFRvIII 2C6 | 1.8 | 178.0 |
| 3 | U87MG-hEGFRvIII 1D1 | 3.0 | 96.4 |
| 4 | U87MG-hEGFRvIII 2C3 | 1.9 | 82.7 |
| 5 | U87MG-hEGFRvIII 1F9 | 1.7 | 68.0 |
| 6 | U87MG-hEGFRvIII 1B3 | 2.3 | 66.9 |

### (III) Preparation of hybridoma cells and antibody screening

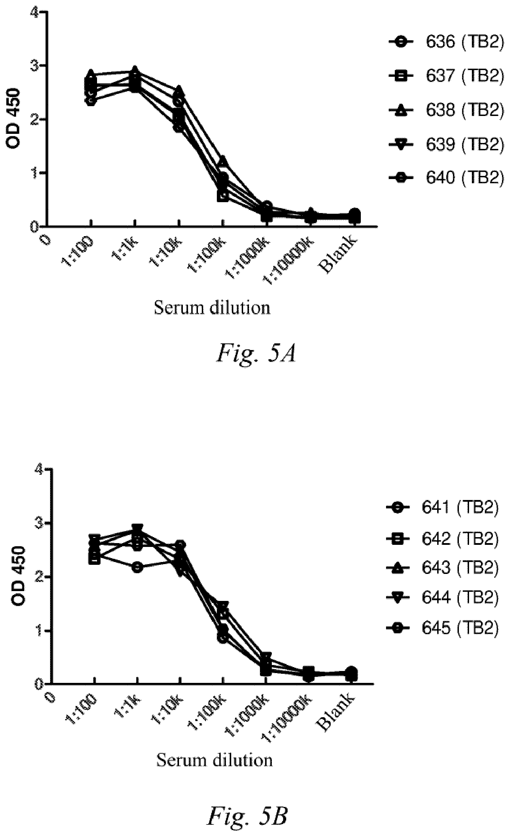

A. 6 to 8 week-old BALB/cAnNCrl mice or SJL/JorllcoCrl mice (purchased from Shanghai SLAC Laboratory Animal Co., Ltd.) are used in the immunization with immunogen A, and the mice are raised under SPF conditions. For the primary immunization, an immunogen A protein is emulsified with a Freund's complete adjuvant and each mouse is intraperitoneally injected at 0.25 ml, namely, each mouse is injected with 50 µg of the immunogen A protein. For the booster immunization, an immunogen A protein is emulsified with a Freund's incomplete adjuvant and each mouse is intraperitoneally injected at 0.25 ml, namely, each mouse is injected with 50 µg of the immunogen A protein. The interval between the primary immunization and the first booster immunization is 2 weeks, and the interval between subsequent booster immunizations is 3 weeks. Blood is collected 1 week after each booster immunization, and the antibody titer and specificity of immunogen A in the serum are detected by ELISA and FACS, with the results as shown in figure 5 and tables 7 and 8. Tables 7 and 8 show that the sera of mice immunized with the human EGFRvIII-hFc all bind to the immunogen to varying degrees, exhibiting antigen-antibody reactions, with the highest dilutability of about one million. The blank control is 1% (w/w) BSA; the batch refers to the sera of mice on the seventh day after the second booster immunization; and the data in the tables is the OD_{450 nm} value.

**Table 7 Serum antibody titer in Balb/c mice immunized with EGFRvIII protein detected by ELISA**

| OD450 nm | Serum dilutability | | | | | | |
|---|---|---|---|---|---|---|---|
| Batch | 1 : 100 | 1 : 10³ | 1 : 10⁴ | 1 : 10⁵ | 1 : 10⁶ | 1 : 10⁷ | Blank control |
| 636 (TB2) | 2.49 | 2.82 | 2.34 | 0.91 | 0.37 | 0.20 | 0.24 |
| 637 (TB2) | 2.64 | 2.63 | 2.07 | 0.57 | 0.20 | 0.18 | 0.17 |
| 638 (TB2) | 2.83 | 2.89 | 2.53 | 1.22 | 0.27 | 0.25 | 0.18 |
| 639 (TB2) | 2.62 | 2.65 | 2.11 | 0.72 | 0.22 | 0.16 | 0.16 |
| 640 (TB2) | 2.35 | 2.59 | 1.85 | 0.85 | 0.26 | 0.16 | 0.17 |

**Table 8 Serum antibody titer in SJL mice immunized with EGFRvIII protein detected by ELISA**

| OD450 nm | Serum dilutability | | | | | | |
|---|---|---|---|---|---|---|---|
| Batch | 1 : 100 | 1 : 10³ | 1 : 10⁴ | 1 : 10⁵ | 1 : 10⁶ | 1 : 10⁷ | Blank control |
| 641 (TB2) | 2.42 | 2.18 | 2.31 | 0.87 | 0.28 | 0.15 | 0.23 |
| 642 (TB2) | 2.34 | 2.73 | 2.33 | 1.31 | 0.36 | 0.23 | 0.17 |
| 643 (TB2) | 2.58 | 2.87 | 2.46 | 1.01 | 0.27 | 0.18 | 0.24 |
| 644 (TB2) | 2.69 | 2.88 | 2.11 | 1.44 | 0.49 | 0.19 | 0.18 |
| 645 (TB2) | 2.63 | 2.58 | 2.60 | 1.03 | 0.25 | 0.17 | 0.21 |

B. 6 to 8 week-old BALB/cAnNCrl mice or SJL/JorllcoCrl mice (purchased from Shanghai SLAC Laboratory Animal Co., Ltd.) are used in the immunization with immunogen B, and the mice are raised under SPF conditions. The HEK293 cell line is transfected with the pIRES plasmid containing the nucleotide sequence encoding the full-length amino acid sequence of human EGFRvIII [see step (II) in example 1] to obtain an HEK293 stable cell line 293F-hEGFRvIII 1C10 containing human EGFRvIII (using X-treme GENE HP DNA Transfection Reagent, which is purchased from Roche, Cat #06 366 236 001, and operating according to the instructions); the cell line is subjected to an expanded culture to 90% confluence in a T-75 cell culture flask; the medium is sucked out, and the cells are washed twice with a DMEM basal medium (purchased from Invitrogen), and then treated with an enzyme-free cell dissociation solution (purchased from Invitrogen) at 37°C until cells can be fallen off from the wall of the culture dish, and the cells are collected. The cells are washed twice with the DMEM basal medium and are counted, and then the cells are diluted with a phosphate buffer (pH 7.2) to 2 × 10⁷ cells/ml. Each mouse is injected intraperitoneally with 0.5 ml of the cell suspension at each immunization. The interval between the first immunization and the second immunization is 2 weeks, and the interval between subsequent immunizations is 3 weeks. In addition to the first immunization, blood is collected 1 week after each immunization, and the antibody titer and specificity in the serum are detected by FACS. After the second booster immunization, the serum antibody titer reaches at least 1 : 1000 detected by FACS.

Before the completion of steps A and B, each selected mouse is the last time immunized intraperitoneally with 100 µg of the purified immunogen A (mice immunized with the immunogen A) or immunogen B (mice immunized with the immunogen B); after 5 days, the mice are sacrificed and spleen cells are collected. NH₄OH is added to a final concentration of 1% (w/w) and red blood cells incorporated in the spleen cells are lysed to obtain a spleen cell suspension. The cells are washed with DMEM basic medium and centrifuged at 1000 rpm for 3 times, then are mixed at a 5 : 1 ratio of living cell number with mouse myeloma cells SP2/0 (purchased from ATCC), then are subjected to cell fusion by using an efficient electrical fusion method (see METHODS IN ENZYMOLOGY, VOL. 220). The fused cells are diluted into a DMEM medium containing 20% fetal bovine serum and 1 × HAT, with the percentage being the mass percentage. Then the cells at 1 × 10⁵/200 µL/well are added to a 96-well cell culture plate and placed in an incubator with 5% CO₂ and 37°C, with the percentage being the volume percentage. After 14 days, the cell fusion plate supernatant is screened by ELISA and Acumen (a microplate cell detection method), and the positive clones with OD₄₅₀nm > 1.0 in ELISA and MFI value > 100 in Acumen are amplified to a 24-well plate, and an expanded culture is performed in DMEM (invitrogen) containing 10% (w/w) HT fetal bovine serum under the conditions of 37°C and 5% (v/v) CO₂. After culturing for 3 days, the culture broth from the expanded culture in the 24-well plate is centrifuged; the supernatant is collected and analyzed for antibody subtypes; and the binding activity in the EGFRvIII protein and EGFRvIII positive cells (for the detection method of the binding activity, please refer to example 3A and example 3B respectively), and the anti-mouse antibody-MMAF indirect cytotoxic killing experiment (for detection method of the indirect cytotoxic killing activity, please refer to example 4) are determined by ELISA and FACS.

According to the screening results of the 24-well plate, hybridoma cells with OD₄₅₀ₙₘ > 1.0 in the ELISA experiment, with the MFI value > 50 in the FACS experiment, and with the killing rate of the hybridoma cell culture supernatant for the EGFRvIII-positive cells reaching 50% in the indirect cytotoxicity killing experiment are selected as eligible positive clones, and eligible hybridoma cells are subcloned in the 96-well plate by a limiting dilution method and cultured in a DMEM medium (purchased from invitrogen) containing 10% (w/w) FBS under conditions of 37°C and 5% (v/v) CO₂. 10 days after subcloning, preliminary screening is performed by ELISA and Acumen, and a single positive monoclone is selected and amplified to a 24-well plate for further cultivation. 3 days later, the FACS is used to determine the positive antigen binding, and the antimouse antibody-MMAF indirect cytotoxicity killing experiment is used to evaluate the biological activity (evaluation criteria: OD₄₅₀ₙₘ > 1.0 in the ELISA experiment, the MFI value > 50 in the FACS experiment, and the killing rate of the hybridoma cell culture supernatant for the EGFRvIII-positive cells reaching 50% in the indirect cytotoxicity killing experiment).

Based on the detection results of the samples in the 24-well plate, the best clones are selected, subjected to an expanded culture in the DMEM medium (purchased from invitrogen) containing 10% (w/w) FBS under conditions of 37°C and 5% (v/v) CO₂, and then cryopreserved in liquid nitrogen to obtain the hybridoma cells of the present invention, which can be used for subsequent antibody production and purification.

### Example 2 Production and purification of lead antibody

The antibody produced by the hybridoma cells has a relatively low concentration which is only about 1 to 10 µg/ml, and the concentration varied greatly. Moreover, various proteins produced by cell culture in the culture medium and fetal bovine serum components contained in the culture medium interfered with many biological activity analysis methods in varying degrees, and therefore, it is necessary to perform a small-scale (1 to 5 mg) antibody production and purification.

The hybridoma cells obtained in example 1 are inoculated into a T-75 cell culture flask and acclimated and passaged for 3 generations by using a production medium (Hybridoma serum free medium, purchased from Invitrogen). When the cells have a good growing status, they are inoculated into a roller bottle for tissue culture. 200 ml of the production medium is added to each 2 liter of the culture roller bottle, with the cell density inoculated being 1.0 × 10⁵/ml. The bottle cap is screwed down, and the roller bottle is placed on a rotary machine in a 37°C incubator at a speed of 3 rpm/min. After a continuous rotating culture for 14 days, the cell culture broth is collected and filtered to remove cells, and the culture supernatant is filtered with a 0.45 micron filter membrane until it is clear. The clear culture supernatant may be purified immediately or cryopreserved at -30°C.

Monoclonal antibodies in the clear culture supernatant (200 mL) of hybridoma cells are purified by using a 2 mL protein A column (purchased from GE Healthcare). The protein G column is first equilibrated with an equilibration buffer (PBS phosphate buffer, pH 7.4), and then the clear culture supernatant is loaded onto the protein A column, with the flow rate controlled at 3 mL/min. After loading, the protein G column is washed with the equilibration buffer at a volume of 4 column bed volumes of the protein G column. The EGFRvIII antibody bound to the protein A column is eluted with an eluent (0.1 M sodium citrate buffer, pH 3.5), and the elution status is monitored with an ultraviolet detector (A280 ultraviolet absorption peak). The eluted antibodies are collected, and 10% 1.0 M Tris-HCl buffer is added to neutralize the pH, with the percentage being the volume percentage, and then immediately dialyzed with the PBS phosphate buffer overnight. The next day, the dialysate is changed once and is further dialyzed for 3 hours. The dialyzed EGFRvIII antibody is collected, aseptically filtered with a 0.22 micron filter, and stored aseptically to obtain a purified EGFRvIII antibody.

The purified EGFRvIII antibody is detected and analyzed for protein concentration (A280/1.4), purity, endotoxin (Lonza kit), etc. The results as shown in table 9 indicate that the endotoxin concentration of the final antibody product is within 1.0 EU/mg.

**Table 9 Quality control of purified EGFRvIII antibody**

| Antibody name | Clone number | Antibody purity | Protein concentration (mg/ml) | Endotoxin (EU/mg) |
|---|---|---|---|---|
| Murine anti-75G7 | 75G7C6 | > 95% | 0.44 | < 1.0 |
| Murine anti-63A10 | 63A10A7 | > 95% | 1.26 | < 1.0 |
| Murine anti-64F1 | 64F1F8 | > 95% | 1.52 | < 1.0 |
| Murine anti-7E5 | 7E5-2A9 | > 95% | 0.81 | < 1.0 |
| Murine anti-43H6 | 43H6A1 | > 95% | 1.08 | < 1.0 |
| Murine anti-46C4 | 46C4A6 | > 95% | 1.43 | < 1.0 |
| Murine anti-49G12 | 49G12G5 | > 95% | 1.16 | < 1.0 |
| Murine anti-51G7 | 51G7C2 | > 95% | 1.07 | < 1.0 |
| Murine anti-53D8 | 53D8H4 | > 95% | 0.93 | < 1.0 |
| Murine anti-54D2 | 54D2G10 | > 95% | 1.29 | < 1.0 |
| Murine anti-56F10 | 56F10G2 | > 95% | 0.92 | < 1.0 |
| Murine anti-59G3 | 59G3F12 | > 95% | 1.29 | < 1.0 |
| Murine anti-64B11 | 64B11F2 | > 95% | 1.11 | < 1.0 |

### Example 3 Detection and identification of lead antibody

### A. Binding of the antibody to an EGFRvIII protein detected by an enzyme-linked immunosorbent assay (ELISA)

The purified EGFRvIII antibody obtained in example 2 is reacted with a human EGFRvIII-hFc protein.

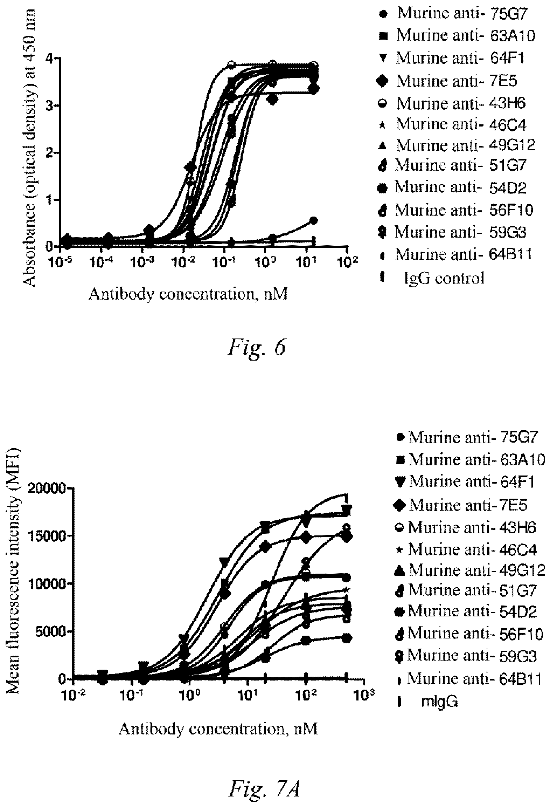

The purified immunogen A (EGFRvIII-hFc) obtained in example 1 (for the preparation method, see step (I) in example 1) is diluted with PBS to a final concentration of 1.0 µg/mL, and then added to a 96-well ELISA plate at 100 µl/well. The plate is sealed with a plastic film and incubated at 4°C overnight. The next day, the plate is washed twice with a plate washing solution [PBS + 0.01% (v/v) Tween20], and added with a blocking solution [PBS + 0.01% (v/v) Tween20 + 1% (w/w) BSA] for blocking 2 hours at room temperature. The blocking solution is discarded, and the purified EGFRvIII antibody obtained in example 2 is added at 100 µl/well. The plate is incubated for 2 hours at 37°C, and then washed 3 times with the plate washing solution [PBS + 0.01% (v/v) Tween20]. The plate is added with an HRP (horseradish peroxidase) labeled secondary antibody (purchased from Sigma), incubated at 37°C for 2 hours, and then washed 3 times with the plate washing solution [PBS + 0.01% (v/v) Tween20]. The plate is added with 100 µl of TMB substrate/well, incubated for 30 minutes at room temperature, and then added with 100 µl of stopping solution (1.0N HCl)/well. A450 nm value is read using an ELISA plate reader (SpectraMax 384plus, purchased from Molecular Device), and the results are shown in figure 6 and table 10. Table 10 shows that the purified antibody binds to the EGFRvIII recombinant protein at an ELISA level. The IgG control is a mouse IgG, and the data in the table is OD₄₅₀ₙₘ value.

**Table 10 Binding reaction of EGFRvIII antibody with human EGFRvIII-hFc protein detected by ELISA**

| OD450 nm | | Antibody concentration (nM) | | | | | | | |
|---|---|---|---|---|---|---|---|---|---|
| Antibody name | Clone number | 100 | 10 | 1 | 0.1 | 0.01 | 0.001 | 0.0001 | Blank |
| Murine anti-75G7 | 75G7C6 | 0.56 | 0.19 | 0.09 | 0.07 | 0.06 | 0.07 | 0.07 | 0.07 |
| Murine anti-63A10 | 63A10A7 | 3.71 | 3.76 | 3.42 | 0.80 | 0.17 | 0.09 | 0.08 | 0.08 |
| Murine anti-64F1 | 64F1F8 | 3.73 | 3.76 | 3.51 | 1.00 | 0.16 | 0.09 | 0.08 | 0.08 |
| Murine anti-7E5 | 7E5-2A9 | 3.36 | 3.14 | 3.18 | 1.68 | 0.35 | 0.17 | 0.15 | 0.19 |
| Murine anti-43H6 | 43H6A1 | 3.85 | 3.87 | 3.86 | 1.39 | 0.25 | 0.11 | 0.09 | 0.10 |
| Murine anti-46C4 | 46C4A6 | 3.83 | 3.85 | 3.50 | 0.72 | 0.15 | 0.09 | 0.08 | 0.08 |
| Murine anti-49G12 | 49G12G5 | 3.79 | 3.80 | 3.20 | 0.64 | 0.14 | 0.13 | 0.08 | 0.08 |
| Murine anti-51G7 | 51G7C2 | 3.68 | 3.69 | 2.44 | 0.46 | 0.15 | 0.09 | 0.08 | 0.09 |
| Murine anti-53D8 | 53D8H4 | 3.72 | 3.69 | 3.20 | 0.64 | 0.16 | 0.10 | 0.09 | 0.09 |
| Murine anti-54D2 | 54D2G10 | 3.61 | 3.60 | 1.33 | 0.22 | 0.09 | 0.08 | 0.08 | 0.08 |
| Murine anti-56F10 | 56F10G2 | 3.61 | 3.53 | 0.98 | 0.18 | 0.11 | 0.10 | 0.09 | 0.11 |
| Murine anti-59G3 | 59G3F12 | 3.71 | 3.67 | 2.68 | 0.49 | 0.13 | 0.08 | 0.08 | 0.08 |
| Murine anti-64B11 | 64B11F2 | 3.64 | 3.63 | 1.58 | 0.24 | 0.09 | 0.08 | 0.08 | 0.08 |
| hIgG | IgG control | 0.11 | 0.10 | 0.09 | 0.09 | 0.09 | 0.09 | 0.09 | 0.09 |

### B. Binding of antibody to EGFRvIII expressing cell detected by fluorescence activated cell sorting (FACS)

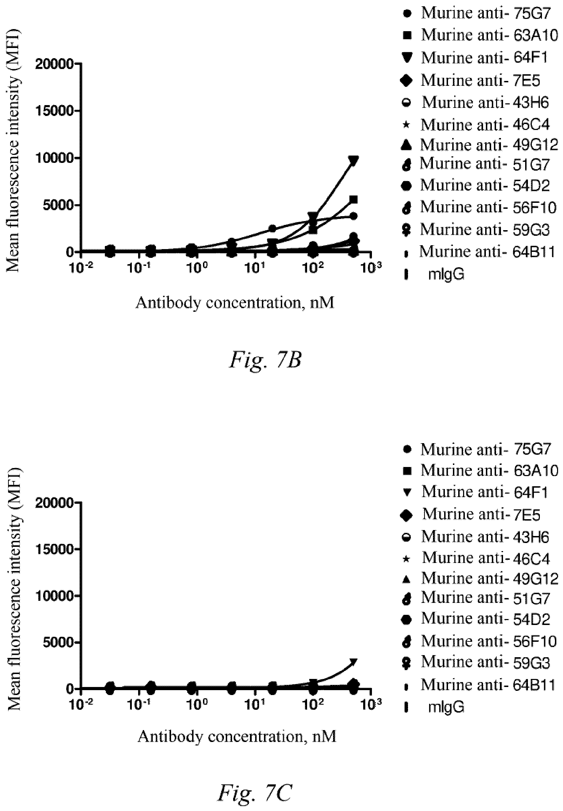

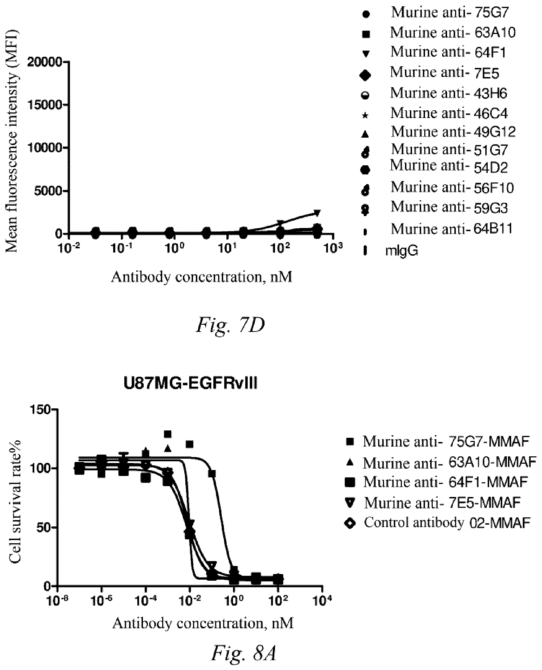

The desired cells are subjected to an expanded culture to 90% confluence in a T-75 cell culture flask; the medium is sucked out, and the cells are washed twice with a HBSS buffer (Hanks Balanced Salt Solution) (purchased from Invitrogen), and then treated with an enzyme-free cell dissociation solution (Versene solution: purchased from Life Technology) and collected. The cells are washed twice with the HBSS buffer and are counted, and then the cells are diluted with the HBSS buffer to 2 × 10⁶ cells/ml, added with 1% goat serum blocking solution, with the percentage being the mass percentage, and incubated on ice for 30 minutes, and then centrifugally washed twice with the HBSS buffer. The collected cells are suspended to 2 × 10⁶ cells/mL with an FACS buffer (HBSS + 1% BSA, with the percentage being the mass percentage), added to a 96-well FACS reaction plate at 100 µL/well, then added with 100 µL/well of the purified EGFRvIII antibody (samples to be tested) obtained in example 2, and incubated on ice for 2 hours. The plate is centrifugally washed twice with the FACS buffer, added with 100 µL/well of a fluorescence (Alexa 488) labeled secondary antibody (purchased from Invitrogen), and then incubated on ice for 1 hour. The plate is centrifugally washed 3 times with the FACS buffer, added with 100 µL/well of a fixing solution [4% (v/v) paraformaldehyde] for suspending the cells, and centrifugally washed twice with the FACS buffer after 10 minutes. The cells are suspended in 100 µL of the FACS buffer, and the results are detected and analyzed by FACS (FACS Calibur, purchased from BD). Data analysis is performed by using the software (CellQuest) to obtain the mean fluorescence intensity (MFI) of the cells. Data is further analyzed by using the software (GraphPad Prism5) to perform data fitting and calculate EC50. The analysis results as shown in table 11 and figure 7 indicate that the antibodies to be tested may bind to the EGFRvIII protein on the surface of U87MG-hEGFRvIII cells (Figure 7A). A431 cells (human epidermal cell cancer cell lines) have a large amount of wild-type EGFR protein expressed on the surface thereof. From figure 7B, it can be seen that the murine anti-75G7, -63A10 and -64F1 may weakly bind to the wild-type EGFR protein on the surface of A431 cells, while the murine anti-7E5 (with the antibody clone number being 7E5-2A9) does not recognize the wild-type EGFR protein, and only specifically recognizes the mutant EGFRvIII protein. U87MG cells (human glioma cell lines) have a small amount of wild-type EGFR protein expressed on the surface thereof, while HEB cells (human glioma cell lines) have a high level of wild-type EGFR protein expression on the surface thereof. The antibodies to be tested have no binding reaction to U87MG and HEB cells, indicating that the murine anti-75G7, -63A10 and -64F1 have a good specificity, and only recognize the mutant EGFRvIII protein and overexpressed wild-type EGFR protein in tumor tissues, but do not recognize the wild-type EGFR protein in normal tissues (Figures 7C and 7D).

**Table 11 Binding reaction of EGFRvIII antibody with U87MG-hEGFRvIII, A431, U87MG and HEB cells detected by FACS**

| Antibody name | Antibody clone number | EC50, nM | | | |
|---|---|---|---|---|---|
| | | U87MG-hEGFRvIII | A431 | HEB | U87MG |
| Murine anti-75G7 | 75G7C6 | 4.8 | Weakly positive | Negative | Negative |
| Murine anti-63A10 | 63A10A7 | 3.1 | Weakly positive | Negative | Negative |
| Murine anti-64F1 | 64F1F8 | 2.0 | Weakly positive | Negative | Negative |
| Murine anti-7E5 | 7E5-2A9 | 2.9 | Negative | Negative | Negative |
| Murine anti-43H6 | 43H6A1 | 4.0 | Negative | Negative | Negative |
| Murine anti-46C4 | 46C4A6 | 13.3 | Negative | Negative | Negative |
| Murine anti-49G12 | 49G12G5 | 7.9 | Negative | Negative | Negative |
| Murine anti-51G7 | 51G7C2 | 26.2 | Negative | Negative | Negative |
| Murine anti-53D8 | 53D8H4 | 6.8 | Negative | Negative | Negative |
| Murine anti-54D2 | 54D2G10 | 19.5 | Negative | Negative | Negative |
| Murine anti-56F10 | 56F10G2 | 12.3 | Negative | Negative | Negative |
| Murine anti-59G3 | 59G3F12 | 40.9 | Negative | Negative | Negative |
| Murine anti-64B11 | 64B11F2 | 25.3 | Negative | Negative | Negative |

### Example 4 Cell killing activity experiment of EGFRvIII antibody-drug conjugate

The antibody is dialyzed with a sodium borate buffer at Ph 6.5 to 8.5, and then a certain proportion of TCEP is added thereto (with TCEP/antibody ratio being 2-10); reduction is performed for 2 to 4 hours at room temperature or 37°C, and excess TCEPs are removed through a G25 desalting filler, and a certain proportion of MC-MMAF (with drug/antibody ratio being 5-20) is added and reacted for 4 hours. Then cysteine is added to neutralize excess drugs, and excess small molecules are removed through G25. A purified antibody-drug conjugate is afforded (for the coupling method, see Doronina, 2006, Bioconjugate Chem.17,114-124). The cytotoxic activity is analyzed after HIC analysis of drug cross-linking rate, purity and other parameters. For comparison, the drug cross-linking rate of all antibody conjugates is 8.

The resulting antibody conjugates are respectively subjected to gradient dilution with a complete medium, and a 96-well cell culture plate is added with 90 µL of the U87MG-EGFRvIII cell suspension at 1000 cells/well, and to each well is further added 10 µL of different concentrations of antibody-drug conjugate dilutions; after continuing the culture for 5 days, the cell viability is detected using the CellTiter-Glo kit (purchased from Promega, and the usage method refers to the product instructions).

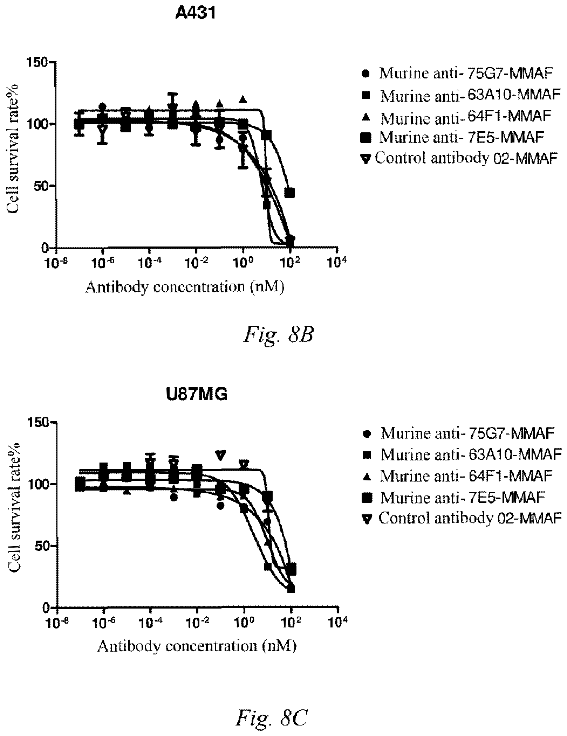

The results are as shown in table 12 and figure 8, wherein IC50 in table 13-1 refers to the median effective dose of cells whose activity is inhibited after drug actions, and the cell killing activity can be reflected by detecting the cell activity. Figure 8 shows the detection of the cell killing activity of the antibody-drug conjugate on the recombinant tumor cell line U87MG-EGFRvIII positive for EGFRvIII. The results show that in an in-vitro experiment, the antibodies to be tested all can produce a good killing effect on U87MG-hEGFRvIII cells having a mutant EGFRvIII protein expressed on the surface thereof (Figure 8A). A431 cells (human epidermal cell cancer cell lines) have a large amount of wild-type EGFR protein expressed on the surface thereof. From figure 8B, it can be seen that the murine anti-75G7, -63A10 and -64F1 have a weaker killing effect on A431. U87MG cells (human glioma cell lines) have a small amount of wild-type EGFR protein expressed on the surface thereof, while HEB cells (human glioma cell lines) have a high level of wild-type EGFR protein expression on the surface thereof. The murine anti-75G7, -63A10 and -64F1 have weak or no killing effect on U87MG and HEB cells (see figure 8C and figure 8D). It is indicated that the murine anti-75G7, -63A10 and -64F1 have a good specificity, can specifically kill tumor tissues expressing the mutant EGFRvIII protein and overexpressing the wild-type EGFR protein, but have no killing effect on normal tissues expressing the wild-type EGFR protein.

**Table 12 Specific killing effect of antibody conjugates on EGFRvIII positive cells detected by a cell killing experiment**

| Antibody conjugate | Antibody clone number | DAR | IC50, nM | | | |
|---|---|---|---|---|---|---|
| | | | U87MG-hEGFRvIII | A431 | HEB | U87MG |
| Murine anti-75G7-MMAF | 75G7C6 | 8 | 0.27 | Weakly positive | Negative | Negative |
| Murine anti-63A10-MMAF | 63A10A7 | 8 | 0.01 | Weakly positive | Negative | Negative |
| Murine anti-64F1-MMAF | 64F1F8 | 8 | 0.01 | Weakly positive | Negative | Negative |
| Murine anti-7E5-MMAF | 7E5-2A9 | 8 | 0.02 | Negative | Negative | Negative |
| Murine anti-43H6-MMAF | 43H6A1 | 8 | 0.01 | Negative | Negative | Negative |
| Murine anti-46C4-MMAF | 46C4A6 | 8 | 0.15 | Negative | Negative | Negative |
| Murine anti-49G12-MMAF | 49G12G5 | 8 | 0.02 | Negative | Negative | Negative |
| Murine anti-53D8-MMAF | 53D8H4 | 8 | 0.04 | Negative | Negative | Negative |
| Murine anti-56F10-MMAF | 56F10G2 | 8 | 0.15 | Negative | Negative | Negative |
| Murine anti-59G3-MMAF | 59G3F12 | 8 | 0.06 | Negative | Negative | Negative |
| Murine anti-64B11-MMAF | 64B11F2 | 8 | 0.04 | Negative | Negative | Negative |

### Example 5 Epitope analysis of EGFRvIII antibody

### A. Analysis of epitope by a competitive ELISA method (epitope binning)

In order to identify the binding sites of antibodies to antigens, EGFRvIII antibodies are grouped using a competitive ELISA method.

The purified antibodies to be tested are diluted with PBS to 1 µg/mL, coated onto a 96-well high-adsorption ELISA plate at 50 µL/well at 4°C overnight, and then blocked with 250 µL of the blocking solution [PBS containing 0.01% (v/v) Tween20 and 1% (w/w) BSA] for one hour at room temperature, and each well is added with 0.05 µg/mL of the biotin-labeled recombinant EGFRvIII protein. Simultaneously, 5 µg/mL of the competitive antibody (i.e., the purified EGFRvIII antibody obtained in example 2) is added thereto, and incubated at 25°C-37°C for 1 to 2 hours. The plate is washed 3 times with the plate washing solution [PBS containing 0.01% (v/v) Tween20], and added with HRP (horseradish peroxidase) labeled streptavidin (purchased from Sigma). The plate is incubated for 0.5 hour at 37°C, and then washed 3 times with the plate washing solution [PBS containing 0.01% (v/v) Tween20]. The plate is added with 100 µL of TMB substrate/well, incubated for 30 minutes at room temperature, and then added with 100 µL of stopping solution (1.0N HCl)/well. A450 nm value is read using an ELISA plate reader (SpectraMax 384 plus, purchased from Molecular Device), and the results are shown in figure 6. The competition rate between antibodies is calculated based on the A450 nm value, and the results are shown in table 13-1. The higher the value of the competition rate, the closer the antigen surfaces of the two antibodies are.

**Table 13-1 Analysis of epitope by a competitive ELISA method (epitope binning)**

| Competition rate | Control antibody 01 | Control antibody 02 | Murine anti-7E5 | Murine anti-64F1 | Murine anti-63A10 | Murine anti-75G7 |
|---|---|---|---|---|---|---|
| Control antibody 01 | 66% | 0% | 4% | 2% | 5% | 16% |
| Control antibody 02 | 2% | 91% | 81% | 74% | 37% | 51% |
| Murine anti-7E5 | -2% | 66% | 85% | 71% | 70% | 23% |
| Murine anti-64F1 | -5% | 69% | 80% | 72% | 81% | 13% |
| Murine anti-63A10 | -3% | 79% | 82% | 73% | 89% | 20% |
| Murine anti-75G7 | -5% | -2% | 3% | -3% | 9% | 67% |

Competitive ELISA results show that epitopes of control antibody 02 and murine anti-7E5, -63A10 and -64F1 are competitive and should be the same or similar epitopes. Since the murine anti-75G7 is not competitive with control antibody 02, the epitope of the murine anti-75G7 should be different from that of the control antibody 02, wherein the control antibody 01 is AMG-595 (purchased from Amgen), and the control antibody 02 is ABT-414 (purchased from Abbvie).

### B. Analysis of epitope by a polypeptide dot matrix ELISA method (epitope mapping)

In order to determine the specific antibody binding epitopes of 4 candidate antibodies of the present invention, a polypeptide dot matrix ELISA method is used to analyze the antigen binding epitopes of the antibodies. A polypeptide of 16 amino acids in length is synthesized by using amino acids at positions 1 to 50 of the EGFRvIII protein as a template, starting from the N-terminus, and moving in a manner of overlapping 15 amino acids. The C-terminus of the polypeptide is added with a biotin label. The polypeptide is synthesized by GL Biochem, with 35 biotin labeled polypeptides synthesized in total. The detailed sequences are shown in table 13-2.

**Table 13-2 Analysis of epitope by a polypeptide dot matrix ELISA method**

| **Polypeptides** | | **Control antibody 01** | | **Control antibody 02** | | **Chimeric antibody 64F1** | | **Chimeric antibody 63A10** | | **Chimeric antibody 75G7** | | **hIgG** | |
|---|---|---|---|---|---|---|---|---|---|---|---|---|---|
| pep01 | LEEKKGNYVV TDHGSC-K (Biotin) | 3.62 | 3.59 | 0.05 | 0.05 | 0.05 | 0.05 | 0.05 | 0.05 | 0.06 | 0.05 | 0.08 | 0.07 |
| pep02 | EEKKGNYVVT DHGSCV-K (Biotin) | 3.32 | 3.17 | 0.05 | 0.05 | 0.05 | 0.05 | 0.05 | 0.07 | 0.05 | 0.05 | 0.06 | 0.06 |
| pep03 | EKKGNYVVTD HGSCVR-K (Biotin) | 0.14 | 0.10 | 0.05 | 0.05 | 0.05 | 0.05 | 0.32 | 0.05 | 0.12 | 0.06 | 0.17 | 0.10 |
| pep04 | KKGNYVVTD HGSCVRA-K (Biotin) | 0.12 | 0.06 | 0.06 | 0.05 | 0.05 | 0.56 | 0.06 | 0.06 | 0.06 | 0.05 | 0.05 | 0.06 |
| pep05 | KGNYVVTDH GSCVRAC-K (Biotin) | 0.05 | 0.15 | 0.05 | 0.05 | 0.05 | 0.82 | 0.15 | 0.17 | 0.06 | 0.44 | 0.05 | 0.06 |
| pep06 | GNYVVTDHGS CVRACG-K (Biotin) | 0.19 | 0.06 | 0.09 | 0.05 | 0.05 | 0.25 | 0.05 | 0.07 | 0.14 | 0.10 | 0.07 | 0.05 |
| pep07 | NYVVTDHGSC VRACGA-K (Biotin) | 0.23 | 0.06 | 0.05 | 0.05 | 0.05 | 0.12 | 0.05 | 0.52 | 0.31 | 0.05 | 0.05 | 0.05 |
| pep08 | YVVTDHGSCV RACGAD-K (Biotin) | 0.08 | 0.05 | 0.05 | 0.05 | 0.05 | 0.05 | 0.05 | 0.05 | 0.05 | 0.05 | 0.05 | 0.05 |
| pep09 | VVTDHGSCVR ACGADS-K (Biotin) | 0.05 | 0.05 | 0.06 | 0.05 | 0.05 | 0.05 | 0.05 | 0.05 | 0.06 | 0.05 | 0.05 | 0.05 |
| pep10 | VTDHGSCVRA CGADSY-K (Biotin) | 0.05 | 0.08 | 0.07 | 0.05 | 0.05 | 0.05 | 0.05 | 0.05 | 0.09 | 0.05 | 0.05 | 0.06 |
| pep11 | TDHGSCVRAC GADSYE-K (Biotin) | 0.05 | 0.09 | 0.06 | 0.06 | 0.05 | 0.05 | 0.05 | 0.05 | 0.08 | 0.08 | 0.05 | 0.06 |
| pep12 | DHGSCVRACG ADSYEM-K (Biotin) | 0.09 | 0.06 | 0.06 | 0.06 | 0.05 | 0.05 | 0.05 | 0.06 | 0.07 | 0.11 | 0.05 | 0.05 |
| pep13 | HGSCVRACGA DSYEME-K (Biotin) | 0.06 | 0.08 | 0.08 | 0.05 | 0.05 | 0.07 | 0.06 | 0.07 | 0.07 | 0.10 | 0.06 | 0.06 |
| pep14 | GSCVRACGAD SYEMEE-K (Biotin) | 0.09 | 0.11 | 0.08 | 0.07 | 0.08 | 0.09 | 0.08 | 0.23 | 0.18 | 0.12 | 0.07 | 0.07 |
| pep15 | SCVRACGADS YEMEED-K (Biotin) | 0.06 | 0.05 | 0.05 | 0.06 | 0.09 | 0.11 | 0.14 | 0.11 | 0.06 | 0.11 | 0.05 | 0.06 |
| pep16 | CVRACGADSY EMEEDG-K (Biotin) | 0.06 | 0.05 | 0.10 | 0.05 | 0.05 | 0.08 | 0.05 | 0.05 | 0.05 | 0.05 | 0.05 | 0.05 |
| pep17 | VRACGADSYE MEEDGV-K (Biotin) | 0.09 | 0.05 | 0.05 | 0.06 | 0.05 | 0.05 | 0.05 | 0.05 | 0.05 | 0.05 | 0.05 | 0.06 |
| pep18 | RACGADSYEM EEDGVR-K (Biotin) | 0.05 | 0.05 | 0.05 | 0.05 | 0.06 | 0.05 | 0.05 | 0.05 | 0.05 | 0.05 | 0.05 | 0.05 |
| pep19 | ACGADSYEME EDGVRK-K (Biotin) | 0.15 | 0.09 | 0.31 | 0.11 | 0.05 | 0.05 | 0.11 | 0.15 | 0.18 | 0.17 | 0.05 | 0.05 |
| pep20 | CGADSYEMEE DGVRKC-K (Biotin) | 0.22 | 0.13 | 3.47 | 3.49 | 1.31 | 1.88 | 1.95 | 2.02 | 0.16 | 0.17 | 0.12 | 0.10 |
| pep21 | GADSYEMEED GVRKCK-K (Biotin) | 0.15 | 0.31 | 0.32 | 0.43 | 0.05 | 0.06 | 0.21 | 0.26 | 0.19 | 0.18 | 0.06 | 0.06 |
| pep22 | ADSYEMEEDG VRKCKK-K (Biotin) | 0.12 | 0.15 | 2.05 | 1.90 | 0.08 | 0.08 | 1.02 | 1.18 | 0.12 | 0.13 | 0.06 | 0.06 |
| pep23 | DSYEMEEDGV RKCKKC-K (Biotin) | 0.06 | 0.06 | 0.19 | 0.16 | 0.07 | 0.07 | 0.19 | 0.20 | 0.05 | 0.05 | 0.06 | 0.06 |
| pep24 | SYEMEEDGVR KCKKCE-K (Biotin) | 0.05 | 0.05 | 0.11 | 0.09 | 0.06 | 0.06 | 0.09 | 0.08 | 0.05 | 0.05 | 0.06 | 0.06 |
| pep25 | YEMEEDGVRK CKKCEG-K (Biotin) | 0.06 | 0.06 | 0.07 | 0.07 | 0.06 | 0.06 | 0.06 | 0.06 | 0.06 | 0.05 | 0.06 | 0.06 |
| pep26 | EMEEDGVRKC KKCEGP-K (Biotin) | 0.25 | 0.28 | 0.40 | 0.33 | 0.05 | 0.08 | 0.33 | 0.33 | 0.20 | 0.33 | 0.32 | 0.06 |
| pep27 | MEEDGVRKC KKCEGPC-K (Biotin) | 0.38 | 0.29 | 0.38 | 0.32 | 0.05 | 0.26 | 0.45 | 0.31 | 0.28 | 0.29 | 0.27 | 0.07 |
| pep28 | EEDGVRKCKK CEGPCR-K (Biotin) | 0.06 | 0.12 | 0.09 | 0.07 | 0.05 | 0.06 | 0.06 | 0.06 | 0.06 | 0.06 | 0.07 | 0.07 |
| pep29 | EDGVRKCKKC EGPCRK-K (Biotin) | 0.06 | 0.08 | 0.07 | 0.07 | 0.06 | 0.06 | 0.06 | 0.05 | 0.05 | 0.09 | 0.07 | 0.07 |
| pep30 | DGVRKCKKCE GPCRKV-K (Biotin) | 0.07 | 0.07 | 0.08 | 0.07 | 0.06 | 0.06 | 0.06 | 0.06 | 0.09 | 0.07 | 0.08 | 0.07 |
| pep31 | GVRKCKKCEG PCRKVC-K (Biotin) | 0.06 | 0.07 | 0.07 | 0.07 | 0.06 | 0.06 | 0.06 | 0.06 | 0.06 | 0.06 | 0.07 | 0.08 |
| pep32 | VRKCKKCEGP CRKVCN-K (Biotin) | 0.06 | 0.07 | 0.07 | 0.07 | 0.06 | 0.06 | 0.13 | 0.06 | 0.06 | 0.06 | 0.07 | 0.08 |
| pep33 | RKCKKCEGPC RKVCNG-K (Biotin) | 0.08 | 0.10 | 0.08 | 0.07 | 0.07 | 0.08 | 0.07 | 0.07 | 0.07 | 0.07 | 0.07 | 0.06 |
| pep34 | KCKKCEGPCR KVCNGI-K (Biotin) | 0.07 | 0.09 | 0.07 | 0.09 | 0.07 | 0.07 | 0.07 | 0.07 | 0.06 | 0.06 | 0.07 | 0.08 |
| pep35 | CKKCEGPCRK VCNGIG-K (Biotin) | 0.07 | 0.07 | 0.07 | 0.07 | 0.09 | 0.07 | 0.07 | 0.07 | 0.07 | 0.07 | 0.07 | 0.07 |

The results of the polypeptide dot matrix ELISA show that the control antibody 01 and murine anti-7E5, murine anti-64F1 and murine anti-63A10 have the same or similar epitope, i.e., CGADSYEMEEDGVRKC. Since the murine anti-75G7 does not bind to the 35 polypeptides, the epitope of the murine anti-75G7 should be different from that of the control antibody 01 and that of the control antibody 02, wherein the control antibody 01 is AMG-595 (purchased from Amgen), and the control antibody 02 is ABT-414 (purchased from Abbvie). Therefore, the epitope of the murine anti-75G7 needs to be further verified by experiments.

### Example 6 Analysis of binding specificity of EGFRvIII antibody

The EGFRvIII antibody can specifically recognize the activated EGFR, i.e., the mutant EGFRvIII and overexpressed wild-type EGFR in tumor tissues, but does not recognize the wild-type EGFR in normal tissues. Therefore, the antibody has an excellent target specificity, which can effectively avoid the harm of the antibody and conjugates thereof to human normal tissues, and effectively gather the antibody and conjugates thereof at the location of the tumor, thereby accurately and effectively killing tumor tissues, and significantly reducing the toxic and side effects of drugs on other normal organs.

### A. Binding specificity of EGFRvIII antibody to human normal tissue cell lines detected by FACS

Seven human normal tissue cell lines are respectively selected from different tissues, and see table 14 for details. The binding of the EGFRvIII antibody to the seven human normal tissue cell lines is detected and analyzed by FACS. The results as shown in figures 9A to 9G indicate that none of four EGFRvIII chimeric antibodies bind to human normal tissue cell lines, and only the chimeric antibody 75G7 weakly binds to HEB and WPMY-1. It is indicated that the four EGFRvIII chimeric antibodies all have a good tissue specificity and do not recognize the wild-type EGFR in normal tissues.

**Table 14 Cell lines derived from 7 different human normal tissues**

| Cell line name | Tissue source |
|---|---|
| HFF-1 | Skin |
| HFL-I | Lung |
| QSG-7701 | Liver |
| HEEC | Esophagus |
| HEB | Brain |
| WPMY-1 | Prostate |
| MCF-10A | Breast |

### B. Binding specificity of the EGFRvIII antibody detected by immunohistochemical staining (IHC)

The human glioma tissue chip (Cat #GL805d) and human multi-organ normal tissue chip (Cat #FDA999n) are both purchased from Alenabio, Co., Ltd. (Xi'an).

The experimental procedure of the immunohistochemical staining is as follows:
1) Baking the slices at 60°C for 2 hours. The purpose of baking the slices is to firmly stick the tissue slices with paraffin on the slide to prevent the slices from falling off during the staining. The purpose is achieved by placing the slices in an incubator at 56°C to 60°C for at least 1 hour; however, the usual temperature of baking slices is 8°C to 60°C and the time thereof is 2 to 6 hours. Since high-temperature drying may accelerate the oxidation of antigens in the tissues, baking slices at a high temperature has a destructive effect on antigens.
2) Performing deparaffinage and hydration. Soaking is carried out in xylene I and II for 15 to 30 minutes to remove the paraffin from the tissues. However, the xylene entering human tissues is immiscible with a water-soluble staining solution, which makes it necessary to gradually replace the xylene in the tissues with a descending gradient of ethanol. The slices are soaked respectively in 100% ethanol I and II for 5 minutes and respectively in 95%, 90%, 80% and 70% ethanol for 2 minutes, rinsed with PBS 3 times, with 3 minutes each time, and placed in distilled water for later use.
3) Antigen repair. The tissue chip is placed in an EDTA solution (pH 9.0) and repair is performed at high temperature for 5 minutes.
4) Blocking endogenous peroxidase. The tissue chip is placed in 3% H₂O₂ at room temperature for 5 minutes.
5) Blocking non-immune serum. The tissue chip is placed in 5% FBS at room temperature for 15 minutes. Antibodies can be adsorbed by charged collagen and connective tissue components in tissue slices, which causes background coloring, and in order to prevent this situation, it is best to select the non-immune serum derived from the same species as the secondary antibody to block the charge before treating the slices with specific antibodies, which prevents the primary antibody from binding to the charge, and inhibits the non-specific background coloring. A common method is to use 2% to 10% sheep serum or 2% to 5% bovine serum albumin for reaction at room temperature for 10 to 30 minutes.
6) Incubating the primary antibody and placing the primary antibody at room temperature for 1 hour.
7) Incubating the secondary antibody and placing the secondary antibody at room temperature for 0.5 hour.
8) Allowing DAB to develop color and placing same at room temperature for 5 minutes.
9) Counterstaining nucleus with hematoxylin and placing same at room temperature for 10 seconds.
10) Sealing for detection.

The results of the immunohistochemical staining are shown in figures 10A to F. The murine anti-80E11 is an antibody against the wild-type EGFR. From figures 10 A to F, it can be seen that the wild-type EGFR is expressed in both human glioma tissues and human multi-organ normal tissues, and the expression level in tumor tissues is higher than that in normal tissues. As shown in table 15, the positive rate of the murine anti-7E5, -64F1 and -63A10 in the human glioma tissue chip is 34%, and the reaction thereof with the human multi-organ normal tissue chip is basically negative, with only a few samples being weakly positive. The reactions of the murine anti-75G7 with the human glioma tissue chip and the human multi-organ normal tissue chip are both negative. The difference between the antigen binding epitope of the murine anti-75G7 and that of other antibodies may be due to the destruction of the antigen binding epitope of the murine anti-75G7 during chip processing. The tissue chips used in this experiment are all embedded in paraffin, which may not be suitable for detecting the murine anti-75G7 antibody. Then a freezing section IHC would be tried.

**Table 15 Response rate of EGFR antibody and EGFRvIII antibody to human glioma tissue chips**

| 70 GBM samples | Anti-EGFR (murine anti-80E11) | anti-EGFRvIII (murine anti-63A10) | anti-EGFRvIII (murine anti-64F1) | anti-EGFRvIII (murine anti-7E5-2) | anti-EGFRvIII (murine anti-75G7) |
|---|---|---|---|---|---|
| Positive samples | 70 | 24 | 24 | 24 | / |
| % positive | 100% | 34% | 34% | 34% | / |

It is proved in the FACS and IHC experiments that the EGFRvIII antibody can specifically recognize the EGFR in tumor tissues, but does not recognize the EGFR in normal tissues. Therefore, the antibody has an excellent target specificity, which can effectively avoid the harm of the antibody and conjugates thereof to human normal tissues, and effectively gather the antibody and conjugates thereof at the location of the tumor, thereby accurately and effectively killing tumor tissues, and significantly reducing the toxic and side effects of drugs on other normal organs.

### Example 7 Determination of amino acid sequences of light and heavy chain variable regions

Total RNA isolation: 5 × 10⁷ hybridoma cells obtained in example 1 are collected by centrifugation, added with 1 mL Trizol and mixed evenly, transferred to a 1.5 mL centrifuge tube, and placed at room temperature for 5 minutes. The tube is added with 0.2 mL of chloroform, shaken for 15 seconds, standing for 10 minutes, and then centrifuged at 4°C and at 12,000 g for 5 minutes, and the supernatant is taken and transferred to a new 1.5 mL centrifuge tube. 0.5 mL isopropanol is added, the liquid in the tube is mixed homogeneously and gently, then the tube is standing at room temperature for 10 minutes, is centrifuged at 4°C and at 12000 g for 15 minutes, then the supernatant is discarded. After adding 1 mL of 75% (v/v) ethanol, the precipitate is gently washed; then the tube is centrifuged at 4°C and at 12000 g for 5 minutes to discard the supernatant; the precipitate is dried in the air, and then the DEPC-treated H₂O is added to perform dissolution (promoting the dissolution at 55°C in water bath for 10 minutes) so as to obtain total RNA.

Reverse transcription and PCR: 1 µg of total RNA is taken to formulate a 20 µL system, to which a reverse transcriptase is added, for reacting at 42°C for 60 minutes and at 7°C for 10 minutes so as to stop the reaction. A 50 µL PCR system is formulated, comprising 1 µL cDNA, 25 pmol of each primer, 1 µL DNA polymerase, a corresponding buffer system, and 250 µmol dNTPs; the PCR program is set: pre-denaturating at 95°C for 3 minutes; denaturating at 95°C for 30 seconds; annealing at 55°C for 30 seconds; extending at 72°C for 35 seconds; after 35 cycles, further extending at 72°C for 5 minutes to obtain a PCR product. The kit used for reverse transcription is PrimeScript RT Master Mix, purchased from Takara (Cat #RR036); and the kit used for PCR includes a Q5 ultra-fidelity enzyme, purchased from NEB (Cat #M0492).

Cloning and sequencing: 5 µL of the PCR product is taken for an agarose gel electrophoresis detection, and the column recovery kit is used to purify the positive test samples, wherein the recovery kit is NucleoSpin® Gel & PCR Clean-up, purchased from MACHEREY-NAGEL (Cat #740609). The ligation reaction is performed as follows: 50 ng of the sample, 50 ng of the T vector, 0.5 µL of the ligase, 1 µL of the buffer, and 10 µL of the reaction system are reacted at 16°C for half an hour to obtain a ligation product. The ligated kit is a T4 DNA ligase, purchased from NEB (Cat #M0402); 5 µL of the ligation product is added to 100 µL of competent cells (Ecos 101 competent cells, purchased from Yeasten, Cat #FYE607), put into an ice bath for 5 minutes, then heat shocked in 42°C water bath for 1 minute, put back on ice for 1 minute, then added with 650 µL of the antibiotic-free SOC medium, and recovered at a speed of 200 RPM on a shaker at 37°C for 30 minutes. 200 µL of the mixture is taken and coated on an LB solid medium containing antibiotics, and incubated in a 37°C incubator overnight. The next day, primers M13F and M13R on the T vector are used to formulate 30 µL of the PCR system for a colony PCR, wherein the colony is dipped with a pipette tip to the PCR reaction system, and then is blown and sucked; and 0.5 µL is sucked out and placed on another LB solid culture dish containing 100 nM ampicillin to preserve the strain. After the PCR reaction is completed, 5 µL is taken for an agarose gel electrophoresis detection, and the positive samples are sequenced and analyzed (wherein the CDR is divided according to the Chothia definition system). The sequencing results are shown in tables 16 and 17.

**Table 16 Numbers of amino acid sequences of EGFRvIII antibody**

| Clone | Heavy chain protein | | | | Light chain protein | | | |
|---|---|---|---|---|---|---|---|---|
| number | Variable region | CDR1 | CDR 2 | CDR 3 | Variable region | CDR 1 | CDR 2 | CDR 3 |
| 75G7C6 | 1 | 2 | 3 | 4 | 5 | 6 | 7 | 8 |
| 63A10A7 | 9 | 10 | 11 | 12 | 13 | 14 | 15 | 16 |
| 64F1F8 | 17 | 18 | 19 | 20 | 21 | 22 | 23 | 24 |
| 7E5-2A9 | 25 | 26 | 27 | 28 | 29 | 30 | 31 | 32 |
| 43H6A1 | 33 | 34 | 35 | 36 | 37 | 38 | 39 | 40 |
| 46C4A6 | 41 | 42 | 43 | 44 | 45 | 46 | 47 | 48 |
| 49G12G5 | 49 | 50 | 51 | 52 | 53 | 54 | 55 | 56 |
| 51G7C2 | 57 | 58 | 59 | 60 | 61 | 62 | 63 | 64 |
| 53D8H4 | 65 | 66 | 67 | 68 | 69 | 70 | 71 | 72 |
| 54D2G10 | 73 | 74 | 75 | 76 | 77 | 78 | 79 | 80 |
| 56F10G2 | 81 | 82 | 83 | 84 | 85 | 86 | 87 | 88 |
| 59G3F12 | 89 | 90 | 91 | 92 | 93 | 94 | 95 | 96 |
| 64B11F2 | 97 | 98 | 99 | 100 | 101 | 102 | 103 | 104 |

The numbers in table 17 are the sequence numbers in the sequence listing. For example, the amino acid sequence of the heavy chain protein variable region of 43H6A1 is SEQ ID No.1, and the amino acid sequence of the CDR1 in the heavy chain protein variable region of 43H6A1 is SEQ ID No.2.

**Table 17 Numbers of nucleotide sequences of EGFRvIII antibody**

| Clone number | Heavy chain protein variable region | Light chain protein variable region |
|---|---|---|
| 75G7C6 | 105 | 106 |
| 63A10A7 | 107 | 108 |
| 64F1F8 | 109 | 110 |
| 7E5-2A9 | 111 | 112 |
| 43H6A1 | 113 | 114 |
| 46C4A6 | 115 | 116 |
| 49G12G5 | 117 | 118 |
| 51G7C2 | 119 | 120 |
| 53D8H4 | 121 | 122 |
| 54D2G10 | 123 | 124 |
| 56F10G2 | 125 | 126 |
| 59G3F12 | 127 | 128 |
| 64B11F2 | 129 | 130 |

The numbers in table 18 are the sequence numbers in the sequence listing. For example, the nucleotide sequence encoding the heavy chain protein variable region of 75G7C6 is SEQ ID No. 105.

### Example 8 Production and purification of EGFRvIII human-murine chimeric antibody

1) Plasmid construction and preparation: The heavy chain variable region sequence of the above-mentioned murine lead antibody is recombined into an expression vector containing a signal peptide and a human heavy chain antibody IgG1 constant region (wherein the expression vector is purchased from Invitrogen, and the recombination steps are also completed by Shanghai ChemPartner Co., Ltd.), and the light chain variable region sequence of the EGFRvIII antibody is recombined into an expression vector containing a signal peptide and a human antibody light chain kappa constant region (wherein the expression vector is purchased from Invitrogen, and the recombination steps are also completed by Shanghai ChemPartner Co., Ltd), thereby obtaining recombinant plasmids (for the experimental principles and steps of the above-mentioned plasmid recombination, see "Molecular Cloning: A Laboratory Manual (Third Edition)", (US) J. Sambrook et al.) which are verified by sequencing. An alkaline lysis kit (purchased from MACHEREY-NAGEL) is used for a medium-amount extraction of recombinant plasmids having a high purity, wherein the recombinant plasmids have a mass of at least 500 µg and filtered through a 0.22 µm filter membrane (purchased from Millopore) for transfection.
2) Cell transfection: 293E cells (purchased from Invitrogen) are cultured in a Freestyle 293 expression medium (purchased from Invitrogen). The shaker is set to 37°C, 130 RPM and 8% CO₂ (v/v). During transfection, the Freestyle 293 expression medium is added with 10% (v/v) F68 (purchased from Invitrogen) to a final F68 concentration of 0.1% (v/v), thereby obtaining a Freestyle 293 expression medium containing 0.1% (v/v) F68 (i.e., a medium A). 5 mL of the medium A and 200 µg/mL PEI (purchased from Sigma) are mixed evenly to obtain a medium B. 5 mL of the medium A and 100 µg of the recombinant plasmids (herein referred to the mixed recombinant plasmids obtained by mixing the above-mentioned heavy chain recombinant plasmid and light chain recombinant plasmid in a conventional equal proportion) obtained in step 1) are mixed evenly to obtain a medium C. After 5 minutes, the medium B and the medium C are combined and mixed evenly, and allowed to stand for 15 minutes to obtain a mixed solution D. 10 mL of the mixed solution D is slowly added to 100 mL of the Freestyle 293 expression medium containing 293E cells until the cell density of 293E is 1.5 × 10⁶ cells/mL, wherein the addition is performed with shaking to avoid excessive concentration of PEI, and the mixture is placed in a shaker for culture. The next day peptone is added to a final concentration of 0.5% (w/v). On days 5 to 7, the antibody titer of the culture broth is measured. On days 6 and 7, the supernatant is collected by centrifugation (3500 RPM, 30 minutes) and filtered through a 0.22 µm filter membrane to obtain the filtered cell supernatant for purification.
3) Antibody purification: The continuously produced endotoxin-free chromatography columns and protein A fillers are treated with 0.1 M NaOH for 30 minutes or rinsed with 5 column volumes of 0.5 M NaOH; the long-term unused column materials and chromatography columns are soaked with 1 M NaOH for at least 1 hour, rinsed with endotoxin-free water to neutrality, and washed with 10-fold column volumes of 1% Triton X100. 5 column volumes of PBS are used for equilibration, and the filtered cell supernatant is loaded onto the column, and the flow-through fluid is collected if necessary. After loaded onto the column, 5-fold column volumes of PBS are used for washing. 5-fold column volumes of 0.1 M Glycine-HCl (pH 3.0) is used for eluting, and the eluate is collected and neutralized with 1/10 volume of 1 M Tris-HCl (1.5 M NaCl) (pH 8.5). The antibodies are harvested, and then dialyzed in 1 × PBS overnight to avoid endotoxin contamination. After dialysis, the concentration is measured using a spectrophotometer or a kit; the purity of the antibodies is measured using HPLC-SEC; and the endotoxin content of the antibodies is detected using an endotoxin detection kit (purchased from Lonza).

Purified EGFRvIII chimeric antibodies 75G7, 63A10, 64F1 and 7E5 are obtained respectively.

The purified EGFRvIII chimeric antibody is detected and analyzed for protein concentration (A280/1.4), purity, endotoxin (Lonza kit), etc. The results as shown in table 18 indicate that the endotoxin concentration of the final antibody product is within 1.0 EU/mg.

**Table 18 Quality control of purified EGFRvIII chimeric antibody**

| Antibody name | Clone number | Antibody purity | Protein concentration (mg/ml) | Endotoxin (EU/mg) |
|---|---|---|---|---|
| Chimeric antibody 75G7 | 75G7C6 | > 95% | 1.44 | < 1.0 |
| Chimeric antibody 63A10 | 63A10A7 | > 95% | 1.26 | < 1.0 |
| Chimeric antibody 64F1 | 64F1F8 | > 95% | 1.02 | < 1.0 |
| Chimeric antibody 7E5 | 7E5-2A9 | > 95% | 1.81 | < 1.0 |

### Example 9 Detection and identification of EGFRvIII chimeric antibody

### A. Binding of the antibody to an EGFRvIII protein detected by an enzyme-linked immunosorbent assay (ELISA)

The purified EGFRvIII chimeric antibody obtained in example 2 is reacted with a human EGFRvIII-hFc protein.

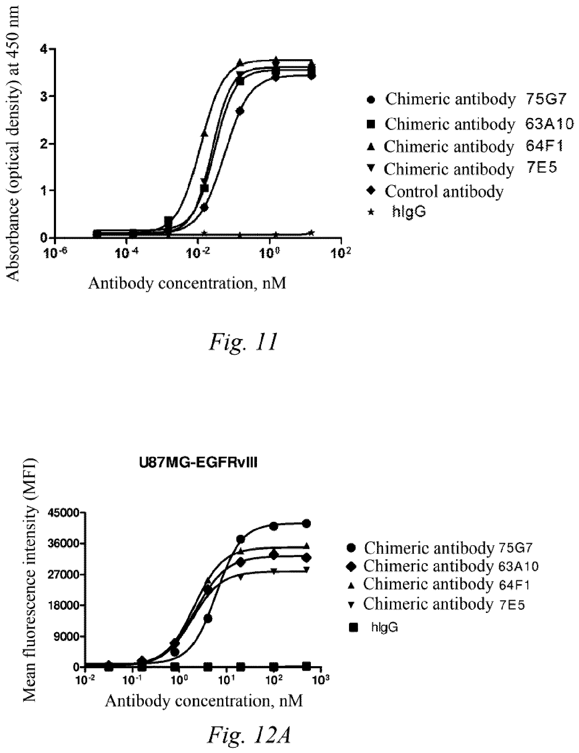

The purified immunogen A (EGFRvIII-hFc) obtained in example 1 (for the preparation method, see step (I) in example 1) is diluted with PBS to a final concentration of 1.0 µg/mL, and then added to a 96-well ELISA plate at 100 µl/well. The plate is sealed with a plastic film and incubated at 4°C overnight. The next day, the plate is washed twice with a plate washing solution [PBS + 0.01% (v/v) Tween20], and added with a blocking solution [PBS + 0.01% (v/v) Tween20 + 1% (w/w) BSA] for blocking 2 hours at room temperature. The blocking solution is discarded, and the purified EGFRvIII chimeric antibody obtained in example 2 is added at 100 µl/well. The plate is incubated for 2 hours at 37°C, and then washed 3 times with the plate washing solution [PBS + 0.01% (v/v) Tween20]. The plate is added with an HRP (horseradish peroxidase) labeled secondary antibody (purchased from Sigma), incubated at 37°C for 2 hours, and then washed 3 times with the plate washing solution [PBS + 0.01% (v/v) Tween20]. The plate is added with 100 µl of TMB substrate/well, incubated for 30 minutes at room temperature, and then added with 100 µl of stopping solution (1.0N HCl)/well. A450 nm value is read using an ELISA plate reader (SpectraMax 384plus, purchased from Molecular Device), and the results are shown in figure 11 and table 19. Table 19 shows that the purified antibody binds to the EGFRvIII recombinant protein at an ELISA level. The IgG control is a mouse IgG, and the data in the table is OD₄₅₀ₙₘ value.

**Table 19 Binding reaction of EGFRvIII chimeric antibody with human EGFRvIII-hFc protein detected by ELISA**

| OD450 nm | | Antibody concentration (nM) | | | | | | | |
|---|---|---|---|---|---|---|---|---|---|
| Antibody name | Clone number | 100 | 10 | 1 | 0.1 | 0.01 | 0.001 | 0.0001 | Blank |
| Chimeric antibody 75G7 | 75G7C6 | 3.44 | 3.41 | 2.69 | 0.65 | 0.15 | 0.08 | 0.07 | 0.08 |
| Chimeric antibody 63A10 | 63A10A7 | 3.55 | 3.55 | 3.33 | 1.05 | 0.37 | 0.11 | 0.07 | 0.06 |
| Chimeric antibody 64F1 | 64F1F8 | 3.70 | 3.78 | 3.72 | 2.23 | 0.35 | 0.11 | 0.08 | 0.08 |
| Chimeric antibody 7E5 | 7E5-2A9 | 3.60 | 3.63 | 3.45 | 1.18 | 0.19 | 0.08 | 0.09 | 0.07 |
| Control antibody | Positive control | 3.44 | 3.41 | 2.69 | 0.65 | 0.15 | 0.08 | 0.07 | 0.08 |
| hIgG | IgG control | 0.11 | 0.07 | 0.06 | 0.1 | 0.06 | 0.06 | 0.07 | 0.06 |

### B. Binding of antibody to EGFRvIII expressing cell detected by fluorescence activated cell sorting (FACS)

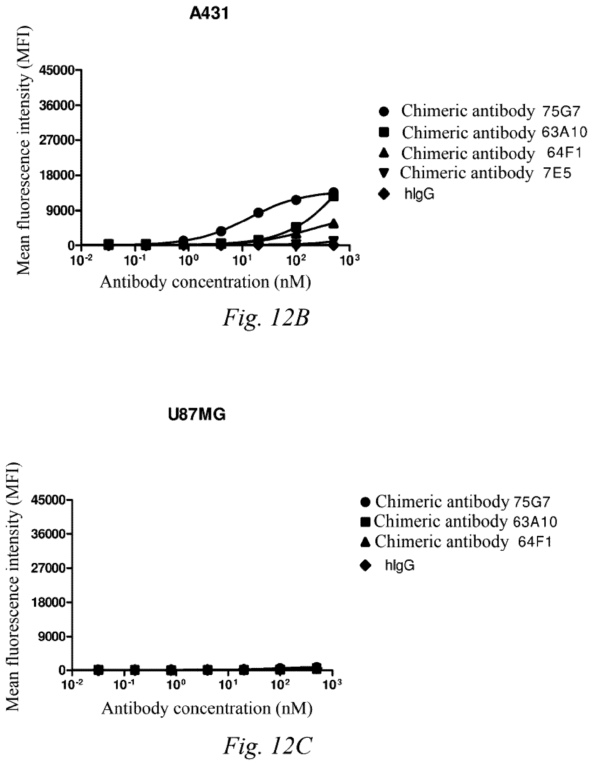

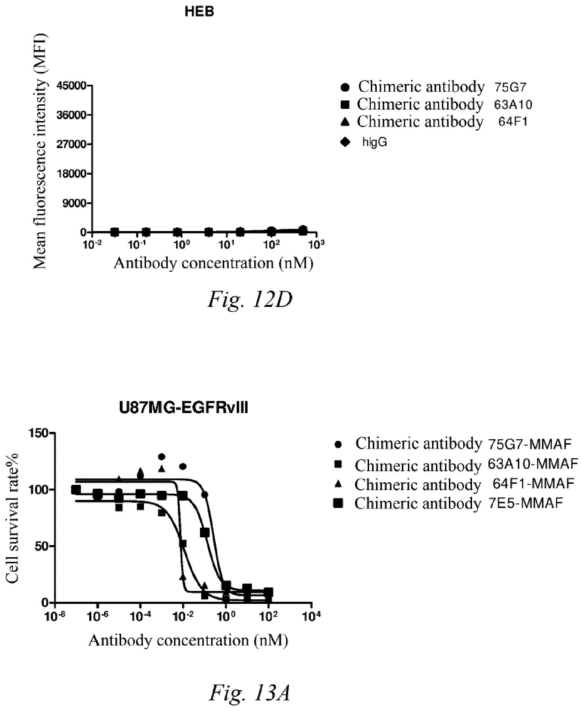

The desired cells are subjected to an expanded culture to 90% confluence in a T-75 cell culture flask; the medium is sucked out, and the cells are washed twice with a HBSS buffer (Hanks Balanced Salt Solution) (purchased from Invitrogen), and then treated with an enzyme-free cell dissociation solution (Versene solution: purchased from Life Technology) and collected. The cells are washed twice with the HBSS buffer and are counted, and then the cells are diluted with the HBSS buffer to 2 × 10⁶ cells/ml, added with 1% goat serum blocking solution, with the percentage being the mass percentage, and incubated on ice for 30 minutes, and then centrifugally washed twice with the HBSS buffer. The collected cells are suspended to 2 × 10⁶ cells/mL with an FACS buffer (HBSS + 1% BSA, with the percentage being the mass percentage), added to a 96-well FACS reaction plate at 100 µL/well, then added with 100 µL/well of the purified EGFRvIII antibody (samples to be tested) obtained in example 2, and incubated on ice for 2 hours. The plate is centrifugally washed twice with the FACS buffer, added with 100 µL/well of a fluorescence (Alexa 488) labeled secondary antibody (purchased from Invitrogen), and then incubated on ice for 1 hour. The plate is centrifugally washed 3 times with the FACS buffer, added with 100 µL/well of a fixing solution [4% (v/v) paraformaldehyde] for suspending the cells, and centrifugally washed twice with the FACS buffer after 10 minutes. The cells are suspended in 100 µL of the FACS buffer, and the results are detected and analyzed by FACS (FACS Calibur, purchased from BD). Data analysis is performed by using the software (CellQuest) to obtain the mean fluorescence intensity (MFI) of the cells. Data is further analyzed by using the software (GraphPad Prism5) to perform data fitting and calculate EC50. The analysis results as shown in table 20 and figures 12A to D indicate that the chimeric antibodies to be tested may bind to the EGFRvIII protein on the surface of U87MG-hEGFRvIII cells (Figure 12A). A431 cells (human epidermal cell cancer cell lines) have a large amount of wild-type EGFR protein expressed on the surface thereof. From figure 12B, it can be seen that chimeric antibodies 75G7, 63A10 and 64F1 may weakly bind to the wild-type EGFR protein on the surface of A431 cells, while the chimeric antibody 7E5 does not recognize the wild-type EGFR protein, and only specifically recognizes the mutant EGFRvIII protein. U87MG cells (human glioma cell lines) have a small amount of wild-type EGFR protein expressed on the surface thereof, while HEB cells (human glioma cell lines) have a high level of wild-type EGFR protein expression on the surface thereof. The antibodies to be tested have no binding reaction to U87MG and HEB cells, indicating that the chimeric antibodies 75G7, 63A10 and 64F1 have a good specificity, and only recognize the mutant EGFRvIII protein and overexpressed wild-type EGFR protein in tumor tissues, but do not recognize the wild-type EGFR protein in normal tissues (Figures 12C and 12D).

**Table 20 Binding reaction of EGFRvIII chimeric antibody with U87MG-hEGFRvIII, A431, U87MG and HEB cells detected by FACS**

| Antibody name | Antibody clone number | EC50, nM | | | |
|---|---|---|---|---|---|
| | | U87MG-hEGFRvIII | A431 | HEB | U87MG |
| Chimeric antibody 75G7 | 75G7C6 | 6.1 | 13.4 | Negative | Negative |
| Chimeric antibody 63A10 | 63A10A7 | 2.2 | 696.0 | Negative | Negative |
| Chimeric antibody 64F1 | 64F1F8 | 2.1 | 180.4 | Negative | Negative |
| Chimeric antibody 7E5 | 7E5-2A9 | 1.9 | Negative | Negative | Negative |

### Example 10 Cell killing activity experiment of EGFRvIII chimeric antibody-drug conjugate

The antibody is dialyzed with a sodium borate buffer at Ph 6.5 to 8.5, and then a certain proportion of TCEP is added thereto (with TCEP/antibody ratio being 2-10); reduction is performed for 2 to 4 hours at room temperature or 37°C, and excess TCEPs are removed through a G25 desalting filler, and a certain proportion of MC-MMAF (with drug/antibody molar ratio being 4.5) is added and reacted for 4 hours. Then cysteine is added to neutralize excess drugs, and excess small molecules are removed through G25. A purified antibody-drug conjugate is afforded (for the coupling method, see Doronina, 2006, Bioconjugate Chem.17,114-124). The cytotoxic activity is analyzed after HIC analysis of drug cross-linking rate, purity and other parameters. For comparison, the drug cross-linking rate of all antibody conjugates is 8 [the DAR (drug-antibody coupling ratio) is adjusted through experiments to reach the preset value, and the exact DAR is detected and determined later].

The resulting antibody conjugates are respectively subjected to gradient dilution with a complete medium, and a 96-well cell culture plate is added with 90 µL of the U87MG-EGFRvIII cell suspension at 1000 cells/well, and to each well is further added 10 µL of different concentrations of antibody-drug conjugate dilutions; after continuing the culture for 5 days, the cell viability is detected using the CellTiter-Glo kit (purchased from Promega, and the usage method refers to the product instructions).

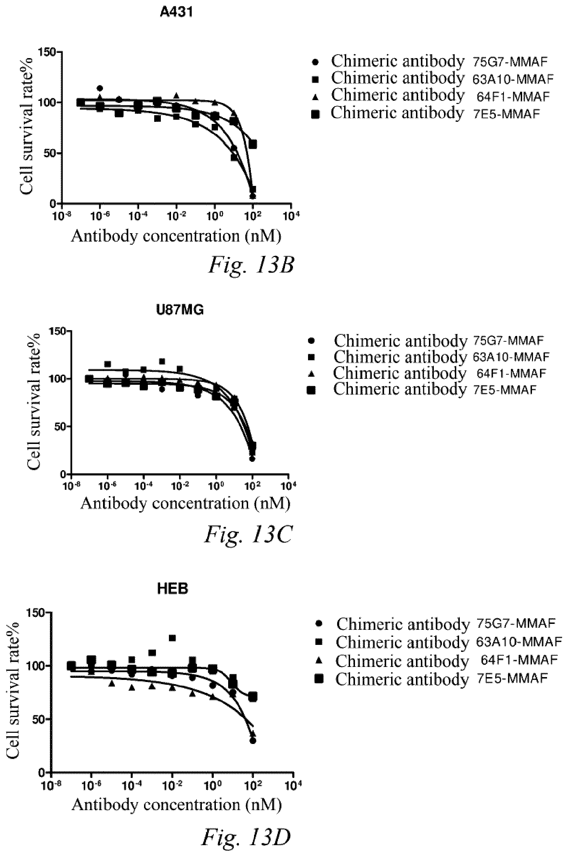

The results are as shown in table 21 and figures 13A to D, wherein IC50 in table 23 refers to the median effective dose of cells whose activity is inhibited after drug actions, and the cell killing activity can be reflected by detecting the cell activity. Figure 13 shows the detection of the cell killing activity of the antibody-drug conjugate on the recombinant tumor cell line U87MG-EGFRvIII positive for EGFRvIII. The results show that in an in-vitro experiment, the antibodies to be tested all can produce a good killing effect on U87MG-hEGFRvIII cells having a mutant EGFRvIII protein expressed on the surface thereof (Figure 13A). A431 cells (human epidermal cell cancer cell lines) have a large amount of wild-type EGFR protein expressed on the surface thereof. From figure 13B, it can be seen that chimeric antibodies 75G7, 63A10 and 64F1 have a weaker killing effect on A431. U87MG cells (human glioma cell lines) have a small amount of wild-type EGFR protein expressed on the surface thereof, while HEB cells (human glioma cell lines) have a high level of wild-type EGFR protein expression on the surface thereof. The chimeric antibodies 75G7, 63A10 and 64F1 have weak or no killing effect on U87MG and HEB cells, see figure 13C and figure 13D. It is indicated that the chimeric antibodies 75G7, 63A10 and 64F1 have a good specificity, can specifically kill tumor tissues expressing the mutant EGFRvIII protein and overexpressing the wild-type EGFR protein, but have no killing effect on normal tissues expressing the wild-type EGFR protein.

**Table 21 Killing effect of chimeric antibody conjugates on EGFRvIII positive cells detected by a cell killing experiment**

| Antibody conjugate | Antibody clone number | DAR | IC50, nM | | | |
|---|---|---|---|---|---|---|
| | | | U87MG-hEGFRvIII | A431 | HEB | U87MG |
| Chimeric antibody 75G7-MMAF | 75G7C6 | 3.40 | 0.27 | Weakly positive | Negative | Negative |
| Chimeric antibody 63A10-MMAF | 63A10A7 | 3.10 | 0.01 | Weakly positive | Negative | Negative |
| Chimeric antibody 64F1-MMAF | 64F1F8 | 4.05 | 0.01 | Weakly positive | Negative | Negative |
| Chimeric antibody 7E5-MMAF | 7E5-2A9 | 3.51 | 0.04 | Negative | Negative | Negative |

### Example 11 In vivo pharmacodynamic study of EGFRvIII chimeric antibody-drug conjugate

Since the expression of EGFRvIII genes may gradually be lost during the *in vitro* cultivation of tumor cells, there is no endogenous tumor cell line that stably expresses the EGFRvIII gene. Therefore, the U87MG-EGFRvIII recombinant cell line is constructed and the U87MG-EGFRvIII subcutaneous xenograft tumor model is established to test the *in vivo* efficacy of the chimeric antibody-drug conjugate.

See example 1 for the construction method of the U87MG-EGFRvIII recombinant cell line. 200 µl (1 × 10⁶ cells) of the U87MG-EGFRvIII cell suspension is inoculated on the right back of Balb/c nude mice; after 7 days, tumor grew to 150 - 250 mm³, and then tumor-bearing mice with appropriate tumor volume are selected, randomly grouped according to the tumor volume before administering drugs per the established schedule. See table 22 for specific research plan and administration dose.

The experimental indicators are to investigate whether tumor growth may be inhibited, delayed or cured. The next day the diameter of the tumor is measured with a vernier caliper. The computational formula of the tumor volume is: V = 0.5 × a × b², where a and b represent the long diameter and short diameter of the tumor, respectively.

The tumor suppressive effect (TGI) of the compounds is evaluated by TGI (%). TGI% = 1 - (TRTVn - TRTV1)/(CRTVn - CRTV1) × 100% (TRTVn: RTV on the last day of the treatment group; CRTV: RTV in the negative control group). The computational formula of the relative tumor volume RTV is RTV = Vt/V0, wherein V0 is the tumor volume measured during administration in different cages (Day 0), and Vt is the tumor volume at each measurement. The percentage value of T/C (%) reflects the tumor growth inhibition rate; according to guiding principles regarding anti-tumor drugs of Center for Drug Evaluation, TGI% ≤ 58% indicates that the drug is effective.

**Table 22 In vivo pharmacodynamic study of EGFRvIII chimeric antibody-drug conjugates**

| Groups | Numbers of animals | Treatment group | Dose (unit: mg/kg) | Injection volume | Route of administration | Administration schedule |
|---|---|---|---|---|---|---|
| 1 | 8 | Solvent control | -- | 10 µl/g | Tail vein injection i.v | Dosing every 4 days × 4 times |
| 2 | 8 | Control antibody 02-MMAE | 3 | 10 µl/g | Tail vein injection i.v | Dosing every 4 days × 4 times |
| 3 | 8 | Control antibody 02-MMAE | 1 | 10 µl/g | Tail vein injection i.v | Dosing every 4 days × 4 times |
| 4 | 8 | Chimeric antibody 63A10-MMAE | 3 | 10 µl/g | Tail vein injection i.v | Dosing every 4 days × 4 times |
| 5 | 8 | Chimeric antibody 63A10-MMAE | 1 | 10 µl/g | Tail vein injection i.v | Dosing every 4 days × 4 times |
| 6 | 8 | Chimeric antibody 75G7-MMAE | 3 | 10 µl/g | Tail vein injection i.v | Dosing every 4 days × 4 times |
| 7 | 8 | Chimeric antibody 75G7-MMAE | 1 | 10 µl/g | Tail vein injection i.v | Dosing every 4 days × 4 times |
| 8 | 8 | Chimeric antibody 64F1-MMAE | 3 | 10 µl/g | Tail vein injection i.v | Dosing every 4 days × 4 times |
| 9 | 8 | Chimeric antibody 64F1-MMAE | 1 | 10 µl/g | Tail vein injection i.v | Dosing every 4 days × 4 times |
| 10 | 8 | Chimeric antibody 7E5-MMAE | 3 | 10 µl/g | Tail vein injection i.v | Dosing every 4 days × 4 times |
| 11 | 8 | Chimeric antibody 7E5-MMAE | 1 | 10 µl/g | Tail vein injection i.v | Dosing every 4 days × 4 times |

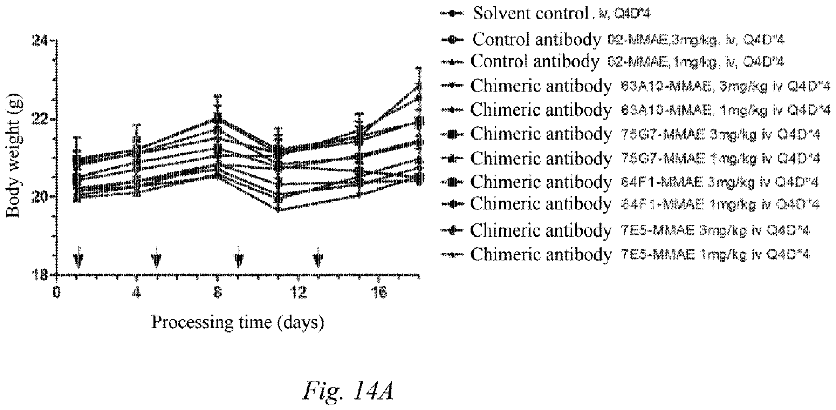

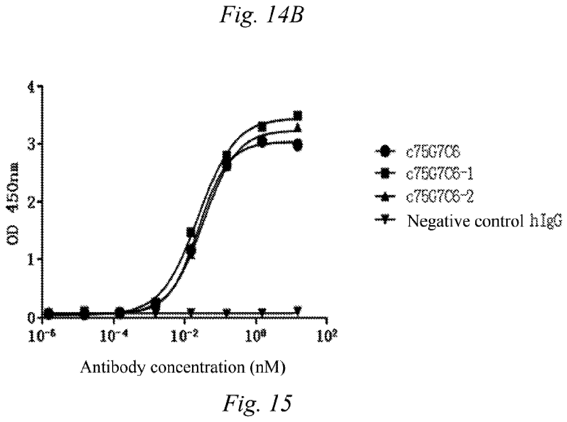

The results are shown in figure 14A and figure 14B: There is no significant change in body weight of all tested mice, indicating that all tested antibody-drug conjugates are well tolerated in U87MG-EGFRvIII xenograft tumor-bearing mice. Compared with the untreated solvent control group, the tumor volume of mice in all groups administrated with antibody-drug conjugates are significantly inhibited; and compared with the low-dose group (1 mg/kg), the high-dose group (3 mg/kg) has a marked dose dependence. In the high-dose group (3 mg/kg), after 18 days of administration (administered every 4 days for a total of 4 times), tumor growth is completely inhibited (TGI% > 100%). The low-dose group (1 mg/kg) also showed a good anti-tumor activity (TGI% > 58%). 4 chimeric antibody-drug conjugates 63A10-MMAE, 75G7-MMAE, 64F1-MMAE and 7E5-MMAE tested in therein all showed a better anti-tumor activity than the control antibody-drug conjugate (control antibody 02-MMAE), with 63A10-MMAE being the best.

### Example 12 Inhibition of introduction of mutations for deamidation, isomerization, and hydrolysis reactions

By analyzing the antibody sequences of 75G7C6 and 63A10A7, it is found that there is a possibility of deamidation reaction of NG at positions 54 and 55 of the CDR2 (SEQ ID No. 3) and isomerization of DG at positions 98 and 99 of the CDR3 (SEQ ID No. 4) in the heavy chain variable region (SEQ ID No. 1) of the 75G7C6 antibody. In order to suppress deamidation, isomerization and hydrolysis, upon calculation and analysis, the NG located in the CDR2 of the 75G7C6 antibody is mutated to NA, and the amino acid sequence of the heavy chain variable region of the 75G7C6 antibody after mutation is as shown in SEQ ID No. 179; or the NG is mutated to QG, and the amino acid sequence of the heavy chain variable region of the 75G7C6 antibody after mutation is as shown in SEQ ID No. 180. Moreover, the DG located in the CDR3 is mutated to SG, and the amino acid sequence of the heavy chain variable region of the 75G7C6 antibody after mutation is as shown in SEQ ID No. 181 (the corresponding nucleic acid sequence is as shown in SEQ ID No. 188); or the DG is mutated to EG, and the amino acid sequence of the heavy chain variable region of the 75G7C6 antibody after mutation is as shown in SEQ ID No. 182 (the corresponding nucleic acid sequence is as shown in SEQ ID No. 131); or the DG is mutated to DA, and the amino acid sequence of the heavy chain variable region of the 75G7C6 antibody after mutation is as shown in SEQ ID No. 183 (the corresponding nucleic acid sequence is as shown in SEQ ID No. 132). Amino acid modifications of removing asparagine residues and aspartoyl residues that are used as sites undergoing deamidation, are intended to be performed by means of the above-mentioned site-directed mutations.

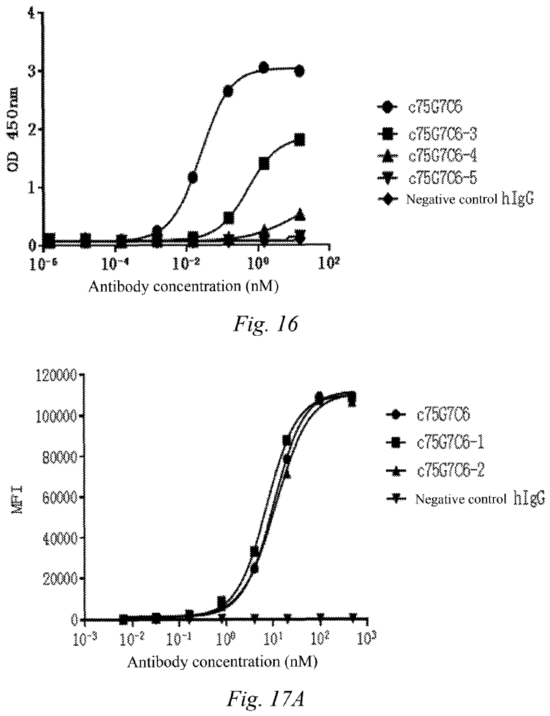

After the site-directed mutant sequences of the heavy chain variable region of the chimeric 75G7C6 antibody are genetically synthesized, plasmid construction, cell transfection, and antibody purification are performed as described in example 8. The purified antibodies are identified for the mutant chimeric antibody binding activity by ELISA and FACS as described in example 9. ELISA identification results are shown in figure 15 and figure 16 and table 23, wherein figure 15 is the ELISA binding activity identification of antibodies after the mutation of the NG of the CDR2 in the heavy chain variable region of the 75G7C6 antibody; figure 16 is the ELISA binding activity identification of antibodies after the mutation of the NG of the CDR3 in the heavy chain variable region of the 75G7C6 antibody; table 23 summarizes the EC50 values of the c75G7C6 wild-type antibody and mutant antibody binding to the EGFRvIII-hFc protein. As can be seen from table 23, the binding activity in EGFRvIII-hFc of the antibody mutants c75G7C6-1 and c75G7C6-2 after the NG at heavy chain CDR2 is mutated to NA or QG is close to that of the wild-type antibody c75G7C6, indicating that the mutation of NG to NA or QG does not affect the binding to EGFRvIII-hFc of the antibodies. However, whether the DG located in CDR3 is mutated to EG, SG or DA, it affects the binding activity in EGFRvIII-hFc, wherein the mutant antibody c75G7C6-3 with DG mutated to EG has a 22-fold decrease in binding ability to EGFRvIII-hFc compared to the wild-type chimeric antibody c75G7C6, and the mutant antibodies c75G7C6-4 and c75G7C6-5 with DG mutated to SG or DA do not bind to the EGFRvIII-hFc, indicating that the mutation of DG to EG, SG or DA seriously affects the binding to EGFRvIII-hFc of the antibodies.

**Table 23. Binding reaction of c75G7C6 chimeric antibody mutant with human EGFRvIII-hFc protein detected by ELISA**

| Antibody name | Point mutation | EC50 (unit: nM) | Maximum OD450 value |
|---|---|---|---|
| hIgG | None | None | 0.08 |
| c75G7C6 | wt (NG & DG) | 0.02 | 3.03 |
| c75G7C6-1 | NG/NA | 0.02 | 3.44 |
| c75G7C6-2 | NG/QG | 0.03 | 3.23 |
| c75G7C6-3 | DG/EG | 0.54 | 1.86 |
| c75G7C6-4 | DG/SG | 7.94 | 0.79 |
| c75G7C6-5 | DG/DA | None | 0.14 |

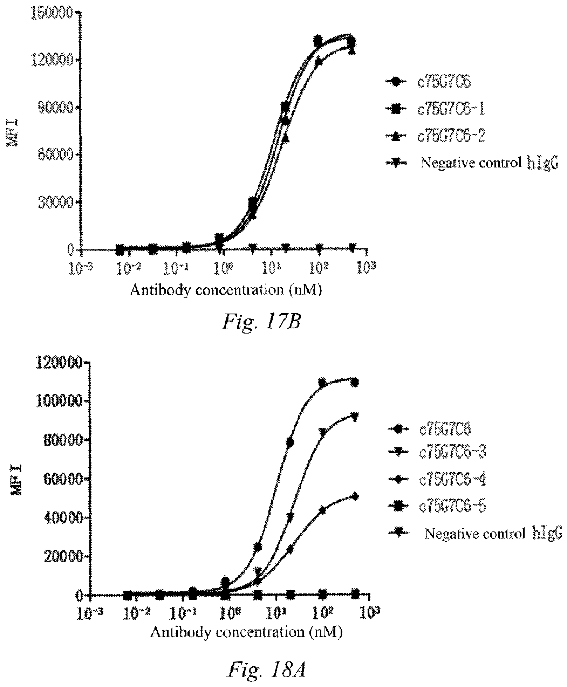

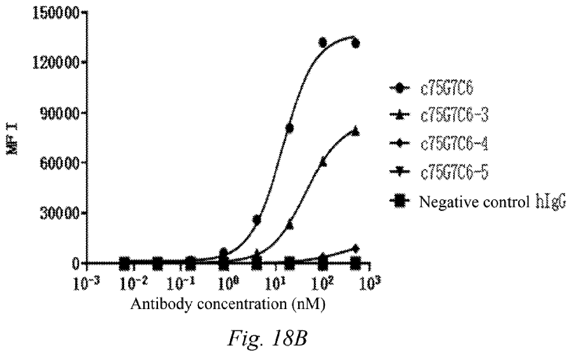

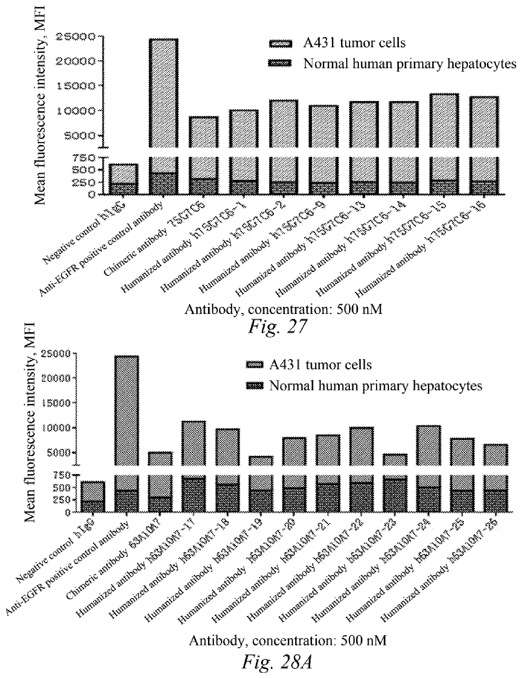

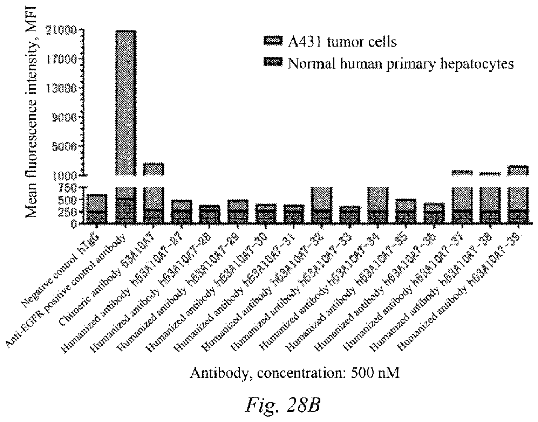

FACS identification results are as shown in figures 17A-B and 28A-B and tables 24 and 25, wherein figure 17A shows the detection of the binding activity in CHOK-EGFRvIII cells of the mutant antibodies c75G7C6-1 and c75G7C6-2 after the NG of the CDR2 in the heavy chain variable region of the 75G7C6 antibody is mutated to NA or QG and the wild-type antibody c75G7C6; figure 17B shows the detection of the binding activity in CHOK-EGFR cells of the mutant antibodies c75G7C6-1 and c75G7C6-2 after the NG of the CDR2 in the heavy chain variable region of the 75G7C6 antibody is mutated to NA or QG and the wild-type antibody c75G7C6; Figure 18A shows the detection of the binding activity in CHOK-EGFRvIII cells of the mutant antibodies c75G7C6-3, c75G7C6-4 and c75G7C6-5 after the DG of the CDR3 in the heavy chain variable region of the 75G7C6 antibody is mutated to SG, EG or DA and the wild-type antibody c75G7C6; figure 18B shows the detection of the binding activity in CHOK-EGFR cells of the mutant antibodies c75G7C6-3, c75G7C6-4 and c75G7C6-5 after the DG of the CDR3 in the heavy chain variable region of the 75G7C6 antibody is mutated to SG, EG or DA and the wild-type antibody c75G7C6. Table 24 summarizes the binding activity in CHOK-EGFRvIII cells of the c75G7C6 wild-type antibodies and mutant antibodies, and table 25 summarizes the binding activity in CHOK-EGFR cells of the c75G7C6 wild-type antibodies and mutant antibodies.

**Table 24. Binding reaction of c75G7C6 chimeric antibody mutants with CHOk1-EGFRvIII cells detected by FACS**

| Antibody name | Point mutation | EC50 (unit: nM) | Maximum mean fluorescence intensity |
|---|---|---|---|
| c75G7C6 | Wild type (NG & DG) | 10.5 | 111769 |
| c75G7C6-1 | NG/NA | 7.452 | 109947 |
| c75G7C6-2 | NG/QG | 11.64 | 111041 |
| c75G7C6-3 | DG/EG | 24.82 | 94071 |
| c75G7C6-4 | DG/SG | 24 | 52584 |
| c75G7C6-5 | DG/DA | None | 296 |
| Negative control hIgG | None | None | 132.6 |

**Table 25. Binding reaction of c75G7C6 chimeric antibody mutants with CHOk1-EGFR cells detected by FACS**

| Antibody name | Point mutation | EC50 (unit: nM) | Maximum mean fluorescence intensity |
|---|---|---|---|
| c75G7C6 | Wild type (NG & DG) | 14.22 | 137153 |
| c75G7C6-1 | NG/NA | 11.27 | 134462 |
| c75G7C6-2 | NG/QG | 16.92 | 130112 |
| c75G7C6-3 | DG/EG | 44.82 | 84723 |
| c75G7C6-4 | DG/SG | None | 8968 |
| c75G7C6-5 | DG/DA | None | 121 |
| Negative control hIgG | None | None | 144 |

As can be seen from table 24 and figures 17A and 18A, the binding activity in CHOk1-EGFRvIII cells of the mutant antibodies c75G7C6-1 and c75G7C6-2 after the NG located at the CDR2 in the heavy chain variable region of the c75G7C6 is mutated to NA or QG is close to that of the wild-type antibody c75G7C6, indicating that the mutation of NG to NA or QG does not affect the binding of the antibody to CHOK-EGFRvIII cells. However, whether the DG located in CDR3 is mutated to EG, SG or DA, it affects the binding activity in CHOK-EGFRvIII of the antibody, wherein the mutant antibodies c75G7C6-3 and c75G7C6-4 with DG mutated to EG or SG has a 2.5-fold decrease in binding ability in CHOK-EGFRvIII compared to the wild-type chimeric antibody c75G7C6, and has a decrease by 16% and 53% respectively in maximum mean fluorescence intensity; the mutant antibody c75G7C6-5 with DG mutated to DA does not bind to CHOK-EGFRvIII, indicating that the mutation of DG to EG, SG or DA seriously affects the binding of the antibody to CHOK-EGFRvIII cells.

As can be seen from table 25 and figures 17B and 18B, the binding activity in CHOk1-EGFR cells of the mutant antibodies c75G7C6-1 and c75G7C6-2 after the NG located at the CDR2 in the heavy chain variable region of the c75G7C6 is mutated to NA or QG is close to that of the wild-type antibody c75G7C6, indicating that the mutation of NG to NA or QG does not affect the binding of the antibody to CHOK-EGFR cells. However, whether the DG located in CDR3 is mutated to EG, SG or DA, it affects the binding activity in CHOK-EGFR of the antibody, wherein the mutant antibody c75G7C6-3 with DG mutated to EG has a 3-fold decrease in binding ability in CHOK-EGFR compared to the wild-type chimeric antibody c75G7C6, and has a decrease by 38% in maximum mean fluorescence intensity; the mutant antibodies c75G7C6-4 and c75G7C6-5 with DG mutated to SG or DA weakly bind to or do not bind to CHOK-EGFR, indicating that the mutation of DG to EG, SG or DA seriously affects the binding of the antibody to CHOK-EGFR cells.

As can be seen from the above-mentioned ELISA and FACS results, the NG located at the CDR2 in the heavy chain variable region of the c75G7C6 can be mutated to NA or QG, and the binding activity in antigens of the mutated antibodies is not affected; however, whether the DG located in CDR3 is mutated to EG, SG or DA, it affects the binding of the antibodies to antigens, in which case mutation is not suitable. Therefore, the antibody c75G7C6-1 with NG of the CDR2 mutated to NA will be selected for follow-up study.

### Example 13 Preparation of humanized EGFRvIII antibody

The heavy and light chain variable region templates of human germline antibodies that best match the non-CDR regions of the above-mentioned chimeric antibodies 63A10A7 and 75G7C6 are selected from the Germline database. The human acceptable sequences of the humanized EGFRvIII antibody are selected from human germline exons V_{H}, J_{H}, Vₖ and Jₖ sequences. The template of the heavy chain variable region of the 63A10A7 antibody is IGHV1-46^{∗}01 of the human germline antibody heavy chain exon V_{H}, and J_{H}-4 of the exon J_{H}; the template of the light chain variable region is IGKV1-39^{∗}01 of the human germline antibody light chain exon V_{K}, and J_{K}-2 of the exon J_{K}. The template of the heavy chain variable region of the 75G7C6 antibody is IGHV1-46^{∗}01 of the human germline antibody heavy chain exon V_{H}, and J_{H}-6b of the exon J_{H}; and the template of the light chain variable region is IGKV3-11^{∗}01 of the human germline antibody light chain exon V_{K}, and J_{K}-4 of the exon J_{K}.

The heavy chain and light chain CDRs of the chimeric antibodies 63A10A7 and 75G7C6 determined according to the Kabat definition are grafted into the selected human germline templates, respectively, to replace the CDR regions of the human germline templates so as to obtain a humanized antibody. Then, based on the three-dimensional structure of the murine antibody, the residues that embed residues and directly interact with the CDR regions, and the residues in framework regions that have an important influence on the conformations of VL and VH are subjected to back mutation to obtain a humanized antibody. Briefly, synthetic overlapping oligonucleotides across the humanized V_{H} or V_{L} domain are generated, and the PCR overlap extension is used to assemble each domain. The restriction site incorporated into the PCR product is used to directionally clone the V_{H} domain into an expression vector containing a signal peptide and a human antibody heavy chain IgG1 constant region, and to directionally clone the V_{L} domain into an expression vector containing a signal peptide and a human antibody light chain kappa constant region, thereby obtaining recombinant plasmids which are verified by sequencing; an alkaline lysis kit (purchased from MACHEREY-NAGEL) is used for a medium-amount extraction of the recombinant plasmids having a high purity, wherein the recombinant plasmids have a mass of at least 500 µg and filtered through a 0.22 µm filter membrane (purchased from Millopore) for transfection.

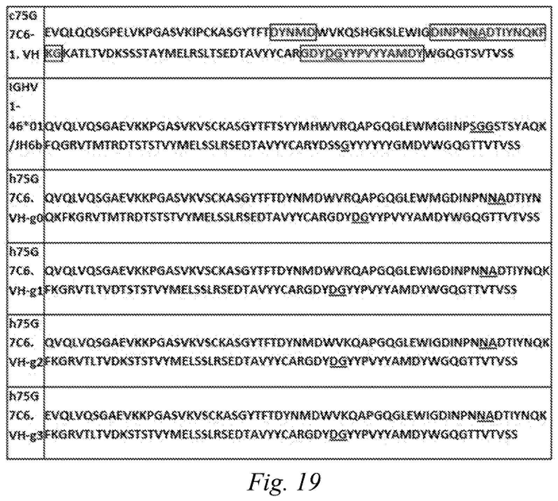

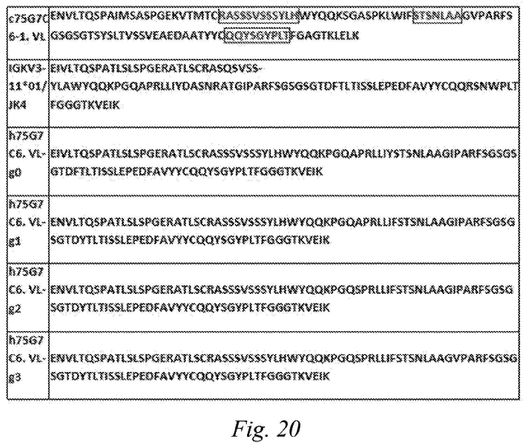

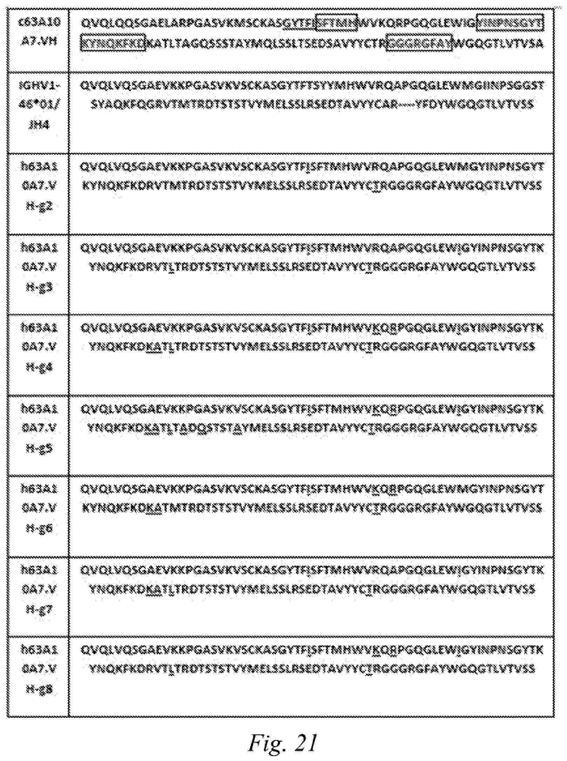

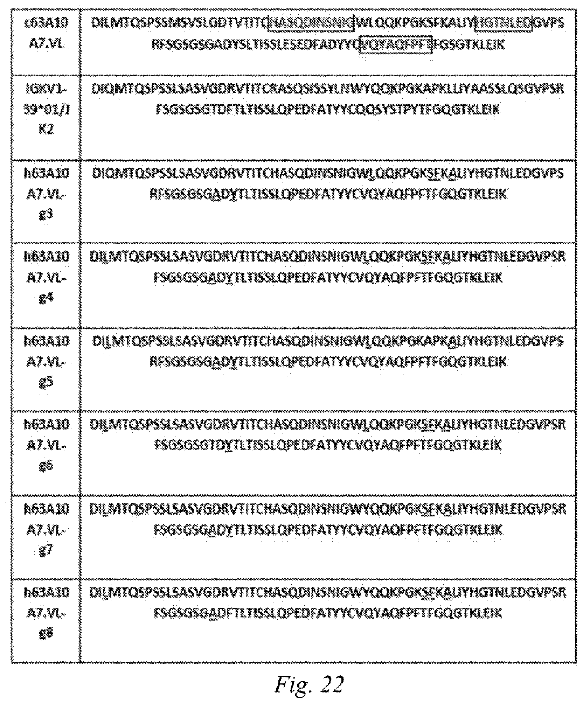

The sequence alignments of the heavy and light chain variable regions of the humanized anti-EGFRVIII antibody variants with the human germline heavy and light chain variable regions and the heavy and light chain variable regions of chimeric antibodies are as shown in figures 19-22, wherein figure 19 is the sequence alignment of the heavy chain variable region h75G7C6.VH of the humanized anti-EGFRvIII antibody h75G7C6 and variants thereof with the chimeric antibody c75G7C6.VH and the human germline VH exon IGHV1-46^{∗}01/JH6b; figure 20 is the sequence alignment of the light chain variable region h75G7C6.VL of the humanized anti-EGFRvIII antibody h75G7C6 and variants thereof with the chimeric antibody c75G7C6.VL and the human germline VL exon IGHV3-11^{∗}01/JK4; figure 21 is the sequence alignment of the heavy chain variable region h63A10A7.VH of the humanized anti-EGFRvIII antibody h63A10A7 and variants thereof with the chimeric antibody c63A10A7.VH and the human germline VH exon IGHV1-46^{∗}01/JH4; figure 22 is the sequence alignment of the light chain variable region h63A10A7.VL of the humanized anti-EGFRvIII antibody h63A10A7 and variants thereof with the chimeric antibody c63A10A7.VL and the human germline VL exon IGKV1-39^{∗}01/JK2, with CDRs in the box.
Human germline heavy chain variable region template IGHV1-46^{∗}01/JH4
   IGHV1-46^{∗}01: 
QVQLVQSGAEVKKPGASVKVSCKASGYTFTSYYMHWVRQAPGQGLEWMGIINPSGG
STSYAQKFQGRVTMTRDTSTSTVYMELSSLRSEDTAVYYCAR (SEQ ID NO. 175);
   JH4: YFDYWGQGTLVTVSS (SEQ ID NO. 189).
Human germline heavy chain variable region template IGHV1-46^{∗}01/JH6b
   IGHV1-46^{∗}01: 
QVQLVQSGAEVKKPGASVKVSCKASGYTFTSYYMHWVRQAPGQGLEWMGIINPSGG
STSYAQKFQGRVTMTRDTSTSTVYMELSSLRSEDTAVYYCAR（SEQ ID NO. 176)
   JH6b: YYYYYGMDVWGQGTTVTVSS (SEQ ID NO. 190)
Human germline light chain variable region template IGKV3-11^{∗}01/JK4
   IGKV3-11^{∗}01: 
EIVLTQSPATLSLSPGERATLSCRASQSVSSYLAWYQQKPGQAPRLLIYDASNRATGIPA
RFSGSGSGTDFTLTISSLEPEDFAVYYCQQRSNWP（SEQ ID NO. 177）;
   JK4: LTFGGGTKVEIK (SEQ ID NO. 191).
Human germline light chain variable region template IGKV1-39^{∗}01/JK2
   IGKV1-39^{∗}01: 
DIQMTQSPSSLSASVGDRVTITCRASQSISSYLNWYQQKPGKAPKLLIYAASSLQSGVPS
RFSGSGSGTDFTLTISSLQPEDFATYYCQQSYSTP（SEQ ID NO. 178）;
   JK2: YTFGQGTKLEIK (SEQ ID NO. 192).

Several framework positions are selected to reintroduce mouse donor residues. Several humanized 75G7C6 and 63A10A7 variants may be generated by incorporating different combinations of mouse framework donor residues into the VH domain or of human CDR residues into the VL domain. Table 26 and table 27 summarize these variants. Table 26 and table 27 show the variable regions but not constant regions of these variants. In tables 26 and 24, beginning with c represents a chimeric antibody, and beginning with h represents a humanized antibody; in the column of donor framework residues and back mutations, for example, "Q1E" in the heavy chain variable region h75G7C6.VH of the humanized anti-EGFRvIII antibody 75G7C6 and variants thereof means that the amino acid at position 1 as shown in figure 19 is mutated from "Q" glutamine to "E" glutamic acid, and the site of back mutation is located in the framework region, and description thereof will not be repeated.

**Table 26**

| Antibody number | Heavy chain name | Human germline antibody heavy chain variable region template VH | Donor framework residues and back mutations | Heavy chain variable region SEQ ID NO. | Light chain name | Human germline antibody light chain variable region template Vk | Donor framework residues and back mutations | Light chain variable region SEQ ID NO. |
|---|---|---|---|---|---|---|---|---|
| c75G7C6 | VH | None | CDR | 1 | VL | None | CDR | 5 |
| h75G7C6-1 | VH-g3 | IGHV1-46^{∗}01/JH6 b | CDR-grafted, Q1E, R38K, M48I, M70L, R72V, T74K | 133 | VL-g0 | IGKHV3-11^{∗}01/JK4 | CDR-grafted, | 137 |
| h75G7C6-2 | VH-g3 | IGHV1-46^{∗}01/JH6 b | CDR-grafted, Q1E, R38K, M48I, M70L, R72V, T74K | 133 | VL-gl | IGKHV3-11^{∗}01/JK4 | CDR-grafted, I2N, Y50F, F72Y | 138 |
| h75G7C6-3 | VH-g3 | IGHV1-46^{∗}01/JH6 b | CDR-grafted, Q1E, R38K, M48I, M70L, R72V, T74K | 133 | VL-g2 | IGKHV3-11^{∗}01/JK4 | CDR-grafted, I2N, A44S, Y50F, F72Y | 139 |
| h75G7C6-4 | VH-g3 | IGHV1-46^{∗}01/JH6 b | CDR-grafted, Q1E, R38K, M48I, M70L, R72V, T74K | 133 | VL-g3 | IGKHV3-11^{∗}01/JK4 | CDR-grafted, I2N, A44S, Y50F, I59V, F72Y | 140 |
| h75G7C6-5 | VH-g0 | IGHV1-46^{∗}01/JH6 b | CDR-grafted, | 134 | VL-g0 | IGKHV3-11^{∗}01/JK4 | CDR-grafted, | 137 |
| h75G7C6-6 | VH-g0 | IGHV1-46^{∗}01/JH6 b | CDR-grafted, | 134 | VL-g1 | IGKHV3-11^{∗}01/JK4 | CDR-grafted, I2N, Y50F, F72Y | 138 |
| h75G7C6-7 | VH-g0 | IGHV1-46^{∗}01/JH6 b | CDR-grafted, | 134 | VL-g2 | IGKHV3-11^{∗}01/JK4 | CDR-grafted, I2N, A44S, Y50F, F72Y | 139 |
| h75G7C6-8 | VH-g0 | IGHV1-46^{∗}01/JH6 b | CDR-grafted, | 134 | VL-g3 | IGKHV3-11^{∗}01/JK4 | CDR-grafted, I2N, A44S, Y50F, I59V, F72Y | 140 |
| h75G7C6-9 | VH-g1 | IGHV1-46^{∗}01/JH6 b | CDR-grafted, M48I, M70L, R72V | 135 | VL-g0 | IGKHV3-11^{∗}01/JK4 | CDR-grafted, | 137 |
| h75G7C6-10 | VH-g1 | IGHV1-46^{∗}01/JH6 b | CDR-grafted, M48I, M70L, R72V | 135 | VL-g1 | IGKHV3-11^{∗}01/JK4 | CDR-grafted, I2N, Y50F, F72Y | 138 |
| h75G7C6-11 | VH-g1 | IGHV1-46^{∗}01/JH6 b | CDR-grafted, M48I, M70L, R72V | 135 | VL-g2 | IGKHV3-11^{∗}01/JK4 | CDR-grafted, I2N, A44S, Y50F, F72Y | 139 |
| h75G7C6-12 | VH-g1 | IGHV1-46^{∗}01/JH6 b | CDR-grafted, M48I, M70L, R72V | 135 | VL-g3 | IGKHV3-11^{∗}01/JK4 | CDR-grafted, I2N, A44S, Y50F, I59V, F72Y | 140 |
| h75G7C6-13 | VH-g2 | IGHV1-46^{∗}01/JH6 b | CDR-grafted, R38K, M48I, M70L, R72V, T74K | 136 | VL-g0 | IGKHV3-11^{∗}01/JK4 | CDR-grafted, | 137 |
| h75G7C6-14 | VH-g2 | IGHV1-46^{∗}01/JH6 b | CDR-grafted, R38K, M48I, M70L, R72V, T74K | 136 | VL-gl | IGKHV3-11^{∗}01/JK4 | CDR-grafted, I2N, Y50F, F72Y | 138 |
| h75G7C6-15 | VH-g2 | IGHV1-46^{∗}01/JH6 b | CDR-grafted, R38K, M48I, M70L, R72V, T74K | 136 | VL-g2 | IGKHV3-11^{∗}01/JK4 | CDR-grafted, I2N, A44S, Y50F, F72Y | 139 |
| h75G7C6-16 | VH-g2 | IGHV1-46^{∗}01/JH6 b | CDR-grafted, R38K, M48I, M70L, R72V, T74K | 136 | VL-g3 | IGKHV3-11^{∗}01/JK4 | CDR-grafted, I2N, A44S, Y50F, I59V, F72Y | 140 |

**Table 27**

| Antibody number | Heavy chain name | Human germline antibody heavy chain variable region template VH | Donor framework residues and back mutations | Heavy chain variable region SEQ ID NO. | Light chain name | Human germline antibody light chain variable region template Vk | Donor framework residues and back mutations | Light chain variable region SEQ ID NO. |
|---|---|---|---|---|---|---|---|---|
| c63A10A7 | VH | None | CDR | 9 | VL | None | CDR | 13 |
| h63A10A7-17 | VH-g4 | IGHV1-46^{∗}01/JH4 | CDR-grafted, T30I, R38K, A40R, M48I,R67K, V68A, M70L, A97T | 143 | VL-g4 | IGKV1-39^{∗}01/JK2 | CDR-grafted, Q3L, Y36L, A43S, P44S, L46A, T69A, F71Y | 149 |
| h63A10A7-18 | VH-g5 | IGHV1-46^{∗}01/JH4 | CDR-grafted, T30I, R38K, A40R, M48I,R67K, V68A, M70L, R72A, T74Q,V79A, A97T | 144 | VL-g4 | IGKV1-39^{∗}01/JK2 | CDR-grafted, Q3L, Y36L, A43S, P44S, L46A, T69A, F71Y | 149 |
| h63A10A7-19 | VH-g6 | IGHV1-46^{∗}01/JH4 | CDR-grafted,T30I, R38K, A40R,R67K, V68A, A97T | 145 | VL-g4 | IGKV1-39^{∗}01/JK2 | CDR-grafted, Q3L, Y36L, A43S, P44S, L46A, T69A, F71Y | 149 |
| h63A10A7-20 | VH-g7 | IGHV1-46^{∗}01/JH4 | CDR-grafted,T30I, M48I,R67K, V68A, M70L, A97T | 146 | VL-g4 | IGKV1-39^{∗}01/JK2 | CDR-grafted, Q3L, Y36L, | 149 |
| h63A10A7-21 | VH-g8 | IGHV1-46^{∗}01/JH4 | CDR-grafted, T30I, R38K, A40R, M48I,M70L, A97T | 147 | VL-g4 | IGKV1-39^{∗}01/JK2 | CDR-grafted, Q3L, Y36L, A43S, P44S, L46A, T69A, F71Y | 149 |
| h63A10A7-22 | VH-g4 | IGHV1-46^{∗}01/JH4 | CDR-grafted, T30I, R38K, A40R, M48I,R67K, V68A, M70L, A97T | 143 | VL-g3 | IGKV1-39^{∗}01/JK2 | CDR-grafted, Y36L, A43S, P44S, L46A, T69A, F71Y | 148 |
| h63A10A7-23 | VH-g4 | IGHV1-46^{∗}01/JH4 | CDR-grafted,T30I, R38K, A40R, M48I,R67K, V68A, M70L, A97T | 143 | VL-g5 | IGKV1-39^{∗}01/JK2 | CDR-grafted, Q3L, Y36L, L46A, T69A, F71Y | 150 |
| h63A10A7-24 | VH-g4 | IGHV1-46^{∗}01/JH4 | CDR-grafted, T30I, R38K, A40R, M48I,R67K, V68A, M70L, A97T | 143 | VL-g6 | IGKV1-39^{∗}01/JK2 | CDR-grafted, Q3L, Y36L, A43S, P44S, L46A, F71Y | 151 |
| h63A10A7-25 | VH-g4 | IGHV1-46^{∗}01/JH4 | CDR-grafted,T30I, R38K, A40R, M48I,R67K, V68A, M70L, A97T | 143 | VL-g7 | IGKV1-39^{∗}01/JK2 | CDR-grafted, Q3L, A43S, P44S, L46A, T69A, F71Y | 152 |
| h63A10A7-26 | VH-g4 | IGHV1-46^{∗}01/JH4 | CDR-grafted,T30I, R38K, A40R, M48I,R67K, V68A, M70L, A97T | 143 | VL-g8 | IGKV1-39^{∗}01/JK2 | CDR-grafted, Q3L, A43S, P44S, L46A, T69A | 153 |
| h63A10A7-27 | VH-g2 | IGHV1-46^{∗}01/JH4 | CDR-grafted, T30I, A97T | 141 | VL-g4 | IGKV1-39^{∗}01/JK2 | CDR-grafted, Q3L, Y36L, A43S, P44S, L46A, T69A, F71Y | 149 |
| h63A10A7-28 | VH-g2 | IGHV1-46^{∗}01/JH4 | CDR-grafted, T30I, A97T | 141 | VL-g5 | IGKV1-39^{∗}01/JK2 | CDR-grafted, Q3L, Y36L, L46A, T69A, F71Y | 150 |
| h63A10A7-29 | VH-g2 | IGHV1-46^{∗}01/JH4 | CDR-grafted, T30I, A97T | 141 | VL-g6 | IGKV1-39^{∗}01/JK2 | CDR-grafted, Q3L, Y36L, A43S, P44S, L46A, F71Y | 151 |
| h63A10A7-30 | VH-g2 | IGHV1-46^{∗}01/JH4 | CDR-grafted, T30I, A97T | 141 | VL-g7 | IGKV1-39^{∗}01/JK2 | CDR-grafted, Q3L, A43S, P44S, L46A, T69A, F71Y | 152 |
| h63A10A7-31 | VH-g2 | IGHV1-46^{∗}01/JH4 | CDR-grafted, T30I, A97T | 141 | VL-g8 | IGKV1-39^{∗}01/JK2 | CDR-grafted, Q3L, A43S, P44S, L46A, T69A | 153 |
| h63A10A7-32 | VH-g3 | IGHV1-46^{∗}01/JH4 | CDR-grafted,T30I, M48I, M70L, A97T | 142 | VL-g4 | IGKV1-39^{∗}01/JK2 | CDR-grafted, Q3L, Y36L, A43S, P44S, L46A, T69A, F71Y | 149 |
| h63A10A7-33 | VH-g3 | IGHV1-46^{∗}01/JH4 | CDR-grafted,T30I, M48I, M70L, A97T | 142 | VL-g5 | IGKV1-39^{∗}01/JK2 | CDR-grafted, Q3L, Y36L, L46A, T69A, F71Y | 150 |
| h63A10A7-34 | VH-g3 | IGHV1-46^{∗}01/JH4 | CDR-grafted, T30I, M48I, M70L, A97T | 142 | VL-g6 | IGKV1-39^{∗}01/JK2 | CDR-grafted, Q3L, Y36L, A43S, P44S, L46A, F71Y | 151 |
| h63A10A7-35 | VH-g3 | IGHV1-46^{∗}01/JH4 | CDR-grafted, T30I, M48I, M70L, A97T | 142 | VL-g7 | IGKV1-39^{∗}01/JK2 | CDR-grafted, Q3L, A43S, P44S, L46A, T69A, F71Y | 152 |
| h63A10A7-36 | VH-g3 | IGHV1-46^{∗}01/JH4 | CDR-grafted,T30I, M48I, M70L, A97T | 142 | VL-g8 | IGKV1-39^{∗}01/JK2 | CDR-grafted, Q3L, A43S, P44S, L46A, T69A | 153 |
| h63A10A7-37 | VH-g6 | IGHV1-46^{∗}01/JH4 | CDR-grafted, T30I, R38K, A40R,R67K, V68A, A97T | 145 | VL-g3 | IGKV1-39^{∗}01/JK2 | CDR-grafted, Y36L, A43S, P44S, L46A, T69A, F71Y | 148 |
| h63A10A7-38 | VH-g7 | IGHV1-46^{∗}01/JH4 | CDR-grafted, T30I, M48I,R67K, V68A, M70L, A97T | 146 | VL-g3 | IGKV1-39^{∗}01/JK2 | CDR-grafted, Y36L, A43S, P44S, L46A, T69A, F71Y | 148 |
| h63A10A7-39 | VH-g8 | IGHV1-46^{∗}01/JH4 | CDR-grafted,T30I, R38K, A40R, M48I,M70L, A97T | 147 | VL-g3 | IGKV1-39^{∗}01/JK2 | CDR-grafted, Y36L, A43S, P44S, L46A, T69A, F71Y | 148 |

| | | | | | | | | |
|---|---|---|---|---|---|---|---|---|
| Note: The meaning of "/" in the table is "and", which refers to a parallel relationship. | | | | | | | | |

cDNAs (i.e., the sequences respectively shown in SEQ ID NO. 154, 155, 156, 157, 158, 159, 160, 161, 162, 163, 164, 165, 166, 167, 168, 169, 170, 171, 172, 173 and 174 in the sequence listing) are synthesized based on the amino acid sequence of the light chain variable region and heavy chain variable region of each humanized antibody; the heavy chain cDNA is digested with FspAI and AfeI, and the light chain cDNA is digested with FspAI and BsiwI; then the cDNA fragments are inserted into an expression vector containing a signal peptide and a human heavy chain antibody IgG1 constant region and an expression vector containing a signal peptide and a human antibody light chain kappa constant region (wherein the expression vectors are purchased from Invitrogen, and the recombination steps are also completed by Shanghai ChemPartner Co., Ltd) through FspAI/AfeI or FspAI/BsiwI restriction enzyme sites, respectively, thereby obtaining recombinant plasmids which are verified by sequencing; an alkaline lysis kit (purchased from MACHEREY-NAGEL) is used for a medium-amount extraction of the recombinant plasmids having a high purity, wherein the recombinant plasmids have a mass of at least 500 µg and filtered through a 0.22 µm filter membrane (purchased from Millopore) for transfection.

Before transfection, 293E cells (purchased from Invitrogen) are cultured in a Freestyle 293 expression medium (purchased from Invitrogen). During transfection, the Freestyle 293 expression medium is added with 10% (v/v) F68 (purchased from Invitrogen) to a final F68 concentration of 0.1% (v/v), thereby obtaining a Freestyle 293 expression medium containing 0.1% (v/v) F68 (i.e., a medium A). 5 mL of the medium A and 200 µg/mL PEI (purchased from Sigma) are mixed evenly to obtain a medium B. 5 mL of the medium A and 100 µg of the heavy and light chain recombinant plasmids (the mass ratio of the heavy chain recombinant plasmid to the light chain recombinant plasmid is in the range of 1 : 1-1 : 3) are mixed evenly to obtain a medium C. After 5 minutes, the medium B and the medium C are combined and mixed evenly, and allowed to stand for 15 minutes to obtain a mixed solution D. 10 mL of the mixed solution D was slowly added to 100 mL of the Freestyle 293 expression medium containing 293E cells until the cell density of 293E is 1.5 × 10⁶ cells/mL, wherein the addition was performed with shaking to avoid excessive concentration of PEI, and the mixture was placed in a shaker for culture, with the shaker being set to 37°C, 130 RPM and 8% CO₂ (v/v). The next day peptone is added to a final concentration of 0.5% (w/v). On days 5 to 7, the antibody titer of the culture broth is measured. On days 6 and 7, the supernatant is collected by centrifugation (3500 RPM, 30 minutes) and filtered through a 0.22 µm filter membrane to obtain the filtered cell supernatant for purification.

During antibody purification, the continuously produced endotoxin-free chromatography columns and protein A fillers (purchased from GE) are rinsed with 5 column volumes of 0.5 M NaOH. Then 5 column volumes of PBS (a PBS buffer, pH 7.4) are used for equilibration to neutrality, and then the filtered cell supernatant is loaded onto the column, and the flow-through fluid is collected if necessary. After loaded onto the column, 5-fold column volumes of PBS are used for washing. 5-fold column volumes of 0.1 M Glycine-HCl (pH 3.0) is used for eluting, and the eluate is collected, and immediately added with 0.1-fold volume of 1 M Tris-HCl (1.5 M NaCl) (pH 8.5) to neutralize the EGFRvIII antibody. All the solutions used above need to be freshly prepared. The EGFRvIII antibody is harvested, and then dialyzed in 1×PBS for 4 hours to avoid endotoxin contamination. After dialysis, the concentration is measured using a spectrophotometer or a kit; the purity of the antibodies is measured using HPLC-SEC; and the endotoxin content of the antibodies is detected using an endotoxin detection kit (purchased from Lonza). The obtained EGFRvIII antibody is subjected to characterization (the operation steps are as described in example 13 below).

### Example 14 Identification of humanized anti-EGFRvIII antibody

### A. Binding of the antibody to an EGFRvIII protein detected by an enzyme-linked immunosorbent assay (ELISA)

The purified humanized anti-EGFRvIII antibody obtained in example 12 is reacted with a human EGFRvIII-hFc protein.

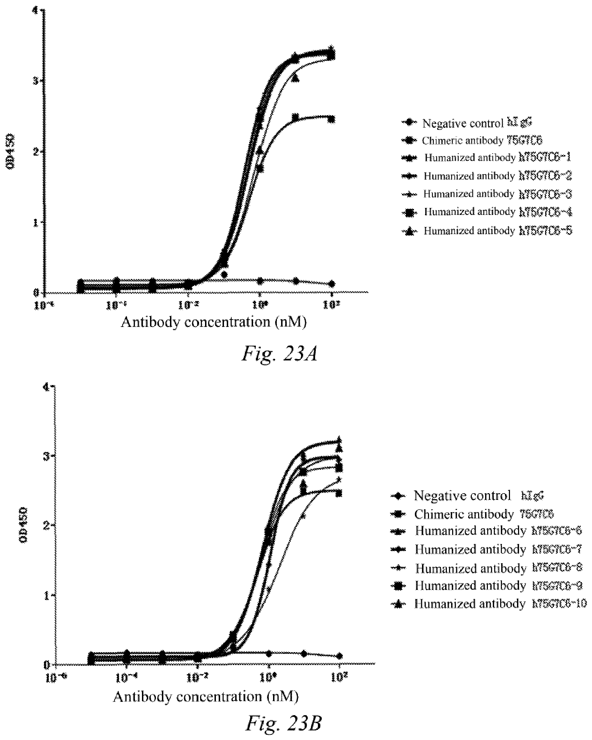

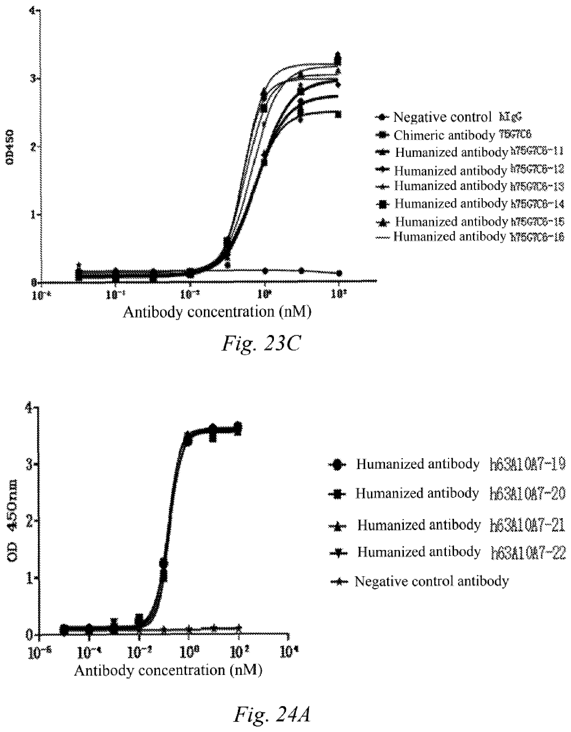

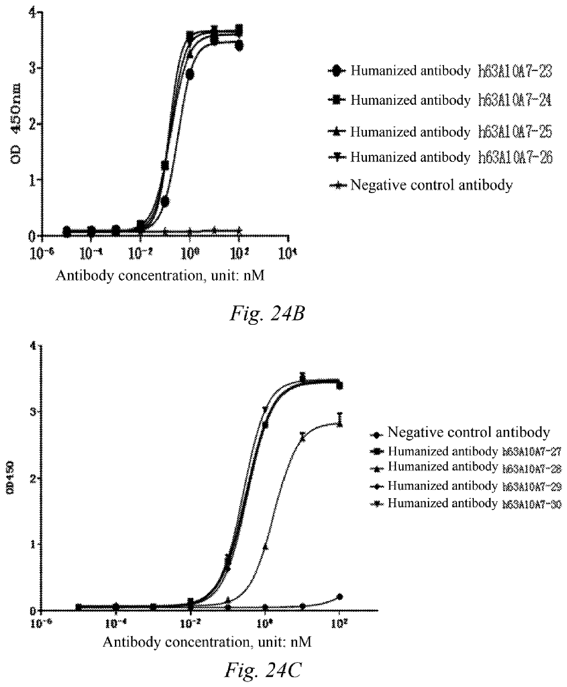

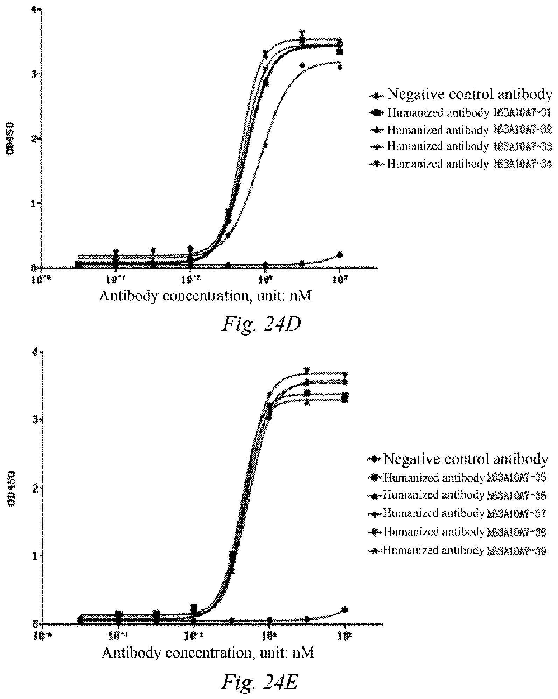

The purified immunogen A (EGFRvIII-hFc) obtained in example 1 (for the preparation method, see step (I) in example 1) is diluted with PBS to a final concentration of 1.0 µg/mL, and then added to a 96-well ELISA plate at 100 µl/well. The plate is sealed with a plastic film and incubated at 4°C overnight. The next day, the plate is washed twice with a plate washing solution [PBS + 0.01% (v/v) Tween20], and added with a blocking solution [PBS + 0.01% (v/v) Tween20 + 1% (w/w) BSA] for blocking 2 hours at room temperature. The blocking solution is discarded, and the purified EGFRvIII chimeric antibody obtained in example 2 is added at 100 µl/well. The plate is incubated for 2 hours at 37°C, and then washed 3 times with the plate washing solution [PBS + 0.01% (v/v) Tween20]. The plate is added with an HRP (horseradish peroxidase) labeled secondary antibody (purchased from Sigma), incubated at 37°C for 2 hours, and then washed 3 times with the plate washing solution [PBS + 0.01% (v/v) Tween20]. The plate is added with 100 µl of TMB substrate/well, incubated for 30 minutes at room temperature, and then added with 100 µl of stopping solution (1.0N HCl)/well. A450 nm value is read using an ELISA plate reader (SpectraMax 384plus, purchased from Molecular Device). The results are shown in figures 23A-C and 24A-E and tables 28 and 29, wherein figures 23A-C show the binding reaction of the purified humanized h75G7C6 variant with a human EGFRVIII-hFc protein, and figures 24A-E show the binding reaction of the purified humanized h63A10A7 variant with a human EGFRVIII-hFc protein. Tables 28 and 29 are the EC50 values calculated based on the OD₄₅₀ₙₘ values of the h75G7C6 variant and h63A10A7 variant, respectively, indicating that the purified humanized EGFRvIII antibody variant has a better binding to the EGFRvIII recombinant protein at the ELISA level.

**Table 28 Binding reaction of humanized h75G7C6 antibody mutant with human EGFRvIII-hFc protein detected by ELISA**

| Antibody name | EC50 (unit: nM) | Maximum OD450 value |
|---|---|---|
| Negative control antibody | None | 0.17 |
| Chimeric antibody c75G7C6 | 0.50 | 2.50 |
| Humanized antibody h75G7C6-1 | 0.49 | 3.41 |
| Humanized antibody h75G7C6-2 | 0.38 | 3.38 |
| Humanized antibody h75G7C6-3 | 0.51 | 3.44 |
| Humanized antibody h75G7C6-4 | 0.45 | 3.37 |
| Humanized antibody h75G7C6-5 | 0.70 | 3.32 |
| Humanized antibody h75G7C6-6 | 0.68 | 3.20 |
| Humanized antibody h75G7C6-7 | 1.10 | 2.97 |
| Humanized antibody h75G7C6-8 | 2.00 | 2.72 |
| Humanized antibody h75G7C6-9 | 0.58 | 2.83 |
| Humanized antibody h75G7C6-10 | 0.66 | 2.98 |
| Humanized antibody h75G7C6-11 | 0.67 | 2.96 |
| Humanized antibody h75G7C6-12 | 0.57 | 2.73 |
| Humanized antibody h75G7C6-13 | 0.48 | 3.16 |
| Humanized antibody h75G7C6-14 | 0.32 | 3.03 |
| Humanized antibody h75G7C6-15 | 0.29 | 3.19 |
| Humanized antibody h75G7C6-16 | 0.27 | 2.97 |

**Table 29 Binding reaction of humanized h63A10A7 antibody mutant with human EGFRvIII-hFc protein detected by ELISA**

| Antibody name | EC50 (unit: nM) | Maximum OD450 value |
|---|---|---|
| Negative control antibody | None | 0.1 |
| Chimeric antibody c63A10A7 | 0.22 | 3.6 |
| Humanized antibody h63A10A7-17 | 0.01 | 3.71 |
| Humanized antibody h63A10A7-18 | 0.01 | 3.71 |
| Humanized antibody h63A10A7-19 | 0.17 | 3.63 |
| Humanized antibody h63A10A7-20 | 0.18 | 3.55 |
| Humanized antibody h63A10A7-21 | 0.17 | 3.60 |
| Humanized antibody h63A10A7-22 | 0.15 | 3.57 |
| Humanized antibody h63A10A7-23 | 0.33 | 3.47 |
| Humanized antibody h63A10A7-24 | 0.15 | 3.67 |
| Humanized antibody h63A10A7-25 | 0.17 | 3.61 |
| Humanized antibody h63A10A7-26 | 0.16 | 3.65 |
| Humanized antibody h63A10A7-27 | 0.31 | 3.5 |
| Humanized antibody h63A10A7-28 | 1.73 | 2.8 |
| Humanized antibody h63A10A7-29 | 0.33 | 3.4 |
| Humanized antibody h63A10A7-30 | 0.26 | 3.5 |
| Humanized antibody h63A10A7-31 | 0.30 | 3.4 |
| Humanized antibody h63A10A7-32 | 0.21 | 3.5 |
| Humanized antibody h63A10A7-33 | 0.72 | 3.2 |
| Humanized antibody h63A10A7-34 | 0.27 | 3.5 |
| Humanized antibody h63A10A7-35 | 0.18 | 3.4 |
| Humanized antibody h63A10A7-36 | 0.21 | 3.3 |
| Humanized antibody h63A10A7-37 | 0.28 | 3.6 |
| Humanized antibody h63A10A7-38 | 0.22 | 3.7 |
| Humanized antibody h63A10A7-39 | 0.24 | 3.6 |

### B. Binding of antibody to EGFRvIII expressing cell detected by fluorescence activated cell sorting (FACS)

The preparation of U87MG-EGFRvIII required for FACS detection is as described in the preparation of the immunogen B in example 1. Normal human primary hepatocytes are purchased from BioreclamationIVT, and A431 tumor cells are purchased from ATCC. The recovered hepatocytes are directly used for FACS detection, and the recovered U87MG-EGFRvIII and A431 cells are subjected to an expanded culture to 90% confluence in a T-75 cell culture flask; the medium is sucked out, and the cells are washed twice with a HBSS buffer (Hanks Balanced Salt Solution) (purchased from Invitrogen), and then treated with an enzyme-free cell dissociation solution (Versene solution: purchased from Life Technology) and collected. The cells are washed twice with the HBSS buffer and are counted, and then the cells are diluted with the HBSS buffer to 2 × 10⁶ cells/ml, added with 1% goat serum blocking solution, with the percentage being the mass percentage, and incubated on ice for 30 minutes, and then centrifugally washed twice with the HBSS buffer. The collected cells are resuspended to 2 × 10⁶ cells/mL with an FACS buffer (HBSS + 2% FBS, with the percentage being the volume percentage), added to a 96-well FACS reaction plate at 100 µL/well, then added with 100 µL/well of the purified EGFRvIII antibody (samples to be tested) obtained in example 12, and incubated on ice for 2 hours. The plate is centrifugally washed twice with the FACS buffer, added with 100 µL/well of a 1 : 1000 diluted fluorescence (Alexa 488) labeled secondary antibody (purchased from Invitrogen), and incubated on ice for 1 hour. The plate is centrifugally washed 3 times with the FACS buffer, added with 100 µL/well of a fixing solution [4% (v/v) paraformaldehyde] to resuspend the cells, and centrifugally washed twice with the FACS buffer after 10 minutes. The cells are resuspended in 100 µL of the FACS buffer, and the results are detected and analyzed by FACS (FACS Calibur, purchased from BD). Data analysis is performed by using the software (CellQuest) to obtain the mean fluorescence intensity (MFI) of the cells. Data is further analyzed by using the software (GraphPad Prism5) to perform data fitting and calculate EC50.

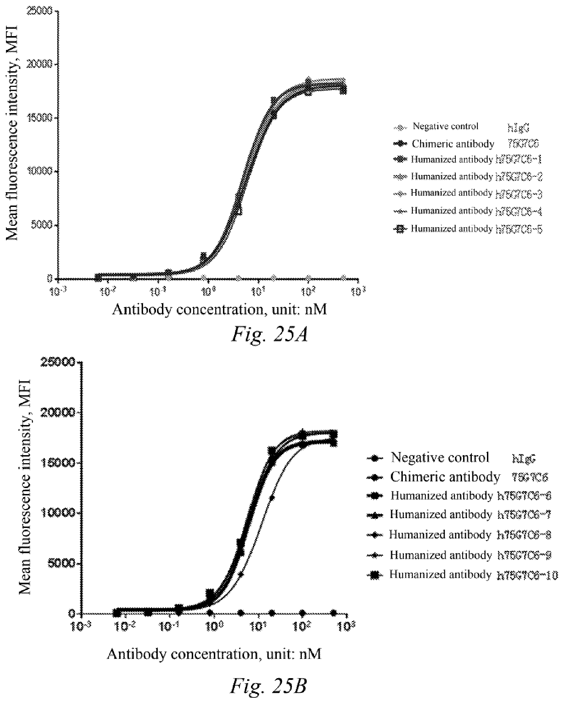

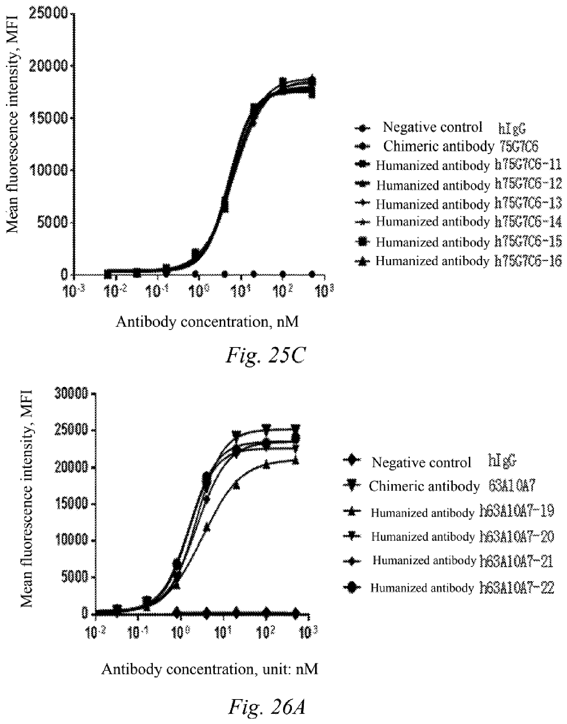

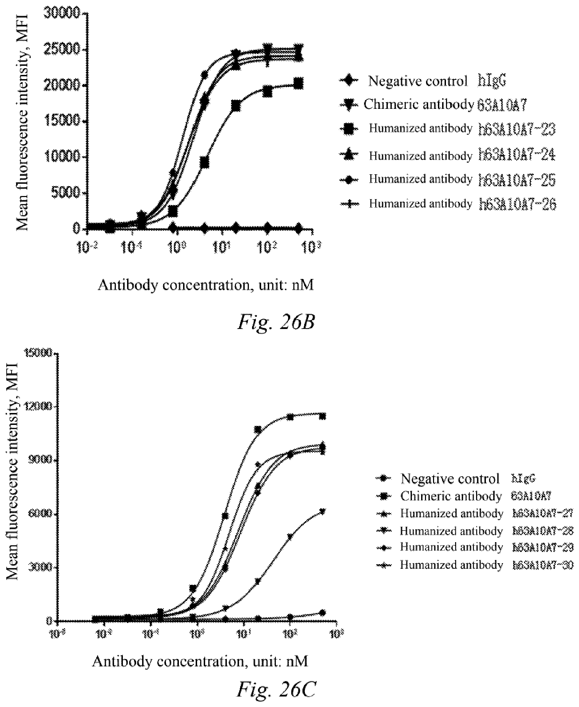

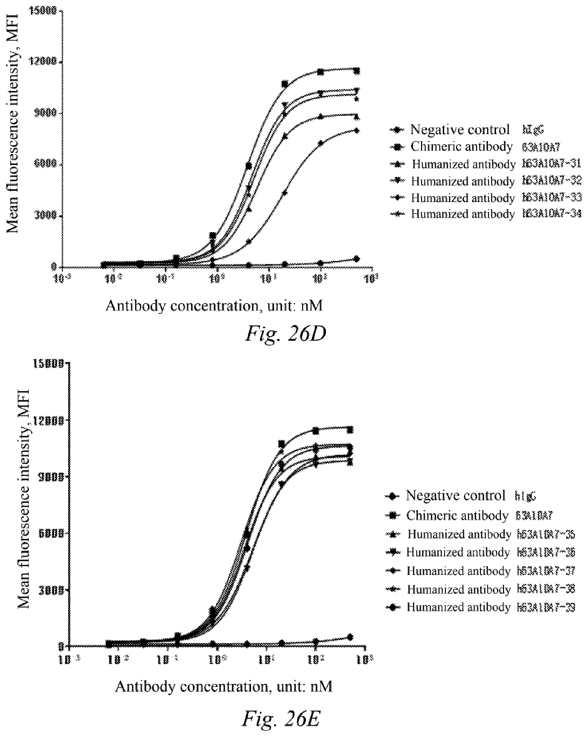

The analysis results are as shown in tables 30 and 31, and figures 25A-C and 26A-E. The humanized antibodies to be tested all can specifically bind to the EGFRvIII protein on the surface of U87MG-hEGFRvIII cells. Figures 25A-C show the binding of the humanized h75G7C6 antibody variant to the EGFRvIII on the surface of U87MG-hEGFRvIII cells. Figures 26A-E show the binding of the humanized h63A10A7 antibody variant to the EGFRvIII on the surface of U87MG-hEGFRvIII cells.

**Table 30. Binding reaction of humanized h75G7C6 antibody variant with EGFRvIII on the surface of U87MG-hEGFRvIII cells detected by FACS**

| | U87MG-EGF RvIII | |
|---|---|---|
| Antibody name | Maximum mean fluorescence intensity | EC50 (unit: nM) |
| Negative control hIgG | 0 | None |
| Chimeric antibody c75G7C6 | 18060 | 5.75 |
| Humanized antibody h75G7C6-1 | 18267 | 4.93 |
| Humanized antibody h75G7C6-2 | 18306 | 5.67 |
| Humanized antibody h75G7C6-3 | 18594 | 5.60 |
| Humanized antibody h75G7C6-4 | 18686 | 5.57 |
| Humanized antibody h75G7C6-5 | 17772 | 6.07 |
| Humanized antibody h75G7C6-6 | 17110 | 5.96 |
| Humanized antibody h75G7C6-7 | 17203 | 5.74 |
| Humanized antibody h75G7C6-8 | 17593 | 11.62 |
| Humanized antibody h75G7C6-9 | 18232 | 5.83 |
| Humanized antibody h75G7C6-10 | 18039 | 5.32 |
| Humanized antibody h75G7C6-11 | 17646 | 5.11 |
| Humanized antibody h75G7C6-12 | 17791 | 5.20 |
| Humanized antibody h75G7C6-13 | 18719 | 6.77 |
| Humanized antibody h75G7C6-14 | 18473 | 6.37 |
| Humanized antibody h75G7C6-15 | 18856 | 6.45 |
| Humanized antibody h75G7C6-16 | 18872 | 6.74 |

**Table 31. Binding reaction of humanized h63A10A7 antibody variant with EGFRvIII on the surface of U87MG-hEGFRvIII cells detected by FACS**

| Antibody name | U87MG-E :GFRvIII | |
|---|---|---|
| | Maximum mean fluorescence intensity | EC50 (unit: nM) |
| Negative control hIgG | 0 | None |
| Chimeric antibody c63A10A7 | 11650 | 3.80 |
| Humanized antibody h63A10A7-19 | 21123 | 3.27 |
| Humanized antibody h63A10A7-20 | 22590 | 1.56 |
| Humanized antibody h63A10A7-21 | 23511 | 2.34 |
| Humanized antibody h63A10A7-22 | 23519 | 1.58 |
| Humanized antibody h63A10A7-23 | 20151 | 4.62 |
| Humanized antibody h63A10A7-24 | 24165 | 1.83 |
| Humanized antibody h63A10A7-25 | 24676 | 1.32 |
| Humanized antibody h63A10A7-26 | 23692 | 1.79 |
| Humanized antibody h63A10A7-27 | 9969 | 7.68 |
| Humanized antibody h63A10A7-28 | 6691 | 43.18 |
| Humanized antibody h63A10A7-29 | 9783 | 8.55 |
| Humanized antibody h63A10A7-30 | 9542 | 4.86 |
| Humanized antibody h63A10A7-31 | 8977 | 5.72 |
| Humanized antibody h63A10A7-32 | 10416 | 4.66 |
| Humanized antibody h63A10A7-33 | 8258 | 17.98 |
| Humanized antibody h63A10A7-34 | 10137 | 5.08 |
| Humanized antibody h63A10A7-35 | 10088 | 3.52 |
| Humanized antibody h63A10A7-36 | 9871 | 5.20 |
| Humanized antibody h63A10A7-37 | 10213 | 5.35 |
| Humanized antibody h63A10A7-38 | 10739 | 3.03 |
| Humanized antibody h63A10A7-39 | 10651 | 4.10 |

In addition, humanized 75G7C6 and 63A10A7 antibody variants can also weakly bind to the wild-type EGFR. A431 cells (human epidermal cell cancer cell line) overexpress a large amount of wild-type EGFR proteins, and normal human primary hepatocytes also have a certain amount of wild-type EGFR protein expressed on the surface thereof; as shown in figure 27 and figure 28A-B, compared with anti-EGFR control antibody Cetuximab, humanized 75G7C6 antibody variants and humanized 63A10A7 antibody variants can all bind to EGFR overexpressed in tumor cells A431, but weakly bind to wild-type EGFR protein on the surface of normal human primary hepatocytes, which may be related to the spatial position of the antigenic determinants of humanized 75G7C6 and 63A10A7 antibodies. However, each variant of the humanized 75G7C6 antibody has a different binding ability regarding the EGFR protein on the surfaces of A431 and normal human primary hepatocytes. As shown in table 32, the antibodies to be tested weakly bind to or do not bind to normal human primary hepatocytes, have an mean fluorescence intensity (MFI) value of around 250, and have a binding ability similar to the negative control hIgG (with MFI value of 219); however, the antibodies to be tested have a high level of binding to the EGFR on the surface of A431 tumor cells, have an mean fluorescence intensity (MFI) value of 10000-13000, indicating that the humanized 75G7C6 antibody selectively binds to the EGFR protein overexpressed on the surface of tumor cells, but does not bind to or weakly binds to the EGFR protein expressed in normal cells, with a 35 to 47-fold selective window.

Similarly, each variant of the humanized 63A10A7 antibody has a different binding ability regarding the EGFR protein on the surfaces of A431 and normal human primary hepatocytes. As shown in figures 28A-B and table 33, some antibodies to be tested (such as humanized antibodies h63A10A7-17 to 26) have a similar binding ability in normal human primary hepatocytes to the anti-EGFR antibody (MFI 433), and have an mean fluorescence intensity (MFI) value of 433-677; therefore, these antibodies to be tested are considered not selective for normal cells expressing the EGFR. However, some antibodies to be tested show a good selectivity for tumor cells and normal cells expressing the EGFR. For example, humanized antibodies h63A10A7-37 to 39 weakly bind to or do not bind to normal human primary hepatocytes, with an mean fluorescence intensity (MFI) value of around 260, and have a similar binding ability to the negative control hIgG (with MFI value of 219), but they can bind to the EGFR on the surface of A431 tumor cells at a certain level, with an mean fluorescence intensity (MFI) value of 1000-2000, indicating that some humanized 63A10A7 antibody variants selectively bind to the EGFR protein overexpressed on the surface of tumor cells, but do not bind to or weakly bind to the EGFR protein expressed in normal cells, with a 5 to 7.5-fold selective window.

**Table 32. Binding reaction of humanized h75G7C6 antibody variant with EGFR on the surfaces of A431 tumor cells and normal human primary hepatocytes detected by FACS**

| Antibody name | A431 tumor cells | Normal primary hepatocytes | Selective window (MFI A431/Hepatocytes) |
|---|---|---|---|
| Negative control hIgG | 388 | 219 | 1.8 |
| Anti-EGFR positive control antibody | 23918 | 433 | 55.2 |
| Chimeric antibody c75G7C6 | 8394 | 317 | 26.5 |
| Humanized antibody h75G7C6-1 | 9780 | 277 | 35.3 |
| Humanized antibody h75G7C6-2 | 11751 | 250 | 47.0 |
| Humanized antibody h75G7C6-9 | 10717 | 238 | 45.0 |
| Humanized antibody h75G7C6-13 | 11451 | 256 | 44.7 |
| Humanized antibody h75G7C6-14 | 11452 | 248 | 46.2 |
| Humanized antibody h75G7C6-15 | 13002 | 287 | 45.3 |
| Humanized antibody h75G7C6-16 | 12433 | 269 | 46.2 |

**Table 33. Binding reaction of humanized h63A10A7 antibody variant with EGFR on the surfaces of A431 tumor cells and normal human primary hepatocytes detected by FACS**

| Antibody name | A431 tumor cells | Normal primary hepatocytes | Selective window (MFI A431/Hepatocytes) |
|---|---|---|---|
| Negative control hIgG | 388 | 219 | 2.0 |
| Anti-EGFR positive control antibody | 23918 | 433 | 55.2 |
| Chimeric antibody c63A10A7 | 4729 | 299 | 15.8 |
| Humanized antibody h63A10A7-17 | 10550 | 677 | 15.6 |
| Humanized antibody h63A10A7-18 | 9137 | 553 | 16.5 |
| Humanized antibody h63A10A7-19 | 3832 | 439 | 8.7 |
| Humanized antibody h63A10A7-20 | 7462 | 486 | 15.4 |
| Humanized antibody h63A10A7-21 | 7927 | 568 | 14.0 |
| Humanized antibody h63A10A7-22 | 9379 | 592 | 15.8 |
| Humanized antibody h63A10A7-23 | 3999 | 661 | 6.0 |
| Humanized antibody h63A10A7-24 | 9861 | 502 | 19.6 |
| Humanized antibody h63A10A7-25 | 7360 | 433 | 17.0 |
| Humanized antibody h63A10A7-26 | 6131 | 435 | 14.1 |
| Humanized antibody h63A10A7-27 | 209 | 262 | 0.8 |
| Humanized antibody h63A10A7-28 | 87 | 268 | 0.3 |
| Humanized antibody h63A10A7-29 | 212 | 262 | 0.8 |
| Humanized antibody h63A10A7-30 | 126 | 260 | 0.5 |
| Humanized antibody h63A10A7-31 | 112 | 254 | 0.4 |
| Humanized antibody h63A10A7-32 | 626 | 267 | 2.3 |
| Humanized antibody h63A10A7-33 | 92 | 252 | 0.4 |
| Humanized antibody h63A10A7-34 | 624 | 254 | 2.5 |
| Humanized antibody h63A10A7-35 | 236 | 253 | 0.9 |
| Humanized antibody h63A10A7-36 | 160 | 245 | 0.7 |
| Humanized antibody h63A10A7-37 | 1321 | 265 | 5.0 |
| Humanized antibody h63A10A7-38 | 1061 | 254 | 4.2 |
| Humanized antibody h63A10A7-39 | 1955 | 261 | 7.5 |

### C. Detection of binding affinity of humanized anti-EGFRvIII antibody

In order to evaluate the binding specificity and affinity of the humanized anti-EGFRvIII antibody to the EGFRvIII and EGFR proteins, on an OctetRED (purchased from Pall) instrument, an anti-human Fc biosensor AHC (purchased from ForteBio) is used to bind the humanized h75G7C6 antibody variant to be tested, which then binds to the EGFRvIII-his (purchased from Sino Biological) or EGFR-his (purchased from Sino Biological) proteins, and detected by means of bio-layer interferometry (BLI) technology. BLI technology involves: detecting the interaction of the molecules fixed on the sensor with the molecules in the solution, which results in that the biofilm thickness is increased, and the interference spectrum curve is shifted along the direction of increased wavelength, wherein the phase shift of light waves can be detected by workstation in real time, and analyzed to quantitatively obtain the changes in the number of molecules on the sensor surface and related concentration and kinetic data. The binding rate (Kₐ), dissociation rate (K_{dis}) and binding affinity (K_{D}) of the humanized h75G7C6 antibody variant to the EGFRvIII and EGFR proteins are as shown in tables 34 and 35, wherein the chimeric antibody c75G7C6 is used as a control.

**Table 34. Binding affinity of humanized h75G7C6 antibody variant to EGFRvIII protein**

| Antibody name | Ka (1/Ms) | Kdis (1/s) | KD (M) |
|---|---|---|---|
| Chimeric antibody c75G7C6 | 9.14E + 04 | 1.32E-04 | 1.45E-09 |
| Humanized antibody h75G7C6-1 | 8.28E + 04 | 1.41E-04 | 1.71E-09 |
| Humanized antibody h75G7C6-2 | 9.06E + 04 | 1.40E-04 | 1.54E-09 |
| Humanized antibody h75G7C6-9 | 9.89E + 04 | 1.75E-04 | 1.77E-09 |
| Humanized antibody h75G7C6-13 | 1.25E + 05 | 1.81E-04 | 1.45E-09 |

**Table 35. Binding affinity of humanized h75G7C6 antibody variant to EGFR protein**

| Antibody name | Ka (1/Ms) | Kdis (1/s) | KD (M) |
|---|---|---|---|
| Chimeric antibody c75G7C6 | 1.65E + 04 | 3.62E-03 | 2.20E-07 |
| Humanized antibody h75G7C6-1 | 1.72E + 04 | 3.53E-03 | 2.05E-07 |
| Humanized antibody h75G7C6-2 | 2.03E + 04 | 4.05E-03 | 2.00E-07 |
| Humanized antibody h75G7C6-9 | 2.02E + 04 | 4.35E-03 | 2.15E-07 |
| Humanized antibody h75G7C6-13 | 2.25E + 04 | 4.76E-03 | 2.11E-07 |

OctetRED results show that the binding affinity to the EGFRvIII and EGFR proteins of the humanized h75G7C6 antibody variant is very close to that of the chimeric antibody c75G7C6, which verifies that the humanized h75G7C6 antibody does not significantly reduce the antigen binding activity compared to the chimeric antibody. Furthermore, the binding affinity of the humanized h75G7C6 antibody variant to the EGFRvIII protein is about 1.5 nM, and the binding affinity thereof to the EGFR protein is about 0.2 uM.

Although the specific embodiments of the present invention have been described above, it will be understood by those skilled in the art that these are merely illustrative, and that various alterations or modifications can be made to these embodiments without departing from the principle and essence of the present invention. Therefore, the scope of protection of the present invention is defined by the appended claims.

## Claims

1. An EGFRvIII antibody, wherein the antibody comprises complementarity determining regions (CDRs): one or more of heavy chain CDR1, heavy chain CDR2 and heavy chain CDR3, and/or one or more of light chain CDR1, light chain CDR2 and light chain CDR3, wherein the amino acid sequence of the heavy chain CDR1 is as shown in SEQ ID No. 2, SEQ ID No. 10, SEQ ID No. 18, SEQ ID No. 26, SEQ ID No. 34, SEQ ID No. 42, SEQ ID No. 50, SEQ ID No. 58, SEQ ID No. 66, SEQ ID No. 74, SEQ ID No. 82, SEQ ID No. 90 or SEQ ID No. 98 in the sequence listing; the amino acid sequence of the heavy chain CDR2 is as shown in SEQ ID No. 3, SEQ ID No. 11, SEQ ID No. 19, SEQ ID No. 27, SEQ ID No. 35, SEQ ID No. 43, SEQ ID No. 51, SEQ ID No. 59, SEQ ID No. 67, SEQ ID No. 75, SEQ ID No. 83, SEQ ID No. 91, SEQ ID No. 99, SEQ ID NO. 184 or SEQ ID NO. 186 in the sequence listing; the amino acid sequence of the heavy chain CDR3 is as shown in SEQ ID No. 4, SEQ ID No. 12, SEQ ID No. 20, SEQ ID No. 28, SEQ ID No. 36, SEQ ID No. 44, SEQ ID No. 52, SEQ ID No. 60, SEQ ID No. 68, SEQ ID No. 76, SEQ ID No. 84, SEQ ID No. 92 or SEQ ID No. 100 in the sequence listing; the amino acid sequence of the light chain CDR1 is as shown in SEQ ID No. 6, SEQ ID No. 14, SEQ ID No. 22, SEQ ID No. 30, SEQ ID No. 38, SEQ ID No. 46, SEQ ID No. 54, SEQ ID No. 62, SEQ ID No. 70, SEQ ID No. 78, SEQ ID No. 86, SEQ ID No. 94 or SEQ ID No. 102 in the sequence listing; the amino acid sequence of the light chain CDR2 is as shown in SEQ ID No. 7, SEQ ID No. 15, SEQ ID No. 23, SEQ ID No. 31, SEQ ID No. 39, SEQ ID No. 47, SEQ ID No. 55, SEQ ID No. 63, SEQ ID No. 71, SEQ ID No. 79, SEQ ID No. 87, SEQ ID No. 95 or SEQ ID No. 103 in the sequence listing; the amino acid sequence of the light chain CDR3 is as shown in SEQ ID No. 8, SEQ ID No. 16, SEQ ID No. 24, SEQ ID No. 32, SEQ ID No. 40, SEQ ID No. 48, SEQ ID No. 56, SEQ ID No. 64, SEQ ID No. 72, SEQ ID No. 80, SEQ ID No. 88, SEQ ID No. 96 or SEQ ID No. 104 in the sequence listing;
or the amino acid sequence of the heavy chain CDR1 is as shown in an amino acid sequence which has at least 80% sequence homology with the amino acid sequence as shown in SEQ ID No. 2, SEQ ID No. 10, SEQ ID No. 18, SEQ ID No. 26, SEQ ID No. 34, SEQ ID No. 42, SEQ ID No. 50, SEQ ID No. 58, SEQ ID No. 66, SEQ ID No. 74, SEQ ID No. 82, SEQ ID No. 90 or SEQ ID No. 98 in the sequence listing; the amino acid sequence of the heavy chain CDR2 is as shown in an amino acid sequence which has at least 80% sequence homology with the amino acid sequence as shown in SEQ ID No. 3, SEQ ID No. 11, SEQ ID No. 19, SEQ ID No. 27, SEQ ID No. 35, SEQ ID No. 43, SEQ ID No. 51, SEQ ID No. 59, SEQ ID No. 67, SEQ ID No. 75, SEQ ID No. 83, SEQ ID No. 91, SEQ ID No. 99, SEQ ID NO. 184 or SEQ ID NO. 186 in the sequence listing; the amino acid sequence of the heavy chain CDR3 is as shown in an amino acid sequence which has at least 80% sequence homology with the amino acid sequence as shown in SEQ ID No. 4, SEQ ID No. 12, SEQ ID No. 20, SEQ ID No. 28, SEQ ID No. 36, SEQ ID No. 44, SEQ ID No. 52, SEQ ID No. 60, SEQ ID No. 68, SEQ ID No. 76, SEQ ID No. 84, SEQ ID No. 92 or SEQ ID No. 100 in the sequence listing; the amino acid sequence of the light chain CDR1 is as shown in an amino acid sequence which has at least 80% sequence homology with the amino acid sequence as shown in SEQ ID No. 6, SEQ ID No. 14, SEQ ID No. 22, SEQ ID No. 30, SEQ ID No. 38, SEQ ID No. 46, SEQ ID No. 54, SEQ ID No. 62, SEQ ID No. 70, SEQ ID No. 78, SEQ ID No. 86, SEQ ID No. 94 or SEQ ID No. 102 in the sequence listing; the amino acid sequence of the light chain CDR2 is as shown in an amino acid sequence which has at least 80% sequence homology with the amino acid sequence as shown in SEQ ID No. 7, SEQ ID No. 15, SEQ ID No. 23, SEQ ID No. 31, SEQ ID No. 39, SEQ ID No. 47, SEQ ID No. 55, SEQ ID No. 63, SEQ ID No. 71, SEQ ID No. 79, SEQ ID No. 87, SEQ ID No. 95 or SEQ ID No. 103 in the sequence listing; the amino acid sequence of the light chain CDR3 is as shown in an amino acid sequence which has at least 80% sequence homology with the amino acid sequence as shown in SEQ ID No. 8, SEQ ID No. 16, SEQ ID No. 24, SEQ ID No. 32, SEQ ID No. 40, SEQ ID No. 48, SEQ ID No. 56, SEQ ID No. 64, SEQ ID No. 72, SEQ ID No. 80, SEQ ID No. 88, SEQ ID No. 96 or SEQ ID No. 104 in the sequence listing.

2. The EGFRvIII antibody as defined in claim 1, wherein the amino acid sequence of the heavy chain CDR1 is as shown in SEQ ID No. 2 in the sequence listing, the amino acid sequence of the heavy chain CDR2 is as shown in SEQ ID No. 3 in the sequence listing, and the amino acid sequence of the heavy chain CDR3 is as shown in SEQ ID No. 4 in the sequence listing; the amino acid sequence of the heavy chain CDR1 is as shown in SEQ ID No. 10 in the sequence listing, the amino acid sequence of the heavy chain CDR2 is as shown in SEQ ID No. 11 in the sequence listing, and the amino acid sequence of the heavy chain CDR3 is as shown in SEQ ID No. 12 in the sequence listing; the amino acid sequence of the heavy chain CDR1 is as shown in SEQ ID No. 18 in the sequence listing, the amino acid sequence of the heavy chain CDR2 is as shown in SEQ ID No. 19 in the sequence listing, and the amino acid sequence of the heavy chain CDR3 is as shown in SEQ ID No. 20 in the sequence listing; the amino acid sequence of the heavy chain CDR1 is as shown in SEQ ID No. 26 in the sequence listing, the amino acid sequence of the heavy chain CDR2 is as shown in SEQ ID No. 27 in the sequence listing, and the amino acid sequence of the heavy chain CDR3 is as shown in SEQ ID No. 28 in the sequence listing; the amino acid sequence of the heavy chain CDR1 is as shown in SEQ ID No. 34 in the sequence listing, the amino acid sequence of the heavy chain CDR2 is as shown in SEQ ID No. 35 in the sequence listing, and the amino acid sequence of the heavy chain CDR3 is as shown in SEQ ID No. 36 in the sequence listing; the amino acid sequence of the heavy chain CDR1 is as shown in SEQ ID No. 42 in the sequence listing, the amino acid sequence of the heavy chain CDR2 is as shown in SEQ ID No. 43 in the sequence listing, and the amino acid sequence of the heavy chain CDR3 is as shown in SEQ ID No. 44 in the sequence listing; the amino acid sequence of the heavy chain CDR1 is as shown in SEQ ID No. 50 in the sequence listing, the amino acid sequence of the heavy chain CDR2 is as shown in SEQ ID No. 51 in the sequence listing, and the amino acid sequence of the heavy chain CDR3 is as shown in SEQ ID No. 52 in the sequence listing; the amino acid sequence of the heavy chain CDR1 is as shown in SEQ ID No. 58 in the sequence listing, the amino acid sequence of the heavy chain CDR2 is as shown in SEQ ID No. 59 in the sequence listing, and the amino acid sequence of the heavy chain CDR3 is as shown in SEQ ID No. 60 in the sequence listing; the amino acid sequence of the heavy chain CDR1 is as shown in SEQ ID No. 66 in the sequence listing, the amino acid sequence of the heavy chain CDR2 is as shown in SEQ ID No. 67 in the sequence listing, and the amino acid sequence of the heavy chain CDR3 is as shown in SEQ ID No. 68 in the sequence listing; the amino acid sequence of the heavy chain CDR1 is as shown in SEQ ID No. 74 in the sequence listing, the amino acid sequence of the heavy chain CDR2 is as shown in SEQ ID No. 75 in the sequence listing, and the amino acid sequence of the heavy chain CDR3 is as shown in SEQ ID No. 76 in the sequence listing; the amino acid sequence of the heavy chain CDR1 is as shown in SEQ ID No. 82 in the sequence listing, the amino acid sequence of the heavy chain CDR2 is as shown in SEQ ID No. 83 in the sequence listing, and the amino acid sequence of the heavy chain CDR3 is as shown in SEQ ID No. 84 in the sequence listing; the amino acid sequence of the heavy chain CDR1 is as shown in SEQ ID No. 90 in the sequence listing, the amino acid sequence of the heavy chain CDR2 is as shown in SEQ ID No. 91 in the sequence listing, and the amino acid sequence of the heavy chain CDR3 is as shown in SEQ ID No. 92 in the sequence listing; the amino acid sequence of the heavy chain CDR1 is as shown in SEQ ID No. 98 in the sequence listing, the amino acid sequence of the heavy chain CDR2 is as shown in SEQ ID No. 99 in the sequence listing, and the amino acid sequence of the heavy chain CDR3 is as shown in SEQ ID No. 100 in the sequence listing; the amino acid sequence of the heavy chain CDR1 is as shown in SEQ ID No. 2 in the sequence listing, the amino acid sequence of the heavy chain CDR2 is as shown in SEQ ID No. 184 in the sequence listing, and the amino acid sequence of the heavy chain CDR3 is as shown in SEQ ID No. 4 in the sequence listing; or the amino acid sequence of the heavy chain CDR1 is as shown in SEQ ID No. 2 in the sequence listing, the amino acid sequence of the heavy chain CDR2 is as shown in SEQ ID No. 186 in the sequence listing, and the amino acid sequence of the heavy chain CDR3 is as shown in SEQ ID No. 4 in the sequence listing;
the amino acid sequence of the light chain CDR1 is as shown in SEQ ID No. 6 in the sequence listing, the amino acid sequence of the light chain CDR2 is as shown in SEQ ID No. 7 in the sequence listing, and the amino acid sequence of the light chain CDR3 is as shown in SEQ ID No. 8 in the sequence listing; the amino acid sequence of the light chain CDR1 is as shown in SEQ ID No. 14 in the sequence listing, the amino acid sequence of the light chain CDR2 is as shown in SEQ ID No. 15 in the sequence listing, and the amino acid sequence of the light chain CDR3 is as shown in SEQ ID No. 16 in the sequence listing; the amino acid sequence of the light chain CDR1 is as shown in SEQ ID No. 22 in the sequence listing, the amino acid sequence of the light chain CDR2 is as shown in SEQ ID No. 23 in the sequence listing, and the amino acid sequence of the light chain CDR3 is as shown in SEQ ID No. 24 in the sequence listing; the amino acid sequence of the light chain CDR1 is as shown in SEQ ID No. 30 in the sequence listing, the amino acid sequence of the light chain CDR2 is as shown in SEQ ID No. 31 in the sequence listing, and the amino acid sequence of the light chain CDR3 is as shown in SEQ ID No. 32 in the sequence listing; the amino acid sequence of the light chain CDR1 is as shown in SEQ ID No. 38 in the sequence listing, the amino acid sequence of the light chain CDR2 is as shown in SEQ ID No. 39 in the sequence listing, and the amino acid sequence of the light chain CDR3 is as shown in SEQ ID No. 40 in the sequence listing; the amino acid sequence of the light chain CDR1 is as shown in SEQ ID No. 46 in the sequence listing, the amino acid sequence of the light chain CDR2 is as shown in SEQ ID No. 47 in the sequence listing, and the amino acid sequence of the light chain CDR3 is as shown in SEQ ID No. 48 in the sequence listing; the amino acid sequence of the light chain CDR1 is as shown in SEQ ID No. 54 in the sequence listing, the amino acid sequence of the light chain CDR2 is as shown in SEQ ID No. 55 in the sequence listing, and the amino acid sequence of the light chain CDR3 is as shown in SEQ ID No. 56 in the sequence listing; the amino acid sequence of the light chain CDR1 is as shown in SEQ ID No. 62 in the sequence listing, the amino acid sequence of the light chain CDR2 is as shown in SEQ ID No. 63 in the sequence listing, and the amino acid sequence of the light chain CDR3 is as shown in SEQ ID No. 64 in the sequence listing; the amino acid sequence of the light chain CDR1 is as shown in SEQ ID No. 70 in the sequence listing, the amino acid sequence of the light chain CDR2 is as shown in SEQ ID No. 71 in the sequence listing, and the amino acid sequence of the light chain CDR3 is as shown in SEQ ID No. 72 in the sequence listing; the amino acid sequence of the light chain CDR1 is as shown in SEQ ID No. 78 in the sequence listing, the amino acid sequence of the light chain CDR2 is as shown in SEQ ID No. 79 in the sequence listing, and the amino acid sequence of the light chain CDR3 is as shown in SEQ ID No. 80 in the sequence listing; the amino acid sequence of the light chain CDR1 is as shown in SEQ ID No. 86 in the sequence listing, the amino acid sequence of the light chain CDR2 is as shown in SEQ ID No. 87 in the sequence listing, and the amino acid sequence of the light chain CDR3 is as shown in SEQ ID No. 88 in the sequence listing; the amino acid sequence of the light chain CDR1 is as shown in SEQ ID No. 94 in the sequence listing, the amino acid sequence of the light chain CDR2 is as shown in SEQ ID No. 95 in the sequence listing, and the amino acid sequence of the light chain CDR3 is as shown in SEQ ID No. 96 in the sequence listing; or the amino acid sequence of the light chain CDR1 is as shown in SEQ ID No. 102 in the sequence listing, the amino acid sequence of the light chain CDR2 is as shown in SEQ ID No. 103 in the sequence listing, and the amino acid sequence of the light chain CDR3 is as shown in SEQ ID No. 104 in the sequence listing.

3. The EGFRvIII antibody as defined in claim 1 or 2, wherein the antibody further comprises an antibody framework region containing a heavy chain framework region and/or a light chain framework region; preferably, the heavy chain framework region is a human or murine antibody heavy chain framework region, and/or the light chain framework region is a human or murine antibody light chain framework region; more preferably, the light chain framework region is a human antibody light chain framework region, preferably a combination of human antibody light chain framework segments of FR1, FR2 and FR3 of IGKV1-39^{∗}01 or IGKV3-11^{∗}01 and segment FR4 of J_{K}-2 or J_{K}-4 of a human germline antibody light chain; and the heavy chain framework region is a human antibody heavy chain framework region, preferably a combination of human antibody heavy chain framework segments of FR1, FR2 and FR3 of IGHV1-46^{∗}01 and FR4 of J_{H}-4 or J_{H}-6b of a human germline antibody heavy chain.

4. The EGFRvIII antibody as defined in claim 3, wherein the EGFRvIII antibody comprises a heavy chain variable region and/or light chain variable region containing the CDRs, wherein the amino acid sequence of the heavy chain variable region is as shown in SEQ ID No. 1, SEQ ID No. 9, SEQ ID No. 17, SEQ ID No. 25, SEQ ID No. 33, SEQ ID No. 41, SEQ ID No. 49, SEQ ID No. 57, SEQ ID No. 65, SEQ ID No. 73, SEQ ID No. 81, SEQ ID No. 89, SEQ ID No. 97, SEQ ID No. 179, SEQ ID No. 180, SEQ ID NO. 133, SEQ ID NO. 134, SEQ ID NO. 135, SEQ ID NO. 136, SEQ ID NO. 141, SEQ ID NO. 142, SEQ ID NO. 143, SEQ ID NO. 144, SEQ ID NO. 145, SEQ ID NO. 146 or SEQ ID NO. 147 in the sequence listing; the amino acid sequence of the light chain variable region is as shown in SEQ ID No. 5, SEQ ID No. 13, SEQ ID No. 21, SEQ ID No. 29, SEQ ID No. 37, SEQ ID No. 45, SEQ ID No. 53, SEQ ID No. 61, SEQ ID No. 69, SEQ ID No. 77, SEQ ID No. 85, SEQ ID No. 93, SEQ ID No. 101, SEQ ID NO. 137, SEQ ID NO. 138, SEQ ID NO. 139, SEQ ID NO. 140, SEQ ID NO. 148, SEQ ID NO. 149, SEQ ID NO. 150, SEQ ID NO. 151, SEQ ID NO. 152 or SEQ ID NO. 153 in the sequence listing;
or the amino acid sequence of the heavy chain variable region is as shown in an amino acid sequence which has at least 80% sequence homology with the amino acid sequence as shown in SEQ ID No. 1, SEQ ID No. 9, SEQ ID No. 17, SEQ ID No. 25, SEQ ID No. 33, SEQ ID No. 41, SEQ ID No. 49, SEQ ID No. 57, SEQ ID No. 65, SEQ ID No. 73, SEQ ID No. 81, SEQ ID No. 89, SEQ ID No. 97, SEQ ID No. 179, SEQ ID No. 180, SEQ ID NO. 133, SEQ ID NO. 134, SEQ ID NO. 135, SEQ ID NO. 136, SEQ ID NO. 141, SEQ ID NO. 142, SEQ ID NO. 143, SEQ ID NO. 144, SEQ ID NO. 145, SEQ ID NO. 146 or SEQ ID NO. 147 in the sequence listing; the sequence of the light chain variable region is as shown in an amino acid sequence which has at least 80% sequence homology with the amino acid sequence as shown in SEQ ID No. 5, SEQ ID No. 13, SEQ ID No. 21, SEQ ID No. 29, SEQ ID No. 37, SEQ ID No. 45, SEQ ID No. 53, SEQ ID No. 61, SEQ ID No. 69, SEQ ID No. 77, SEQ ID No. 85, SEQ ID No. 93, SEQ ID No. 101, SEQ ID NO. 137, SEQ ID NO. 138, SEQ ID NO. 139, SEQ ID NO. 140, SEQ ID NO. 148, SEQ ID NO. 149, SEQ ID NO. 150, SEQ ID NO. 151, SEQ ID NO. 152 or SEQ ID NO. 153 in the sequence listing.

5. The EGFRvIII antibody as defined in claim 4, wherein the amino acid sequence of the heavy chain variable region is as shown in SEQ ID No. 1 in the sequence listing, and the amino acid sequence of the light chain variable region is as shown in SEQ ID No. 5 in the sequence listing; the amino acid sequence of the heavy chain variable region is as shown in SEQ ID No. 9 in the sequence listing, and the amino acid sequence of the light chain variable region is as shown in SEQ ID No. 13 in the sequence listing; the amino acid sequence of the heavy chain variable region is as shown in SEQ ID No. 17 in the sequence listing, and the amino acid sequence of the light chain variable region is as shown in SEQ ID No. 21 in the sequence listing; the amino acid sequence of the heavy chain variable region is as shown in SEQ ID No. 25 in the sequence listing, and the amino acid sequence of the light chain variable region is as shown in SEQ ID No. 29 in the sequence listing; the amino acid sequence of the heavy chain variable region is as shown in SEQ ID No. 33 in the sequence listing, and the amino acid sequence of the light chain variable region is as shown in SEQ ID No. 37 in the sequence listing; the amino acid sequence of the heavy chain variable region is as shown in SEQ ID No. 41 in the sequence listing, and the amino acid sequence of the light chain variable region is as shown in SEQ ID No. 45 in the sequence listing; the amino acid sequence of the heavy chain variable region is as shown in SEQ ID No. 49 in the sequence listing, and the amino acid sequence of the light chain variable region is as shown in SEQ ID No. 53 in the sequence listing; the amino acid sequence of the heavy chain variable region is as shown in SEQ ID No. 57 in the sequence listing, and the amino acid sequence of the light chain variable region is as shown in SEQ ID No. 61 in the sequence listing; the amino acid sequence of the heavy chain variable region is as shown in SEQ ID No. 65 in the sequence listing, and the amino acid sequence of the light chain variable region is as shown in SEQ ID No. 69 in the sequence listing; the amino acid sequence of the heavy chain variable region is as shown in SEQ ID No. 73 in the sequence listing, and the amino acid sequence of the light chain variable region is as shown in SEQ ID No. 77 in the sequence listing; the amino acid sequence of the heavy chain variable region is as shown in SEQ ID No. 81 in the sequence listing, and the amino acid sequence of the light chain variable region is as shown in SEQ ID No. 85 in the sequence listing; the amino acid sequence of the heavy chain variable region is as shown in SEQ ID No. 89 in the sequence listing, and the amino acid sequence of the light chain variable region is as shown in SEQ ID No. 93 in the sequence listing; the amino acid sequence of the heavy chain variable region is as shown in SEQ ID No. 97 in the sequence listing, and the amino acid sequence of the light chain variable region is as shown in SEQ ID No. 101 in the sequence listing; the amino acid sequence of the heavy chain variable region is as shown in SEQ ID No. 179 in the sequence listing, and the amino acid sequence of the light chain variable region is as shown in SEQ ID No. 5 in the sequence listing; the amino acid sequence of the heavy chain variable region is as shown in SEQ ID No. 180 in the sequence listing, and the amino acid sequence of the light chain variable region is as shown in SEQ ID No. 5 in the sequence listing; the amino acid sequence of the heavy chain variable region is a sequence as shown in SEQ ID No. 133 in the sequence listing, and the amino acid sequence of the light chain variable region is a sequence as shown in SEQ ID No. 137 in the sequence listing; the amino acid sequence of the heavy chain variable region is a sequence as shown in SEQ ID No. 133 in the sequence listing, and the amino acid sequence of the light chain variable region is a sequence as shown in SEQ ID No. 138 in the sequence listing; the amino acid sequence of the heavy chain variable region is a sequence as shown in SEQ ID No. 133 in the sequence listing, and the amino acid sequence of the light chain variable region is a sequence as shown in SEQ ID No. 139 in the sequence listing; the amino acid sequence of the heavy chain variable region is a sequence as shown in SEQ ID No. 133 in the sequence listing, and the amino acid sequence of the light chain variable region is a sequence as shown in SEQ ID No. 140 in the sequence listing; the amino acid sequence of the heavy chain variable region is a sequence as shown in SEQ ID No. 134 in the sequence listing, and the amino acid sequence of the light chain variable region is a sequence as shown in SEQ ID No. 137 in the sequence listing; the amino acid sequence of the heavy chain variable region is a sequence as shown in SEQ ID No. 134 in the sequence listing, and the amino acid sequence of the light chain variable region is a sequence as shown in SEQ ID No. 138 in the sequence listing; the amino acid sequence of the heavy chain variable region is a sequence as shown in SEQ ID No. 134 in the sequence listing, and the amino acid sequence of the light chain variable region is a sequence as shown in SEQ ID No. 139 in the sequence listing; the amino acid sequence of the heavy chain variable region is a sequence as shown in SEQ ID No. 134 in the sequence listing, and the amino acid sequence of the light chain variable region is a sequence as shown in SEQ ID No. 140 in the sequence listing; the amino acid sequence of the heavy chain variable region is a sequence as shown in SEQ ID No. 135 in the sequence listing, and the amino acid sequence of the light chain variable region is a sequence as shown in SEQ ID No. 137 in the sequence listing; the amino acid sequence of the heavy chain variable region is a sequence as shown in SEQ ID No. 135 in the sequence listing, and the amino acid sequence of the light chain variable region is a sequence as shown in SEQ ID No. 138 in the sequence listing; the amino acid sequence of the heavy chain variable region is a sequence as shown in SEQ ID No. 135 in the sequence listing, and the amino acid sequence of the light chain variable region is a sequence as shown in SEQ ID No. 139 in the sequence listing; the amino acid sequence of the heavy chain variable region is a sequence as shown in SEQ ID No. 135 in the sequence listing, and the amino acid sequence of the light chain variable region is a sequence as shown in SEQ ID No. 140 in the sequence listing; the amino acid sequence of the heavy chain variable region is a sequence as shown in SEQ ID No. 136 in the sequence listing, and the amino acid sequence of the light chain variable region is a sequence as shown in SEQ ID No. 137 in the sequence listing; the amino acid sequence of the heavy chain variable region is a sequence as shown in SEQ ID No. 136 in the sequence listing, and the amino acid sequence of the light chain variable region is a sequence as shown in SEQ ID No. 138 in the sequence listing; the amino acid sequence of the heavy chain variable region is a sequence as shown in SEQ ID No. 136 in the sequence listing, and the amino acid sequence of the light chain variable region is a sequence as shown in SEQ ID No. 139 in the sequence listing; the amino acid sequence of the heavy chain variable region is a sequence as shown in SEQ ID No. 136 in the sequence listing, and the amino acid sequence of the light chain variable region is a sequence as shown in SEQ ID No. 140 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 144 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 145 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 146 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 147 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 148 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 150 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 151 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 152 in the sequence listing; the amino acid sequence of the heavy chain variable region is a sequence as shown in SEQ ID No. 143 in the sequence listing, and the amino acid sequence of the light chain variable region is a sequence as shown in SEQ ID No. 153 in the sequence listing; the amino acid sequence of the heavy chain variable region is a sequence as shown in SEQ ID No. 141 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 141 in the sequence listing, and the amino acid sequence of the light chain variable region is a sequence as shown in SEQ ID No. 150 in the sequence listing; the amino acid sequence of the heavy chain variable region is a sequence as shown in SEQ ID No. 141 in the sequence listing, and the amino acid sequence of the light chain variable region is a sequence as shown in SEQ ID No. 151 in the sequence listing; the amino acid sequence of the heavy chain variable region is a sequence as shown in SEQ ID No. 141 in the sequence listing, and the amino acid sequence of the light chain variable region is a sequence as shown in SEQ ID No. 152 in the sequence listing; the amino acid sequence of the heavy chain variable region is a sequence as shown in SEQ ID No. 141 in the sequence listing, and the amino acid sequence of the light chain variable region is a sequence as shown in SEQ ID No. 153 in the sequence listing; the amino acid sequence of the heavy chain variable region is a sequence as shown in SEQ ID No. 142 in the sequence listing, and the amino acid sequence of the light chain variable region is a sequence as shown in SEQ ID No. 149 in the sequence listing; the amino acid sequence of the heavy chain variable region is a sequence as shown in SEQ ID No. 142 in the sequence listing, and the amino acid sequence of the light chain variable region is a sequence as shown in SEQ ID No. 150 in the sequence listing; the amino acid sequence of the heavy chain variable region is a sequence as shown in SEQ ID No. 142 in the sequence listing, and the amino acid sequence of the light chain variable region is a sequence as shown in SEQ ID No. 151 in the sequence listing; the amino acid sequence of the heavy chain variable region is a sequence as shown in SEQ ID No. 142 in the sequence listing, and the amino acid sequence of the light chain variable region is a sequence as shown in SEQ ID No. 152 in the sequence listing; the amino acid sequence of the heavy chain variable region is a sequence as shown in SEQ ID No. 142 in the sequence listing, and the amino acid sequence of the light chain variable region is a sequence as shown in SEQ ID No. 153 in the sequence listing; the amino acid sequence of the heavy chain variable region is a sequence as shown in SEQ ID No. 145 in the sequence listing, and the amino acid sequence of the light chain variable region is a sequence as shown in SEQ ID No. 148 in the sequence listing; the amino acid sequence of the heavy chain variable region is a sequence as shown in SEQ ID No. 146 in the sequence listing, and the amino acid sequence of the light chain variable region is a sequence as shown in SEQ ID No. 148 in the sequence listing; or the amino acid sequence of the heavy chain variable region is a sequence as shown in SEQ ID No. 147 in the sequence listing, and the amino acid sequence of the light chain variable region is a sequence as shown in SEQ ID No. 148 in the sequence listing.

6. The EGFRvIII antibody as defined in any one of claims 1 to 5, wherein the EGFRvIII antibody further comprises an antibody heavy chain constant region and/or an antibody light chain constant region, wherein the antibody heavy chain constant region is preferably a human or mouse antibody heavy chain constant region, and the antibody light chain constant region is preferably a human or mouse antibody light chain constant region.

7. The EGFRvIII antibody as defined in any one of claims 1 to 6, wherein the EGFRvIII antibody is a monoclonal antibody, full-length antibody protein, antigen-antibody binding domain protein fragment, bispecific antibody, multispecific antibody, single chain antibody fragment, single domain antibody or single-domain antibody of the EGFRvIII.

8. A nucleic acid, wherein the nucleic acid encodes the EGFRvIII antibody as defined in any one of claims 1 to 7.

9. The nucleic acid as defined in claim 8, wherein the nucleotide sequence of the nucleic acid encoding the heavy chain variable region is as shown in SEQ ID No. 105, SEQ ID No. 107, SEQ ID No. 109, SEQ ID No. 111, SEQ ID No. 113, SEQ ID No. 115, SEQ ID No. 117, SEQ ID No. 119, SEQ ID No. 121, SEQ ID No. 123, SEQ ID No. 125, SEQ ID No. 127, SEQ ID No. 129, SEQ ID No. 185, SEQ ID No. 154, SEQ ID No. 155, SEQ ID No. 156, SEQ ID No. 157, SEQ ID No. 162, SEQ ID No. 163, SEQ ID No. 164, SEQ ID No. 165, SEQ ID No. 166, SEQ ID No. 167 or SEQ ID No. 168 in the sequence listing; and/or the nucleotide sequence of the nucleic acid encoding the light chain variable region is as shown in SEQ ID No. 106, SEQ ID No. 108, SEQ ID No. 110, SEQ ID No. 112, SEQ ID No. 114, SEQ ID No. 116, SEQ ID No. 118, SEQ ID No. 120, SEQ ID No. 122, SEQ ID No. 124, SEQ ID No. 126, SEQ ID No. 128, SEQ ID No. 130, SEQ ID No. 158, SEQ ID No. 159, SEQ ID No. 160, SEQ ID No. 161, SEQ ID No. 169, SEQ ID No. 170, SEQ ID No. 171, SEQ ID No. 172, SEQ ID No. 173 or SEQ ID No. 174 in the sequence listing.

10. The nucleic acid as defined in claim 9, wherein the nucleotide sequence of the nucleic acid encoding the heavy chain variable region is as shown in SEQ ID No. 105 in the sequence listing, and the nucleotide sequence of the nucleic acid encoding the light chain variable region is as shown in SEQ ID No. 106 in the sequence listing; the nucleotide sequence of the nucleic acid encoding the heavy chain variable region is as shown in SEQ ID No. 107 in the sequence listing, and the nucleotide sequence of the nucleic acid encoding the light chain variable region is as shown in SEQ ID No. 108 in the sequence listing; the nucleotide sequence of the nucleic acid encoding the heavy chain variable region is as shown in SEQ ID No. 109 in the sequence listing, and the nucleotide sequence of the nucleic acid encoding the light chain variable region is as shown in SEQ ID No. 110 in the sequence listing; the nucleotide sequence of the nucleic acid encoding the heavy chain variable region is as shown in SEQ ID No. 111 in the sequence listing, and the nucleotide sequence of the nucleic acid encoding the light chain variable region is as shown in SEQ ID No. 112 in the sequence listing; the nucleotide sequence of the nucleic acid encoding the heavy chain variable region is as shown in SEQ ID No. 113 in the sequence listing, and the nucleotide sequence of the nucleic acid encoding the light chain variable region is as shown in SEQ ID No. 114 in the sequence listing; the nucleotide sequence of the nucleic acid encoding the heavy chain variable region is as shown in SEQ ID No. 115 in the sequence listing, and the nucleotide sequence of the nucleic acid encoding the light chain variable region is as shown in SEQ ID No. 116 in the sequence listing; the nucleotide sequence of the nucleic acid encoding the heavy chain variable region is as shown in SEQ ID No. 117 in the sequence listing, and the nucleotide sequence of the nucleic acid encoding the light chain variable region is as shown in SEQ ID No. 118 in the sequence listing; the nucleotide sequence of the nucleic acid encoding the heavy chain variable region is as shown in SEQ ID No. 119 in the sequence listing, and the nucleotide sequence of the nucleic acid encoding the light chain variable region is as shown in SEQ ID No. 120 in the sequence listing; the nucleotide sequence of the nucleic acid encoding the heavy chain variable region is as shown in SEQ ID No. 121 in the sequence listing, and the nucleotide sequence of the nucleic acid encoding the light chain variable region is as shown in SEQ ID No. 122 in the sequence listing; the nucleotide sequence of the nucleic acid encoding the heavy chain variable region is as shown in SEQ ID No. 123 in the sequence listing, and the nucleotide sequence of the nucleic acid encoding the light chain variable region is as shown in SEQ ID No. 124 in the sequence listing; the nucleotide sequence of the nucleic acid encoding the heavy chain variable region is as shown in SEQ ID No. 125 in the sequence listing, and the nucleotide sequence of the nucleic acid encoding the light chain variable region is as shown in SEQ ID No126 in the sequence listing; the nucleotide sequence of the nucleic acid encoding the heavy chain variable region is as shown in SEQ ID No. 127 in the sequence listing, and the nucleotide sequence of the nucleic acid encoding the light chain variable region is as shown in SEQ ID No. 128 in the sequence listing; the nucleotide sequence of the nucleic acid encoding the heavy chain variable region is as shown in SEQ ID No. 129 in the sequence listing, and the nucleotide sequence of the nucleic acid encoding the light chain variable region is as shown in SEQ ID No. 130 in the sequence listing; the nucleotide sequence of the nucleic acid encoding the heavy chain variable region is as shown in SEQ ID No. 185 in the sequence listing, and the nucleotide sequence of the nucleic acid encoding the light chain variable region is as shown in SEQ ID No. 106 in the sequence listing; the nucleotide sequence of the nucleic acid encoding the heavy chain variable region is as shown in SEQ ID No. 154 in the sequence listing, and the nucleotide sequence of the nucleic acid encoding the light chain variable region is as shown in SEQ ID No. 158 in the sequence listing; the nucleotide sequence of the nucleic acid encoding the heavy chain variable region is as shown in SEQ ID No. 154 in the sequence listing, and the nucleotide sequence of the nucleic acid encoding the light chain variable region is as shown in SEQ ID No. 159 in the sequence listing; the nucleotide sequence of the nucleic acid encoding the heavy chain variable region is as shown in SEQ ID No. 154 in the sequence listing, and the nucleotide sequence of the nucleic acid encoding the light chain variable region is as shown in SEQ ID No. 160 in the sequence listing; the nucleotide sequence of the nucleic acid encoding the heavy chain variable region is as shown in SEQ ID No. 154 in the sequence listing, and the nucleotide sequence of the nucleic acid encoding the light chain variable region is as shown in SEQ ID No. 161 in the sequence listing; the nucleotide sequence of the nucleic acid encoding the heavy chain variable region is as shown in SEQ ID No. 155 in the sequence listing, and the nucleotide sequence of the nucleic acid encoding the light chain variable region is as shown in SEQ ID No. 158 in the sequence listing; the nucleotide sequence of the nucleic acid encoding the heavy chain variable region is as shown in SEQ ID No. 155 in the sequence listing, and the nucleotide sequence of the nucleic acid encoding the light chain variable region is as shown in SEQ ID No. 159 in the sequence listing; the nucleotide sequence of the nucleic acid encoding the heavy chain variable region is as shown in SEQ ID No. 155 in the sequence listing, and the nucleotide sequence of the nucleic acid encoding the light chain variable region is as shown in SEQ ID No. 160 in the sequence listing; the nucleotide sequence of the nucleic acid encoding the heavy chain variable region is as shown in SEQ ID No. 155 in the sequence listing, and the nucleotide sequence of the nucleic acid encoding the light chain variable region is as shown in SEQ ID No. 161 in the sequence listing; the nucleotide sequence of the nucleic acid encoding the heavy chain variable region is as shown in SEQ ID No. 156 in the sequence listing, and the nucleotide sequence of the nucleic acid encoding the light chain variable region is as shown in SEQ ID No. 158 in the sequence listing; the nucleotide sequence of the nucleic acid encoding the heavy chain variable region is as shown in SEQ ID No. 156 in the sequence listing, and the nucleotide sequence of the nucleic acid encoding the light chain variable region is as shown in SEQ ID No. 159 in the sequence listing; the nucleotide sequence of the nucleic acid encoding the heavy chain variable region is as shown in SEQ ID No. 156 in the sequence listing, and the nucleotide sequence of the nucleic acid encoding the light chain variable region is as shown in SEQ ID No. 160 in the sequence listing; the nucleotide sequence of the nucleic acid encoding the heavy chain variable region is as shown in SEQ ID No. 156 in the sequence listing, and the nucleotide sequence of the nucleic acid encoding the light chain variable region is as shown in SEQ ID No. 161 in the sequence listing; the nucleotide sequence of the nucleic acid encoding the heavy chain variable region is as shown in SEQ ID No. 157 in the sequence listing, and the nucleotide sequence of the nucleic acid encoding the light chain variable region is as shown in SEQ ID No. 158 in the sequence listing; the nucleotide sequence of the nucleic acid encoding the heavy chain variable region is as shown in SEQ ID No. 157 in the sequence listing, and the nucleotide sequence of the nucleic acid encoding the light chain variable region is as shown in SEQ ID No. 159 in the sequence listing; the nucleotide sequence of the nucleic acid encoding the heavy chain variable region is as shown in SEQ ID No. 157 in the sequence listing, and the nucleotide sequence of the nucleic acid encoding the light chain variable region is as shown in SEQ ID No. 160 in the sequence listing; the nucleotide sequence of the nucleic acid encoding the heavy chain variable region is as shown in SEQ ID No. 157 in the sequence listing, and the nucleotide sequence of the nucleic acid encoding the light chain variable region is as shown in SEQ ID No. 161 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 165 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 166 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 167 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 168 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 169 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 171 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 172 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 173 in the sequence listing; the nucleotide sequence of the nucleic acid encoding the heavy chain variable region is as shown in SEQ ID No. 164 in the sequence listing, and the nucleotide sequence of the nucleic acid encoding the light chain variable region is as shown in SEQ ID No. 174 in the sequence listing; the nucleotide sequence of the nucleic acid encoding the heavy chain variable region is as shown in SEQ ID No. 162 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 162 in the sequence listing, and the nucleotide sequence of the nucleic acid encoding the light chain variable region is as shown in SEQ ID No. 171 in the sequence listing; the nucleotide sequence of the nucleic acid encoding the heavy chain variable region is as shown in SEQ ID No. 162 in the sequence listing, and the nucleotide sequence of the nucleic acid encoding the light chain variable region is as shown in SEQ ID No. 172 in the sequence listing; the nucleotide sequence of the nucleic acid encoding the heavy chain variable region is as shown in SEQ ID No. 162 in the sequence listing, and the nucleotide sequence of the nucleic acid encoding the light chain variable region is as shown in SEQ ID No. 173 in the sequence listing; the nucleotide sequence of the nucleic acid encoding the heavy chain variable region is as shown in SEQ ID No. 162 in the sequence listing, and the nucleotide sequence of the nucleic acid encoding the light chain variable region is as shown in SEQ ID No. 174 in the sequence listing; the nucleotide sequence of the nucleic acid encoding the heavy chain variable region is as shown in SEQ ID No. 163 in the sequence listing, and the nucleotide sequence of the nucleic acid encoding the light chain variable region is as shown in SEQ ID No. 170 in the sequence listing; the nucleotide sequence of the nucleic acid encoding the heavy chain variable region is as shown in SEQ ID No. 163 in the sequence listing, and the nucleotide sequence of the nucleic acid encoding the light chain variable region is as shown in SEQ ID No. 171 in the sequence listing; the nucleotide sequence of the nucleic acid encoding the heavy chain variable region is as shown in SEQ ID No. 163 in the sequence listing, and the nucleotide sequence of the nucleic acid encoding the light chain variable region is as shown in SEQ ID No. 172 in the sequence listing; the nucleotide sequence of the nucleic acid encoding the heavy chain variable region is as shown in SEQ ID No. 163 in the sequence listing, and the nucleotide sequence of the nucleic acid encoding the light chain variable region is as shown in SEQ ID No. 173 in the sequence listing; the nucleotide sequence of the nucleic acid encoding the heavy chain variable region is as shown in SEQ ID No. 163 in the sequence listing, and the nucleotide sequence of the nucleic acid encoding the light chain variable region is as shown in SEQ ID No. 174 in the sequence listing; the nucleotide sequence of the nucleic acid encoding the heavy chain variable region is as shown in SEQ ID No. 166 in the sequence listing, and the nucleotide sequence of the nucleic acid encoding the light chain variable region is as shown in SEQ ID No. 169 in the sequence listing; the nucleotide sequence of the nucleic acid encoding the heavy chain variable region is as shown in SEQ ID No. 167 in the sequence listing, and the nucleotide sequence of the nucleic acid encoding the light chain variable region is as shown in SEQ ID No. 169 in the sequence listing; the nucleotide sequence of the nucleic acid encoding the heavy chain variable region is as shown in SEQ ID No. 168 in the sequence listing, and the nucleotide sequence of the nucleic acid encoding the light chain variable region is as shown in SEQ ID No. 169 in the sequence listing.

11. A recombinant expression vector comprising the nucleic acid as defined in any one of claims 8 to 10.

12. A recombinant expression transformant comprising the recombinant expression vector as defined in claim 11.

13. A method for preparing the EGFRvIII antibody, comprising the following steps: culturing the recombinant expression transformant as defined in claim 12, and obtaining the EGFRvIII antibody from the culture.

14. An immunoconjugate, wherein the immunoconjugate comprises the EGFRvIII antibody as defined in any one of claims 1 to 7 covalently attached to a cytotoxic agent.

15. The immunoconjugate as defined in claim 14, wherein 1 equivalent of the EGFRvIII antibody as defined in any one of claims 1 to 7 is linked to y equivalent of the cytotoxic agent via x equivalent of a linker, and the immunoconjugate has a structure as shown in formula 1,
Ab-(L)ₓ-(D)_{y} formula 1
where Ab is the EGFRvIII antibody as defined in any one of claims 1 to 7; L is a linker; D is a cytotoxic agent; x is a natural number, preferably 1 to 20; y is a natural number greater than 0, preferably 1 to 20; x and y are each independently and more preferably 2w, with w being an integer of 1 to 5, further preferably 3 and 4; and the ratio of x and y is preferably 1 : 1.

16. The immunoconjugate as defined in claim 15, wherein the linker L comprises the structure of formula 2, which is the remaining part in L after the leaving group leaves:
(CO-Alk¹-Sp¹-Ar-Sp²-Alk²-C(Z¹)=Q-Sp) formula 2
preferably, the linker L is maleimidocaproyl (MC) or maleimidocaproyl-L-valine-L-citrulline-p-aminobenzyl alcohol (MC-VC-PAB); and/or the D is monomethylauristatin F (MMAF) or monomethylauristatin E (MMAE).

17. The immunoconjugate as defined in any one of claims 14 to 16, wherein in the formula 1, x = y = n, and the structure of the immunoconjugate is as shown in formula 3-1 or 3-2, 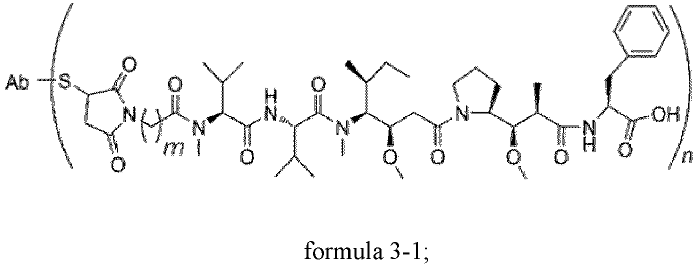 in the formula 3-1, m is 1 to 10, preferably m is 5, and L is maleimidocaproyl; D is monomethylauristatin F (MMAF); where n is a natural number, preferably an integer of 1 to 20, and more preferably 2w, with w being an integer of 1 to 5, further preferably 3 and 4; 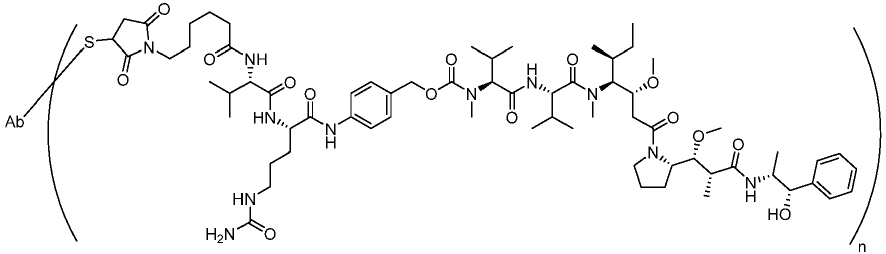 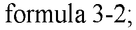 in the formula 3-2, m is 1 to 10, preferably m is 5, and L is maleimidocaproyl-L-valine-L-citrulline-p-aminobenzyl alcohol; D is monomethylauristatin E (MMAE); where n is a natural number, preferably an integer of 1 to 20, and more preferably 2w, with w being an integer of 1 to 5, further preferably 3 and 4.

18. A pharmaceutical composition, wherein the pharmaceutical composition comprises the EGFRvIII antibody as defined in any one of claims 1 to 7 or the immunoconjugate as defined in any one of claims 14 to 17, and a pharmaceutically acceptable carrier.

19. The pharmaceutical composition as defined in claim 18, wherein the pharmaceutical composition comprises 0.01% to 99.99% of the EGFRvIII antibody as defined in any one of claims 1 to 7 or the immunoconjugate as defined in any one of claims 14 to 17, and 0.01% to 99.99% of the pharmaceutically acceptable carrier, wherein the percentage is a mass percentage accounting for the pharmaceutical composition.

20. Use of the EGFRvIII antibody as defined in any one of claims 1 to 7, or the immunoconjugate as defined in any one of claims 14 to 17, or the pharmaceutical composition as defined in claim 18 or 19 in the preparation of an anti-tumor drug.

21. A method for detecting a cell overexpressing an EGFRvIII protein, wherein the method comprises the following steps: contacting the EGFRvIII antibody as defined in any one of claims 1 to 7 with a sample to be detected *in vitro,* then detecting the binding of the EGFRvIII antibody as defined in any one of claims 1 to 7 to the sample to be detected.

22. A composition for detecting a cell overexpressing an EGFRvIII protein, wherein the composition comprises the EGFRvIII antibody as defined in any one of claims 1 to 7 as an active ingredient.

23. Use of the EGFRvIII antibody as defined in any one of claims 1 to 7, or the immunoconjugate as defined in any one of claims 14 to 17, or the pharmaceutical composition as defined in claim 18 or 19 in the preparation of a drug for preventing or treating a disease related to abnormal expression or function of the EGFRvIII; preferably, the disease related to abnormal expression or function of the EGFRvIII is a tumor, wherein the tumor is preferably bladder cancer, brain cancer, head and neck cancer, pancreatic cancer, lung cancer, breast cancer, ovarian cancer, colon cancer, prostate cancer, or renal cancer.


SEQUENCE LISTING

<110>  ¿­»Ý¿Æ¼¼•¢Õ¹£¨ÉÏº££©ÓÐÏÞ¹«Ë¾

<120>  Ò»ÖÖEGFRvIII¿¹Ìå¼°ÆäÅ¼Áª Îï¡¢ÖÆ±¸•½•¨ºÍÓ¦ÓÃ

<130>  P184017307C

<150>  201711329680.1
<151>  2017-12-13

<160>  192

<170>  PatentIn version 3.5

<210>  1
<211>  124
<212>  PRT
<213>  Mus musculus

<400>  1

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Asn Gly Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Ser Val Thr Val Ser Ser
        115                 120


<210>  2
<211>  7
<212>  PRT
<213>  Mus musculus

<400>  2

Gly Tyr Thr Phe Thr Asp Tyr
1               5

<210>  3
<211>  6
<212>  PRT
<213>  Mus musculus

<400>  3

Asn Pro Asn Asn Gly Asp
1               5


<210>  4
<211>  15
<212>  PRT
<213>  Mus musculus

<400>  4

Gly Asp Tyr Asp Gly Tyr Tyr Pro Val Tyr Tyr Ala Met Asp Tyr
1               5                   10                  15


<210>  5
<211>  108
<212>  PRT
<213>  Mus musculus

<400>  5

Glu Asn Val Leu Thr Gln Ser Pro Ala Ile Met Ser Ala Ser Pro Gly
1               5                   10                  15


Glu Lys Val Thr Met Thr Cys Arg Ala Ser Ser Ser Val Ser Ser Ser
            20                  25                  30


Tyr Leu His Trp Tyr Gln Gln Lys Ser Gly Ala Ser Pro Lys Leu Trp
        35                  40                  45


Ile Phe Ser Thr Ser Asn Leu Ala Ala Gly Val Pro Ala Arg Phe Ser
    50                  55                  60


Gly Ser Gly Ser Gly Thr Ser Tyr Ser Leu Thr Val Ser Ser Val Glu
65                  70                  75                  80


Ala Glu Asp Ala Ala Thr Tyr Tyr Cys Gln Gln Tyr Ser Gly Tyr Pro
                85                  90                  95


Leu Thr Phe Gly Ala Gly Thr Lys Leu Glu Leu Lys
            100                 105


<210>  6
<211>  12
<212>  PRT
<213>  Mus musculus

<400>  6

Arg Ala Ser Ser Ser Val Ser Ser Ser Tyr Leu His
1               5                   10


<210>  7
<211>  7
<212>  PRT
<213>  Mus musculus

<400>  7

Ser Thr Ser Asn Leu Ala Ala
1               5


<210>  8
<211>  9
<212>  PRT
<213>  Mus musculus

<400>  8

Gln Gln Tyr Ser Gly Tyr Pro Leu Thr
1               5


<210>  9
<211>  117
<212>  PRT
<213>  Mus musculus

<400>  9

Gln Val Gln Leu Gln Gln Ser Gly Ala Glu Leu Ala Arg Pro Gly Ala
1               5                   10                  15


Ser Val Lys Met Ser Cys Lys Ala Ser Gly Tyr Thr Phe Ile Ser Phe
            20                  25                  30


Thr Met His Trp Val Lys Gln Arg Pro Gly Gln Gly Leu Glu Trp Ile
        35                  40                  45


Gly Tyr Ile Asn Pro Asn Ser Gly Tyr Thr Lys Tyr Asn Gln Lys Phe
    50                  55                  60


Lys Asp Lys Ala Thr Leu Thr Ala Gly Gln Ser Ser Ser Thr Ala Tyr
65                  70                  75                  80


Met Gln Leu Ser Ser Leu Thr Ser Glu Asp Ser Ala Val Tyr Tyr Cys
                85                  90                  95


Thr Arg Gly Gly Gly Arg Gly Phe Ala Tyr Trp Gly Gln Gly Thr Leu
            100                 105                 110

Val Thr Val Ser Ala
        115

<210>  10
<211>  7
<212>  PRT
<213>  Mus musculus

<400>  10

Gly Tyr Thr Phe Ile Ser Phe
1               5

<210>  11
<211>  6
<212>  PRT
<213>  Mus musculus

<400>  11

Asn Pro Asn Ser Gly Tyr
1               5

<210>  12
<211>  8
<212>  PRT
<213>  Mus musculus

<400>  12

Gly Gly Gly Arg Gly Phe Ala Tyr
1               5

<210>  13
<211>  107
<212>  PRT
<213>  Mus musculus

<400>  13

Asp Ile Leu Met Thr Gln Ser Pro Ser Ser Met Ser Val Ser Leu Gly
1               5                   10                  15

Asp Thr Val Thr Ile Thr Cys His Ala Ser Gln Asp Ile Asn Ser Asn
            20                  25                  30

Ile Gly Trp Leu Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Ser Leu Thr Ile Ser Ser Leu Glu Ser
65                  70                  75                  80

Glu Asp Phe Ala Asp Tyr Tyr Cys Val Gln Tyr Ala Gln Phe Pro Phe
                85                  90                  95

Thr Phe Gly Ser Gly Thr Lys Leu Glu Ile Lys
            100                 105

<210>  14
<211>  11
<212>  PRT
<213>  Mus musculus

<400>  14

His Ala Ser Gln Asp Ile Asn Ser Asn Ile Gly
1               5                   10

<210>  15
<211>  7
<212>  PRT
<213>  Mus musculus

<400>  15

His Gly Thr Asn Leu Glu Asp
1               5

<210>  16
<211>  9
<212>  PRT
<213>  Mus musculus

<400>  16

Val Gln Tyr Ala Gln Phe Pro Phe Thr
1               5

<210>  17
<211>  117
<212>  PRT
<213>  Mus musculus

<400>  17

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Ser Ser Tyr
            20                  25                  30

Thr Met His Trp Ala Lys Gln Arg Pro Gly Gln Gly Leu Glu Trp Ile
        35                  40                  45

Gly Tyr Ile Asn Pro Ser Ser Gly Tyr Thr Lys Tyr Asn Gln Lys Phe

50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ile Val Tyr Tyr Cys
                85                  90                  95

Ala Arg Gly Gly Gly Arg Gly Phe Ala Tyr Trp Gly Gln Gly Thr Leu
            100                 105                 110

Val Thr Val Ser Ala
        115

<210>  18
<211>  7
<212>  PRT
<213>  Mus musculus

<400>  18

Gly Tyr Thr Phe Ser Ser Tyr
1               5

<210>  19
<211>  6
<212>  PRT
<213>  Mus musculus

<400>  19

Asn Pro Ser Ser Gly Tyr
1               5

<210>  20
<211>  8
<212>  PRT
<213>  Mus musculus

<400>  20

Gly Gly Gly Arg Gly Phe Ala Tyr
1               5

<210>  21
<211>  106
<212>  PRT
<213>  Mus musculus

<400>  21

Gln Ile Val Leu Thr Gln Ser Pro Ala Ile Met Ser Ala Ser Pro Gly
1               5                   10                  15

Glu Arg Val Thr Met Thr Cys Ser Ala Ser Ser Ser Val Ser Tyr Met
            20                  25                  30

Tyr Trp Tyr Gln Gln Lys Pro Gly Ser Ser Pro Arg Leu Leu Ile Tyr
        35                  40                  45

Asp Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Val Glu Ala Glu
65                  70                  75                  80

Asp Ala Ala Thr Tyr Tyr Cys Gln Gln Trp Ser Ser Tyr Ser Leu Thr
                85                  90                  95

Phe Gly Ala Gly Thr Lys Leu Glu Leu Lys
            100                 105

<210>  22
<211>  10
<212>  PRT
<213>  Mus musculus

<400>  22

Ser Ala Ser Ser Ser Val Ser Tyr Met Tyr
1               5                   10

<210>  23
<211>  7
<212>  PRT
<213>  Mus musculus

<400>  23

Asp Thr Ser Asn Leu Ala Ser
1               5

<210>  24
<211>  9
<212>  PRT
<213>  Mus musculus

<400>  24

Gln Gln Trp Ser Ser Tyr Ser Leu Thr
1               5

<210>  25
<211>  114
<212>  PRT
<213>  Mus musculus

<400>  25

Glu Ile Gln Leu Gln Gln Ser Gly Pro Glu Leu Val Lys Pro Gly Ala
1               5                   10                  15

Ser Val Lys Val Ser Cys Lys Ala Ser Gly Tyr Ser Phe Thr Asp Tyr
            20                  25                  30

Asn Met Tyr Trp Val Lys Gln Ser His Gly Lys Ser Leu Glu Trp Ile
        35                  40                  45

Gly Tyr Val Asp Pro Ser Asn Gly Gly Thr Asn Tyr Asn Gln Lys Phe
    50                  55                  60

Lys Gly Lys Ala Thr Leu Thr Val Asp Lys Ser Ser Ser Thr Ala Phe
65                  70                  75                  80

Met His Leu Asn Ser Leu Thr Ser Glu Asp Ser Ala Val Tyr Tyr Cys
                85                  90                  95

Ala Arg Gly Phe Phe Asp Tyr Trp Gly Gln Gly Thr Thr Leu Thr Val
            100                 105                 110

Ser Ser

<210>  26
<211>  7
<212>  PRT
<213>  Mus musculus

<400>  26

Gly Tyr Ser Phe Thr Asp Tyr
1               5

<210>  27
<211>  6
<212>  PRT
<213>  Mus musculus

<400>  27

Asp Pro Ser Asn Gly Gly
1               5

<210>  28
<211>  5
<212>  PRT
<213>  Mus musculus

<400>  28

Gly Phe Phe Asp Tyr

1               5

<210>  29
<211>  113
<212>  PRT
<213>  Mus musculus

<400>  29

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Asn Arg Glu Ser Gly Val
    50                  55                  60

Pro Asp Arg Phe Thr Gly Ser Gly Ser Gly Lys Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Arg Tyr Pro Val Thr Phe Gly Ala Gly Thr Lys Leu Glu Leu
            100                 105                 110

Lys

<210>  30
<211>  17
<212>  PRT
<213>  Mus musculus

<400>  30

Lys Ser Ser Gln Ser Leu Leu Asn Ser Gly Asn Gln Lys Asn Tyr Leu
1               5                   10                  15

Thr

<210>  31
<211>  7
<212>  PRT
<213>  Mus musculus

<400>  31

Trp Ala Ser Asn Arg Glu Ser
1               5

<210>  32
<211>  9
<212>  PRT
<213>  Mus musculus

<400>  32

Gln Asn Asp Tyr Arg Tyr Pro Val Thr
1               5

<210>  33
<211>  133
<212>  PRT
<213>  Mus musculus

<400>  33

Cys Leu Gln Val Asp Asp Ser Asn Thr Thr His Tyr Arg Ala Ser Ser
1               5                   10                  15

Gly Ile Asn Ala Glu Tyr Ala Gly Gly Pro Gly Leu Val Lys Pro Ser
            20                  25                  30

Gln Ser Leu Ser Leu Thr Cys Thr Val Thr Asp Tyr Ser Ile Thr Ser
        35                  40                  45

Asp Tyr Ala Trp Asn Trp Ile Arg Gln Phe Pro Gly Asn Lys Leu Glu
    50                  55                  60

Trp Met Gly Tyr Ile Ser Tyr Ser Gly Asn Thr Asn Tyr Asn Pro Ser
65                  70                  75                  80

Leu Lys Ser Arg Ile Ser Ile Thr Arg Asp Thr Ser Lys Asn Gln Phe
                85                  90                  95

Phe Leu Gln Leu Asn Ser Val Thr Thr Glu Asp Thr Ala Thr Tyr Tyr
            100                 105                 110

Cys Ala Arg Leu Gly Arg Gly Phe Ala Tyr Trp Gly Gln Gly Thr Leu
        115                 120                 125

Val Thr Val Ser Ala
    130

<210>  34
<211>  8
<212>  PRT
<213>  Mus musculus

<400>  34

Asp Tyr Ser Ile Thr Ser Asp Tyr
1               5


<210>  35
<211>  5
<212>  PRT
<213>  Mus musculus

<400>  35

Ser Tyr Ser Gly Asn
1               5


<210>  36
<211>  7
<212>  PRT
<213>  Mus musculus

<400>  36

Leu Gly Arg Gly Phe Ala Tyr
1               5


<210>  37
<211>  107
<212>  PRT
<213>  Mus musculus

<400>  37

Asp Ile Leu Met Thr Gln Ser Pro Ser Ser Met Ser Val Ser Leu Gly
1               5                   10                  15


Asp Thr Val Ser Ile Thr Cys His Ala Ser Gln Asp Ile Asn Ser Asn
            20                  25                  30


Ile Gly Trp Leu Gln Gln Lys Pro Gly Thr Ser Phe Lys Gly Leu Ile
        35                  40                  45


Tyr His Gly Arg Asn Leu Glu Asp Gly Val Pro Ser Arg Phe Ser Gly
    50                  55                  60


Ser Gly Ser Gly Glu Asp Tyr Ser Leu Thr Ile Ser Ser Leu Glu Ser
65                  70                  75                  80


Glu Asp Phe Ala Asp Tyr Tyr Cys Val Gln Tyr Ala His Phe Pro Pro
                85                  90                  95


Thr Phe Gly Ala Gly Thr Lys Leu Glu Leu Lys
            100                 105

<210>  38
<211>  11
<212>  PRT
<213>  Mus musculus

<400>  38

His Ala Ser Gln Asp Ile Asn Ser Asn Ile Gly
1               5                   10


<210>  39
<211>  7
<212>  PRT
<213>  Mus musculus

<400>  39

His Gly Arg Asn Leu Glu Asp
1               5


<210>  40
<211>  9
<212>  PRT
<213>  Mus musculus

<400>  40

Val Gln Tyr Ala His Phe Pro Pro Thr
1               5


<210>  41
<211>  113
<212>  PRT
<213>  Mus musculus

<400>  41

Glu Val His Leu Gln Gln Ser Val Ala Glu Leu Val Arg Pro Gly Ala
1               5                   10                  15

Ser Val Lys Leu Ser Cys Thr Ala Ser Gly Phe Asn Ile Lys Asn Thr
            20                  25                  30

Tyr Met His Trp Val Lys Gln Arg Pro Glu Gln Gly Leu Glu Trp Ile
        35                  40                  45

Gly Arg Ile Asp Pro Ala Asn Gly Asn Thr Lys Tyr Ala Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Ser Leu Thr Ser Glu Asp Thr Ala Ile Tyr Tyr Cys
                85                  90                  95

Ala Ser Asp Phe Asp Tyr Trp Gly Gln Gly Thr Thr Leu Thr Val Ser
            100                 105                 110

Ser

<210>  42
<211>  7
<212>  PRT
<213>  Mus musculus

<400>  42

Gly Phe Asn Ile Lys Asn Thr
1               5

<210>  43
<211>  6
<212>  PRT
<213>  Mus musculus

<400>  43

Asp Pro Ala Asn Gly Asn
1               5

<210>  44
<211>  4
<212>  PRT
<213>  Mus musculus

<400>  44

Asp Phe Asp Tyr
1

<210>  45
<211>  112
<212>  PRT
<213>  Mus musculus

<400>  45

Asp Val Leu Met Thr Gln Thr Pro Leu Ser Leu Pro Val Ser Leu Gly
1               5                   10                  15

Asp Gln Val Ser Ile Ser Cys Arg Ser Ser Gln Ser Ile Val Asn Ser
            20                  25                  30

Asn Gly Asn Thr His Leu Glu Trp Tyr Leu Gln Lys Pro Gly Gln Ser
        35                  40                  45

Pro Lys Val Leu Ile Tyr Lys Val Ser Lys Arg Phe Ser Gly Val Pro

50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser His Val Pro Tyr Thr Phe Gly Gly Gly Thr Lys Leu Glu Ile Lys
            100                 105                 110

<210>  46
<211>  16
<212>  PRT
<213>  Mus musculus

<400>  46

Arg Ser Ser Gln Ser Ile Val Asn Ser Asn Gly Asn Thr His Leu Glu
1               5                   10                  15

<210>  47
<211>  7
<212>  PRT
<213>  Mus musculus

<400>  47

Lys Val Ser Lys Arg Phe Ser
1               5

<210>  48
<211>  9
<212>  PRT
<213>  Mus musculus

<400>  48

Phe Gln Gly Ser His Val Pro Tyr Thr
1               5

<210>  49
<211>  117
<212>  PRT
<213>  Mus musculus

<400>  49

Glu Val Gln Leu Leu Gln Ser Gly Ala Glu Leu Val Lys Pro Gly Ala
1               5                   10                  15

Ser Val Lys Leu Ser Cys Thr Ala Ser Gly Phe Asn Ile Lys Asp Tyr
            20                  25                  30

Tyr Met His Trp Val Lys Gln Arg Thr Glu Gln Gly Leu Glu Trp Ile
        35                  40                  45

Gly Arg Ile Asp Pro Glu Asp Gly Glu Thr Lys Tyr Ala Pro Lys Phe
    50                  55                  60

Gln Gly Lys Ala Thr Met Thr Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Ser Ser Ala Tyr Tyr Gly Arg Asn Tyr Trp Gly Gln Gly Thr Thr
            100                 105                 110

Leu Thr Val Ser Ser
        115

<210>  50
<211>  7
<212>  PRT
<213>  Mus musculus

<400>  50

Gly Phe Asn Ile Lys Asp Tyr
1               5

<210>  51
<211>  6
<212>  PRT
<213>  Mus musculus

<400>  51

Asp Pro Glu Asp Gly Glu
1               5

<210>  52
<211>  8
<212>  PRT
<213>  Mus musculus

<400>  52

Ser Ala Tyr Tyr Gly Arg Asn Tyr
1               5

<210>  53
<211>  108
<212>  PRT
<213>  Mus musculus

<400>  53

Gln Ile Val Leu Thr Gln Ser Pro Ala Ile Met Ser Ala Ser Pro Gly
1               5                   10                  15

Glu Lys Val Thr Leu Thr Cys Ser Ala Ser Ser Ser Leu Ser Ser Ser
            20                  25                  30

Tyr Leu Tyr Trp Tyr Gln Gln Lys Pro Gly Ser Ser Pro Lys Leu Trp
        35                  40                  45

Ile Tyr Ser Thr Ser Asn Leu Ala Ser Gly Val Pro Ala Arg Phe Ser
    50                  55                  60

Gly Ser Gly Ser Gly Thr Ser Tyr Ser Leu Thr Ile Ser Ser Met Glu
65                  70                  75                  80

Ala Glu Asp Ala Ala Ser Tyr Phe Cys His Gln Trp Ser Ser Tyr Pro
                85                  90                  95

Tyr Thr Phe Gly Gly Gly Thr Lys Leu Glu Ile Lys
            100                 105


<210>  54
<211>  12
<212>  PRT
<213>  Mus musculus

<400>  54

Ser Ala Ser Ser Ser Leu Ser Ser Ser Tyr Leu Tyr
1               5                   10


<210>  55
<211>  7
<212>  PRT
<213>  Mus musculus

<400>  55

Ser Thr Ser Asn Leu Ala Ser
1               5


<210>  56
<211>  9
<212>  PRT
<213>  Mus musculus

<400>  56

His Gln Trp Ser Ser Tyr Pro Tyr Thr
1               5


<210>  57

<211>  117
<212>  PRT
<213>  Mus musculus

<400>  57

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Phe
            20                  25                  30

Tyr Ile His Trp Val Lys Gln Arg Thr Glu Gln Gly Leu Glu Trp Ile
        35                  40                  45

Gly Arg Ile Asp Pro Glu Asp Gly Glu Thr Lys Tyr Ala Pro Lys Phe
    50                  55                  60

Gln Asp Lys Ala Thr Ile Ile Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Ser Ser Ser Tyr Tyr Gly Asn Asn Tyr Trp Gly Gln Gly Thr Thr
            100                 105                 110

Leu Thr Val Ser Ser
        115


<210>  58
<211>  7
<212>  PRT
<213>  Mus musculus

<400>  58

Gly Phe Asn Ile Lys Asp Phe
1               5


<210>  59
<211>  6
<212>  PRT
<213>  Mus musculus

<400>  59

Asp Pro Glu Asp Gly Glu
1               5


<210>  60
<211>  8
<212>  PRT

<213>  Mus musculus

<400>  60

Ser Ser Tyr Tyr Gly Asn Asn Tyr
1               5


<210>  61
<211>  108
<212>  PRT
<213>  Mus musculus

<400>  61

Gln Ile Val Leu Thr Gln Ser Pro Ala Ile Met Ser Ala Ser Pro Gly
1               5                   10                  15


Glu Lys Val Thr Leu Thr Cys Ser Ala Ser Ser Ser Val Ser Ser Asn
            20                  25                  30


Tyr Leu Tyr Trp Tyr Gln Gln Lys Pro Gly Ser Ser Pro Lys Leu Trp
        35                  40                  45


Ile Tyr Ser Thr Ser Asn Leu Ala Ser Gly Val Pro Ala Arg Phe Ser
    50                  55                  60


Gly Ser Gly Ser Gly Thr Ser Tyr Ser Leu Thr Ile Ser Ser Met Glu
65                  70                  75                  80


Ala Glu Asp Ala Ala Ser Tyr Phe Cys His Gln Trp Ser Ser Phe Pro
                85                  90                  95


Tyr Thr Phe Gly Gly Gly Thr Lys Leu Glu Ile Lys
            100                 105


<210>  62
<211>  12
<212>  PRT
<213>  Mus musculus

<400>  62

Ser Ala Ser Ser Ser Val Ser Ser Asn Tyr Leu Tyr
1               5                   10


<210>  63
<211>  7
<212>  PRT
<213>  Mus musculus

<400>  63

Ser Thr Ser Asn Leu Ala Ser
1               5

<210>  64
<211>  9
<212>  PRT
<213>  Mus musculus

<400>  64

His Gln Trp Ser Ser Phe Pro Tyr Thr
1               5

<210>  65
<211>  117
<212>  PRT
<213>  Mus musculus

<400>  65

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr
            20                  25                  30

Thr Met His Trp Val Lys Gln Arg Pro Gly Gln Gly Leu Glu Trp Ile
        35                  40                  45

Gly Tyr Ile Asn Pro Ser Ser Gly Tyr Thr Lys Tyr Asn Gln Ile Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ala
        115

<210>  66
<211>  7
<212>  PRT
<213>  Mus musculus

<400>  66

Gly Tyr Thr Phe Thr Ser Tyr
1               5

<210>  67
<211>  6
<212>  PRT
<213>  Mus musculus

<400>  67

Asn Pro Ser Ser Gly Tyr
1               5


<210>  68
<211>  8
<212>  PRT
<213>  Mus musculus

<400>  68

Gly Gly Gly Arg Gly Phe Ala Tyr
1               5


<210>  69
<211>  107
<212>  PRT
<213>  Mus musculus

<400>  69

Asp Ile Leu Met Thr Gln Ser Pro Ser Ser Met Ser Val Ser Leu Gly
1               5                   10                  15

Asp Thr Val Ser Ile Thr Cys His Ala Ser Gln Asp Ile Asn Ser Asn
            20                  25                  30

Ile Gly Trp Leu Gln Gln Lys Pro Gly Lys Ser Phe Lys Gly Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Ser Leu Thr Ile Ser Ser Leu Glu Ser
65                  70                  75                  80

Glu Asp Phe Ala Asp Tyr Tyr Cys Val Gln Tyr Ala Gln Phe Pro Phe
                85                  90                  95

Thr Phe Gly Ser Gly Thr Lys Leu Glu Ile Lys
            100                 105


<210>  70
<211>  11
<212>  PRT
<213>  Mus musculus

<400>  70

His Ala Ser Gln Asp Ile Asn Ser Asn Ile Gly
1               5                   10

<210>  71
<211>  7
<212>  PRT
<213>  Mus musculus

<400>  71

His Gly Thr Asn Leu Glu Asp
1               5

<210>  72
<211>  9
<212>  PRT
<213>  Mus musculus

<400>  72

Val Gln Tyr Ala Gln Phe Pro Phe Thr
1               5

<210>  73
<211>  116
<212>  PRT
<213>  Mus musculus

<400>  73

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Tyr
            20                  25                  30

Tyr Met His Trp Val Lys Gln Arg Thr Glu Gln Gly Leu Glu Trp Ile
        35                  40                  45

Gly Arg Ile Asp Pro Glu Asp Gly Glu Thr Lys Tyr Ala Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Asn Leu Pro Ser Glu Asp Thr Ala Val Tyr Tyr Cys
                85                  90                  95

Ala Cys Arg Thr Gly Gly Phe Ala Tyr Trp Gly Gln Gly Thr Leu Val
            100                 105                 110

Thr Val Ser Ala

        115

<210>  74
<211>  7
<212>  PRT
<213>  Mus musculus

<400>  74

Gly Phe Asn Ile Lys Asp Tyr
1               5

<210>  75
<211>  6
<212>  PRT
<213>  Mus musculus

<400>  75

Asp Pro Glu Asp Gly Glu
1               5

<210>  76
<211>  7
<212>  PRT
<213>  Mus musculus

<400>  76

Arg Thr Gly Gly Phe Ala Tyr
1               5

<210>  77
<211>  106
<212>  PRT
<213>  Mus musculus

<400>  77

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Leu
            20                  25                  30

Tyr Trp Tyr Gln Gln Lys Pro Gly Ser Ser Pro Ser Leu Leu Ile Tyr
        35                  40                  45

Asp Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Trp Ser Arg Asn Pro Trp Thr
                85                  90                  95

Phe Gly Gly Gly Thr Lys Leu Glu Ile Lys
            100                 105

<210>  78
<211>  10
<212>  PRT
<213>  Mus musculus

<400>  78

Ser Ala Ser Ser Ser Val Ser Tyr Met Tyr
1               5                   10

<210>  79
<211>  7
<212>  PRT
<213>  Mus musculus

<400>  79

Asp Thr Ser Asn Leu Ala Ser
1               5

<210>  80
<211>  9
<212>  PRT
<213>  Mus musculus

<400>  80

Gln Gln Trp Ser Ser Tyr Ser Leu Thr
1               5

<210>  81
<211>  116
<212>  PRT
<213>  Mus musculus

<400>  81

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Phe
            20                  25                  30

Tyr Met His Trp Val Lys Gln Arg Thr Glu Gln Gly Leu Glu Trp Ile
        35                  40                  45

Gly Arg Ile Asp Pro Glu Asp Gly Glu Thr Lys Tyr Ala Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Gly Cys Arg Thr Gly Gly Phe Ala Tyr Trp Gly Gln Gly Thr Leu Val
            100                 105                 110

Thr Val Ser Ala
        115

<210>  82
<211>  7
<212>  PRT
<213>  Mus musculus

<400>  82

Gly Phe Asn Ile Lys Asp Phe
1               5

<210>  83
<211>  6
<212>  PRT
<213>  Mus musculus

<400>  83

Asp Pro Glu Asp Gly Glu
1               5

<210>  84
<211>  7
<212>  PRT
<213>  Mus musculus

<400>  84

Arg Thr Gly Gly Phe Ala Tyr
1               5

<210>  85
<211>  106
<212>  PRT
<213>  Mus musculus

<400>  85

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Leu
            20                  25                  30

Tyr Trp Tyr Gln Gln Lys Pro Gly Ser Ser Pro Ser Leu Leu Ile Tyr
        35                  40                  45

Asp Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Trp Ser Arg Asn Pro Trp Thr
                85                  90                  95

Phe Gly Gly Gly Thr Lys Leu Glu Ile Lys
            100                 105

<210>  86
<211>  10
<212>  PRT
<213>  Mus musculus

<400>  86

Ser Ala Ser Ser Ser Val Ser Tyr Leu Tyr
1               5                   10

<210>  87
<211>  7
<212>  PRT
<213>  Mus musculus

<400>  87

Asp Thr Ser Asn Leu Ala Ser
1               5

<210>  88
<211>  9
<212>  PRT
<213>  Mus musculus

<400>  88

Gln Gln Trp Ser Arg Asn Pro Trp Thr
1               5

<210>  89
<211>  117
<212>  PRT
<213>  Mus musculus

<400>  89

Asp Val Gln Leu Gln Glu Ser Gly Pro Gly Leu Val Lys Pro Ser Gln

1               5                   10                  15

Ser Leu Ser Leu Thr Cys Ser Val Thr Gly Tyr Ser Ile Thr Ser Gly
            20                  25                  30

Tyr Tyr Trp His Trp Ile Arg Gln Phe Pro Glu Asn Lys Leu Glu Trp
        35                  40                  45

Met Gly Tyr Ile Ser Tyr Asp Gly Ser Asn Ile Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Thr Glu Asp Thr Ala Thr Tyr Tyr Cys
                85                  90                  95

Ala Ile Asn Ser Asp Tyr Ala Leu Asp Tyr Trp Gly Gln Gly Thr Ser
            100                 105                 110

Val Thr Val Ser Ser
        115

<210>  90
<211>  8
<212>  PRT
<213>  Mus musculus

<400>  90

Gly Tyr Ser Ile Thr Ser Gly Tyr
1               5

<210>  91
<211>  5
<212>  PRT
<213>  Mus musculus

<400>  91

Ser Tyr Asp Gly Ser
1               5

<210>  92
<211>  8
<212>  PRT
<213>  Mus musculus

<400>  92

Asn Ser Asp Tyr Ala Leu Asp Tyr
1               5

<210>  93
<211>  106
<212>  PRT
<213>  Mus musculus

<400>  93

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

Tyr Trp Tyr Gln Gln Lys Pro Gly Ser Ser Pro Arg Leu Leu Ile Tyr
        35                  40                  45

Asp Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Trp Ser Ser Tyr Ser Leu Thr
                85                  90                  95

Phe Gly Ala Gly Thr Lys Leu Glu Leu Lys
            100                 105


<210>  94
<211>  10
<212>  PRT
<213>  Mus musculus

<400>  94

Ser Ala Ser Ser Ser Val Ser Tyr Met Tyr
1               5                   10


<210>  95
<211>  7
<212>  PRT
<213>  Mus musculus

<400>  95

Asp Thr Ser Asn Leu Ala Ser
1               5


<210>  96
<211>  9
<212>  PRT
<213>  Mus musculus

<400>  96

Gln Gln Trp Ser Ser Tyr Ser Leu Thr
1               5

<210>  97
<211>  116
<212>  PRT
<213>  Mus musculus

<400>  97

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Tyr
            20                  25                  30

Tyr Met His Trp Val Lys Gln Arg Thr Glu Gln Gly Leu Glu Trp Ile
        35                  40                  45

Gly Arg Ile Asp Pro Glu Asp Gly Glu Thr Lys Tyr Ala Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Asn Leu Pro Ser Glu Asp Thr Ala Val Tyr Tyr Cys
                85                  90                  95

Gly Cys Arg Thr Gly Gly Phe Ala Tyr Trp Gly Gln Gly Thr Leu Val
            100                 105                 110

Thr Val Ser Ala
        115

<210>  98
<211>  7
<212>  PRT
<213>  Mus musculus

<400>  98

Gly Phe Asn Ile Lys Asp Tyr
1               5

<210>  99
<211>  6
<212>  PRT
<213>  Mus musculus

<400>  99

Asp Pro Glu Asp Gly Glu
1               5

<210>  100
<211>  7
<212>  PRT
<213>  Mus musculus

<400>  100

Arg Thr Gly Gly Phe Ala Tyr
1               5

<210>  101
<211>  107
<212>  PRT
<213>  Mus musculus

<400>  101

Asp Ile Leu Met Thr Gln Ser Pro Ser Ser Met Ser Val Ser Leu Gly
1               5                   10                  15

Asp Thr Val Ser Ile Thr Cys His Ala Ser Gln Gly Ile Asn Ser Asn
            20                  25                  30

Ile Gly Trp Leu Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Ser Leu Thr Ile Ser Ser Leu Glu Ser
65                  70                  75                  80

Glu Asp Phe Ala Asp Tyr Tyr Cys Val Gln Tyr Ala Gln Phe Pro Phe
                85                  90                  95

Thr Phe Gly Ser Gly Thr Glu Leu Glu Ile Lys
            100                 105

<210>  102
<211>  11
<212>  PRT
<213>  Mus musculus

<400>  102

His Ala Ser Gln Gly Ile Asn Ser Asn Ile Gly
1               5                   10

<210>  103
<211>  7

<212>  PRT
<213>  Mus musculus

<400>  103

His Gly Thr Asn Leu Glu Asp
1               5


<210>  104
<211>  9
<212>  PRT
<213>  Mus musculus

<400>  104

Val Gln Tyr Ala Gln Phe Pro Phe Thr
1               5


<210>  105
<211>  372
<212>  DNA
<213>  Mus musculus

<400>  105
   gaggtccagc tgcaacagtc tggacctgag ctggtgaagc ctggggcttc agtgaagata      60
   ccctgcaagg cttctggata cacattcact gactacaaca tggactgggt gaagcagagc     120
   catggaaaga gccttgagtg gattggagat attaatccta acaatggtga tactatctac     180
   aaccagaagt tcaagggcaa ggccacattg actgtagaca agtcctccag cacagcctac     240
   atggagctcc gcagcctgac atctgaggac actgcagtct attactgtgc aaggggggac     300
   tatgatggtt actacccggt ttactatgct atggactatt ggggtcaagg aacctcagtc     360
   accgtctcct ca                                                         372


<210>  106
<211>  324
<212>  DNA
<213>  Mus musculus

<400>  106
gaaaatgtgc tcacccagtc tccagcaatc atgtctgcat ctccagggga aaaggtcacc      60

atgacctgca gggccagctc aagtgtaagt tccagttact tgcactggta ccagcagaag     120

tcaggtgcct cccccaaact ctggattttt agcacatcca acttggctgc tggagtccct     180

gctcgcttca gtggcagtgg gtctgggacc tcttactctc tcacagtcag cagtgtggag     240

gctgaagatg ctgccactta ttactgccag cagtacagtg gttacccact cacgttcggt     300

gctgggacca agctggagct gaaa                                            324


<210>  107
<211>  351
<212>  DNA

<213>  Mus musculus

<400>  107
caggtccagc tgcagcagtc tggggctgaa ctggcaagac ctggcgcctc agtgaagatg      60

tcctgcaagg cttctggcta cacctttatt agcttcacga tgcactgggt aaaacagagg      120

cctggacagg gtctggaatg gattggatac attaatccta acagtggtta tactaagtac      180

aatcagaagt tcaaggacaa ggccacattg actgcaggcc aatcctccag cacagcctac      240

atgcaactga gcagcctgac atctgaggac tctgcagtct attactgtac aagagggggg      300

ggacgagggt ttgcttactg gggccaaggg actctggtca ctgtctctgc a      351


<210>  108
<211>  321
<212>  DNA
<213>  Mus musculus

<400>  108
gacatcctga tgacccaatc tccatcctcc atgtctgtat ctctgggaga cacagtcacc      60

atcacttgcc atgcaagtca ggacattaac agtaatatag ggtggttgca gcagaaacca      120

gggaaatcat ttaaggccct gatctatcat ggaaccaact tggaagatgg agttccatca      180

aggttcagtg gcagtggatc tggagcagac tattctctca ccatcagcag cctggaatct      240

gaagattttg cagactatta ctgtgtacag tatgctcagt ttccattcac gttcggctcg      300

gggacaaagt tggaaataaa a      321


<210>  109
<211>  351
<212>  DNA
<213>  Mus musculus

<400>  109
caggtccagc tgcagcagtc tggggctgaa ctggcaagac ctggtgcctc agtgaagatg      60

tcctgcaagg cttctggcta cacctttcct agctacacga tgcactgggc aaaacagagg      120

cctggacagg gtcttgaatg gattgggtac attaatccta gcagtggtta tacaaagtac      180

aatcagaagt tcaaggacaa ggccacattg actgcagaca aatcctccag cacagcctat      240

atgcaactga gcagcctgac atctgaggac tctatagtct attactgtgc aagagggggg      300

ggacgagggt ttgcttactg gggccaaggg actctggtca ctgtctctgc a      351


<210>  110
<211>  318
<212>  PRT
<213>  Mus musculus

<400>  110

Cys Ala Ala Ala Thr Thr Gly Thr Thr Cys Thr Cys Ala Cys Cys Cys
1               5                   10                  15

Ala Gly Thr Cys Thr Cys Cys Ala Gly Cys Ala Ala Thr Cys Ala Thr
            20                  25                  30

Gly Thr Cys Thr Gly Cys Ala Thr Cys Thr Cys Cys Ala Gly Gly Gly
        35                  40                  45

Gly Ala Gly Ala Gly Gly Gly Thr Cys Ala Cys Cys Ala Thr Gly Ala
    50                  55                  60

Cys Cys Thr Gly Cys Ala Gly Thr Gly Cys Cys Ala Gly Cys Thr Cys
65                  70                  75                  80

Ala Ala Gly Thr Gly Thr Ala Ala Gly Thr Thr Ala Cys Ala Thr Gly
                85                  90                  95

Thr Ala Cys Thr Gly Gly Thr Ala Cys Cys Ala Gly Cys Ala Gly Ala
            100                 105                 110

Ala Gly Cys Cys Ala Gly Gly Ala Thr Cys Cys Thr Cys Cys Cys Cys
        115                 120                 125

Cys Ala Gly Ala Cys Thr Cys Cys Thr Gly Ala Thr Thr Thr Ala Thr
    130                 135                 140

Gly Ala Cys Ala Cys Ala Thr Cys Cys Ala Ala Cys Cys Thr Gly Gly
145                 150                 155                 160

Cys Thr Thr Cys Thr Gly Gly Ala Gly Thr Cys Cys Cys Thr Gly Thr
                165                 170                 175

Thr Cys Gly Cys Thr Thr Cys Ala Gly Thr Gly Gly Cys Ala Gly Thr
            180                 185                 190

Gly Gly Gly Thr Cys Thr Gly Gly Gly Ala Cys Cys Thr Cys Thr Thr
        195                 200                 205

Ala Thr Thr Cys Thr Cys Thr Cys Ala Cys Ala Ala Thr Cys Ala Gly
    210                 215                 220

Cys Cys Gly Ala Gly Thr Gly Gly Ala Gly Gly Cys Thr Gly Ala Ala
225                 230                 235                 240

Gly Ala Thr Gly Cys Cys Gly Cys Cys Ala Cys Thr Thr Ala Thr Thr
                245                 250                 255

Ala Cys Thr Gly Cys Cys Ala Gly Cys Ala Gly Thr Gly Gly Ala Gly

260                 265                 270

Thr Ala Gly Thr Thr Ala Cys Thr Cys Ala Cys Thr Cys Ala Cys Gly
        275                 280                 285

Thr Thr Cys Gly Gly Thr Gly Cys Thr Gly Gly Gly Ala Cys Cys Ala
    290                 295                 300

Ala Gly Cys Thr Gly Gly Ala Gly Cys Thr Gly Ala Ala Ala
305                 310                 315

<210>  111
<211>  342
<212>  DNA
<213>  Mus musculus

<400>  111
gagatccagc tgcagcagtc tggacctgag ctggtgaagc ctggggcttc agtgaaggta      60

tcctgcaagg cttctggtta ctcattcact gactacaaca tgtactgggt gaagcagagc     120

catggaaaga gccttgagtg gattggatat gttgatcctt ccaatggtgg tactaactac     180

aaccagaagt tcaagggcaa ggccacattg actgttgaca agtcctccag cacagccttc     240

atgcatctca acagcctgac atctgaggac tctgcagtct attactgtgc aagagggttc     300

tttgactact ggggccaagg caccactctc acagtctcct ca                        342

<210>  112
<211>  339
<212>  DNA
<213>  Mus musculus

<400>  112
gacattgtga tgacacagtc tccatcctcc ctgactgtga cagcaggaga gaaggtcact      60

atgagctgca agtccagtca gagtctgtta aacagtggaa atcaaaagaa ctacttgacc     120

tggtaccagc agaaaccagg gcagcctcct aaactgttga tctactgggc atccaatagg     180

gaatctgggg tccctgatcg cttcacaggc agtggatctg gaaaagattt cactctcacc     240

atcagcagtg tgcaggctga agacctggca gtttattact gtcagaatga ttataggtat     300

ccggtcacgt tcggtgctgg gaccaagctg gagctgaaa                            339

<210>  113
<211>  399
<212>  DNA
<213>  Mus musculus

<400>  113
tgcctgcagg tcgacgattc taatacgact cactataggg caagcagtgg tatcaacgca      60

gagtacgcgg ggggacctgg cctggtgaaa ccttctcagt ctctgtccct cacctgcact     120

gtcactgact actcaatcac cagtgattat gcctggaact ggatccggca gtttccagga 180

aacaaactgg agtggatggg ttacataagc tacagtggta acactaacta caacccatct 240

ctcaaaagtc gaatctctat cacccgagac acatccaaga accagttctt cctgcagttg 300

aattctgtga ctactgagga cacagccaca tattactgtg caagactggg acgagggttt 360

gcttactggg gccaagggac tctggtcact gtctctgca 399

<210> 114
<211> 321
<212> DNA
<213> Mus musculus

<400> 114
gacatcctga tgacccaatc tccatcctcc atgtctgtat ctctgggaga cacagtcagc 60

atcacttgcc atgcaagtca ggacattaac agtaatattg ggtggctgca gcagaaacca 120

gggacatcat ttaagggcct gatctatcat ggaagaaact tggaagatgg agttccatca 180

aggttcagcg gcagtggatc tggagaagat tattctctca ccatcagcag cctggaatct 240

gaagattttg cagactatta ctgtgtacag tatgctcatt ttcctcccac gttcggtgct 300

gggaccaaac tggagctgaa g 321

<210> 115
<211> 339
<212> DNA
<213> Mus musculus

<400> 115
gaggttcacc tgcagcagtc tgtggccgag cttgtaaggc caggggcctc agtcaagttg 60

tcctgcacag cttctggctt caacattaaa aacacctata tgcactgggt gaagcagagg 120

cctgaacagg gcctggagtg gattggaagg attgatcctg cgaatggtaa tactaaatat 180

gccccgaagt tccagggcaa ggccactata actgcagaca catcctccaa cacagcctac 240

ctgcagctca gcagcctgac atctgaggac actgccatct attactgtgc tagtgacttt 300

gactactggg gccaaggcac cactctcaca gtctcctca 339

<210> 116
<211> 336
<212> DNA
<213> Mus musculus

<400> 116
gatgttttga tgacccaaac tccactctcc ctgcctgtca gtcttggaga tcaagtctcc 60

atctcttgca gatctagtca gagcattgta aatagtaatg gaaataccca tttagaatgg 120

tacctgcaga aaccaggcca gtctccaaag gtcctgatct acaaagtttc caaacgattt 180

tctggggtcc cagacaggtt cagtggcagt ggatcaggga cagatttcac actcaagatc 240

agcagagtgg aggctgagga tctgggagtt tattactgct ttcaaggttc acatgttccg 300

tacacgttcg gaggggggac caagctggaa ataaaa 336

<210> 117
<211> 351
<212> DNA
<213> Mus musculus

<400> 117
gaggttcagc tgctgcagtc tggggcagaa cttgtgaagc caggggcctc agtcaagttg 60

tcctgcacag cttctggctt caacattaaa gactactata tgcactgggt gaaacagagg 120

actgaacagg gcctggagtg gattggaagg attgatcctg aggatggtga aactaaatat 180

gccccgaaat tccagggcaa ggccactatg acagcggaca catcctccaa cacagcctac 240

ctgcagctca gcagcctgac atctgaggac actgccgtct attattgtgc tagttccgcc 300

tactacggtc gtaactactg gggccaaggc accactctca cagtctcctc c 351

<210> 118
<211> 324
<212> DNA
<213> Mus musculus

<400> 118
caaattgttc tcacccagtc tccagcaatc atgtctgcat ctcctgggga gaaggtcacc 60

ttgacctgca gtgccagttc tagtttaagt tccagctact tgtactggta ccagcagaag 120

ccaggatcct cccccaaact ctggatttat agcacatcca acctggcttc tggagtccct 180

gctcgcttca gtggcagtgg gtctgggacc tcttactctc tcacaatcag cagcatggag 240

gctgaagatg ctgcctctta tttctgccat cagtggagta gttacccgta cacgttcgga 300

ggggggacca agctggaaat aaaa 324

<210> 119
<211> 351
<212> DNA
<213> Mus musculus

<400> 119
gaggttcagc tgcagcagtc tggggcagag cttgtgaagc caggggcctc agtcaagttg 60

tcctgcacag cttctggctt caacattaaa gacttctata tacactgggt gaagcagagg 120

actgaacagg gcctggagtg gattggaagg attgatcctg aggatggtga aactaaatat 180

gccccgaaat tccaggacaa ggccactata atagcagaca catcctccaa cacagcctac 240

ctgcagctca gcagcctgac atctgaggac actgccgtct attactgtgc tagctcctcc 300

tactacggta ataactactg gggccaaggc accactctca cagtctcctc a 351

<210> 120

<211>  324
<212>  DNA
<213>  Mus musculus

<400>  120
caaattgttc tcacccagtc tccagcaatc atgtctgcat ctcctgggga gaaggtcacc      60

ttgacctgca gtgccagctc aagtgtaagt tccaactact tgtactggta ccagcagaag     120

ccaggatcct cccccaaact ctggatttat agcacatcca acctggcttc tggagtccct     180

gctcgcttca gtggcagtgg gtctgggacc tcttactctc tcacaatcag cagcatggag     240

gctgaagatg ctgcctctta tttctgccat cagtggagta gtttcccgta cacgttcgga     300

ggggggacca agctggaaat aaaa                                            324


<210>  121
<211>  351
<212>  DNA
<213>  Mus musculus

<400>  121
caggtccagc tgcagcagtc tggggctgaa ctggcaagac ctggtgcctc agtgaagatg      60

tcctgcaagg cttctggcta cacctttact agttacacga tgcactgggt aaaacagagg     120

cctggacagg gtctggaatg gattggatac attaatccta gcagtggtta tactaagtac     180

aatcagattt tcaaggacaa ggccacattg actgcagaca aatcctccag cacagcctac     240

atgcaactga gcagcctgac atctgaggac tctgcagtgt attactgtac aagagggggg     300

ggacgagggt ttgcttactg gggccaaggg actctggtca ctgtctctgc a              351


<210>  122
<211>  321
<212>  DNA
<213>  Mus musculus

<400>  122
gacatcctga tgacccaatc tccatcctcc atgtctgtat ctctgggaga cacagtcagc      60

atcacttgcc atgcaagtca ggacattaac agtaatatag ggtggttgca gcagaaacca     120

gggaaatcat ttaagggcct gatctatcat ggaaccaact tggaagatgg agttccatca     180

aggttcagtg gcagtggatc tggagcagat tattctctca ccatcagcag cctggaatct     240

gaagattttg cagactatta ctgtgtacag tatgctcagt ttccattcac gttcggctcg     300

gggacaaagt tggaaataaa a                                               321


<210>  123
<211>  348
<212>  DNA
<213>  Mus musculus

<400>  123
gaggttcagc tgcagcagtc tggggcagag cttgtgaagc caggggcctc agtcaagttg      60

tcctgcacag cttctggctt caatattaaa gactactata tgcactgggt gaagcagagg 120

actgaacagg gcctggagtg gattggaagg attgatcctg aggatggtga aactaaatat 180

gccccgaaat tccagggcaa ggccactata acagcagaca catcctccaa cacagcctac 240

ctgcagctca gcaacctgcc atctgaggac actgccgtct attactgtgc ttgtaggacg 300

ggggggtttg cttactgggg ccaagggact ctggtcactg tctctgca 348

<210> 124
<211> 318
<212> DNA
<213> Mus musculus

<400> 124
caaattgttc tcacccagtc tccagcaatc atgtctgcat ctccagggga gaaggtcacc 60

atgacctgca gtgccagctc aagtgtaagt tacatgtact ggtaccagca gaagccagga 120

tcctccccca gactcctgat ttatgacaca tccaacctgg cttctggagt ccctgttcgc 180

ttcagtggca gtgggtctgg gacctcttat tctctcacaa tcagccgagt ggaggctgaa 240

gatgccgcca cttattactg ccagcagtgg agtagttact cactcacgtt cggtgctggg 300

accaagctgg agctgaaa 318

<210> 125
<211> 348
<212> DNA
<213> Mus musculus

<400> 125
gaggttcagc tgcagcagtc tggggcagag cttgtgaagc caggggcctc agtcaagttg 60

tcctgcacag cttctggctt caacattaaa gacttctata tgcactgggt gaagcagagg 120

actgaacagg gcctggagtg gattggaagg attgatcctg aggatggtga aactaaatat 180

gccccgaaat tccagggcaa ggccactata acagcagata catcctccaa cacagcctac 240

ctgcagctca gcagcctgac atctgaggac actgccgtct attactgtgg ttgtaggacg 300

ggggggtttg cgtactgggg ccaagggact ctggtcactg tctctgca 348

<210> 126
<211> 318
<212> DNA
<213> Mus musculus

<400> 126
caaattgttc tcacccagtc tccagcaatc atgtctgcat ctccagggga gaaggtcacc 60

atgacctgca gtgccagctc aagtgtaagt tacctatact ggtaccagca gaagccagga 120

tcctccccca gcctcctgat ttatgacaca tccaacctgg cttctggagt ccctgttcgc 180

ttcagtggca gtgggtctgg gacctcttac tctctcacaa tcagccgaat ggaggctgaa 240

gatgctgcca cttattattg ccagcagtgg agtagaaacc catggacgtt cggtggaggc 300

accaagctgg aaatcaaa 318

<210> 127
<211> 351
<212> DNA
<213> Mus musculus

<400> 127
gatgtacaac ttcaggagtc aggacctggc ctcgtgaaac cttctcagtc tctgtctctc 60

acctgctctg tcactggcta ctccatcacc agtggttatt actggcactg gatccggcag 120

tttccagaaa acaaactgga atggatgggc tacataagct acgatggtag caatatctac 180

aacccatctc tcaaaaatcg aatctccatc actcgtgaca catctaagaa ccagtttttc 240

ctgaagttga attctgtgac tactgaggac acagccacat attactgtgc aattaactcg 300

gactatgctt tggactactg gggtcaagga acctcagtca ccgtctcctc a 351

<210> 128
<211> 318
<212> DNA
<213> Mus musculus

<400> 128
caaattgttc tcacccagtc tccagcaatc atgtctgcat ctccagggga gaaggtcacc 60

atgacctgca gtgccagctc aagtgtgagt tacatgtact ggtaccagca gaagccagga 120

tcctccccca gactcctgat ttatgacaca tccaacctgg cttctggagt ccctgttcgc 180

ttcagtggca gtgggtctgg gacctcttac tctctcacaa tcagccgaat ggaggctgaa 240

gatgctgcca cttattactg ccagcagtgg agtagttact cactcacgtt cggtgctggg 300

accaagctgg agctgaaa 318

<210> 129
<211> 348
<212> DNA
<213> Mus musculus

<400> 129
gaggttcagc tgcagcagtc tggggcagaa cttgtgaagc caggggcctc agtcaagttg 60

tcctgcacag cttctggctt caacattaaa gactactata tgcactgggt gaagcagagg 120

actgaacagg gcctggagtg gattggaagg attgatcctg aggatggtga aactaaatat 180

gccccgaaat tccagggcaa ggccactata acagcagaca catcctccaa cacagcctac 240

ctgcagctca gcaacctgcc atctgaggac actgccgtct attactgtgg ttgtaggacg 300

ggggggtttg cttactgggg ccaagggact ctggtcactg tctctgca 348

<210>  130
<211>  321
<212>  DNA
<213>  Mus musculus

<400>  130
gacatcctga tgacccaatc tccatcctcc atgtctgtat ctctgggaga cacagtcagc      60

atcacttgcc atgcaagtca gggcattaac agtaatatag ggtggttgca gcagaaacca     120

gggaaatcat ttaaggccct gatctatcat ggaaccaact tggaagatgg agttccgtca     180

aggttcagtg gcagtggatc tggagcagat tattctctca ccatcagcag cctggaatct     240

gaagattttg cagactatta ctgtgtacag tatgctcagt ttccattcac gttcggctcg     300

gggactgagt tggaaataaa a                                               321


<210>  131
<211>  372
<212>  DNA
<213>  Artificial Sequence

<220>
<223>  Ç¶°Ï¿¹Ìå75G7C6ÖØÁ´¿É±äÇø¶¨µãÍ»±ä£¨DG/EG£©

<400>  131
gaggtccagc tgcaacagtc tggacctgag ctggtgaagc ctggggcttc agtgaagata      60

ccctgcaagg cttctggata cacattcact gactacaaca tggactgggt gaagcagagc     120

catggaaaga gccttgagtg gattggagat attaatccta acaatggtga tactatctac     180

aaccagaagt tcaagggcaa ggccacattg actgtagaca agtcctccag cacagcctac     240

atggagctcc gcagcctgac atctgaggac actgcagtct attactgtgc aaggggggac     300

tatgaaggtt actacccggt ttactatgct atggactatt ggggtcaagg aacctcagtc     360

accgtctcct ca                                                         372


<210>  132
<211>  372
<212>  DNA
<213>  Artificial Sequence

<220>
<223>  Ç¶°Ï¿¹Ìå75G7C6ÖØÁ´¿É±äÇø¶¨µãÍ»±ä£¨DG/DA£©

<400>  132
gaggtccagc tgcaacagtc tggacctgag ctggtgaagc ctggggcttc agtgaagata      60

ccctgcaagg cttctggata cacattcact gactacaaca tggactgggt gaagcagagc     120

catggaaaga gccttgagtg gattggagat attaatccta acaatggtga tactatctac     180

aaccagaagt tcaagggcaa ggccacattg actgtagaca agtcctccag cacagcctac     240

atggagctcc gcagcctgac atctgaggac actgcagtct attactgtgc aaggggggac     300

tatgatgctt actacccggt ttactatgct atggactatt ggggtcaagg aacctcagtc     360

accgtctcct ca                                                          372

<210>  133
<211>  124
<212>  PRT
<213>  Artificial Sequence

<220>
<223>  h75G7C6. VH-g0

<400>  133

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Asn Met Asp Trp Val Arg Gln Ala Pro Gly Gln Gly Leu Glu Trp Met
        35                  40                  45

Gly Asp Ile Asn Pro Asn Asn Ala Asp Thr Ile Tyr Asn Gln Lys Phe
    50                  55                  60

Lys Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Thr Val Thr Val Ser Ser
        115                 120


<210>  134
<211>  124
<212>  PRT
<213>  Artificial Sequence

<220>
<223>  h75G7C6. VH-g1

<400>  134

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Asn Met Asp Trp Val Arg Gln Ala Pro Gly Gln Gly Leu Glu Trp Ile
        35                  40                  45

Gly Asp Ile Asn Pro Asn Asn Ala Asp Thr Ile Tyr Asn Gln Lys Phe
    50                  55                  60

Lys Gly Arg Val Thr Leu Thr Val Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Thr Val Thr Val Ser Ser
        115                 120

<210> 135
<211> 124
<212> PRT
<213> Artificial Sequence

<220>
<223> h75G7C6. VH-g2

<400> 135

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Asn Met Asp Trp Val Lys Gln Ala Pro Gly Gln Gly Leu Glu Trp Ile
        35                  40                  45

Gly Asp Ile Asn Pro Asn Asn Ala Asp Thr Ile Tyr Asn Gln Lys Phe
    50                  55                  60

Lys Gly Arg Val Thr Leu Thr Val Asp Lys Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Thr Val Thr Val Ser Ser
        115                 120

<210>  136
<211>  124
<212>  PRT
<213>  Artificial Sequence

<220>
<223>  h75G7C6. VH-g3

<400>  136

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Asn Met Asp Trp Val Lys Gln Ala Pro Gly Gln Gly Leu Glu Trp Ile
        35                  40                  45

Gly Asp Ile Asn Pro Asn Asn Ala Asp Thr Ile Tyr Asn Gln Lys Phe
    50                  55                  60

Lys Gly Arg Val Thr Leu Thr Val Asp Lys Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Thr Val Thr Val Ser Ser
        115                 120

<210>  137
<211>  108
<212>  PRT
<213>  Artificial Sequence

<220>
<223>  h75G7C6. VL-g0

<400>  137

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Ser Ser Val Ser Ser Ser
            20                  25                  30

Tyr Leu His Trp Tyr Gln Gln Lys Pro Gly Gln Ala Pro Arg Leu Leu
        35                  40                  45

Ile Tyr Ser Thr Ser Asn Leu Ala Ala Gly Ile Pro Ala Arg Phe Ser
    50                  55                  60

Gly Ser Gly Ser Gly Thr Asp Phe Thr Leu Thr Ile Ser Ser Leu Glu
65                  70                  75                  80

Pro Glu Asp Phe Ala Val Tyr Tyr Cys Gln Gln Tyr Ser Gly Tyr Pro
                85                  90                  95

Leu Thr Phe Gly Gly Gly Thr Lys Val Glu Ile Lys
            100                 105

<210>  138
<211>  108
<212>  PRT
<213>  Artificial Sequence

<220>
<223>  h75G7C6. VL-g1

<400>  138

Glu Asn Val Leu Thr Gln Ser Pro Ala Thr Leu Ser Leu Ser Pro Gly
1               5                   10                  15

Glu Arg Ala Thr Leu Ser Cys Arg Ala Ser Ser Ser Val Ser Ser Ser
            20                  25                  30

Tyr Leu His Trp Tyr Gln Gln Lys Pro Gly Gln Ala Pro Arg Leu Leu
        35                  40                  45

Ile Phe Ser Thr Ser Asn Leu Ala Ala Gly Ile Pro Ala Arg Phe Ser
    50                  55                  60

Gly Ser Gly Ser Gly Thr Asp Tyr Thr Leu Thr Ile Ser Ser Leu Glu
65                  70                  75                  80

Pro Glu Asp Phe Ala Val Tyr Tyr Cys Gln Gln Tyr Ser Gly Tyr Pro
                85                  90                  95

Leu Thr Phe Gly Gly Gly Thr Lys Val Glu Ile Lys
            100                 105

<210>  139
<211>  108
<212>  PRT
<213>  Artificial Sequence

<220>
<223>  h75G7C6. VL-g2

<400>  139

Glu Asn Val Leu Thr Gln Ser Pro Ala Thr Leu Ser Leu Ser Pro Gly
1               5                   10                  15

Glu Arg Ala Thr Leu Ser Cys Arg Ala Ser Ser Ser Val Ser Ser Ser
            20                  25                  30

Tyr Leu His Trp Tyr Gln Gln Lys Pro Gly Gln Ser Pro Arg Leu Leu
        35                  40                  45

Ile Phe Ser Thr Ser Asn Leu Ala Ala Gly Ile Pro Ala Arg Phe Ser
    50                  55                  60

Gly Ser Gly Ser Gly Thr Asp Tyr Thr Leu Thr Ile Ser Ser Leu Glu
65                  70                  75                  80

Pro Glu Asp Phe Ala Val Tyr Tyr Cys Gln Gln Tyr Ser Gly Tyr Pro
                85                  90                  95

Leu Thr Phe Gly Gly Gly Thr Lys Val Glu Ile Lys
            100                 105


<210>  140
<211>  108
<212>  PRT
<213>  Artificial Sequence

<220>
<223>  h75G7C6. VL-g3

<400>  140

Glu Asn Val Leu Thr Gln Ser Pro Ala Thr Leu Ser Leu Ser Pro Gly
1               5                   10                  15

Glu Arg Ala Thr Leu Ser Cys Arg Ala Ser Ser Ser Val Ser Ser Ser
            20                  25                  30

Tyr Leu His Trp Tyr Gln Gln Lys Pro Gly Gln Ser Pro Arg Leu Leu
        35                  40                  45

Ile Phe Ser Thr Ser Asn Leu Ala Ala Gly Val Pro Ala Arg Phe Ser
    50                  55                  60

Gly Ser Gly Ser Gly Thr Asp Tyr Thr Leu Thr Ile Ser Ser Leu Glu
65                  70                  75                  80

Pro Glu Asp Phe Ala Val Tyr Tyr Cys Gln Gln Tyr Ser Gly Tyr Pro
                85                  90                  95

Leu Thr Phe Gly Gly Gly Thr Lys Val Glu Ile Lys
            100                 105

<210>  141
<211>  117
<212>  PRT
<213>  Artificial Sequence

<220>
<223>  h63A10A7.VH-g2

<400>  141

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Arg Gln Ala Pro Gly Gln Gly Leu Glu Trp Met
        35                  40                  45

Gly Tyr Ile Asn Pro Asn Ser Gly Tyr Thr Lys Tyr Asn Gln Lys Phe
    50                  55                  60

Lys Asp Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  142
<211>  117
<212>  PRT
<213>  Artificial Sequence

<220>
<223>  h63A10A7.VH-g3

<400>  142

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Arg Gln Ala Pro Gly Gln Gly Leu Glu Trp Ile
        35                  40                  45

Gly Tyr Ile Asn Pro Asn Ser Gly Tyr Thr Lys Tyr Asn Gln Lys Phe
    50                  55                  60

Lys Asp Arg Val Thr Leu Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  143
<211>  117
<212>  PRT
<213>  Artificial Sequence

<220>
<223>  h63A10A7.VH-g4

<400>  143

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Lys Gln Arg Pro Gly Gln Gly Leu Glu Trp Ile
        35                  40                  45

Gly Tyr Ile Asn Pro Asn Ser Gly Tyr Thr Lys Tyr Asn Gln Lys Phe
    50                  55                  60

Lys Asp Lys Ala Thr Leu Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  144
<211>  117
<212>  PRT
<213>  Artificial Sequence

<220>
<223>  h63A10A7.VH-g5

<400>  144

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Lys Gln Arg Pro Gly Gln Gly Leu Glu Trp Ile
        35                  40                  45

Gly Tyr Ile Asn Pro Asn Ser Gly Tyr Thr Lys Tyr Asn Gln Lys Phe
    50                  55                  60

Lys Asp Lys Ala Thr Leu Thr Ala Asp Gln Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  145
<211>  117
<212>  PRT
<213>  Artificial Sequence

<220>
<223>  h63A10A7.VH-g6

<400>  145

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Lys Gln Arg Pro Gly Gln Gly Leu Glu Trp Met
        35                  40                  45

Gly Tyr Ile Asn Pro Asn Ser Gly Tyr Thr Lys Tyr Asn Gln Lys Phe
    50                  55                  60

Lys Asp Lys Ala Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  146
<211>  117
<212>  PRT
<213>  Artificial Sequence

<220>
<223>  h63A10A7.VH-g7

<400>  146

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Arg Gln Ala Pro Gly Gln Gly Leu Glu Trp Ile
        35                  40                  45

Gly Tyr Ile Asn Pro Asn Ser Gly Tyr Thr Lys Tyr Asn Gln Lys Phe
    50                  55                  60

Lys Asp Lys Ala Thr Leu Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  147
<211>  117
<212>  PRT
<213>  Artificial Sequence

<220>
<223>  h63A10A7.VH-g8

<400>  147

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Lys Gln Arg Pro Gly Gln Gly Leu Glu Trp Ile
        35                  40                  45

Gly Tyr Ile Asn Pro Asn Ser Gly Tyr Thr Lys Tyr Asn Gln Lys Phe
    50                  55                  60

Lys Asp Arg Val Thr Leu Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  148
<211>  107
<212>  PRT
<213>  Artificial Sequence

<220>
<223>  h63A10A7.VL-g3

<400>  148

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Leu Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105

<210>  149
<211>  107
<212>  PRT
<213>  Artificial Sequence

<220>
<223>  h63A10A7.VL-g4

<400>  149

Asp Ile Leu Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Leu Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105

<210>  150
<211>  107
<212>  PRT
<213>  Artificial Sequence

<220>
<223>  h63A10A7.VL-g5

<400>  150

Asp Ile Leu Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Leu Gln Gln Lys Pro Gly Lys Ala Pro Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105


<210>  151
<211>  107
<212>  PRT
<213>  Artificial Sequence

<220>
<223>  h63A10A7.VL-g 6

<400>  151

Asp Ile Leu Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Leu Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105

<210>  152
<211>  107
<212>  PRT
<213>  Artificial Sequence

<220>
<223>  h63A10A7.VL-g 7

<400>  152

Asp Ile Leu Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Tyr Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105

<210>  153
<211>  107
<212>  PRT
<213>  Artificial Sequence

<220>
<223>  h63A10A7.VL-g8

<400>  153

Asp Ile Leu Met Thr Gln Ser Pro Ser Ser Leu Ser Ala Ser Val Gly

1               5                   10                  15

Asp Arg Val Thr Ile Thr Cys His Ala Ser Gln Asp Ile Asn Ser Asn
            20                  25                  30

Ile Gly Trp Tyr Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Phe Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105

<210>  154
<211>  372
<212>  DNA
<213>  Artificial Sequence

<220>
<223>  h75G7C6. VH-g0

<400>  154
caagttcagc tggtgcagtc cggagccgag gtgaagaaac ccggcgccag cgtgaaggtg      60

agctgcaagg ccagcggcta caccttcacc gactacaaca tggactgggt gagacaagct     120

cccggtcaag gtttagagtg gatgggcgac atcaacccca acaacgccga caccatctac     180

aaccagaagt tcaaaggtcg tgtgaccatg actcgtgaca ccagcacctc caccgtgtac     240

atggagctga gctctttaag gtccgaggac accgccgtgt actactgcgc tcgtggcgac     300

tacgacggct actaccccgt gtactacgcc atggactact ggggccaagg taccacagtg     360

accgtgagct cc                                                         372

<210>  155
<211>  372
<212>  DNA
<213>  Artificial Sequence

<220>
<223>  h75G7C6. VH-g1

<400>  155
caagttcagc tggtgcagag cggcgctgaa gtgaagaagc ccggcgccag cgtgaaggtg      60

agctgcaagg ccagcggcta caccttcacc gactacaaca tggactgggt gaggcaagct     120

cccggtcaag gtttagagtg gatcggcgac attaacccca acaacgccga caccatctac 180

aaccagaagt tcaagggtcg tgtgacttta accgtggaca ccagcaccag caccgtgtac 240

atggagctga gctctttaag aagcgaggac accgccgtgt actactgcgc tcgtggcgac 300

tacgacggct actaccccgt gtactacgcc atggactatt ggggccaagg tacaaccgtg 360

accgtgagca gc 372

<210> 156
<211> 372
<212> DNA
<213> Artificial Sequence

<220>
<223> h75G7C6. VH-g2

<400> 156
caagttcagc tggtgcagag cggcgctgaa gtgaagaagc ccggcgccag cgtgaaggtg 60

agctgcaagg ccagcggcta caccttcacc gactacaaca tggactgggt gaagcaagct 120

cccggtcaag gtttagagtg gattggcgac atcaacccca acaacgccga caccatctac 180

aaccagaagt tcaagggtcg tgtgacttta accgtggaca agagcaccag caccgtgtac 240

atggagctga gctctttaag gagcgaggac accgccgtgt actactgcgc tcgtggcgac 300

tacgacggct actaccccgt gtactacgcc atggactact ggggccaagg taccacagtg 360

accgtgagca gc 372

<210> 157
<211> 372
<212> DNA
<213> Artificial Sequence

<220>
<223> h75G7C6. VH-g3

<400> 157
gaggtgcagc tggtgcagag cggcgctgaa gtgaagaagc ccggcgccag cgtgaaggtg 60

agctgcaagg ccagcggcta caccttcacc gactacaaca tggactgggt gaagcaagct 120

cccggtcaag gtttagagtg gatcggcgac atcaacccca acaacgccga caccatctac 180

aaccagaagt tcaagggtcg tgtgacttta acagtggaca aaagcacctc caccgtgtac 240

atggagctgt cctctttaag gtccgaggac accgccgtgt actactgcgc tcgtggcgac 300

tacgacggct actaccccgt gtactacgcc atggactact ggggccaagg taccaccgtg 360

accgtgagca gc 372

<210> 158
<211> 324
<212> DNA

<213>  Artificial Sequence

<220>
<223>  h75G7C6. VL-g0

<400>  158
gagatcgtgc tgacccagtc ccccgctaca ctgtctttat cccccggtga gagagccact      60

ttaagctgca gagccagcag cagcgtgagc agcagctatt tacactggta ccagcagaag     120

cccggacaag ctcccagact gctgatctac agcaccagca atttagctgc cggcatcccc     180

gctcgtttca gcggaagcgg aagcggcacc gacttcactt taaccatctc ctctttagag     240

cccgaggact tcgccgtgta ctactgccag cagtacagcg gctaccctct gaccttcggc     300

ggaggcacca aggtggagat taag                                            324


<210>  159
<211>  324
<212>  DNA
<213>  Artificial Sequence

<220>
<223>  h75G7C6. VL-g1

<400>  159
gagaacgtgc tgacccagag ccccgccaca ctgtctttaa gccccggtga gagggccact      60

ttaagctgca gagccagcag cagcgtgagc agcagctatt tacactggta ccagcagaaa     120

cccggccaag ctcccagact gctgatcttc agcaccagca atttagctgc cggcatcccc     180

gctagattta gcggcagcgg cagcggaacc gactacactt taaccatcag ctctttagaa     240

cccgaggact tcgccgtgta ctactgccag cagtacagcg gctatcctct gaccttcggc     300

ggcggcacca aggtggagat caag                                            324


<210>  160
<211>  324
<212>  DNA
<213>  Artificial Sequence

<220>
<223>  h75G7C6. VL-g2

<400>  160
gagaacgtgc tgacccagag ccccgctact ttatctttaa gccccggaga aagggccact      60

ttaagctgta gggccagcag cagcgtgagc agctcctatt tacactggta ccagcagaag     120

cccggtcaga gccctcgtct gctgatcttc agcacaagca atttagccgc cggcatcccc     180

gctagattct ccggaagcgg cagcggcacc gactacactt taaccatctc ctctttagag     240

cccgaggact tcgccgtgta ctactgccag cagtactccg gctacccttt aacctttggc     300

ggaggcacca aggtggagat caag                                            324

<210> 161
<211> 324
<212> DNA
<213> Artificial Sequence

<220>
<223> h75G7C6. VL-g3

<400> 161
gagaacgtgc tgacccagag ccccgctact ttatctttaa gccccggtga gagagccact 60

ttaagctgtc gtgccagcag cagcgtgagc tccagctatt tacactggta ccagcagaag 120

cccggccaga gccccagact gctgatcttc agcaccagca atctggccgc cggagtgccc 180

gctagattca gcggcagcgg aagcggcacc gactacactt taaccatcag ctctttagag 240

cccgaggact tcgccgtgta ctactgccag cagtacagcg gctacccttt aacattcggc 300

ggaggcacca aggtggagat caag 324


<210> 162
<211> 351
<212> DNA
<213> Artificial Sequence

<220>
<223> h63A10A7.VH-g2

<400> 162
caggtgcagc tggtgcagtc aggagcagaa gtgaagaagc ccggagccag cgtgaaagtg 60

tcttgcaagg ccagcggcta caccttcatc agcttcacca tgcattgggt ccggcaggct 120

ccaggacagg gactcgagtg gatgggatac atcaacccca acagcggcta caccaagtac 180

aaccagaagt tcaaggaccg cgtgaccatg accagagaca ccagcaccag caccgtgtac 240

atggagctga gcagcctgag gagcgaggat acagccgtgt actattgcac caggggcgga 300

ggcagaggat tcgcttattg gggccaggga acactggtga cagtgtccag c 351


<210> 163
<211> 351
<212> DNA
<213> Artificial Sequence

<220>
<223> h63A10A7.VH-g3

<400> 163
caggtgcagc tggtgcagtc aggagcagaa gtgaagaagc ccggagccag cgtgaaagtg 60

tcttgcaagg ccagcggcta caccttcatc agcttcacca tgcattgggt ccggcaggct 120

ccaggacagg gactcgagtg gattggctac atcaacccca acagcggcta caccaagtac 180

aaccagaagt tcaaggaccg cgtgaccctg accagagaca ccagcaccag caccgtgtac 240

atggagctga gcagcctgag gagcgaggat acagccgtgt actattgcac caggggcgga 300

ggcagaggat tcgcttattg gggccaggga acactggtga cagtgtccag c 351

<210> 164
<211> 354
<212> DNA
<213> Artificial Sequence

<220>
<223> h63A10A7.VH-g4

<400> 164
caggtgcagc tggtgcagtc aggagcagaa gtgaagaagc ccggagccag cgtgaaagtg 60

tcttgcaagg ccagcggcta caccttcatc agcttcacca tgcattgggt caagcagagg 120

ccaggacagg gactcgagtg gattggctac atcaacccca acagcggcta caccaagtac 180

aaccagaagt tcaaggacaa ggccaccctg accagagaca ccagcaccag caccgtgtac 240

atggagctga gcagcctgag gagcgaggat acagccgtgt actattgcac caggggcgga 300

ggcagaggat tcgcttattg gggccaggga acactggtga cagtgtccag cgct 354

<210> 165
<211> 354
<212> DNA
<213> Artificial Sequence

<220>
<223> h63A10A7.VH-g5

<400> 165
caggtgcagc tggtgcagtc aggagcagaa gtgaagaagc ccggagccag cgtgaaagtg 60

tcttgcaagg ccagcggcta caccttcatc agcttcacca tgcattgggt caagcagagg 120

ccaggacagg gactcgagtg gattggctac atcaacccca acagcggcta caccaagtac 180

aaccagaagt tcaaggacaa ggccaccctg acagccgatc agtctaccag caccgcctac 240

atggagctga gctctctgag gagcgaggac acagccgtgt actattgcac caggggcgga 300

ggcagaggat tcgcttattg gggccaggga acactggtga cagtgtccag cgct 354

<210> 166
<211> 354
<212> DNA
<213> Artificial Sequence

<220>
<223> h63A10A7.VH-g6, DNA

<400> 166
caggtgcagc tggtgcagtc aggagcagaa gtgaagaagc ccggagccag cgtgaaagtg 60

tcttgcaagg ccagcggcta caccttcatc agcttcacca tgcattgggt caagcagagg 120

ccaggacagg gactcgagtg gatgggctac atcaacccca acagcggcta caccaagtac 180

aaccagaagt tcaaggacaa ggccaccatg accagagaca ccagcaccag caccgtgtac 240

atggagctga gcagcctgag gagcgaggat acagccgtgt actattgcac caggggcgga 300

ggcagaggat tcgcttattg ggccagggga acactggtga cagtgtccag cgct 354

<210> 167
<211> 354
<212> DNA
<213> Artificial Sequence

<220>
<223> h63A10A7.VH-g7

<400> 167
caggtgcagc tggtgcagtc aggagcagaa gtgaagaagc ccggagccag cgtgaaagtg 60

tcttgcaagg ccagcggcta caccttcatc agcttcacca tgcattgggt ccggcaggct 120

ccaggacagg gactcgagtg gattggctac atcaacccca acagcggcta caccaagtac 180

aaccagaagt tcaaggacaa ggccaccctg accagagaca ccagcaccag caccgtgtac 240

atggagctga gcagcctgag gagcgaggat acagccgtgt actattgcac caggggcgga 300

ggcagaggat tcgcttattg ggccagggga acactggtga cagtgtccag cgct 354

<210> 168
<211> 354
<212> DNA
<213> Artificial Sequence

<220>
<223> h63A10A7.VH-g8

<400> 168
caggtgcagc tggtgcagtc aggagcagaa gtgaagaagc ccggagccag cgtgaaagtg 60

tcttgcaagg ccagcggcta caccttcatc agcttcacca tgcattgggt caagcagagg 120

ccaggacagg gactcgagtg gattggctac atcaacccca acagcggcta caccaagtac 180

aaccagaagt tcaaggaccg cgtgaccctg accagagaca ccagcaccag caccgtgtac 240

atggagctga gcagcctgag gagcgaggat acagccgtgt actattgcac caggggcgga 300

ggcagaggat tcgcttattg ggccagggga acactggtga cagtgtccag cgct 354

<210> 169
<211> 321
<212> DNA
<213> Artificial Sequence

<220>
<223> h63A10A7.VL-g3

<400> 169
gacatccaga tgacccagag ccctagcagc ctgagcgcta gcgtgggaga tagagtgacc 60

atcacttgcc acgccagcca ggacatcaac agcaacatcg gttggctgca gcagaagccc 120

ggcaagagct tcaaggccct gatctaccac ggcacaaatc tggaggacgg agtgcctagc 180

agattcagcg gcagcggaag cggagccgat tacaccctga ccatcagcag cctgcagcca 240

gaggacttcg ccacctacta ctgcgtgcag tacgcccagt tccccttcac cttcggacag 300

ggaaccaagc tggagatcaa g 321

<210> 170
<211> 327
<212> DNA
<213> Artificial Sequence

<220>
<223> h63A10A7.VL-g4

<400> 170
gacatcctga tgacccagag ccctagcagc ctgagcgcta gcgtgggaga tagagtgacc 60

atcacttgcc acgccagcca ggacatcaac agcaacatcg gttggctgca gcagaagccc 120

ggcaagagct tcaaggccct gatctaccac ggcacaaatc tggaggacgg agtgcctagc 180

agattcagcg gcagcggaag cggagccgat tacaccctga ccatcagcag cctgcagcca 240

gaggacttcg ccacctacta ctgcgtgcag tacgcccagt tccccttcac cttcggacag 300

ggaaccaagc tggagatcaa gcgtacg 327

<210> 171
<211> 327
<212> DNA
<213> Artificial Sequence

<220>
<223> h63A10A7.VL-g5

<400> 171
gacatcctga tgacccagag ccctagcagc ctgagcgcca gcgtgggaga tagagtgacc 60

atcacttgcc acgccagcca ggacatcaac agcaacatcg gttggctgca gcagaagccc 120

ggcaaggctc ctaaggccct gatctaccac ggcacaaatc tggaggacgg agtgcctagc 180

agattcagcg gcagcggaag cggagccgat tacaccctga ccatcagcag cctgcagcca 240

gaggacttcg ccacctacta ctgcgtgcag tacgcccagt tccccttcac cttcggacag 300

ggaaccaagc tggagatcaa gcgtacg 327

<210> 172
<211> 328
<212> DNA
<213> Artificial Sequence

<220>
<223> h63A10A7.VL-g6

<400> 172
ggacatcctg atgacccaga gccctagcag cctgagcgcc agcgtgggag atagagtgac 60

catcacttgc cacgccagcc aggacatcaa cagcaacatc ggttggctgc agcagaagcc 120

cggcaagagc ttcaaggccc tgatctacca cggcacaaat ctggaggacg gagtgcctag 180

cagattcagc ggcagcggaa gcggaacaga ttacaccctg accatcagca gcctgcagcc 240

agaggacttc gccacctact actgcgtgca gtacgcccag ttccccttca ccttcggaca 300

gggaaccaag ctggagatca agcgtacg 328

<210> 173
<211> 327
<212> DNA
<213> Artificial Sequence

<220>
<223> h63A10A7.VL-g7

<400> 173
gacatcctga tgacccagag ccctagcagc ctgagcgcca gcgtgggaga tagagtgacc 60

atcacttgcc acgccagcca ggacatcaac agcaacatcg gttggtatca gcagaagccc 120

ggcaagagct tcaaggccct gatctaccac ggcacaaatc tggaggacgg agtgcctagc 180

agattcagcg gcagcggaag cggagccgat tacaccctga ccatcagcag cctgcagcca 240

gaggacttcg ccacctacta ctgcgtgcag tacgcccagt tccccttcac cttcggacag 300

ggaaccaagc tggagatcaa gcgtacg 327

<210> 174
<211> 328
<212> DNA
<213> Artificial Sequence

<220>
<223> h63A10A7.VL-g8

<400> 174
gacatcctga tgacccagag ccctagcagc ctgagcgcca gcgtgggaga tagagtgacc 60

atcacttgcc acgccagcca ggacatcaac agcaacatcg gttggtatca gcagaagccc 120

ggcaagagct tcaaggccct gatctaccac ggcacaaatc tggaggacgg agtgcctagc 180

agattcagcg gcagcggaag cggagccgat ttcaccctga ccatcagcag cctgcagcca 240

gaggacttcg ccacctacta ctgcgtgcag tacgcccagt tccccttcac cttcggacag 300

ggaaccaagc tggagatcaa gcgtacgg 328

<210> 175
<211> 98
<212> PRT
<213> Homo sapiens

<400> 175

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg

<210>  176
<211>  98
<212>  PRT
<213>  Homo sapiens

<400>  176

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg

<210>  177
<211>  95
<212>  PRT
<213>  Homo sapiens

<400>  177

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro
                85                  90                  95

<210>  178
<211>  95
<212>  PRT
<213>  Homo sapiens

<400>  178

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro
                85                  90                  95

<210>  179

<211>  124
<212>  PRT
<213>  Artificial Sequence

<220>
<223>  Ç¶°Ï¿¹Ìå75G7C6ÖØÁ´¿É±äÇø¶¨µãÍ»±ä£¨NG/NA£©

<400>  179

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Asn Ala Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Ser Val Thr Val Ser Ser
        115                 120


<210>  180
<211>  124
<212>  PRT
<213>  Artificial Sequence

<220>
<223>  Ç¶°Ï¿¹Ìå75G7C6ÖØÁ´¿É±äÇø¶¨µãÍ»±ä£¨NG/QG£©

<400>  180

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Gln Gly Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Ser Val Thr Val Ser Ser
        115                 120

<210>  181
<211>  124
<212>  PRT
<213>  Artificial Sequence

<220>
<223>  Ç¶°Ï¿¹Ìå75G7C6ÖØÁ´¿É±äÇø¶¨µãÍ»±ä£¨DG/SG£©

<400>  181

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Asn Gly Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Ser Gly Tyr Tyr Pro Val Tyr Tyr Ala Met Asp
            100                 105                 110

Tyr Trp Gly Gln Gly Thr Ser Val Thr Val Ser Ser
        115                 120

<210>  182

<211>  124
<212>  PRT
<213>  Artificial Sequence

<220>
<223>  Ç¶°Ï¿¹Ìå75G7C6ÖØÁ´¿É±äÇø¶¨µãÍ»±ä£¨DG/EG£©

<400>  182

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Asn Gly Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Glu Gly Tyr Tyr Pro Val Tyr Tyr Ala Met Asp
            100                 105                 110

Tyr Trp Gly Gln Gly Thr Ser Val Thr Val Ser Ser
        115                 120


<210>  183
<211>  124
<212>  PRT
<213>  Artificial Sequence

<220>
<223>  Ç¶°Ï¿¹Ìå75G7C6ÖØÁ´¿É±äÇø¶¨µãÍ»±ä£¨DG/DA£©

<400>  183

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Asn Gly Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Asp Ala Tyr Tyr Pro Val Tyr Tyr Ala Met Asp
            100                 105                 110

Tyr Trp Gly Gln Gly Thr Ser Val Thr Val Ser Ser
        115                 120

<210>  184
<211>  6
<212>  PRT
<213>  Artificial Sequence

<220>
<223>  Ç¶°Ï¿¹Ìå75G7C6ÖØÁ´¿É±äÇøCDR2¶¨µãÍ»±äÐòÁÐ£¨NG/NA£©

<400>  184

Asn Pro Asn Asn Ala Asp
1               5

<210>  185
<211>  372
<212>  DNA
<213>  Artificial Sequence

<220>
<223>  Ç¶°Ï¿¹Ìå75G7C6ÖØÁ´¿É±äÇø¶¨µãÍ»±ä£¨NG/NA£©

<400>  185
gaggtccagc tgcaacagtc tggacctgag ctggtgaagc ctggggcttc agtgaagata      60

ccctgcaagg cttctggata cacattcact gactacaaca tggactgggt gaagcagagc     120

catggaaaga gccttgagtg gattggagat attaatccta acaatgctga tactatctac     180

aaccagaagt tcaagggcaa ggccacattg actgtagaca agtcctccag cacagcctac     240

atggagctcc gcagcctgac atctgaggac actgcagtct attactgtgc aaggggggac      300

tatgatggtt actacccggt ttactatgct atggactatt ggggtcaagg aacctcagtc     360

accgtctcct ca                                                         372

<210>  186
<211>  6
<212>  PRT
<213>  Artificial Sequence

<220>
<223>  Ç¶°Ï¿¹Ìå75G7C6ÖØÁ´¿É±äÇøCDR2¶¨µãÍ»±ä

<400>  186

Asn Pro Asn Gln Gly Asp
1               5


<210>  187
<211>  372
<212>  DNA
<213>  Artificial Sequence

<220>
<223>  Ç¶°Ï¿¹Ìå75G7C6ÖØÁ´¿É±äÇø¶¨µãÍ»±ä£¨NG/QG£©

<400>  187
gaggtccagc tgcaacagtc tggacctgag ctggtgaagc ctggggcttc agtgaagata      60

ccctgcaagg cttctggata cacattcact gactacaaca tggactgggt gaagcagagc     120

catggaaaga gccttgagtg gattggagat attaatccta accaaggtga tactatctac     180

aaccagaagt tcaagggcaa ggccacattg actgtagaca agtcctccag cacagcctac     240

atggagctcc gcagcctgac atctgaggac actgcagtct attactgtgc aaggggggac      300

tatgatggtt actacccggt ttactatgct atggactatt ggggtcaagg aacctcagtc     360

accgtctcct ca                                                         372


<210>  188
<211>  372
<212>  DNA
<213>  Artificial Sequence

<220>
<223>  Ç¶°Ï¿¹Ìå75G7C6ÖØÁ´¿É±äÇø¶¨µãÍ»±ä£¨DG/SG£©

<400>  188
gaggtccagc tgcaacagtc tggacctgag ctggtgaagc ctggggcttc agtgaagata      60

ccctgcaagg cttctggata cacattcact gactacaaca tggactgggt gaagcagagc     120

catggaaaga gccttgagtg gattggagat attaatccta acaatggtga tactatctac     180

aaccagaagt tcaagggcaa ggccacattg actgtagaca agtcctccag cacagcctac     240

atggagctcc gcagcctgac atctgaggac actgcagtct attactgtgc aaggggggac      300

tattctggtt actacccggt ttactatgct atggactatt ggggtcaagg aacctcagtc     360

accgtctcct ca                                                         372


<210>  189
<211>  15
<212>  PRT
<213>  Homo sapiens

<400>  189

Tyr Phe Asp Tyr Trp Gly Gln Gly Thr Leu Val Thr Val Ser Ser
1               5                   10                  15

<210>  190
<211>  20
<212>  PRT
<213>  Homo sapiens

<400>  190

Tyr Tyr Tyr Tyr Tyr Gly Met Asp Val Trp Gly Gln Gly Thr Thr Val
1               5                   10                  15

Thr Val Ser Ser
            20

<210>  191
<211>  12
<212>  PRT
<213>  Homo sapiens

<400>  191

Leu Thr Phe Gly Gly Gly Thr Lys Val Glu Ile Lys
1               5                   10

<210>  192
<211>  12
<212>  PRT
<213>  Homo sapiens

<400>  192

Tyr Thr Phe Gly Gln Gly Thr Lys Leu Glu Ile Lys
1               5                   10

SEQUENCE LISTING

<110>  XDCEXPLORER (SHANGHAI) CO., LTD

<120>  EGFRvIII Antibody and Conjugate, and Preparation Method and Use
       thereof

<130>  P20411031EP

<150>  201711329680.1
<151>  2017-12-13

<160>  192

<170>  PatentIn version 3.5

<210>  1
<211>  124
<212>  PRT
<213>  Mus musculus

<400>  1

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Asn Gly Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Ser Val Thr Val Ser Ser
        115                 120

<210>  2
<211>  7
<212>  PRT

<213>  Mus musculus

<400>  2

Gly Tyr Thr Phe Thr Asp Tyr
1               5


<210>  3
<211>  6
<212>  PRT
<213>  Mus musculus

<400>  3

Asn Pro Asn Asn Gly Asp
1               5


<210>  4
<211>  15
<212>  PRT
<213>  Mus musculus

<400>  4

Gly Asp Tyr Asp Gly Tyr Tyr Pro Val Tyr Tyr Ala Met Asp Tyr
1               5                   10                  15


<210>  5
<211>  108
<212>  PRT
<213>  Mus musculus

<400>  5

Glu Asn Val Leu Thr Gln Ser Pro Ala Ile Met Ser Ala Ser Pro Gly
1               5                   10                  15


Glu Lys Val Thr Met Thr Cys Arg Ala Ser Ser Ser Val Ser Ser Ser
            20                  25                  30


Tyr Leu His Trp Tyr Gln Gln Lys Ser Gly Ala Ser Pro Lys Leu Trp
        35                  40                  45


Ile Phe Ser Thr Ser Asn Leu Ala Ala Gly Val Pro Ala Arg Phe Ser
    50                  55                  60


Gly Ser Gly Ser Gly Thr Ser Tyr Ser Leu Thr Val Ser Ser Val Glu
65                  70                  75                  80


Ala Glu Asp Ala Ala Thr Tyr Tyr Cys Gln Gln Tyr Ser Gly Tyr Pro

85                  90                  95

Leu Thr Phe Gly Ala Gly Thr Lys Leu Glu Leu Lys
            100                 105

<210>  6
<211>  12
<212>  PRT
<213>  Mus musculus

<400>  6

Arg Ala Ser Ser Ser Val Ser Ser Ser Tyr Leu His
1               5                   10

<210>  7
<211>  7
<212>  PRT
<213>  Mus musculus

<400>  7

Ser Thr Ser Asn Leu Ala Ala
1               5

<210>  8
<211>  9
<212>  PRT
<213>  Mus musculus

<400>  8

Gln Gln Tyr Ser Gly Tyr Pro Leu Thr
1               5

<210>  9
<211>  117
<212>  PRT
<213>  Mus musculus

<400>  9

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Ile Ser Phe
            20                  25                  30

Thr Met His Trp Val Lys Gln Arg Pro Gly Gln Gly Leu Glu Trp Ile
        35                  40                  45

Gly Tyr Ile Asn Pro Asn Ser Gly Tyr Thr Lys Tyr Asn Gln Lys Phe
    50                  55                  60

Lys Asp Lys Ala Thr Leu Thr Ala Gly Gln Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ala
        115

<210>  10
<211>  7
<212>  PRT
<213>  Mus musculus

<400>  10

Gly Tyr Thr Phe Ile Ser Phe
1               5

<210>  11
<211>  6
<212>  PRT
<213>  Mus musculus

<400>  11

Asn Pro Asn Ser Gly Tyr
1               5

<210>  12
<211>  8
<212>  PRT
<213>  Mus musculus

<400>  12

Gly Gly Gly Arg Gly Phe Ala Tyr
1               5

<210>  13
<211>  107
<212>  PRT
<213>  Mus musculus

<400>  13

Asp Ile Leu Met Thr Gln Ser Pro Ser Ser Met Ser Val Ser Leu Gly
1               5                   10                  15

Asp Thr Val Thr Ile Thr Cys His Ala Ser Gln Asp Ile Asn Ser Asn
            20                  25                  30

Ile Gly Trp Leu Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Ser Leu Thr Ile Ser Ser Leu Glu Ser
65                  70                  75                  80

Glu Asp Phe Ala Asp Tyr Tyr Cys Val Gln Tyr Ala Gln Phe Pro Phe
                85                  90                  95

Thr Phe Gly Ser Gly Thr Lys Leu Glu Ile Lys
            100                 105


<210>  14
<211>  11
<212>  PRT
<213>  Mus musculus

<400>  14

His Ala Ser Gln Asp Ile Asn Ser Asn Ile Gly
1               5                   10


<210>  15
<211>  7
<212>  PRT
<213>  Mus musculus

<400>  15

His Gly Thr Asn Leu Glu Asp
1               5


<210>  16
<211>  9
<212>  PRT
<213>  Mus musculus

<400>  16

Val Gln Tyr Ala Gln Phe Pro Phe Thr
1               5


<210>  17
<211>  117
<212>  PRT
<213>  Mus musculus

<400>  17

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Ser Ser Tyr
            20                  25                  30

Thr Met His Trp Ala Lys Gln Arg Pro Gly Gln Gly Leu Glu Trp Ile
        35                  40                  45

Gly Tyr Ile Asn Pro Ser Ser Gly Tyr Thr Lys Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ile Val Tyr Tyr Cys
                85                  90                  95

Ala Arg Gly Gly Gly Arg Gly Phe Ala Tyr Trp Gly Gln Gly Thr Leu
            100                 105                 110

Val Thr Val Ser Ala
        115


<210>  18
<211>  7
<212>  PRT
<213>  Mus musculus

<400>  18

Gly Tyr Thr Phe Ser Ser Tyr
1               5


<210>  19
<211>  6

<212>  PRT
<213>  Mus musculus

<400>  19

Asn Pro Ser Ser Gly Tyr
1               5


<210>  20
<211>  8
<212>  PRT
<213>  Mus musculus

<400>  20

Gly Gly Gly Arg Gly Phe Ala Tyr
1               5


<210>  21
<211>  106
<212>  PRT
<213>  Mus musculus

<400>  21

Gln Ile Val Leu Thr Gln Ser Pro Ala Ile Met Ser Ala Ser Pro Gly
1               5                   10                  15


Glu Arg Val Thr Met Thr Cys Ser Ala Ser Ser Ser Val Ser Tyr Met
            20                  25                  30


Tyr Trp Tyr Gln Gln Lys Pro Gly Ser Ser Pro Arg Leu Leu Ile Tyr
        35                  40                  45


Asp Thr Ser Asn Leu Ala Ser Gly Val Pro Val Arg Phe Ser Gly Ser
    50                  55                  60


Gly Ser Gly Thr Ser Tyr Ser Leu Thr Ile Ser Arg Val Glu Ala Glu
65                  70                  75                  80


Asp Ala Ala Thr Tyr Tyr Cys Gln Gln Trp Ser Ser Tyr Ser Leu Thr
                85                  90                  95


Phe Gly Ala Gly Thr Lys Leu Glu Leu Lys
            100                 105


<210>  22
<211>  10
<212>  PRT

<213>  Mus musculus

<400>  22

Ser Ala Ser Ser Ser Val Ser Tyr Met Tyr
1               5                   10


<210>  23
<211>  7
<212>  PRT
<213>  Mus musculus

<400>  23

Asp Thr Ser Asn Leu Ala Ser
1               5


<210>  24
<211>  9
<212>  PRT
<213>  Mus musculus

<400>  24

Gln Gln Trp Ser Ser Tyr Ser Leu Thr
1               5


<210>  25
<211>  114
<212>  PRT
<213>  Mus musculus

<400>  25

Glu Ile Gln Leu Gln Gln Ser Gly Pro Glu Leu Val Lys Pro Gly Ala
1               5                   10                  15


Ser Val Lys Val Ser Cys Lys Ala Ser Gly Tyr Ser Phe Thr Asp Tyr
            20                  25                  30


Asn Met Tyr Trp Val Lys Gln Ser His Gly Lys Ser Leu Glu Trp Ile
        35                  40                  45


Gly Tyr Val Asp Pro Ser Asn Gly Gly Thr Asn Tyr Asn Gln Lys Phe
    50                  55                  60


Lys Gly Lys Ala Thr Leu Thr Val Asp Lys Ser Ser Ser Thr Ala Phe
65                  70                  75                  80


Met His Leu Asn Ser Leu Thr Ser Glu Asp Ser Ala Val Tyr Tyr Cys

85                  90                  95

Ala Arg Gly Phe Phe Asp Tyr Trp Gly Gln Gly Thr Thr Leu Thr Val
            100                 105                 110

Ser Ser

<210>  26
<211>  7
<212>  PRT
<213>  Mus musculus

<400>  26

Gly Tyr Ser Phe Thr Asp Tyr
1               5

<210>  27
<211>  6
<212>  PRT
<213>  Mus musculus

<400>  27

Asp Pro Ser Asn Gly Gly
1               5

<210>  28
<211>  5
<212>  PRT
<213>  Mus musculus

<400>  28

Gly Phe Phe Asp Tyr
1               5

<210>  29
<211>  113
<212>  PRT
<213>  Mus musculus

<400>  29

Asp Ile Val Met Thr Gln Ser Pro Ser Ser Leu Thr Val Thr Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Leu Thr Trp Tyr Gln Gln Lys Pro Gly Gln
        35                  40                  45

Pro Pro Lys Leu Leu Ile Tyr Trp Ala Ser Asn Arg Glu Ser Gly Val
    50                  55                  60

Pro Asp Arg Phe Thr Gly Ser Gly Ser Gly Lys Asp Phe Thr Leu Thr
65                  70                  75                  80

Ile Ser Ser Val Gln Ala Glu Asp Leu Ala Val Tyr Tyr Cys Gln Asn
                85                  90                  95

Asp Tyr Arg Tyr Pro Val Thr Phe Gly Ala Gly Thr Lys Leu Glu Leu
            100                 105                 110

Lys

<210>  30
<211>  17
<212>  PRT
<213>  Mus musculus

<400>  30

Lys Ser Ser Gln Ser Leu Leu Asn Ser Gly Asn Gln Lys Asn Tyr Leu
1               5                   10                  15

Thr

<210>  31
<211>  7
<212>  PRT
<213>  Mus musculus

<400>  31

Trp Ala Ser Asn Arg Glu Ser
1               5

<210>  32
<211>  9
<212>  PRT
<213>  Mus musculus

<400>  32

Gln Asn Asp Tyr Arg Tyr Pro Val Thr
1               5

<210>  33
<211>  133
<212>  PRT
<213>  Mus musculus

<400>  33

Cys Leu Gln Val Asp Asp Ser Asn Thr Thr His Tyr Arg Ala Ser Ser
1               5                   10                  15

Gly Ile Asn Ala Glu Tyr Ala Gly Gly Pro Gly Leu Val Lys Pro Ser
            20                  25                  30

Gln Ser Leu Ser Leu Thr Cys Thr Val Thr Asp Tyr Ser Ile Thr Ser
        35                  40                  45

Asp Tyr Ala Trp Asn Trp Ile Arg Gln Phe Pro Gly Asn Lys Leu Glu
    50                  55                  60

Trp Met Gly Tyr Ile Ser Tyr Ser Gly Asn Thr Asn Tyr Asn Pro Ser
65                  70                  75                  80

Leu Lys Ser Arg Ile Ser Ile Thr Arg Asp Thr Ser Lys Asn Gln Phe
                85                  90                  95

Phe Leu Gln Leu Asn Ser Val Thr Thr Glu Asp Thr Ala Thr Tyr Tyr
            100                 105                 110

Cys Ala Arg Leu Gly Arg Gly Phe Ala Tyr Trp Gly Gln Gly Thr Leu
        115                 120                 125

Val Thr Val Ser Ala
    130

<210>  34
<211>  8
<212>  PRT
<213>  Mus musculus

<400>  34

Asp Tyr Ser Ile Thr Ser Asp Tyr
1               5

<210>  35
<211>  5
<212>  PRT
<213>  Mus musculus

<400>  35

Ser Tyr Ser Gly Asn
1               5


<210>  36
<211>  7
<212>  PRT
<213>  Mus musculus

<400>  36

Leu Gly Arg Gly Phe Ala Tyr
1               5


<210>  37
<211>  107
<212>  PRT
<213>  Mus musculus

<400>  37

Asp Ile Leu Met Thr Gln Ser Pro Ser Ser Met Ser Val Ser Leu Gly
1               5                   10                  15

Asp Thr Val Ser Ile Thr Cys His Ala Ser Gln Asp Ile Asn Ser Asn
            20                  25                  30

Ile Gly Trp Leu Gln Gln Lys Pro Gly Thr Ser Phe Lys Gly Leu Ile
        35                  40                  45

Tyr His Gly Arg Asn Leu Glu Asp Gly Val Pro Ser Arg Phe Ser Gly
    50                  55                  60

Ser Gly Ser Gly Glu Asp Tyr Ser Leu Thr Ile Ser Ser Leu Glu Ser
65                  70                  75                  80

Glu Asp Phe Ala Asp Tyr Tyr Cys Val Gln Tyr Ala His Phe Pro Pro
                85                  90                  95

Thr Phe Gly Ala Gly Thr Lys Leu Glu Leu Lys
            100                 105


<210>  38

<211>  11
<212>  PRT
<213>  Mus musculus

<400>  38

His Ala Ser Gln Asp Ile Asn Ser Asn Ile Gly
1               5                   10


<210>  39
<211>  7
<212>  PRT
<213>  Mus musculus

<400>  39

His Gly Arg Asn Leu Glu Asp
1               5


<210>  40
<211>  9
<212>  PRT
<213>  Mus musculus

<400>  40

Val Gln Tyr Ala His Phe Pro Pro Thr
1               5


<210>  41
<211>  113
<212>  PRT
<213>  Mus musculus

<400>  41

Glu Val His Leu Gln Gln Ser Val Ala Glu Leu Val Arg Pro Gly Ala
1               5                   10                  15


Ser Val Lys Leu Ser Cys Thr Ala Ser Gly Phe Asn Ile Lys Asn Thr
            20                  25                  30


Tyr Met His Trp Val Lys Gln Arg Pro Glu Gln Gly Leu Glu Trp Ile
        35                  40                  45


Gly Arg Ile Asp Pro Ala Asn Gly Asn Thr Lys Tyr Ala Pro Lys Phe
    50                  55                  60


Gln Gly Lys Ala Thr Ile Thr Ala Asp Thr Ser Ser Asn Thr Ala Tyr
65                  70                  75                  80

Leu Gln Leu Ser Ser Leu Thr Ser Glu Asp Thr Ala Ile Tyr Tyr Cys
                85                  90                  95

Ala Ser Asp Phe Asp Tyr Trp Gly Gln Gly Thr Thr Leu Thr Val Ser
            100                 105                 110

Ser

<210>  42
<211>  7
<212>  PRT
<213>  Mus musculus

<400>  42

Gly Phe Asn Ile Lys Asn Thr
1               5

<210>  43
<211>  6
<212>  PRT
<213>  Mus musculus

<400>  43

Asp Pro Ala Asn Gly Asn
1               5

<210>  44
<211>  4
<212>  PRT
<213>  Mus musculus

<400>  44

Asp Phe Asp Tyr
1

<210>  45
<211>  112
<212>  PRT
<213>  Mus musculus

<400>  45

Asp Val Leu Met Thr Gln Thr Pro Leu Ser Leu Pro Val Ser Leu Gly
1               5                   10                  15

Asp Gln Val Ser Ile Ser Cys Arg Ser Ser Gln Ser Ile Val Asn Ser

20                  25                  30

Asn Gly Asn Thr His Leu Glu Trp Tyr Leu Gln Lys Pro Gly Gln Ser
        35                  40                  45

Pro Lys Val Leu Ile Tyr Lys Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Tyr Cys Phe Gln Gly
                85                  90                  95

Ser His Val Pro Tyr Thr Phe Gly Gly Gly Thr Lys Leu Glu Ile Lys
            100                 105                 110

<210>  46
<211>  16
<212>  PRT
<213>  Mus musculus

<400>  46

Arg Ser Ser Gln Ser Ile Val Asn Ser Asn Gly Asn Thr His Leu Glu
1               5                   10                  15

<210>  47
<211>  7
<212>  PRT
<213>  Mus musculus

<400>  47

Lys Val Ser Lys Arg Phe Ser
1               5

<210>  48
<211>  9
<212>  PRT
<213>  Mus musculus

<400>  48

Phe Gln Gly Ser His Val Pro Tyr Thr
1               5

<210>  49
<211>  117

<212>  PRT
<213>  Mus musculus

<400>  49

Glu Val Gln Leu Leu Gln Ser Gly Ala Glu Leu Val Lys Pro Gly Ala
1               5                   10                  15

Ser Val Lys Leu Ser Cys Thr Ala Ser Gly Phe Asn Ile Lys Asp Tyr
            20                  25                  30

Tyr Met His Trp Val Lys Gln Arg Thr Glu Gln Gly Leu Glu Trp Ile
        35                  40                  45

Gly Arg Ile Asp Pro Glu Asp Gly Glu Thr Lys Tyr Ala Pro Lys Phe
    50                  55                  60

Gln Gly Lys Ala Thr Met Thr Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Ser Ser Ala Tyr Tyr Gly Arg Asn Tyr Trp Gly Gln Gly Thr Thr
            100                 105                 110

Leu Thr Val Ser Ser
        115


<210>  50
<211>  7
<212>  PRT
<213>  Mus musculus

<400>  50

Gly Phe Asn Ile Lys Asp Tyr
1               5


<210>  51
<211>  6
<212>  PRT
<213>  Mus musculus

<400>  51

Asp Pro Glu Asp Gly Glu
1               5

<210>  52
<211>  8
<212>  PRT
<213>  Mus musculus

<400>  52

Ser Ala Tyr Tyr Gly Arg Asn Tyr
1               5

<210>  53
<211>  108
<212>  PRT
<213>  Mus musculus

<400>  53

Gln Ile Val Leu Thr Gln Ser Pro Ala Ile Met Ser Ala Ser Pro Gly
1               5                   10                  15

Glu Lys Val Thr Leu Thr Cys Ser Ala Ser Ser Ser Leu Ser Ser Ser
            20                  25                  30

Tyr Leu Tyr Trp Tyr Gln Gln Lys Pro Gly Ser Ser Pro Lys Leu Trp
        35                  40                  45

Ile Tyr Ser Thr Ser Asn Leu Ala Ser Gly Val Pro Ala Arg Phe Ser
    50                  55                  60

Gly Ser Gly Ser Gly Thr Ser Tyr Ser Leu Thr Ile Ser Ser Met Glu
65                  70                  75                  80

Ala Glu Asp Ala Ala Ser Tyr Phe Cys His Gln Trp Ser Ser Tyr Pro
                85                  90                  95

Tyr Thr Phe Gly Gly Gly Thr Lys Leu Glu Ile Lys
            100                 105

<210>  54
<211>  12
<212>  PRT
<213>  Mus musculus

<400>  54

Ser Ala Ser Ser Ser Leu Ser Ser Ser Tyr Leu Tyr
1               5                   10

<210>  55
<211>  7
<212>  PRT
<213>  Mus musculus

<400>  55

Ser Thr Ser Asn Leu Ala Ser
1               5


<210>  56
<211>  9
<212>  PRT
<213>  Mus musculus

<400>  56

His Gln Trp Ser Ser Tyr Pro Tyr Thr
1               5


<210>  57
<211>  117
<212>  PRT
<213>  Mus musculus

<400>  57

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Phe
            20                  25                  30

Tyr Ile His Trp Val Lys Gln Arg Thr Glu Gln Gly Leu Glu Trp Ile
        35                  40                  45

Gly Arg Ile Asp Pro Glu Asp Gly Glu Thr Lys Tyr Ala Pro Lys Phe
    50                  55                  60

Gln Asp Lys Ala Thr Ile Ile Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Ser Ser Ser Tyr Tyr Gly Asn Asn Tyr Trp Gly Gln Gly Thr Thr
            100                 105                 110

Leu Thr Val Ser Ser

        115

<210>  58
<211>  7
<212>  PRT
<213>  Mus musculus

<400>  58

Gly Phe Asn Ile Lys Asp Phe
1               5

<210>  59
<211>  6
<212>  PRT
<213>  Mus musculus

<400>  59

Asp Pro Glu Asp Gly Glu
1               5

<210>  60
<211>  8
<212>  PRT
<213>  Mus musculus

<400>  60

Ser Ser Tyr Tyr Gly Asn Asn Tyr
1               5

<210>  61
<211>  108
<212>  PRT
<213>  Mus musculus

<400>  61

Gln Ile Val Leu Thr Gln Ser Pro Ala Ile Met Ser Ala Ser Pro Gly
1               5                   10                  15

Glu Lys Val Thr Leu Thr Cys Ser Ala Ser Ser Ser Val Ser Ser Asn
            20                  25                  30

Tyr Leu Tyr Trp Tyr Gln Gln Lys Pro Gly Ser Ser Pro Lys Leu Trp
        35                  40                  45

Ile Tyr Ser Thr Ser Asn Leu Ala Ser Gly Val Pro Ala Arg Phe Ser
    50                  55                  60

Gly Ser Gly Ser Gly Thr Ser Tyr Ser Leu Thr Ile Ser Ser Met Glu
65                  70                  75                  80

Ala Glu Asp Ala Ala Ser Tyr Phe Cys His Gln Trp Ser Ser Phe Pro
                85                  90                  95

Tyr Thr Phe Gly Gly Gly Thr Lys Leu Glu Ile Lys
            100                 105

<210>  62
<211>  12
<212>  PRT
<213>  Mus musculus

<400>  62

Ser Ala Ser Ser Ser Val Ser Ser Asn Tyr Leu Tyr
1               5                   10

<210>  63
<211>  7
<212>  PRT
<213>  Mus musculus

<400>  63

Ser Thr Ser Asn Leu Ala Ser
1               5

<210>  64
<211>  9
<212>  PRT
<213>  Mus musculus

<400>  64

His Gln Trp Ser Ser Phe Pro Tyr Thr
1               5

<210>  65
<211>  117
<212>  PRT
<213>  Mus musculus

<400>  65

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Ser Tyr

                20                  25                  30

Thr Met His Trp Val Lys Gln Arg Pro Gly Gln Gly Leu Glu Trp Ile
        35                  40                  45

Gly Tyr Ile Asn Pro Ser Ser Gly Tyr Thr Lys Tyr Asn Gln Ile Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ala
        115

<210>  66
<211>  7
<212>  PRT
<213>  Mus musculus

<400>  66

Gly Tyr Thr Phe Thr Ser Tyr
1               5

<210>  67
<211>  6
<212>  PRT
<213>  Mus musculus

<400>  67

Asn Pro Ser Ser Gly Tyr
1               5

<210>  68
<211>  8
<212>  PRT
<213>  Mus musculus

<400>  68

Gly Gly Gly Arg Gly Phe Ala Tyr
1               5

<210>  69
<211>  107
<212>  PRT
<213>  Mus musculus

<400>  69

Asp Ile Leu Met Thr Gln Ser Pro Ser Ser Met Ser Val Ser Leu Gly
1               5                   10                  15

Asp Thr Val Ser Ile Thr Cys His Ala Ser Gln Asp Ile Asn Ser Asn
            20                  25                  30

Ile Gly Trp Leu Gln Gln Lys Pro Gly Lys Ser Phe Lys Gly Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Ser Leu Thr Ile Ser Ser Leu Glu Ser
65                  70                  75                  80

Glu Asp Phe Ala Asp Tyr Tyr Cys Val Gln Tyr Ala Gln Phe Pro Phe
                85                  90                  95

Thr Phe Gly Ser Gly Thr Lys Leu Glu Ile Lys
            100                 105


<210>  70
<211>  11
<212>  PRT
<213>  Mus musculus

<400>  70

His Ala Ser Gln Asp Ile Asn Ser Asn Ile Gly
1               5                   10


<210>  71
<211>  7
<212>  PRT
<213>  Mus musculus

<400>  71

His Gly Thr Asn Leu Glu Asp
1               5

<210>  72
<211>  9
<212>  PRT
<213>  Mus musculus

<400>  72

Val Gln Tyr Ala Gln Phe Pro Phe Thr
1               5


<210>  73
<211>  116
<212>  PRT
<213>  Mus musculus

<400>  73

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Tyr
            20                  25                  30

Tyr Met His Trp Val Lys Gln Arg Thr Glu Gln Gly Leu Glu Trp Ile
        35                  40                  45

Gly Arg Ile Asp Pro Glu Asp Gly Glu Thr Lys Tyr Ala Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Asn Leu Pro Ser Glu Asp Thr Ala Val Tyr Tyr Cys
                85                  90                  95

Ala Cys Arg Thr Gly Gly Phe Ala Tyr Trp Gly Gln Gly Thr Leu Val
            100                 105                 110

Thr Val Ser Ala
        115


<210>  74
<211>  7
<212>  PRT
<213>  Mus musculus

<400>  74

Gly Phe Asn Ile Lys Asp Tyr
1               5

<210>  75
<211>  6
<212>  PRT
<213>  Mus musculus

<400>  75

Asp Pro Glu Asp Gly Glu
1               5

<210>  76
<211>  7
<212>  PRT
<213>  Mus musculus

<400>  76

Arg Thr Gly Gly Phe Ala Tyr
1               5

<210>  77
<211>  106
<212>  PRT
<213>  Mus musculus

<400>  77

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Leu
            20                  25                  30

Tyr Trp Tyr Gln Gln Lys Pro Gly Ser Ser Pro Ser Leu Leu Ile Tyr
        35                  40                  45

Asp Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Trp Ser Arg Asn Pro Trp Thr
                85                  90                  95

Phe Gly Gly Gly Thr Lys Leu Glu Ile Lys

            100                 105

<210> 78
<211> 10
<212> PRT
<213> Mus musculus

<400> 78

Ser Ala Ser Ser Ser Val Ser Tyr Met Tyr
1               5                   10

<210> 79
<211> 7
<212> PRT
<213> Mus musculus

<400> 79

Asp Thr Ser Asn Leu Ala Ser
1               5

<210> 80
<211> 9
<212> PRT
<213> Mus musculus

<400> 80

Gln Gln Trp Ser Ser Tyr Ser Leu Thr
1               5

<210> 81
<211> 116
<212> PRT
<213> Mus musculus

<400> 81

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Phe
            20                  25                  30

Tyr Met His Trp Val Lys Gln Arg Thr Glu Gln Gly Leu Glu Trp Ile
        35                  40                  45

Gly Arg Ile Asp Pro Glu Asp Gly Glu Thr Lys Tyr Ala Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Gly Cys Arg Thr Gly Gly Phe Ala Tyr Trp Gly Gln Gly Thr Leu Val
            100                 105                 110

Thr Val Ser Ala
        115

<210>  82
<211>  7
<212>  PRT
<213>  Mus musculus

<400>  82

Gly Phe Asn Ile Lys Asp Phe
1               5

<210>  83
<211>  6
<212>  PRT
<213>  Mus musculus

<400>  83

Asp Pro Glu Asp Gly Glu
1               5

<210>  84
<211>  7
<212>  PRT
<213>  Mus musculus

<400>  84

Arg Thr Gly Gly Phe Ala Tyr
1               5

<210>  85
<211>  106
<212>  PRT
<213>  Mus musculus

<400>  85

Gln Ile Val Leu Thr Gln Ser Pro Ala Ile Met Ser Ala Ser Pro Gly

1               5                   10                  15

Glu Lys Val Thr Met Thr Cys Ser Ala Ser Ser Ser Val Ser Tyr Leu
            20                  25                  30

Tyr Trp Tyr Gln Gln Lys Pro Gly Ser Ser Pro Ser Leu Leu Ile Tyr
        35                  40                  45

Asp Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Trp Ser Arg Asn Pro Trp Thr
                85                  90                  95

Phe Gly Gly Gly Thr Lys Leu Glu Ile Lys
            100                 105

<210>  86
<211>  10
<212>  PRT
<213>  Mus musculus

<400>  86

Ser Ala Ser Ser Ser Val Ser Tyr Leu Tyr
1               5                   10

<210>  87
<211>  7
<212>  PRT
<213>  Mus musculus

<400>  87

Asp Thr Ser Asn Leu Ala Ser
1               5

<210>  88
<211>  9
<212>  PRT
<213>  Mus musculus

<400>  88

Gln Gln Trp Ser Arg Asn Pro Trp Thr
1               5

<210>  89
<211>  117
<212>  PRT
<213>  Mus musculus

<400>  89

Asp Val Gln Leu Gln Glu Ser Gly Pro Gly Leu Val Lys Pro Ser Gln
1               5                   10                  15

Ser Leu Ser Leu Thr Cys Ser Val Thr Gly Tyr Ser Ile Thr Ser Gly
            20                  25                  30

Tyr Tyr Trp His Trp Ile Arg Gln Phe Pro Glu Asn Lys Leu Glu Trp
        35                  40                  45

Met Gly Tyr Ile Ser Tyr Asp Gly Ser Asn Ile Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Thr Glu Asp Thr Ala Thr Tyr Tyr Cys
                85                  90                  95

Ala Ile Asn Ser Asp Tyr Ala Leu Asp Tyr Trp Gly Gln Gly Thr Ser
            100                 105                 110

Val Thr Val Ser Ser
        115


<210>  90
<211>  8
<212>  PRT
<213>  Mus musculus

<400>  90

Gly Tyr Ser Ile Thr Ser Gly Tyr
1               5


<210>  91
<211>  5
<212>  PRT
<213>  Mus musculus

<400>  91

Ser Tyr Asp Gly Ser
1               5

<210>  92
<211>  8
<212>  PRT
<213>  Mus musculus

<400>  92

Asn Ser Asp Tyr Ala Leu Asp Tyr
1               5

<210>  93
<211>  106
<212>  PRT
<213>  Mus musculus

<400>  93

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

Tyr Trp Tyr Gln Gln Lys Pro Gly Ser Ser Pro Arg Leu Leu Ile Tyr
        35                  40                  45

Asp Thr Ser Asn Leu Ala Ser Gly Val Pro Val Arg Phe Ser Gly Ser
    50                  55                  60

Gly Ser Gly Thr Ser Tyr Ser Leu Thr Ile Ser Arg Met Glu Ala Glu
65                  70                  75                  80

Asp Ala Ala Thr Tyr Tyr Cys Gln Gln Trp Ser Ser Tyr Ser Leu Thr
                85                  90                  95

Phe Gly Ala Gly Thr Lys Leu Glu Leu Lys
            100                 105

<210>  94
<211>  10
<212>  PRT
<213>  Mus musculus

<400>  94

Ser Ala Ser Ser Ser Val Ser Tyr Met Tyr
1               5                   10

<210>  95
<211>  7
<212>  PRT
<213>  Mus musculus

<400>  95

Asp Thr Ser Asn Leu Ala Ser
1               5

<210>  96
<211>  9
<212>  PRT
<213>  Mus musculus

<400>  96

Gln Gln Trp Ser Ser Tyr Ser Leu Thr
1               5

<210>  97
<211>  116
<212>  PRT
<213>  Mus musculus

<400>  97

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Tyr
            20                  25                  30

Tyr Met His Trp Val Lys Gln Arg Thr Glu Gln Gly Leu Glu Trp Ile
        35                  40                  45

Gly Arg Ile Asp Pro Glu Asp Gly Glu Thr Lys Tyr Ala Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Asn Leu Pro Ser Glu Asp Thr Ala Val Tyr Tyr Cys
                85                  90                  95

Gly Cys Arg Thr Gly Gly Phe Ala Tyr Trp Gly Gln Gly Thr Leu Val

            100                 105                 110

Thr Val Ser Ala
        115

<210> 98
<211> 7
<212> PRT
<213> Mus musculus

<400> 98

Gly Phe Asn Ile Lys Asp Tyr
1               5

<210> 99
<211> 6
<212> PRT
<213> Mus musculus

<400> 99

Asp Pro Glu Asp Gly Glu
1               5

<210> 100
<211> 7
<212> PRT
<213> Mus musculus

<400> 100

Arg Thr Gly Gly Phe Ala Tyr
1               5

<210> 101
<211> 107
<212> PRT
<213> Mus musculus

<400> 101

Asp Ile Leu Met Thr Gln Ser Pro Ser Ser Met Ser Val Ser Leu Gly
1               5                   10                  15

Asp Thr Val Ser Ile Thr Cys His Ala Ser Gln Gly Ile Asn Ser Asn
            20                  25                  30

Ile Gly Trp Leu Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Ser Leu Thr Ile Ser Ser Leu Glu Ser
65                  70                  75                  80

Glu Asp Phe Ala Asp Tyr Tyr Cys Val Gln Tyr Ala Gln Phe Pro Phe
                85                  90                  95

Thr Phe Gly Ser Gly Thr Glu Leu Glu Ile Lys
            100                 105

<210>  102
<211>  11
<212>  PRT
<213>  Mus musculus

<400>  102

His Ala Ser Gln Gly Ile Asn Ser Asn Ile Gly
1               5                   10

<210>  103
<211>  7
<212>  PRT
<213>  Mus musculus

<400>  103

His Gly Thr Asn Leu Glu Asp
1               5

<210>  104
<211>  9
<212>  PRT
<213>  Mus musculus

<400>  104

Val Gln Tyr Ala Gln Phe Pro Phe Thr
1               5

<210>  105
<211>  372
<212>  DNA
<213>  Mus musculus

<400>  105
gaggtccagc tgcaacagtc tggacctgag ctggtgaagc ctggggcttc agtgaagata        60

ccctgcaagg cttctggata cacattcact gactacaaca tggactgggt gaagcagagc 120

catggaaaga gccttgagtg gattggagat attaatccta acaatggtga tactatctac 180

aaccagaagt tcaagggcaa ggccacattg actgtagaca agtcctccag cacagcctac 240

atggagctcc gcagcctgac atctgaggac actgcagtct attactgtgc aaggggggac 300

tatgatggtt actacccggt ttactatgct atggactatt ggggtcaagg aacctcagtc 360

accgtctcct ca 372

<210> 106
<211> 324
<212> DNA
<213> Mus musculus

<400> 106
gaaaatgtgc tcacccagtc tccagcaatc atgtctgcat ctccagggga aaaggtcacc 60

atgacctgca gggccagctc aagtgtaagt tccagttact tgcactggta ccagcagaag 120

tcaggtgcct cccccaaact ctggattttt agcacatcca acttggctgc tggagtccct 180

gctcgcttca gtggcagtgg gtctgggacc tcttactctc tcacagtcag cagtgtggag 240

gctgaagatg ctgccactta ttactgccag cagtacagtg gttacccact cacgttcggt 300

gctgggacca agctggagct gaaa 324

<210> 107
<211> 351
<212> DNA
<213> Mus musculus

<400> 107
caggtccagc tgcagcagtc tggggctgaa ctggcaagac ctggcgcctc agtgaagatg 60

tcctgcaagg cttctggcta cacctttatt agcttcacga tgcactgggt aaaacagagg 120

cctggacagg gtctggaatg gattggatac attaatccta acagtggtta tactaagtac 180

aatcagaagt tcaaggacaa ggccacattg actgcaggcc aatcctccag cacagcctac 240

atgcaactga gcagcctgac atctgaggac tctgcagtct attactgtac aagagggggg 300

ggacgagggt ttgcttactg gggccaaggg actctggtca ctgtctctgc a 351

<210> 108
<211> 321
<212> DNA
<213> Mus musculus

<400> 108
gacatcctga tgacccaatc tccatcctcc atgtctgtat ctctgggaga cacagtcacc 60

atcacttgcc atgcaagtca ggacattaac agtaatatag ggtggttgca gcagaaacca 120

gggaaatcat ttaaggccct gatctatcat ggaaccaact tggaagatgg agttccatca 180

aggttcagtg gcagtggatc tggagcagac tattctctca ccatcagcag cctggaatct 240

gaagattttg cagactatta ctgtgtacag tatgctcagt ttccattcac gttcggctcg 300

gggacaaagt tggaaataaa a 321

<210> 109
<211> 351
<212> DNA
<213> Mus musculus

<400> 109
caggtccagc tgcagcagtc tggggctgaa ctggcaagac ctggtgcctc agtgaagatg 60

tcctgcaagg cttctggcta caccttttct agctacacga tgcactgggc aaaacagagg 120

cctggacagg gtcttgaatg gattgggtac attaatccta gcagtggtta tacaaagtac 180

aatcagaagt tcaaggacaa ggccacattg actgcagaca aatcctccag cacagcctat 240

atgcaactga gcagcctgac atctgaggac tctatagtct attactgtgc aagagggggg 300

ggacgagggt ttgcttactg gggccaaggg actctggtca ctgtctctgc a 351

<210> 110
<211> 318
<212> PRT
<213> Mus musculus

<400> 110

Cys Ala Ala Ala Thr Thr Gly Thr Thr Cys Thr Cys Ala Cys Cys Cys
1               5                   10                  15

Ala Gly Thr Cys Thr Cys Cys Ala Gly Cys Ala Ala Thr Cys Ala Thr
            20                  25                  30

Gly Thr Cys Thr Gly Cys Ala Thr Cys Thr Cys Cys Ala Gly Gly Gly
        35                  40                  45

Gly Ala Gly Ala Gly Gly Gly Thr Cys Ala Cys Cys Ala Thr Gly Ala
    50                  55                  60

Cys Cys Thr Gly Cys Ala Gly Thr Gly Cys Cys Ala Gly Cys Thr Cys
65                  70                  75                  80

Ala Ala Gly Thr Gly Thr Ala Ala Gly Thr Thr Ala Cys Ala Thr Gly
                85                  90                  95

Thr Ala Cys Thr Gly Gly Thr Ala Cys Cys Ala Gly Cys Ala Gly Ala
            100                 105                 110

Ala Gly Cys Cys Ala Gly Gly Ala Thr Cys Cys Thr Cys Cys Cys Cys
        115                 120                 125

Cys Ala Gly Ala Cys Thr Cys Cys Thr Gly Ala Thr Thr Thr Ala Thr
    130                 135                 140

Gly Ala Cys Ala Cys Ala Thr Cys Cys Ala Ala Cys Cys Thr Gly Gly
145                 150                 155                 160

Cys Thr Thr Cys Thr Gly Gly Ala Gly Thr Cys Cys Cys Thr Gly Thr
                165                 170                 175

Thr Cys Gly Cys Thr Thr Cys Ala Gly Thr Gly Gly Cys Ala Gly Thr
            180                 185                 190

Gly Gly Gly Thr Cys Thr Gly Gly Gly Ala Cys Cys Thr Cys Thr Thr
        195                 200                 205

Ala Thr Thr Cys Thr Cys Thr Cys Ala Cys Ala Ala Thr Cys Ala Gly
    210                 215                 220

Cys Cys Gly Ala Gly Thr Gly Gly Ala Gly Gly Cys Thr Gly Ala Ala
225                 230                 235                 240

Gly Ala Thr Gly Cys Cys Gly Cys Cys Ala Cys Thr Thr Ala Thr Thr
                245                 250                 255

Ala Cys Thr Gly Cys Cys Ala Gly Cys Ala Gly Thr Gly Gly Ala Gly
            260                 265                 270

Thr Ala Gly Thr Thr Ala Cys Thr Cys Ala Cys Thr Cys Ala Cys Gly
        275                 280                 285

Thr Thr Cys Gly Gly Thr Gly Cys Thr Gly Gly Gly Ala Cys Cys Ala
    290                 295                 300

Ala Gly Cys Thr Gly Gly Ala Gly Cys Thr Gly Ala Ala Ala
305                 310                 315

<210>  111
<211>  342
<212>  DNA
<213>  Mus musculus

<400>  111
gagatccagc tgcagcagtc tggacctgag ctggtgaagc ctggggcttc agtgaaggta      60

tcctgcaagg cttctggtta ctcattcact gactacaaca tgtactgggt gaagcagagc     120

catggaaaga gccttgagtg gattggatat gttgatcctt ccaatggtgg tactaactac     180

aaccagaagt tcaagggcaa ggccacattg actgttgaca agtcctccag cacagccttc     240

atgcatctca acagcctgac atctgaggac tctgcagtct attactgtgc aagagggttc     300

tttgactact ggggccaagg caccactctc acagtctcct ca                        342


<210>  112
<211>  339
<212>  DNA
<213>  Mus musculus

<400>  112
gacattgtga tgacacagtc tccatcctcc ctgactgtga cagcaggaga gaaggtcact      60

atgagctgca agtccagtca gagtctgtta aacagtggaa atcaaaagaa ctacttgacc     120

tggtaccagc agaaaccagg gcagcctcct aaactgttga tctactgggc atccaatagg     180

gaatctgggg tccctgatcg cttcacaggc agtggatctg gaaaagattt cactctcacc     240

atcagcagtg tgcaggctga agacctggca gtttattact gtcagaatga ttataggtat     300

ccggtcacgt tcggtgctgg gaccaagctg gagctgaaa                            339


<210>  113
<211>  399
<212>  DNA
<213>  Mus musculus

<400>  113
tgcctgcagg tcgacgattc taatacgact cactataggg caagcagtgg tatcaacgca      60

gagtacgcgg ggggacctgg cctggtgaaa ccttctcagt ctctgtccct cacctgcact     120

gtcactgact actcaatcac cagtgattat gcctggaact ggatccggca gtttccagga     180

aacaaactgg agtggatggg ttacataagc tacagtggta acactaacta caacccatct     240

ctcaaaagtc gaatctctat cacccgagac acatccaaga accagttctt cctgcagttg     300

aattctgtga ctactgagga cacagccaca tattactgtg caagactggg acgagggttt     360

gcttactggg gccaagggac tctggtcact gtctctgca                              399

<210>  114
<211>  321
<212>  DNA
<213>  Mus musculus

<400>  114
gacatcctga tgacccaatc tccatcctcc atgtctgtat ctctgggaga cacagtcagc       60

atcacttgcc atgcaagtca ggacattaac agtaatattg ggtggctgca gcagaaacca      120

gggacatcat ttaagggcct gatctatcat ggaagaaact tggaagatgg agttccatca      180

aggttcagcg gcagtggatc tggagaagat tattctctca ccatcagcag cctggaatct      240

gaagattttg cagactatta ctgtgtacag tatgctcatt ttcctcccac gttcggtgct      300

gggaccaaac tggagctgaa g                                                321

<210>  115
<211>  339
<212>  DNA
<213>  Mus musculus

<400>  115
gaggttcacc tgcagcagtc tgtggccgag cttgtaaggc caggggcctc agtcaagttg       60

tcctgcacag cttctggctt caacattaaa aacacctata tgcactgggt gaagcagagg      120

cctgaacagg gcctggagtg gattggaagg attgatcctg cgaatggtaa tactaaatat      180

gccccgaagt tccagggcaa ggccactata actgcagaca catcctccaa cacagcctac      240

ctgcagctca gcagcctgac atctgaggac actgccatct attactgtgc tagtgacttt      300

gactactggg gccaaggcac cactctcaca gtctcctca                             339

<210>  116
<211>  336
<212>  DNA
<213>  Mus musculus

<400>  116
gatgttttga tgacccaaac tccactctcc ctgcctgtca gtcttggaga tcaagtctcc       60

atctcttgca gatctagtca gagcattgta aatagtaatg gaaataccca tttagaatgg      120

tacctgcaga aaccaggcca gtctccaaag gtcctgatct acaaagtttc caaccgattt      180

tctggggtcc cagacaggtt cagtggcagt ggatcaggga cagatttcac actcaagatc      240

agcagagtgg aggctgagga tctgggagtt tattactgct ttcaaggttc acatgttccg      300

tacacgttcg gaggggggac caagctggaa ataaaa                                336

<210>  117
<211>  351
<212>  DNA
<213>  Mus musculus

<400>  117
gaggttcagc tgctgcagtc tggggcagaa cttgtgaagc caggggcctc agtcaagttg      60

tcctgcacag cttctggctt caacattaaa gactactata tgcactgggt gaaacagagg     120

actgaacagg gcctggagtg gattggaagg attgatcctg aggatggtga aactaaatat     180

gccccgaaat tccagggcaa ggccactatg acagcggaca catcctccaa cacagcctac     240

ctgcagctca gcagcctgac atctgaggac actgccgtct attattgtgc tagttccgcc     300

tactacggtc gtaactactg gggccaaggc accactctca cagtctcctc c              351


<210>  118
<211>  324
<212>  DNA
<213>  Mus musculus

<400>  118
caaattgttc tcacccagtc tccagcaatc atgtctgcat ctcctgggga gaaggtcacc      60

ttgacctgca gtgccagttc tagtttaagt tccagctact tgtactggta ccagcagaag     120

ccaggatcct cccccaaact ctggatttat agcacatcca acctggcttc tggagtccct     180

gctcgcttca gtggcagtgg gtctgggacc tcttactctc tcacaatcag cagcatggag     240

gctgaagatg ctgcctctta tttctgccat cagtggagta gttacccgta cacgttcgga     300

ggggggacca agctggaaat aaaa                                            324


<210>  119
<211>  351
<212>  DNA
<213>  Mus musculus

<400>  119
gaggttcagc tgcagcagtc tggggcagag cttgtgaagc caggggcctc agtcaagttg      60

tcctgcacag cttctggctt caacattaaa gacttctata tacactgggt gaagcagagg     120

actgaacagg gcctggagtg gattggaagg attgatcctg aggatggtga aactaaatat     180

gccccgaaat tccaggacaa ggccactata atagcagaca catcctccaa cacagcctac     240

ctgcagctca gcagcctgac atctgaggac actgccgtct attactgtgc tagctcctcc     300

tactacggta ataactactg gggccaaggc accactctca cagtctcctc a              351

<210>  120
<211>  324
<212>  DNA
<213>  Mus musculus

<400>  120
caaattgttc tcacccagtc tccagcaatc atgtctgcat ctcctgggga gaaggtcacc        60

ttgacctgca gtgccagctc aagtgtaagt tccaactact tgtactggta ccagcagaag       120

ccaggatcct cccccaaact ctggatttat agcacatcca acctggcttc tggagtccct       180

gctcgcttca gtggcagtgg gtctgggacc tcttactctc tcacaatcag cagcatggag       240

gctgaagatg ctgcctctta tttctgccat cagtggagta gtttcccgta cacgttcgga       300

ggggggacca agctggaaat aaaa                                              324


<210>  121
<211>  351
<212>  DNA
<213>  Mus musculus

<400>  121
caggtccagc tgcagcagtc tggggctgaa ctggcaagac ctggtgcctc agtgaagatg        60

tcctgcaagg cttctggcta cacctttact agttacacga tgcactgggt aaaacagagg       120

cctggacagg gtctggaatg gattggatac attaatccta gcagtggtta tactaagtac       180

aatcagattt tcaaggacaa ggccacattg actgcagaca aatcctccag cacagcctac       240

atgcaactga gcagcctgac atctgaggac tctgcagtgt attactgtac aagagggggg       300

ggacgagggt ttgcttactg gggccaaggg actctggtca ctgtctctgc a                351


<210>  122
<211>  321
<212>  DNA
<213>  Mus musculus

<400>  122
gacatcctga tgacccaatc tccatcctcc atgtctgtat ctctgggaga cacagtcagc        60

atcacttgcc atgcaagtca ggacattaac agtaatatag ggtggttgca gcagaaacca       120

gggaaatcat ttaagggcct gatctatcat ggaaccaact tggaagatgg agttccatca       180

aggttcagtg gcagtggatc tggagcagat tattctctca ccatcagcag cctggaatct       240

gaagattttg cagactatta ctgtgtacag tatgctcagt ttccattcac gttcggctcg       300

gggacaaagt tggaaataaa a                                                 321

<210>  123
<211>  348
<212>  DNA
<213>  Mus musculus

<400>  123
gaggttcagc tgcagcagtc tggggcagag cttgtgaagc caggggcctc agtcaagttg      60

tcctgcacag cttctggctt caatattaaa gactactata tgcactgggt gaagcagagg     120

actgaacagg gcctggagtg gattggaagg attgatcctg aggatggtga aactaaatat     180

gccccgaaat tccagggcaa ggccactata acagcagaca catcctccaa cacagcctac     240

ctgcagctca gcaacctgcc atctgaggac actgccgtct attactgtgc ttgtaggacg     300

gggggtttg cttactgggg ccaaggact ctggtcactg tctctgca                    348


<210>  124
<211>  318
<212>  DNA
<213>  Mus musculus

<400>  124
caaattgttc tcacccagtc tccagcaatc atgtctgcat ctccagggga gaaggtcacc      60

atgacctgca gtgccagctc aagtgtaagt tacatgtact ggtaccagca gaagccagga     120

tcctccccca gactcctgat ttatgacaca tccaacctgg cttctggagt ccctgttcgc     180

ttcagtggca gtgggtctgg gacctcttat tctctcacaa tcagccgagt ggaggctgaa     240

gatgccgcca cttattactg ccagcagtgg agtagttact cactcacgtt cggtgctggg     300

accaagctgg agctgaaa                                                   318


<210>  125
<211>  348
<212>  DNA
<213>  Mus musculus

<400>  125
gaggttcagc tgcagcagtc tggggcagag cttgtgaagc caggggcctc agtcaagttg      60

tcctgcacag cttctggctt caacattaaa gacttctata tgcactgggt gaagcagagg     120

actgaacagg gcctggagtg gattggaagg attgatcctg aggatggtga aactaaatat     180

gccccgaaat tccagggcaa ggccactata acagcagata catcctccaa cacagcctac     240

ctgcagctca gcagcctgac atctgaggac actgccgtct attactgtgg ttgtaggacg     300

gggggtttg cgtactgggg ccaaggact ctggtcactg tctctgca                    348


<210>  126

<211>  318
<212>  DNA
<213>  Mus musculus

<400>  126
caaattgttc tcacccagtc tccagcaatc atgtctgcat ctccagggga gaaggtcacc      60

atgacctgca gtgccagctc aagtgtaagt tacctatact ggtaccagca gaagccagga     120

tcctccccca gcctcctgat ttatgacaca tccaacctgg cttctggagt ccctgttcgc     180

ttcagtggca gtgggtctgg gacctcttac tctctcacaa tcagccgaat ggaggctgaa     240

gatgctgcca cttattattg ccagcagtgg agtagaaacc catggacgtt cggtggaggc     300

accaagctgg aaatcaaa                                                   318


<210>  127
<211>  351
<212>  DNA
<213>  Mus musculus

<400>  127
gatgtacaac ttcaggagtc aggacctggc ctcgtgaaac cttctcagtc tctgtctctc      60

acctgctctg tcactggcta ctccatcacc agtggttatt actggcactg gatccggcag     120

tttccagaaa acaaactgga atggatgggc tacataagct acgatggtag caatatctac     180

aacccatctc tcaaaaatcg aatctccatc actcgtgaca catctaagaa ccagtttttc     240

ctgaagttga attctgtgac tactgaggac acagccacat attactgtgc aattaactcg     300

gactatgctt tggactactg gggtcaagga acctcagtca ccgtctcctc a              351


<210>  128
<211>  318
<212>  DNA
<213>  Mus musculus

<400>  128
caaattgttc tcacccagtc tccagcaatc atgtctgcat ctccagggga gaaggtcacc      60

atgacctgca gtgccagctc aagtgtgagt tacatgtact ggtaccagca gaagccagga     120

tcctccccca gactcctgat ttatgacaca tccaacctgg cttctggagt ccctgttcgc     180

ttcagtggca gtgggtctgg gacctcttac tctctcacaa tcagccgaat ggaggctgaa     240

gatgctgcca cttattactg ccagcagtgg agtagttact cactcacgtt cggtgctggg     300

accaagctgg agctgaaa                                                   318


<210>  129
<211>  348

<212>  DNA
<213>  Mus musculus

<400>  129
   gaggttcagc tgcagcagtc tggggcagaa cttgtgaagc caggggcctc agtcaagttg       60
   tcctgcacag cttctggctt caacattaaa gactactata tgcactgggt gaagcagagg      120
   actgaacagg gcctggagtg gattggaagg attgatcctg aggatggtga aactaaatat      180
   gccccgaaat tccagggcaa ggccactata acagcagaca catcctccaa cacagcctac      240
   ctgcagctca gcaacctgcc atctgaggac actgccgtct attactgtgg ttgtaggacg      300
   ggggggtttg cttactgggg ccaagggact ctggtcactg tctctgca                     348


<210>  130
<211>  321
<212>  DNA
<213>  Mus musculus

<400>  130
gacatcctga tgacccaatc tccatcctcc atgtctgtat ctctgggaga cacagtcagc       60

atcacttgcc atgcaagtca gggcattaac agtaatatag ggtggttgca gcagaaacca      120

gggaaatcat ttaaggccct gatctatcat ggaaccaact tggaagatgg agttccgtca      180

aggttcagtg gcagtggatc tggagcagat tattctctca ccatcagcag cctggaatct      240

gaagattttg cagactatta ctgtgtacag tatgctcagt ttccattcac gttcggctcg      300

gggactgagt tggaaataaa a                                                321


<210>  131
<211>  372
<212>  DNA
<213>  Artificial Sequence

<220>
<223>  Site-directed mutation in the heavy chain variable region of
chimeric antibody 75G7C6 (DG/EG)

<400>  131
gaggtccagc tgcaacagtc tggacctgag ctggtgaagc ctggggcttc agtgaagata       60

ccctgcaagg cttctggata cacattcact gactacaaca tggactgggt gaagcagagc      120

catggaaaga gccttgagtg gattggagat attaatccta acaatggtga tactatctac      180

aaccagaagt tcaagggcaa ggccacattg actgtagaca agtcctccag cacagcctac      240

atggagctcc gcagcctgac atctgaggac actgcagtct attactgtgc aaggggggac      300

tatgaaggtt actacccggt ttactatgct atggactatt ggggtcaagg aacctcagtc      360

accgtctcct ca                                                          372

<210>  132
<211>  372
<212>  DNA
<213>  Artificial Sequence

<220>
<223> Site-directed mutation in the heavy chain variable region of
chimeric antibody 75G7C6 (DG/DA)

<400>  132
gaggtccagc tgcaacagtc tggacctgag ctggtgaagc ctggggcttc agtgaagata      60

ccctgcaagg cttctggata cacattcact gactacaaca tggactgggt gaagcagagc     120

catggaaaga gccttgagtg gattggagat attaatccta acaatggtga tactatctac     180

aaccagaagt tcaagggcaa ggccacattg actgtagaca agtcctccag cacagcctac     240

atggagctcc gcagcctgac atctgaggac actgcagtct attactgtgc aaggggggac     300

tatgatgctt actacccggt ttactatgct atggactatt ggggtcaagg aacctcagtc     360

accgtctcct ca                                                          372

<210>  133
<211>  124
<212>  PRT
<213>  Artificial Sequence

<220>
<223>  h75G7C6. VH-g0

<400>  133

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Asn Met Asp Trp Val Arg Gln Ala Pro Gly Gln Gly Leu Glu Trp Met
        35                  40                  45

Gly Asp Ile Asn Pro Asn Asn Ala Asp Thr Ile Tyr Asn Gln Lys Phe
    50                  55                  60

Lys Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Thr Val Thr Val Ser Ser
        115                 120

<210>  134
<211>  124
<212>  PRT
<213>  Artificial Sequence

<220>
<223>  h75G7C6. VH-g1

<400>  134

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Asn Met Asp Trp Val Arg Gln Ala Pro Gly Gln Gly Leu Glu Trp Ile
        35                  40                  45

Gly Asp Ile Asn Pro Asn Asn Ala Asp Thr Ile Tyr Asn Gln Lys Phe
    50                  55                  60

Lys Gly Arg Val Thr Leu Thr Val Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Thr Val Thr Val Ser Ser
        115                 120

<210>  135
<211>  124
<212>  PRT
<213>  Artificial Sequence

<220>
<223>  h75G7C6. VH-g2

<400>  135

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Asn Met Asp Trp Val Lys Gln Ala Pro Gly Gln Gly Leu Glu Trp Ile
        35                  40                  45

Gly Asp Ile Asn Pro Asn Asn Ala Asp Thr Ile Tyr Asn Gln Lys Phe
    50                  55                  60

Lys Gly Arg Val Thr Leu Thr Val Asp Lys Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Thr Val Thr Val Ser Ser
        115                 120


<210>  136
<211>  124
<212>  PRT
<213>  Artificial Sequence

<220>
<223>  h75G7C6. VH-g3

<400>  136

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Asn Met Asp Trp Val Lys Gln Ala Pro Gly Gln Gly Leu Glu Trp Ile
        35                  40                  45

Gly Asp Ile Asn Pro Asn Asn Ala Asp Thr Ile Tyr Asn Gln Lys Phe
    50                  55                  60

Lys Gly Arg Val Thr Leu Thr Val Asp Lys Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Thr Val Thr Val Ser Ser
        115                 120

<210>  137
<211>  108
<212>  PRT
<213>  Artificial Sequence

<220>
<223>  h75G7C6. VL-g0

<400>  137

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Ser Ser Val Ser Ser Ser
            20                  25                  30

Tyr Leu His Trp Tyr Gln Gln Lys Pro Gly Gln Ala Pro Arg Leu Leu
        35                  40                  45

Ile Tyr Ser Thr Ser Asn Leu Ala Ala Gly Ile Pro Ala Arg Phe Ser
    50                  55                  60

Gly Ser Gly Ser Gly Thr Asp Phe Thr Leu Thr Ile Ser Ser Leu Glu
65                  70                  75                  80

Pro Glu Asp Phe Ala Val Tyr Tyr Cys Gln Gln Tyr Ser Gly Tyr Pro
                85                  90                  95

Leu Thr Phe Gly Gly Gly Thr Lys Val Glu Ile Lys
            100                 105

<210>  138
<211>  108
<212>  PRT
<213>  Artificial Sequence

<220>
<223>  h75G7C6. VL-g1

<400>  138

Glu Asn Val Leu Thr Gln Ser Pro Ala Thr Leu Ser Leu Ser Pro Gly
1               5                   10                  15

Glu Arg Ala Thr Leu Ser Cys Arg Ala Ser Ser Ser Val Ser Ser Ser
            20                  25                  30

Tyr Leu His Trp Tyr Gln Gln Lys Pro Gly Gln Ala Pro Arg Leu Leu
        35                  40                  45

Ile Phe Ser Thr Ser Asn Leu Ala Ala Gly Ile Pro Ala Arg Phe Ser
    50                  55                  60

Gly Ser Gly Ser Gly Thr Asp Tyr Thr Leu Thr Ile Ser Ser Leu Glu
65                  70                  75                  80

Pro Glu Asp Phe Ala Val Tyr Tyr Cys Gln Gln Tyr Ser Gly Tyr Pro
                85                  90                  95

Leu Thr Phe Gly Gly Gly Thr Lys Val Glu Ile Lys
            100                 105


<210>  139
<211>  108
<212>  PRT
<213>  Artificial Sequence

<220>
<223>  h75G7C6. VL-g2

<400>  139

Glu Asn Val Leu Thr Gln Ser Pro Ala Thr Leu Ser Leu Ser Pro Gly
1               5                   10                  15

Glu Arg Ala Thr Leu Ser Cys Arg Ala Ser Ser Ser Val Ser Ser Ser
            20                  25                  30

Tyr Leu His Trp Tyr Gln Gln Lys Pro Gly Gln Ser Pro Arg Leu Leu
        35                  40                  45

Ile Phe Ser Thr Ser Asn Leu Ala Ala Gly Ile Pro Ala Arg Phe Ser
    50                  55                  60

Gly Ser Gly Ser Gly Thr Asp Tyr Thr Leu Thr Ile Ser Ser Leu Glu
65                  70                  75                  80

Pro Glu Asp Phe Ala Val Tyr Tyr Cys Gln Gln Tyr Ser Gly Tyr Pro
                85                  90                  95

Leu Thr Phe Gly Gly Gly Thr Lys Val Glu Ile Lys
            100                 105

<210> 140
<211> 108
<212> PRT
<213> Artificial Sequence

<220>
<223> h75G7C6. VL-g3

<400> 140

Glu Asn Val Leu Thr Gln Ser Pro Ala Thr Leu Ser Leu Ser Pro Gly
1               5                   10                  15

Glu Arg Ala Thr Leu Ser Cys Arg Ala Ser Ser Ser Val Ser Ser Ser
            20                  25                  30

Tyr Leu His Trp Tyr Gln Gln Lys Pro Gly Gln Ser Pro Arg Leu Leu
        35                  40                  45

Ile Phe Ser Thr Ser Asn Leu Ala Ala Gly Val Pro Ala Arg Phe Ser
    50                  55                  60

Gly Ser Gly Ser Gly Thr Asp Tyr Thr Leu Thr Ile Ser Ser Leu Glu
65                  70                  75                  80

Pro Glu Asp Phe Ala Val Tyr Tyr Cys Gln Gln Tyr Ser Gly Tyr Pro
                85                  90                  95

Leu Thr Phe Gly Gly Gly Thr Lys Val Glu Ile Lys
            100                 105

<210>  141
<211>  117
<212>  PRT
<213>  Artificial Sequence

<220>
<223>  h63A10A7.VH-g2

<400>  141

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Arg Gln Ala Pro Gly Gln Gly Leu Glu Trp Met
        35                  40                  45

Gly Tyr Ile Asn Pro Asn Ser Gly Tyr Thr Lys Tyr Asn Gln Lys Phe
    50                  55                  60

Lys Asp Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115


<210>  142
<211>  117
<212>  PRT
<213>  Artificial Sequence

<220>
<223>  h63A10A7.VH-g3

<400>  142

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Arg Gln Ala Pro Gly Gln Gly Leu Glu Trp Ile
        35                  40                  45

Gly Tyr Ile Asn Pro Asn Ser Gly Tyr Thr Lys Tyr Asn Gln Lys Phe
    50                  55                  60

Lys Asp Arg Val Thr Leu Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  143
<211>  117
<212>  PRT
<213>  Artificial Sequence

<220>
<223>  h63A10A7.VH-g4

<400>  143

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Lys Gln Arg Pro Gly Gln Gly Leu Glu Trp Ile
        35                  40                  45

Gly Tyr Ile Asn Pro Asn Ser Gly Tyr Thr Lys Tyr Asn Gln Lys Phe
    50                  55                  60

Lys Asp Lys Ala Thr Leu Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  144
<211>  117
<212>  PRT
<213>  Artificial Sequence

<220>
<223>  h63A10A7.VH-g5

<400>  144

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Lys Gln Arg Pro Gly Gln Gly Leu Glu Trp Ile
        35                  40                  45

Gly Tyr Ile Asn Pro Asn Ser Gly Tyr Thr Lys Tyr Asn Gln Lys Phe
    50                  55                  60

Lys Asp Lys Ala Thr Leu Thr Ala Asp Gln Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  145
<211>  117
<212>  PRT
<213>  Artificial Sequence

<220>

<223>  h63A10A7.VH-g6

<400>  145

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Lys Gln Arg Pro Gly Gln Gly Leu Glu Trp Met
        35                  40                  45

Gly Tyr Ile Asn Pro Asn Ser Gly Tyr Thr Lys Tyr Asn Gln Lys Phe
    50                  55                  60

Lys Asp Lys Ala Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115


<210>  146
<211>  117
<212>  PRT
<213>  Artificial Sequence

<220>
<223>  h63A10A7.VH-g7

<400>  146

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Arg Gln Ala Pro Gly Gln Gly Leu Glu Trp Ile
        35                  40                  45

Gly Tyr Ile Asn Pro Asn Ser Gly Tyr Thr Lys Tyr Asn Gln Lys Phe
    50                  55                  60

Lys Asp Lys Ala Thr Leu Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  147
<211>  117
<212>  PRT
<213>  Artificial Sequence

<220>
<223>  h63A10A7.VH-g8

<400>  147

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Ser Phe
            20                  25                  30

Thr Met His Trp Val Lys Gln Arg Pro Gly Gln Gly Leu Glu Trp Ile
        35                  40                  45

Gly Tyr Ile Asn Pro Asn Ser Gly Tyr Thr Lys Tyr Asn Gln Lys Phe
    50                  55                  60

Lys Asp Arg Val Thr Leu Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Thr Arg Gly Gly Gly Arg Gly Phe Ala Tyr Trp Gly Gln Gly Thr Leu
            100                 105                 110

Val Thr Val Ser Ser
        115

<210>  148
<211>  107
<212>  PRT
<213>  Artificial Sequence

<220>
<223>  h63A10A7.VL-g3

<400>  148

Asp Ile Gln Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Leu Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105

<210>  149
<211>  107
<212>  PRT
<213>  Artificial Sequence

<220>
<223>  h63A10A7.VL-g4

<400>  149

Asp Ile Leu Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Leu Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105

<210>  150
<211>  107
<212>  PRT
<213>  Artificial Sequence

<220>
<223>  h63A10A7.VL-g5

<400>  150

Asp Ile Leu Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Leu Gln Gln Lys Pro Gly Lys Ala Pro Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105

<210>  151
<211>  107
<212>  PRT
<213>  Artificial Sequence

<220>
<223>  h63A10A7.VL-g 6

<400>  151

Asp Ile Leu Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Leu Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105


<210>  152
<211>  107
<212>  PRT
<213>  Artificial Sequence

<220>
<223>  h63A10A7.VL-g 7

<400>  152

Asp Ile Leu Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Tyr Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Tyr Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105

<210>  153
<211>  107
<212>  PRT
<213>  Artificial Sequence

<220>
<223>  h63A10A7.VL-g8

<400>  153

Asp Ile Leu Met Thr Gln Ser Pro Ser Ser Leu Ser Ala Ser Val Gly
1               5                   10                  15

Asp Arg Val Thr Ile Thr Cys His Ala Ser Gln Asp Ile Asn Ser Asn
            20                  25                  30

Ile Gly Trp Tyr Gln Gln Lys Pro Gly Lys Ser Phe Lys Ala Leu Ile
        35                  40                  45

Tyr His Gly Thr Asn Leu Glu Asp Gly Val Pro Ser Arg Phe Ser Gly
    50                  55                  60

Ser Gly Ser Gly Ala Asp Phe Thr Leu Thr Ile Ser Ser Leu Gln Pro
65                  70                  75                  80

Glu Asp Phe Ala Thr Tyr Tyr Cys Val Gln Tyr Ala Gln Phe Pro Phe
                85                  90                  95

Thr Phe Gly Gln Gly Thr Lys Leu Glu Ile Lys
            100                 105

<210>  154
<211>  372
<212>  DNA
<213>  Artificial Sequence

<220>
<223>  h75G7C6. VH-g0

<400>  154
caagttcagc tggtgcagtc cggagccgag gtgaagaaac ccggcgccag cgtgaaggtg        60

agctgcaagg ccagcggcta caccttcacc gactacaaca tggactgggt gagacaagct       120

cccggtcaag gtttagagtg gatgggcgac atcaacccca acaacgccga caccatctac       180

aaccagaagt tcaaaggtcg tgtgaccatg actcgtgaca ccagcacctc caccgtgtac       240

atggagctga gctctttaag gtccgaggac accgccgtgt actactgcgc tcgtggcgac       300

tacgacggct actaccccgt gtactacgcc atggactact ggggccaagg taccacagtg       360

accgtgagct cc                                                           372


<210>  155
<211>  372
<212>  DNA
<213>  Artificial Sequence

<220>
<223>  h75G7C6. VH-g1

<400>  155
caagttcagc tggtgcagag cggcgctgaa gtgaagaagc ccggcgccag cgtgaaggtg        60

agctgcaagg ccagcggcta caccttcacc gactacaaca tggactgggt gaggcaagct       120

cccggtcaag gtttagagtg gatcggcgac attaacccca acaacgccga caccatctac       180

aaccagaagt tcaagggtcg tgtgacttta accgtggaca ccagcaccag caccgtgtac       240

atggagctga gctctttaag aagcgaggac accgccgtgt actactgcgc tcgtggcgac       300

tacgacggct actaccccgt gtactacgcc atggactatt ggggccaagg tacaaccgtg       360

accgtgagca gc                                                           372


<210>  156
<211>  372
<212>  DNA
<213>  Artificial Sequence

<220>
<223>  h75G7C6. VH-g2

<400>  156
caagttcagc tggtgcagag cggcgctgaa gtgaagaagc ccggcgccag cgtgaaggtg        60

agctgcaagg ccagcggcta caccttcacc gactacaaca tggactgggt gaagcaagct 120

cccggtcaag gtttagagtg gattggcgac atcaacccca acaacgccga caccatctac 180

aaccagaagt tcaagggtcg tgtgacttta accgtggaca agagcaccag caccgtgtac 240

atggagctga gctctttaag gagcgaggac accgccgtgt actactgcgc tcgtggcgac 300

tacgacggct actaccccgt gtactacgcc atggactact ggggccaagg taccacagtg 360

accgtgagca gc 372

<210> 157
<211> 372
<212> DNA
<213> Artificial Sequence

<220>
<223> h75G7C6. VH-g3

<400> 157
gaggtgcagc tggtgcagag cggcgctgaa gtgaagaagc ccggcgccag cgtgaaggtg 60

agctgcaagg ccagcggcta caccttcacc gactacaaca tggactgggt gaagcaagct 120

cccggtcaag gtttagagtg gatcggcgac atcaacccca acaacgccga caccatctac 180

aaccagaagt tcaagggtcg tgtgacttta acagtggaca aaagcacctc caccgtgtac 240

atggagctgt cctctttaag gtccgaggac accgccgtgt actactgcgc tcgtggcgac 300

tacgacggct actaccccgt gtactacgcc atggactact ggggccaagg taccaccgtg 360

accgtgagca gc 372

<210> 158
<211> 324
<212> DNA
<213> Artificial Sequence

<220>
<223> h75G7C6. VL-g0

<400> 158
gagatcgtgc tgacccagtc ccccgctaca ctgtctttat cccccggtga gagagccact 60

ttaagctgca gagccagcag cagcgtgagc agcagctatt tacactggta ccagcagaag 120

cccggacaag ctcccagact gctgatctac agcaccagca atttagctgc cggcatcccc 180

gctcgtttca gcggaagcgg aagcggcacc gacttcactt taaccatctc ctctttagag 240

cccgaggact tcgccgtgta ctactgccag cagtacagcg gctaccctct gaccttcggc 300

ggaggcacca aggtggagat taag 324

<210>  159
<211>  324
<212>  DNA
<213>  Artificial Sequence

<220>
<223>  h75G7C6. VL-g1

<400>  159
gagaacgtgc tgacccagag ccccgccaca ctgtctttaa gccccggtga gagggccact        60

ttaagctgca gagccagcag cagcgtgagc agcagctatt tacactggta ccagcagaaa       120

cccggccaag ctcccagact gctgatcttc agcaccagca atttagctgc cggcatcccc       180

gctagattta gcggcagcgg cagcggaacc gactacactt taaccatcag ctctttagaa       240

cccgaggact tcgccgtgta ctactgccag cagtacagcg gctatcctct gaccttcggc       300

ggcggcacca aggtggagat caag                                              324


<210>  160
<211>  324
<212>  DNA
<213>  Artificial Sequence

<220>
<223>  h75G7C6. VL-g2

<400>  160
gagaacgtgc tgacccagag ccccgctact ttatctttaa gccccggaga aagggccact        60

ttaagctgta gggccagcag cagcgtgagc agctcctatt tacactggta ccagcagaag       120

cccggtcaga gccctcgtct gctgatcttc agcacaagca atttagccgc cggcatcccc       180

gctagattct ccggaagcgg cagcggcacc gactacactt taaccatctc ctctttagag       240

cccgaggact tcgccgtgta ctactgccag cagtactccg gctacccttt aacctttggc       300

ggaggcacca aggtggagat caag                                              324


<210>  161
<211>  324
<212>  DNA
<213>  Artificial Sequence

<220>
<223>  h75G7C6. VL-g3

<400>  161
gagaacgtgc tgacccagag ccccgctact ttatctttaa gccccggtga gagagccact        60

ttaagctgtc gtgccagcag cagcgtgagc tccagctatt tacactggta ccagcagaag       120

cccggccaga gccccagact gctgatcttc agcaccagca atctggccgc cggagtgccc 180

gctagattca gcggcagcgg aagcggcacc gactacactt taaccatcag ctctttagag 240

cccgaggact tcgccgtgta ctactgccag cagtacagcg gctacccttt aacattcggc 300

ggaggcacca aggtggagat caag 324

<210> 162
<211> 351
<212> DNA
<213> Artificial Sequence

<220>
<223> h63A10A7.VH-g2

<400> 162
caggtgcagc tggtgcagtc aggagcagaa gtgaagaagc ccggagccag cgtgaaagtg 60

tcttgcaagg ccagcggcta caccttcatc agcttcacca tgcattgggt ccggcaggct 120

ccaggacagg gactcgagtg gatgggatac atcaacccca acagcggcta caccaagtac 180

aaccagaagt tcaaggaccg cgtgaccatg accagagaca ccagcaccag caccgtgtac 240

atggagctga gcagcctgag gagcgaggat acagccgtgt actattgcac caggggcgga 300

ggcagaggat tcgcttattg gggccaggga acactggtga cagtgtccag c 351

<210> 163
<211> 351
<212> DNA
<213> Artificial Sequence

<220>
<223> h63A10A7.VH-g3

<400> 163
caggtgcagc tggtgcagtc aggagcagaa gtgaagaagc ccggagccag cgtgaaagtg 60

tcttgcaagg ccagcggcta caccttcatc agcttcacca tgcattgggt ccggcaggct 120

ccaggacagg gactcgagtg gattggctac atcaacccca acagcggcta caccaagtac 180

aaccagaagt tcaaggaccg cgtgaccctg accagagaca ccagcaccag caccgtgtac 240

atggagctga gcagcctgag gagcgaggat acagccgtgt actattgcac caggggcgga 300

ggcagaggat tcgcttattg gggccaggga acactggtga cagtgtccag c 351

<210> 164
<211> 354
<212> DNA
<213> Artificial Sequence

<220>
<223>  h63A10A7.VH-g4

<400>  164
caggtgcagc tggtgcagtc aggagcagaa gtgaagaagc ccggagccag cgtgaaagtg        60

tcttgcaagg ccagcggcta caccttcatc agcttcacca tgcattgggt caagcagagg       120

ccaggacagg gactcgagtg gattggctac atcaacccca acagcggcta caccaagtac       180

aaccagaagt tcaaggacaa ggccaccctg accagagaca ccagcaccag caccgtgtac       240

atggagctga gcagcctgag gagcgaggat acagccgtgt actattgcac caggggcgga       300

ggcagaggat tcgcttattg gggccaggga acactggtga cagtgtccag cgct             354


<210>  165
<211>  354
<212>  DNA
<213>  Artificial Sequence

<220>
<223>  h63A10A7.VH-g5

<400>  165
caggtgcagc tggtgcagtc aggagcagaa gtgaagaagc ccggagccag cgtgaaagtg        60

tcttgcaagg ccagcggcta caccttcatc agcttcacca tgcattgggt caagcagagg       120

ccaggacagg gactcgagtg gattggctac atcaacccca acagcggcta caccaagtac       180

aaccagaagt tcaaggacaa ggccaccctg acagccgatc agtctaccag caccgcctac       240

atggagctga gctctctgag gagcgaggac acagccgtgt actattgcac caggggcgga       300

ggcagaggat tcgcttattg gggccaggga acactggtga cagtgtccag cgct             354


<210>  166
<211>  354
<212>  DNA
<213>  Artificial Sequence

<220>
<223>  h63A10A7.VH-g6, DNA

<400>  166
caggtgcagc tggtgcagtc aggagcagaa gtgaagaagc ccggagccag cgtgaaagtg        60

tcttgcaagg ccagcggcta caccttcatc agcttcacca tgcattgggt caagcagagg       120

ccaggacagg gactcgagtg gatgggctac atcaacccca acagcggcta caccaagtac       180

aaccagaagt tcaaggacaa ggccaccatg accagagaca ccagcaccag caccgtgtac       240

atggagctga gcagcctgag gagcgaggat acagccgtgt actattgcac caggggcgga       300

ggcagaggat tcgcttattg gggccaggga acactggtga cagtgtccag cgct 354

<210> 167
<211> 354
<212> DNA
<213> Artificial Sequence

<220>
<223> h63A10A7.VH-g7

<400> 167
caggtgcagc tggtgcagtc aggagcagaa gtgaagaagc ccggagccag cgtgaaagtg 60

tcttgcaagg ccagcggcta caccttcatc agcttcacca tgcattgggt ccggcaggct 120

ccaggacagg gactcgagtg gattggctac atcaacccca acagcggcta caccaagtac 180

aaccagaagt tcaaggacaa ggccaccctg accagagaca ccagcaccag caccgtgtac 240

atggagctga gcagcctgag gagcgaggat acagccgtgt actattgcac caggggcgga 300

ggcagaggat tcgcttattg gggccaggga acactggtga cagtgtccag cgct 354

<210> 168
<211> 354
<212> DNA
<213> Artificial Sequence

<220>
<223> h63A10A7.VH-g8

<400> 168
caggtgcagc tggtgcagtc aggagcagaa gtgaagaagc ccggagccag cgtgaaagtg 60

tcttgcaagg ccagcggcta caccttcatc agcttcacca tgcattgggt caagcagagg 120

ccaggacagg gactcgagtg gattggctac atcaacccca acagcggcta caccaagtac 180

aaccagaagt tcaaggaccg cgtgaccctg accagagaca ccagcaccag caccgtgtac 240

atggagctga gcagcctgag gagcgaggat acagccgtgt actattgcac caggggcgga 300

ggcagaggat tcgcttattg gggccaggga acactggtga cagtgtccag cgct 354

<210> 169
<211> 321
<212> DNA
<213> Artificial Sequence

<220>
<223> h63A10A7.VL-g3

<400> 169
gacatccaga tgacccagag ccctagcagc ctgagcgcta gcgtgggaga tagagtgacc 60

atcacttgcc acgccagcca ggacatcaac agcaacatcg gttggctgca gcagaagccc 120

ggcaagagct tcaaggccct gatctaccac ggcacaaatc tggaggacgg agtgcctagc 180

agattcagcg gcagcggaag cggagccgat tacaccctga ccatcagcag cctgcagcca 240

gaggacttcg ccacctacta ctgcgtgcag tacgcccagt tccccttcac cttcggacag 300

ggaaccaagc tggagatcaa g 321

<210> 170
<211> 327
<212> DNA
<213> Artificial Sequence

<220>
<223> h63A10A7.VL-g4

<400> 170
gacatcctga tgacccagag ccctagcagc ctgagcgcta gcgtgggaga tagagtgacc 60

atcacttgcc acgccagcca ggacatcaac agcaacatcg gttggctgca gcagaagccc 120

ggcaagagct tcaaggccct gatctaccac ggcacaaatc tggaggacgg agtgcctagc 180

agattcagcg gcagcggaag cggagccgat tacaccctga ccatcagcag cctgcagcca 240

gaggacttcg ccacctacta ctgcgtgcag tacgcccagt tccccttcac cttcggacag 300

ggaaccaagc tggagatcaa gcgtacg 327

<210> 171
<211> 327
<212> DNA
<213> Artificial Sequence

<220>
<223> h63A10A7.VL-g5

<400> 171
gacatcctga tgacccagag ccctagcagc ctgagcgcca gcgtgggaga tagagtgacc 60

atcacttgcc acgccagcca ggacatcaac agcaacatcg gttggctgca gcagaagccc 120

ggcaaggctc ctaaggccct gatctaccac ggcacaaatc tggaggacgg agtgcctagc 180

agattcagcg gcagcggaag cggagccgat tacaccctga ccatcagcag cctgcagcca 240

gaggacttcg ccacctacta ctgcgtgcag tacgcccagt tccccttcac cttcggacag 300

ggaaccaagc tggagatcaa gcgtacg 327

<210> 172
<211> 328

<212>  DNA
<213>  Artificial Sequence

<220>
<223>  h63A10A7.VL-g6

<400>  172
ggacatcctg atgacccaga gccctagcag cctgagcgcc agcgtgggag atagagtgac      60

catcacttgc cacgccagcc aggacatcaa cagcaacatc ggttggctgc agcagaagcc     120

cggcaagagc ttcaaggccc tgatctacca cggcacaaat ctggaggacg gagtgcctag     180

cagattcagc ggcagcggaa gcggaacaga ttacaccctg accatcagca gcctgcagcc     240

agaggacttc gccacctact actgcgtgca gtacgcccag ttccccttca ccttcggaca     300

gggaaccaag ctggagatca agcgtacg                                        328


<210>  173
<211>  327
<212>  DNA
<213>  Artificial Sequence

<220>
<223>  h63A10A7.VL-g7

<400>  173
gacatcctga tgacccagag ccctagcagc ctgagcgcca gcgtgggaga tagagtgacc      60

atcacttgcc acgccagcca ggacatcaac agcaacatcg gttggtatca gcagaagccc     120

ggcaagagct tcaaggccct gatctaccac ggcacaaatc tggaggacgg agtgcctagc     180

agattcagcg gcagcggaag cggagccgat tacaccctga ccatcagcag cctgcagcca     240

gaggacttcg ccacctacta ctgcgtgcag tacgcccagt tccccttcac cttcggacag     300

ggaaccaagc tggagatcaa gcgtacg                                         327


<210>  174
<211>  328
<212>  DNA
<213>  Artificial Sequence

<220>
<223>  h63A10A7.VL-g8

<400>  174
gacatcctga tgacccagag ccctagcagc ctgagcgcca gcgtgggaga tagagtgacc      60

atcacttgcc acgccagcca ggacatcaac agcaacatcg gttggtatca gcagaagccc     120

ggcaagagct tcaaggccct gatctaccac ggcacaaatc tggaggacgg agtgcctagc     180

agattcagcg gcagcggaag cggagccgat ttcaccctga ccatcagcag cctgcagcca     240

gaggacttcg ccacctacta ctgcgtgcag tacgcccagt tccccttcac cttcggacag 300

ggaaccaagc tggagatcaa gcgtacgg 328

<210> 175
<211> 98
<212> PRT
<213> Homo sapiens

<400> 175

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg

<210> 176
<211> 98
<212> PRT
<213> Homo sapiens

<400> 176

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Met His Trp Val Arg Gln Ala Pro Gly Gln Gly Leu Glu Trp Met
        35                  40                  45

Gly Ile Ile Asn Pro Ser Gly Gly Ser Thr Ser Tyr Ala Gln Lys Phe
    50                  55                  60

Gln Gly Arg Val Thr Met Thr Arg Asp Thr Ser Thr Ser Thr Val Tyr
65                  70                  75                  80

Met Glu Leu Ser Ser Leu Arg Ser Glu Asp Thr Ala Val Tyr Tyr Cys
                85                  90                  95

Ala Arg

<210>  177
<211>  95
<212>  PRT
<213>  Homo sapiens

<400>  177

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro
                85                  90                  95

<210>  178
<211>  95
<212>  PRT
<213>  Homo sapiens

<400>  178

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro
                85                  90                  95

<210>  179
<211>  124
<212>  PRT
<213>  Artificial Sequence

<220>
<223> Site-directed mutation in the heavy chain variable region of
chimeric antibody 75G7C6 (NG/NA)

<400>  179

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Asn Ala Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Asp Gly Tyr Tyr Pro Val Tyr Tyr Ala Met Asp

            100                 105                 110

Tyr Trp Gly Gln Gly Thr Ser Val Thr Val Ser Ser
        115                 120

<210> 180
<211> 124
<212> PRT
<213> Artificial Sequence

<220>
<223> Site-directed mutation in the heavy chain variable region of
chimeric antibody 75G7C6 (NG/QG)

<400> 180

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Gln Gly Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Asp Gly Tyr Tyr Pro Val Tyr Tyr Ala Met Asp
            100                 105                 110

Tyr Trp Gly Gln Gly Thr Ser Val Thr Val Ser Ser
        115                 120

<210> 181
<211> 124
<212> PRT
<213> Artificial Sequence

<220>
<223> Site-directed mutation in the heavy chain variable region of
chimeric antibody 75G7C6 (DG/SG)

<400>  181

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Asn Gly Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Ser Gly Tyr Tyr Pro Val Tyr Tyr Ala Met Asp
            100                 105                 110

Tyr Trp Gly Gln Gly Thr Ser Val Thr Val Ser Ser
        115                 120


<210>  182
<211>  124
<212>  PRT
<213>  Artificial Sequence

<220>
<223> Site-directed mutation in the heavy chain variable region of
chimeric antibody 75G7C6 (DG/EG)

<400>  182

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Asn Gly Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Glu Gly Tyr Tyr Pro Val Tyr Tyr Ala Met Asp
            100                 105                 110

Tyr Trp Gly Gln Gly Thr Ser Val Thr Val Ser Ser
        115                 120

<210>  183
<211>  124
<212>  PRT
<213>  Artificial Sequence

<220>
<223> Site-directed mutation in the heavy chain variable region of
chimeric antibody 75G7C6 (DG/DA)

<400>  183

Glu Val Gln Leu Gln Gln Ser Gly Pro Glu Leu Val Lys Pro Gly Ala
1               5                   10                  15

Ser Val Lys Ile Pro Cys Lys Ala Ser Gly Tyr Thr Phe Thr Asp Tyr
            20                  25                  30

Asn Met Asp Trp Val Lys Gln Ser His Gly Lys Ser Leu Glu Trp Ile
        35                  40                  45

Gly Asp Ile Asn Pro Asn Asn Gly Asp Thr Ile Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Thr Ala Val Tyr Tyr Cys
                85                  90                  95

Ala Arg Gly Asp Tyr Asp Ala Tyr Tyr Pro Val Tyr Tyr Ala Met Asp
            100                 105                 110

Tyr Trp Gly Gln Gly Thr Ser Val Thr Val Ser Ser
        115                 120

<210>  184
<211>  6
<212>  PRT
<213>  Artificial Sequence

<220>
<223> Site-directed mutation in heavy chain variable region CDR2 of
chimeric antibody 75G7C6 (NG/NA)

<400>  184

Asn Pro Asn Asn Ala Asp
1               5

<210>  185
<211>  372
<212>  DNA
<213>  Artificial Sequence

<220>
<223> Site-directed mutation in the heavy chain variable region of
chimeric antibody 75G7C6 (NG/NA)

<400>  185
gaggtccagc tgcaacagtc tggacctgag ctggtgaagc ctggggcttc agtgaagata      60

ccctgcaagg cttctggata cacattcact gactacaaca tggactgggt gaagcagagc     120

catggaaaga gccttgagtg gattggagat attaatccta acaatgctga tactatctac     180

aaccagaagt tcaagggcaa ggccacattg actgtagaca agtcctccag cacagcctac     240

atggagctcc gcagcctgac atctgaggac actgcagtct attactgtgc aaggggggac     300

tatgatggtt actacccggt ttactatgct atggactatt ggggtcaagg aacctcagtc     360

accgtctcct ca                                                         372

<210>  186
<211>  6
<212>  PRT
<213>  Artificial Sequence

<220>
<223> Site-directed mutation in heavy chain variable region CDR2 of
chimeric antibody 75G7C6

<400>  186

Asn Pro Asn Gln Gly Asp
1               5

<210>  187
<211>  372
<212>  DNA
<213>  Artificial Sequence

<220>
<223> Site-directed mutation in the heavy chain variable region of
chimeric antibody 75G7C6 (NG/QG)

<400>  187
gaggtccagc tgcaacagtc tggacctgag ctggtgaagc ctggggcttc agtgaagata      60

ccctgcaagg cttctggata cacattcact gactacaaca tggactgggt gaagcagagc     120

catggaaaga gccttgagtg gattggagat attaatccta accaaggtga tactatctac     180

aaccagaagt tcaagggcaa ggccacattg actgtagaca agtcctccag cacagcctac     240

atggagctcc gcagcctgac atctgaggac actgcagtct attactgtgc aaggggggac     300

tatgatggtt actacccggt ttactatgct atggactatt ggggtcaagg aacctcagtc     360

accgtctcct ca                                                         372


<210>  188
<211>  372
<212>  DNA
<213>  Artificial Sequence

<220>
<223> Site-directed mutation in the heavy chain variable region of
chimeric antibody 75G7C6 (DG/SG)

<400>  188
gaggtccagc tgcaacagtc tggacctgag ctggtgaagc ctggggcttc agtgaagata      60

ccctgcaagg cttctggata cacattcact gactacaaca tggactgggt gaagcagagc     120

catggaaaga gccttgagtg gattggagat attaatccta acaatggtga tactatctac     180

aaccagaagt tcaagggcaa ggccacattg actgtagaca agtcctccag cacagcctac     240

atggagctcc gcagcctgac atctgaggac actgcagtct attactgtgc aaggggggac     300

tattctggtt actacccggt ttactatgct atggactatt ggggtcaagg aacctcagtc     360

accgtctcct ca                                                         372


<210>  189
<211>  15
<212>  PRT
<213>  Homo sapiens

<400>  189

Tyr Phe Asp Tyr Trp Gly Gln Gly Thr Leu Val Thr Val Ser Ser
1               5                   10                  15

<210>  190
<211>  20
<212>  PRT
<213>  Homo sapiens

<400>  190

Tyr Tyr Tyr Tyr Tyr Gly Met Asp Val Trp Gly Gln Gly Thr Thr Val
1               5                   10                  15

Thr Val Ser Ser
            20

<210>  191
<211>  12
<212>  PRT
<213>  Homo sapiens

<400>  191

Leu Thr Phe Gly Gly Gly Thr Lys Val Glu Ile Lys
1               5                   10

<210>  192
<211>  12
<212>  PRT
<213>  Homo sapiens

<400>  192

Tyr Thr Phe Gly Gln Gly Thr Lys Leu Glu Ile Lys
1               5                   10